# EUROPEAN PATENT APPLICATION

(11) **EP 4 578 852 A1**
(43) Date of publication of application: **02.07.2025**
(21) Application number: 23856701.0
(22) Date of filing: 24.08.2023
(51) Int. Cl.: C07D 405/12, C07D 405/14, C07D 407/14, A61K 31/443, A61P 29/00

(54) **HETEROCYCLIC COMPOUND, METHOD FOR PREPARING SAME, AND PHARMACEUTICAL USE THEREOF**

(30) Priority: 24.08.2022 CN 202211019508; 30.09.2022 CN 202211211152; 08.12.2022 CN 202211570844; 15.02.2023 CN 202310117126; 28.02.2023 CN 202310176022; 20.03.2023 CN 202310272811; 28.04.2023 CN 202310479094; 16.05.2023 CN 202310550829; 26.05.2023 CN 202310605968; 08.06.2023 CN 202310675380; 21.06.2023 CN 202310740371
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: LI, Xin, Shanghai 200245 (CN); CHEN, Yang, Shanghai 200245 (CN); SHEN, Feng, Shanghai 200245 (CN); HE, Feng, Shanghai 200245 (CN)
(74) Representative: Regimbeau
(86) International application number: PCT/CN2023/114740
(87) International publication number: WO 2024/041613

(57) **Abstract**

The present disclosure relates to a heterocyclic compound, a method for preparing same, and pharmaceutical use thereof. Specifically, the present disclosure relates to a heterocyclic compound represented by general formula (I), a method for preparing same, a pharmaceutical composition comprising same, and use thereof as a therapeutic agent, particularly, use thereof as an Nav inhibitor and use thereof in preparing a medicament for treating and/or alleviating pain and a pain-related disease. Groups in general formula (I) are as defined in the description.

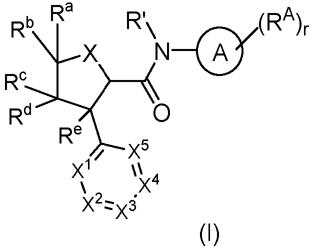

## Description

### TECHNICAL FIELD

The present disclosure pertains to the field of pharmaceuticals and relates to a heterocyclic compound, a preparation method therefor, and pharmaceutical use thereof. In particular, the present disclosure relates to a heterocyclic compound represented by general formula (I), a preparation method therefor, a pharmaceutical composition comprising the compound, use thereof as a Nav inhibitor, and use thereof in the preparation of a medicament for treating and/or alleviating pain and pain-related diseases.

### BACKGROUND

Pain is a complex physiological and psychological activity and is one of the most common clinical symptoms. The International Association for the Study of Pain defines that "pain is an unpleasant sensory and emotional experience associated with actual or potential tissue damage; it is a personal experience." Pain can serve as a warning signal, alerting an organism to potential danger; it plays an essential protective role in an organism's normal life activities. Meanwhile, pain is also a common clinical symptom. After the external stimuli causing pain disappear, intense or persistent pain can cause physiological dysfunctions, significantly impacting a living organism's quality of life. Statistics show that approximately one fifth of the world population suffers from moderate to severe chronic pain.

Pain originates from nociceptors in the peripheral nervous system. These are free nerve endings that are widely distributed throughout the skin, muscles, joints, and internal organ tissues. They can convert thermal, mechanical, or chemical stimulation into nerve impulses (action potentials) and transmit them through afferent nerve fibers to their cell bodies located in the dorsal root ganglia (DRG). From there, the impulses are finally transmitted to higher neural centers, causing the sensation of pain. The generation and transmission of action potentials in neurons depend on voltage-gated sodium channels (Nav) on the cell membrane. When the cell membrane is depolarized, these sodium channels become activated and open, causing the influx of sodium ions, which further depolarizes the cell membrane, resulting in the generation of an action potential. Therefore, inhibiting abnormal sodium channel activity contributes to the treatment and alleviation of pain.

Nav channels are transmembrane ion channel proteins. These proteins consist of an α subunit with a molecular weight of 260 kD and a β subunit with a molecular weight of 30-40 kD. According to the α subunit, they can be classified into 9 subtypes: Nav1.1 to Nav1.9. The different subtypes exhibit different tissue distribution and electrophysiological and pharmacological characteristics. Sodium channels are categorized into tetrodotoxin-sensitive (TTX-S) and tetrodotoxin-resistant (TTX-R), depending on whether they can be effectively inhibited by nanomoles of tetrodotoxin (TTX). Nav1.1, Nav1.2, Nav1.3, and Nav1.7 are TTX-S, and they are encoded by genes located on human chromosome 2q23-24 and highly expressed in neurons. Nav1.5, Nav1.8, and Nav1.9 are TTX-R, and they are encoded by genes located on human chromosome 3p21-24. Nav1.5 mainly exists in cardiac muscle cells, while Nav1.8 and Nav1.9 exist in the peripheral nervous system. Nav1.4 and Nav1.6 are both TTX-S, and they exist in large amounts in skeletal muscle and the central nervous system, respectively. The local anesthetic lidocaine alleviates pain by inhibiting Nav channels. Non-selective Nav inhibitors, such as lamotrigine, lacosamide, and mexiletine, have been successfully used to treat chronic pain.

Nav1.8 is TTX-R, and it is encoded by the SCN10A gene, mainly exists in trigeminal ganglion neurons and DRG neurons, and is electrophysiologically characterized by slow inactivation and quick recovery. In neurons expressing Nav1.8, the upstroke of the action potential is mainly caused by Nav1.8 currents. In some models for studying neuropathic pain, nerve injury can lead to increased expression levels of Nav1.8 in axons and neuronal cell bodies. Using Nav1.8 antisense oligonucleotides to reduce Nav1.8 expression can also significantly alleviate pain. After carrageenan is injected into the paws of rats, an increase in Nav1.8 expression is observed in DRG neurons. Mice in which Nav1.8 is knocked out do not exhibit normal visceral inflammatory pain. In humans, gain-of-function mutations in the Nav1.8 gene can lead to peripheral neuralgia. According to a series of animal experiments and human genetic evidence, selective inhibition of Nav1.8 has the potential to become a novel analgesic therapy. It can be used to treat various types of pain, such as inflammatory pain, neuropathic pain, postsurgical pain, and cancer pain. Lacking subtype selectivity, clinically used Nav inhibitors can inhibit sodium channels expressed in the heart and the central nervous system; therefore, their therapeutic windows are relatively narrow, limiting their application. As Nav1.8 is mainly distributed in the peripheral nervous system, selectively inhibiting Nav1.8 can effectively reduce side effects. Therefore, it is necessary to develop Nav1.8 inhibitors with higher activity, better selectivity, better pharmacokinetic properties, and fewer side effects.

Patent applications of disclosed Nav1.8 inhibitor compounds include WO2021113627A1, WO2022256622A1, WO2022256676A1, WO2022256679A1, WO2022256842A1, and WO2022256702A1.

### SUMMARY

The present disclosure aims to provide a compound represented by general formula (I) or a pharmaceutically acceptable salt thereof: 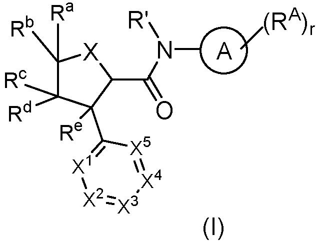 wherein:
ring A is phenyl or 6-membered heteroaryl;
each R^{A} is identical or different and is independently selected from the group consisting of a deuterium atom, halogen, hydroxy, cyano, alkyl, alkoxy, haloalkyl, haloalkoxy, hydroxyalkyl, hydroxyalkoxy, alkoxyalkyl, alkenyl, alkynyl, -NR³R⁴, -alkylene-NR³R⁴, -O-alkylene-NR³R⁴, -C(=NR⁵)R⁶, -S(O)ᵥNR³R⁴, -NR^{S}S(O)ᵥR⁶, -S(O)ᵥR⁶,-S(=NR⁵)(O)R⁶, -NR⁵C(O)R⁶, -NR⁵C(O)NR³R⁴, -C(O)NR⁵-OR⁶, -P(O)R⁷R⁸, -C(O)NR⁵-NR³R⁴, -C(=NR⁵)NR⁵-OR⁶, -C(O)NR⁵-alkylene-Cy, -C(=NR⁵)NR³R⁴, -C(O)-C(O)-NR³R⁴, -S(=NR⁵)NR³R⁴, -S(=NR⁵)R⁶, -S(=NR⁵)(O)NR³R⁴, -Si(O)NR³R⁴, -Si(R⁶)₃,-OR⁶, cycloalkyl, heterocyclyl, aryl, heteroaryl, cycloalkylalkyl, heterocyclylalkyl, -C(O)-cycloalkyl, -C(O)-heterocyclyl, -alkylene-O-alkylene-cycloalkyl, -alkylene-O-cycloalkyl, -O-alkylene-heteroaryl, and -O-alkylene-heterocyclyl, wherein the alkyl, alkoxy, alkoxyalkyl, alkenyl, alkynyl, alkylene, cycloalkyl, heterocyclyl, aryl, heteroaryl, cycloalkylalkyl, and heterocyclylalkyl are each independently and optionally substituted with one or more R⁰¹;
Cy is cycloalkyl or heterocyclyl, and the cycloalkyl and heterocyclyl are each independently and optionally substituted with one or more substituents selected from the group consisting of a deuterium atom, halogen, hydroxy, cyano, oxo, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, and heteroaryl;
each R³, R⁴, and R⁵ is identical or different and is independently selected from the group consisting of a hydrogen atom, alkyl, deuterated alkyl, alkoxy, deuterated alkoxy, alkenyl, alkynyl, NR²⁰R²¹, C(O)NR²⁰R²¹, NR²²C(O)R²³, C(O)R²³, C(O)OR²³, OC(O)R²³, S(O)ᵥR²³, S(O)ᵥOR²³, OS(O)ᵥR²³, S(O)ᵥNR²⁰R²¹, OR²³, cycloalkyl, heterocyclyl, aryl, and heteroaryl, wherein the alkyl, alkoxy, alkenyl, alkynyl, cycloalkyl, heterocyclyl, aryl, and heteroaryl are each independently and optionally substituted with one or more R⁰¹;
alternatively, R³ and R⁴, together with the nitrogen atom to which they are attached, form heterocyclyl; the heterocyclyl is optionally substituted with one or more R⁰¹;
each R⁶, R⁷, and R⁸ is identical or different and is independently selected from the group consisting of a hydrogen atom, alkyl, alkoxy, haloalkyl, deuterated alkyl, haloalkoxy, deuterated alkoxy, hydroxyalkyl, alkenyl, alkynyl, NR²⁰R²¹, C(O)NR²⁰R²¹, NR²²C(O)R²³, C(O)R²³, C(O)OR²³, OC(O)R²³, S(O)ᵥR²³, S(O)ᵥOR²³, OS(O)ᵥR²³, S(O)ᵥNR²⁰R²¹, OR²³, cycloalkyl, heterocyclyl, cycloalkylalkyl, heterocyclylalkyl, aryl, and heteroaryl; the alkyl, alkoxy, alkenyl, alkynyl, cycloalkyl, heterocyclyl, cycloalkylalkyl, heterocyclylalkyl, aryl, and heteroaryl are each independently and optionally substituted with one or more R⁰¹;
each R⁰¹ is identical or different and is independently selected from the group consisting of a deuterium atom, halogen, hydroxy, cyano, oxo, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, heteroaryl, =CR^{7a}R^{8a}, -NR³R⁴, -alkylene-NR³R⁴, -O-alkylene-NR³R⁴, -C(=NR⁵)R⁶, -S(O)ᵥNR³R⁴, -NR^{S}S(O)ᵥR⁶, -S(O)ᵥR⁶, -S(=NR⁵)(O)R⁶, -NR⁵C(O)R⁶,-NR⁵C(O)NR³R⁴, -C(O)NR⁵-OR⁶, -P(O)R⁷R⁸, -C(O)NR⁵-NR³R⁴, -C(=NR⁵)NR⁵-OR⁶,-C(O)NR⁵-alkylene-Cy, -C(=NR⁵)NR³R⁴, -C(O)-C(O)-NR³R⁴, -S(=NR⁵)NR³R⁴,-S(=NR⁵)R⁶, -S(=NR⁵)(O)NR³R⁴, -Si(O)NR³R⁴, -OR⁶, -Si(R^{6a})₃, -O-alkylene-heteroaryl, and -O-alkylene-heterocyclyl; the alkyl, alkenyl, alkynyl, alkoxy, alkylene, cycloalkyl, heterocyclyl, aryl, and heteroaryl are each independently and optionally substituted with one or more substituents selected from the group consisting of a deuterium atom, halogen, hydroxy, cyano, oxo, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, and heteroaryl;
each R^{6a}, R^{7a}, and R^{8a} is identical or different and is independently selected from the group consisting of a hydrogen atom, a deuterium atom, halogen, hydroxy, cyano, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, and heteroaryl;
R' is selected from the group consisting of a hydrogen atom, alkyl, haloalkyl, deuterated alkyl, alkoxy, deuterated alkoxy, hydroxyalkyl, cycloalkyl, and heterocyclyl;
X is O or S;
R^{a} and R^{b} are identical or different and are each independently selected from the group consisting of a hydrogen atom, a deuterium atom, halogen, hydroxy, cyano, amino, alkyl, deuterated alkyl, alkenyl, alkynyl, alkoxy, deuterated alkoxy, haloalkyl, haloalkoxy, hydroxyalkyl, cycloalkyl, heterocyclyl, cycloalkyloxy, and heterocyclyloxy; the alkyl, alkoxy, alkenyl, alkynyl, cycloalkyl, heterocyclyl, cycloalkyloxy, and heterocyclyloxy are optionally substituted with one or more R⁰²;
with the proviso that R^{a} and R^{b} are not simultaneously hydrogen;
R^{c} and R^{d} are identical or different and are each independently selected from the group consisting of a hydrogen atom, a deuterium atom, halogen, hydroxy, cyano, amino, alkyl, alkenyl, alkynyl, alkoxy, haloalkyl, deuterated alkyl, haloalkoxy, deuterated alkoxy, hydroxyalkyl, cycloalkyl, heterocyclyl, cycloalkyloxy, and heterocyclyloxy; the alkyl, alkoxy, alkenyl, alkynyl, cycloalkyl, heterocyclyl, cycloalkyloxy, and heterocyclyloxy are optionally substituted with one or more R⁰²;
alternatively, R^{a} and R^{b}, together with the carbon atom to which they are attached, form cycloalkyl or heterocyclyl; alternatively, R^{c} and R^{d}, together with the carbon atom to which they are attached, form cycloalkyl or heterocyclyl; wherein the cycloalkyl or heterocyclyl is independently and optionally substituted with one or more R⁰²;
R^{e} is selected from the group consisting of a hydrogen atom, a deuterium atom, halogen, hydroxy, cyano, amino, alkyl, alkoxy, haloalkyl, haloalkoxy, deuterated alkyl, deuterated alkoxy, and hydroxyalkyl;
each R⁰² is identical or different and is independently selected from the group consisting of a deuterium atom, halogen, hydroxy, cyano, oxo, amino, an amide group, alkyl, alkenyl, alkynyl, alkoxy, haloalkyl, haloalkoxy, hydroxyalkyl, cycloalkyl, heterocyclyl, aryl, and heteroaryl;
each R²⁰, R²¹, and R²² is identical or different and is independently selected from the group consisting of a hydrogen atom, alkyl, deuterated alkyl, alkoxy, deuterated alkoxy, alkenyl, alkynyl, cycloalkyl, heterocyclyl, aryl, and heteroaryl; the alkyl, alkoxy, alkenyl, alkynyl, cycloalkyl, heterocyclyl, aryl, and heteroaryl are each independently and optionally substituted with one or more groups selected from the group consisting of a deuterium atom, halogen, hydroxy, cyano, amino, alkyl, alkoxy, haloalkyl, haloalkoxy, and hydroxyalkyl;
each R²³ is identical or different and is independently selected from the group consisting of a hydrogen atom, alkyl, deuterated alkyl, alkoxy, deuterated alkoxy, alkenyl, alkynyl, cycloalkyl, heterocyclyl, aryl, and heteroaryl; the alkyl, alkoxy, alkenyl, alkynyl, cycloalkyl, heterocyclyl, aryl, and heteroaryl are each independently and optionally substituted with one or more groups selected from the group consisting of a deuterium atom, halogen, hydroxy, cyano, amino, alkyl, alkoxy, haloalkyl, haloalkoxy, and hydroxyalkyl;
X¹ is CR^{X1} or N;
X² is CR^{X2} or N;
X³ is CR^{X3} or N;
X⁴ is CR^{X4} or N;
X⁵ is CR^{x5} or N;
X¹, X², X³, X⁴, and X⁵ are not simultaneously N;
R^{X1}, R^{X2}, R^{X3}, R^{X4}, and R^{X5} are identical or different and are each independently selected from the group consisting of a hydrogen atom, a deuterium atom, halogen, hydroxy, cyano, amino, an amide group, nitro, alkyl, alkenyl, alkynyl, alkoxy, haloalkyl, deuterated alkyl, haloalkoxy, deuterated alkoxy, hydroxyalkyl, cycloalkyl, heterocyclyl, -O-(CH₂)ₙ-cycloalkyl, -O-(CH₂)ₛ-heterocyclyl, aryl, and heteroaryl, wherein the alkyl, alkoxy, alkenyl, alkynyl, cycloalkyl, heterocyclyl, aryl, and heteroaryl are each independently and optionally substituted with one or more R⁰³;
each R⁰³ is identical or different and is independently selected from the group consisting of a deuterium atom, halogen, hydroxy, cyano, oxo, amino, an amide group, alkyl, alkenyl, alkynyl, alkoxy, haloalkyl, haloalkoxy, hydroxyalkyl, cycloalkyl, heterocyclyl, aryl, and heteroaryl;
each v is identical or different and is independently selected from the group consisting of 0, 1, and 2;
n is selected from the group consisting of 0, 1, 2, 3, 4, and 5;
s is selected from the group consisting of 0, 1, 2, 3, 4, and 5; and
r is selected from the group consisting of 0, 1, 2, 3, 4, and 5;
with the proviso that 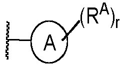 is not

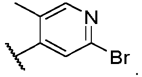

The present disclosure aims to provide a compound represented by general formula (I) or a pharmaceutically acceptable salt thereof: 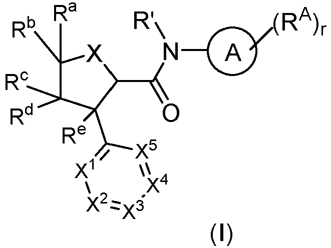 wherein:
ring A is phenyl or 6-membered heteroaryl;
each R^{A} is identical or different and is independently selected from the group consisting of a deuterium atom, halogen, hydroxy, cyano, alkyl, alkoxy, haloalkyl, haloalkoxy, hydroxyalkyl, hydroxyalkoxy, alkoxyalkyl, alkenyl, alkynyl, -NR³R⁴, -alkylene-NR³R⁴, -O-alkylene-NR³R⁴, -C(=NR⁵)R⁶, -S(O)ᵥNR³R⁴, -NR^{S}S(O)ᵥR⁶, -S(O)ᵥR⁶, - S(=NR⁵)(O)R⁶, -NR⁵C(O)R⁶, -NR⁵C(O)NR³R⁴, -C(O)NR⁵-OR⁶, -P(O)R⁷R⁸, -C(O)NR⁵-NR³R⁴, -C(=NR⁵)NR⁵-OR⁶, -C(O)NR⁵-alkylene-Cy, -C(=NR⁵)NR³R⁴, -C(O)-C(O)-NR³R⁴, -S(=NR⁵)NR³R⁴, -S(=NR⁵)R⁶, -S(=NR⁵)(O)NR³R⁴, -Si(O)NR³R⁴, -Si(R⁶)₃,-OR⁶, cycloalkyl, heterocyclyl, aryl, heteroaryl, cycloalkylalkyl, heterocyclylalkyl, -C(O)-cycloalkyl, -C(O)-heterocyclyl, -alkylene-O-alkylene-cycloalkyl, -alkylene-O-cycloalkyl, -O-alkylene-heteroaryl, and -O-alkylene-heterocyclyl, wherein the alkyl, alkoxy, alkoxyalkyl, alkenyl, alkynyl, alkylene, cycloalkyl, heterocyclyl, aryl, heteroaryl, cycloalkylalkyl, and heterocyclylalkyl are each independently and optionally substituted with one or more R⁰¹;
Cy is cycloalkyl or heterocyclyl, and the cycloalkyl and heterocyclyl are each independently and optionally substituted with one or more substituents selected from the group consisting of a deuterium atom, halogen, hydroxy, cyano, oxo, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, and heteroaryl;
each R³, R⁴, and R⁵ is identical or different and is independently selected from the group consisting of a hydrogen atom, alkyl, alkenyl, alkynyl, NR²⁰R²¹, C(O)NR²⁰R²¹, NR²²C(O)R²³, C(O)R²³, C(O)OR²³, OC(O)R²³, S(O)ᵥR²³, S(O)ᵥOR²³, OS(O)ᵥR²³, S(O)ᵥNR²⁰R²¹, OR²³, cycloalkyl, heterocyclyl, aryl, and heteroaryl, wherein the alkyl, alkenyl, alkynyl, cycloalkyl, heterocyclyl, aryl, and heteroaryl are each independently and optionally substituted with one or more R⁰¹;
alternatively, R³ and R⁴, together with the nitrogen atom to which they are attached, form heterocyclyl; the heterocyclyl is optionally substituted with one or more R⁰¹;
each R⁶, R⁷, and R⁸ is identical or different and is independently selected from the group consisting of a hydrogen atom, alkyl, alkoxy, haloalkyl, haloalkoxy, hydroxyalkyl, alkenyl, alkynyl, NR²⁰R²¹, C(O)NR²⁰R²¹, NR²²C(O)R²³, C(O)R²³, C(O)OR²³, OC(O)R²³, S(O)ᵥR²³, S(O)ᵥOR²³, OS(O)ᵥR²³, S(O)ᵥNR²⁰R²¹, OR²³, cycloalkyl, heterocyclyl, cycloalkylalkyl, heterocyclylalkyl, aryl, and heteroaryl; the alkyl, alkoxy, alkenyl, alkynyl, cycloalkyl, heterocyclyl, cycloalkylalkyl, heterocyclylalkyl, aryl, and heteroaryl are each independently and optionally substituted with one or more R⁰¹;
each R⁰¹ is identical or different and is independently selected from the group consisting of a deuterium atom, halogen, hydroxy, cyano, oxo, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, heteroaryl, =CR^{7a}R^{8a}, -NR³R⁴, -alkylene-NR³R⁴, -O-alkylene-NR³R⁴, -C(=NR⁵)R⁶, -S(O)ᵥNR³R⁴, -NR^{S}S(O)ᵥR⁶, -S(O)ᵥR⁶, -S(=NR⁵)(O)R⁶, -NR⁵C(O)R⁶,-NR⁵C(O)NR³R⁴, -C(O)NR⁵-OR⁶, -P(O)R⁷R⁸, -C(O)NR⁵-NR³R⁴, -C(=NR⁵)NR⁵-OR⁶,-C(O)NR⁵-alkylene-Cy, -C(=NR⁵)NR³R⁴, -C(O)-C(O)-NR³R⁴, -S(=NR⁵)NR³R⁴,-S(=NR⁵)R⁶, -S(=NR⁵)(O)NR³R⁴, -Si(O)NR³R⁴, -OR⁶, -Si(R^{6a})₃, -O-alkylene-heteroaryl, and -O-alkylene-heterocyclyl; the alkyl, alkenyl, alkynyl, alkoxy, alkylene, cycloalkyl, heterocyclyl, aryl, and heteroaryl are each independently and optionally substituted with one or more substituents selected from the group consisting of a deuterium atom, halogen, hydroxy, cyano, oxo, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, and heteroaryl;
each R^{6a}, R^{7a}, and R^{8a} is identical or different and is independently selected from the group consisting of a hydrogen atom, a deuterium atom, halogen, hydroxy, cyano, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, and heteroaryl;
R' is selected from the group consisting of a hydrogen atom, alkyl, haloalkyl, hydroxyalkyl, cycloalkyl, and heterocyclyl;
X is O or S;
R^{a} and R^{b} are identical or different and are each independently selected from the group consisting of a hydrogen atom, a deuterium atom, halogen, hydroxy, cyano, amino, alkyl, alkenyl, alkynyl, alkoxy, haloalkyl, haloalkoxy, hydroxyalkyl, cycloalkyl, heterocyclyl, cycloalkyloxy, and heterocyclyloxy; the cycloalkyl, heterocyclyl, cycloalkyloxy, and heterocyclyloxy are optionally substituted with one or more R⁰²;
with the proviso that R^{a} and R^{b} are not simultaneously hydrogen;
R^{c} and R^{d} are identical or different and are each independently selected from the group consisting of a hydrogen atom, halogen, hydroxy, cyano, amino, alkyl, alkenyl, alkynyl, alkoxy, haloalkyl, haloalkoxy, hydroxyalkyl, cycloalkyl, heterocyclyl, cycloalkyloxy, and heterocyclyloxy; the cycloalkyl, heterocyclyl, cycloalkyloxy, and heterocyclyloxy are optionally substituted with one or more R⁰²;
alternatively, R^{a} and R^{b}, together with the carbon atom to which they are attached, form cycloalkyl or heterocyclyl; alternatively, R^{c} and R^{d}, together with the carbon atom to which they are attached, form cycloalkyl or heterocyclyl; wherein the cycloalkyl or heterocyclyl is independently and optionally substituted with one or more R⁰²;
R^{e} is selected from the group consisting of a hydrogen atom, a deuterium atom, halogen, hydroxy, cyano, amino, alkyl, alkoxy, haloalkyl, haloalkoxy, and hydroxyalkyl;
each R⁰² is identical or different and is independently selected from the group consisting of a deuterium atom, halogen, hydroxy, cyano, oxo, amino, an amide group, alkyl, alkenyl, alkynyl, alkoxy, haloalkyl, haloalkoxy, hydroxyalkyl, cycloalkyl, heterocyclyl, aryl, and heteroaryl;
each R²⁰, R²¹, and R²² is identical or different and is independently selected from the group consisting of a hydrogen atom, alkyl, alkoxy, alkenyl, alkynyl, cycloalkyl, heterocyclyl, aryl, and heteroaryl; the alkyl, alkoxy, alkenyl, alkynyl, cycloalkyl, heterocyclyl, aryl, and heteroaryl are each independently and optionally substituted with one or more groups selected from the group consisting of halogen, hydroxy, cyano, amino, alkyl, alkoxy, haloalkyl, haloalkoxy, and hydroxyalkyl;
each R²³ is identical or different and is independently selected from the group consisting of a hydrogen atom, alkyl, alkoxy, alkenyl, alkynyl, cycloalkyl, heterocyclyl, aryl, and heteroaryl; the alkyl, alkoxy, alkenyl, alkynyl, cycloalkyl, heterocyclyl, aryl, and heteroaryl are each independently and optionally substituted with one or more groups selected from the group consisting of halogen, hydroxy, cyano, amino, alkyl, alkoxy, haloalkyl, haloalkoxy, and hydroxyalkyl;
X¹ is CR^{X1} or N;
X² is CR^{X2} or N;
X³ is CR^{X3} or N;
X⁴ is CR^{X4} or N;
X⁵ is CR^{X5} or N;
X¹, X², X³, X⁴, and X⁵ are not simultaneously N;
R^{X1}, R^{X2}, R^{X3}, R^{X4}, and R^{X5} are identical or different and are each independently selected from the group consisting of a hydrogen atom, a deuterium atom, halogen, hydroxy, cyano, amino, an amide group, nitro, alkyl, alkenyl, alkynyl, alkoxy, haloalkyl, haloalkoxy, hydroxyalkyl, cycloalkyl, heterocyclyl, -O-(CH₂)ₙ-cycloalkyl, -O-(CH₂)ₛ-heterocyclyl, aryl, and heteroaryl, wherein the cycloalkyl, heterocyclyl, aryl, and heteroaryl are each independently and optionally substituted with one or more R⁰³;
each R⁰³ is identical or different and is independently selected from the group consisting of a deuterium atom, halogen, hydroxy, cyano, oxo, amino, an amide group, alkyl, alkenyl, alkynyl, alkoxy, haloalkyl, haloalkoxy, hydroxyalkyl, cycloalkyl, heterocyclyl, aryl, and heteroaryl;
each v is identical or different and is independently selected from the group consisting of 0, 1, and 2;
n is selected from the group consisting of 0, 1, 2, 3, 4, and 5;
s is selected from the group consisting of 0, 1, 2, 3, 4, and 5; and
r is selected from the group consisting of 0, 1, 2, 3, 4, and 5;
with the proviso that 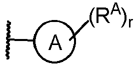 is not

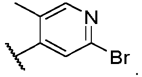

The present disclosure aims to provide a compound represented by general formula (I) or a pharmaceutically acceptable salt thereof: 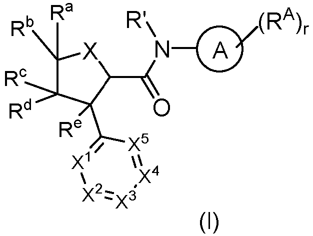 wherein:
ring A is phenyl or 6-membered heteroaryl;
each R^{A} is identical or different and is independently selected from the group consisting of a deuterium atom, halogen, hydroxy, cyano, alkyl, alkoxy, haloalkyl, haloalkoxy, hydroxyalkyl, hydroxyalkoxy, alkenyl, alkynyl, -NR³R⁴, -alkylene-NR³R⁴, -O-alkylene-NR³R⁴, -C(=NR⁵)R⁶, -S(O)ᵥNR³R⁴, -NR^{S}S(O)ᵥR⁶, -S(O)ᵥR⁶, -S(=NR⁵)(O)R⁶,-NR⁵C(O)R⁶, -NR⁵C(O)NR³R⁴, -C(O)NR⁵-OR⁶, -P(O)R⁷R⁸, -C(O)NR⁵-NR³R⁴,-C(=NR⁵)NR⁵-OR⁶, -C(O)NR⁵-alkylene-Cy, -C(=NR⁵)NR³R⁴, -C(O)-C(O)-NR³R⁴,-S(=NR⁵)NR³R⁴, cycloalkyl, heterocyclyl, aryl, heteroaryl, -O-alkylene-heteroaryl, and-O-alkylene-heterocyclyl, wherein the alkenyl, alkynyl, alkylene, cycloalkyl, heterocyclyl, aryl, and heteroaryl are each independently and optionally substituted with one or more R⁰¹;
Cy is cycloalkyl or 3- to 4-membered heterocyclyl, and the cycloalkyl or 3- to 4-membered heterocyclyl is independently and optionally substituted with one or more substituents selected from the group consisting of a deuterium atom, halogen, hydroxy, cyano, oxo, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, and heteroaryl;
each R³, R⁴, and R⁵ is identical or different and is independently selected from the group consisting of a hydrogen atom, alkyl, alkenyl, alkynyl, NR²⁰R²¹, C(O)NR²⁰R²¹, NR²²C(O)R²³, C(O)R²³, C(O)OR²³, OC(O)R²³, S(O)ᵥR²³, S(O)ᵥOR²³, OS(O)ᵥR²³, S(O)ᵥNR²⁰R²¹, NR²²S(O)ᵥR²³, OR²³, cycloalkyl, heterocyclyl, aryl, and heteroaryl, wherein the alkyl, alkenyl, alkynyl, cycloalkyl, heterocyclyl, aryl, and heteroaryl are each independently and optionally substituted with one or more R⁰¹;
alternatively, R³ and R⁴, together with the nitrogen atom to which they are attached, form heterocyclyl; the heterocyclyl is optionally substituted with one or more R⁰¹;
each R⁶, R⁷, and R⁸ is identical or different and is independently selected from the group consisting of a hydrogen atom, alkyl, alkoxy, haloalkyl, haloalkoxy, hydroxyalkyl, alkenyl, alkynyl, NR²⁰R²¹, C(O)NR²⁰R²¹, NR²²C(O)R²³, C(O)R²³, C(O)OR²³, OC(O)R²³, S(O)ᵥR²³, S(O)ᵥOR²³, OS(O)ᵥR²³, S(O)ᵥNR²⁰R²¹, NR²²S(O)ᵥR²³, OR²³, cycloalkyl, heterocyclyl, cycloalkylalkyl, heterocyclylalkyl, aryl, and heteroaryl; the alkyl, alkoxy, alkenyl, alkynyl, cycloalkyl, heterocyclyl, cycloalkylalkyl, heterocyclylalkyl, aryl, and heteroaryl are each independently and optionally substituted with one or more substituents selected from the group consisting of a deuterium atom, halogen, hydroxy, cyano, oxo, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, and heteroaryl;
each R⁰¹ is identical or different and is independently selected from the group consisting of a deuterium atom, halogen, hydroxy, cyano, oxo, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, and heteroaryl;
R' is selected from the group consisting of a hydrogen atom, alkyl, haloalkyl, hydroxyalkyl, cycloalkyl, and heterocyclyl;
X is O or S;
R^{a} and R^{b} are identical or different and are each independently selected from the group consisting of a hydrogen atom, a deuterium atom, halogen, hydroxy, cyano, amino, alkyl, alkenyl, alkynyl, alkoxy, haloalkyl, haloalkoxy, hydroxyalkyl, cycloalkyl, heterocyclyl, cycloalkyloxy, and heterocyclyloxy; the cycloalkyl, heterocyclyl, cycloalkyloxy, and heterocyclyloxy are optionally substituted with one or more R⁰²;
with the proviso that R^{a} and R^{b} are not simultaneously hydrogen;
R^{c} and R^{d} are identical or different and are each independently selected from the group consisting of a hydrogen atom, halogen, hydroxy, cyano, amino, alkyl, alkenyl, alkynyl, alkoxy, haloalkyl, haloalkoxy, hydroxyalkyl, cycloalkyl, heterocyclyl, cycloalkyloxy, and heterocyclyloxy; the cycloalkyl, heterocyclyl, cycloalkyloxy, and heterocyclyloxy are optionally substituted with one or more R⁰²;
alternatively, R^{a} and R^{b}, together with the carbon atom to which they are attached, form cycloalkyl or heterocyclyl; alternatively, R^{c} and R^{d}, together with the carbon atom to which they are attached, form cycloalkyl or heterocyclyl; wherein the cycloalkyl or heterocyclyl is independently and optionally substituted with one or more R⁰²;
R^{e} is selected from the group consisting of a hydrogen atom, a deuterium atom, halogen, hydroxy, cyano, amino, alkyl, alkoxy, haloalkyl, haloalkoxy, and hydroxyalkyl;
each R⁰² is identical or different and is independently selected from the group consisting of a deuterium atom, halogen, hydroxy, cyano, oxo, amino, an amide group, alkyl, alkenyl, alkynyl, alkoxy, haloalkyl, haloalkoxy, hydroxyalkyl, cycloalkyl, heterocyclyl, aryl, and heteroaryl;
each R²⁰, R²¹, and R²² is identical or different and is independently selected from the group consisting of a hydrogen atom, alkyl, alkoxy, alkenyl, alkynyl, cycloalkyl, heterocyclyl, aryl, and heteroaryl; the alkyl, alkoxy, alkenyl, alkynyl, cycloalkyl, heterocyclyl, aryl, and heteroaryl are each independently and optionally substituted with one or more groups selected from the group consisting of halogen, hydroxy, cyano, amino, alkyl, alkoxy, haloalkyl, haloalkoxy, and hydroxyalkyl;
each R²³ is identical or different and is independently selected from the group consisting of a hydrogen atom, alkyl, alkoxy, alkenyl, alkynyl, cycloalkyl, heterocyclyl, aryl, and heteroaryl; the alkyl, alkoxy, alkenyl, alkynyl, cycloalkyl, heterocyclyl, aryl, and heteroaryl are each independently and optionally substituted with one or more groups selected from the group consisting of halogen, hydroxy, cyano, amino, alkyl, alkoxy, haloalkyl, haloalkoxy, and hydroxyalkyl;
X¹ is CR^{X1} or N;
X² is CR^{X2} or N;
X³ is CR^{X3} or N;
X⁴ is CR^{X4} or N;
X⁵ is CR^{x5} or N;
X¹, X², X³, X⁴, and X⁵ are not simultaneously N;
R^{X1}, R^{X2}, R^{X3}, R^{X4}, and R^{X5} are identical or different and are each independently selected from the group consisting of a hydrogen atom, a deuterium atom, halogen, hydroxy, cyano, amino, an amide group, nitro, alkyl, alkenyl, alkynyl, alkoxy, haloalkyl, haloalkoxy, hydroxyalkyl, cycloalkyl, heterocyclyl, -O-(CH₂)ₙ-cycloalkyl, -O-(CH₂)ₛ-heterocyclyl, aryl, and heteroaryl, wherein the cycloalkyl, heterocyclyl, aryl, and heteroaryl are each independently and optionally substituted with one or more R⁰³;
each R⁰³ is identical or different and is independently selected from the group consisting of a deuterium atom, halogen, hydroxy, cyano, oxo, amino, an amide group, alkyl, alkenyl, alkynyl, alkoxy, haloalkyl, haloalkoxy, hydroxyalkyl, cycloalkyl, heterocyclyl, aryl, and heteroaryl;
each v is identical or different and is independently selected from the group consisting of 0, 1, and 2;
n is selected from the group consisting of 0, 1, 2, 3, 4, and 5;
s is selected from the group consisting of 0, 1, 2, 3, 4, and 5; and
r is selected from the group consisting of 0, 1, 2, 3, 4, and 5;
with the proviso that 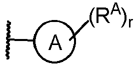 is not

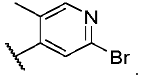

The present disclosure aims to provide a compound represented by general formula (I) or a pharmaceutically acceptable salt thereof: 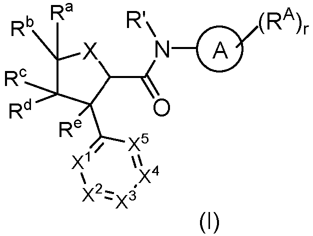 wherein:
ring A is phenyl or 6-membered heteroaryl;
each R^{A} is identical or different and is independently selected from the group consisting of halogen, hydroxy, cyano, alkyl, alkoxy, haloalkyl, haloalkoxy, hydroxyalkyl, hydroxyalkoxy, alkenyl, -NR³R⁴, -alkylene-NR³R⁴, -O-alkylene-NR³R⁴, -C(=NR⁵)R⁶,-S(O)ᵥNR³R⁴, -NR⁵S(O)ᵥR⁶, -S(O)ᵥR⁶, -S(=NR⁵)(O)R⁶, -NR⁵C(O)R⁶, -NR⁵C(O)NR³R⁴,-C(O)NR⁵-OR⁶, -P(O)R⁷R⁸, -C(O)NR⁵-NR³R⁴, -C(=NR⁵)NR⁵-OR⁶, -C(O)NR⁵-alkylene-Cy, -C(=NR⁵)NR³R⁴, -C(O)-C(O)-NR³R⁴, cycloalkyl, heterocyclyl, aryl, heteroaryl, -O-alkylene-heteroaryl, and -O-alkylene-heterocyclyl, wherein the alkenyl, alkylene, cycloalkyl, heterocyclyl, aryl, and heteroaryl are each independently and optionally substituted with one or more R⁰¹;
Cy is cycloalkyl or 3- to 4-membered heterocyclyl, and the cycloalkyl or 3- to 4-membered heterocyclyl is independently and optionally substituted with one or more substituents selected from the group consisting of halogen, hydroxy, cyano, oxo, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, and heteroaryl;
R³, R⁴, and R⁵ are identical or different and are each independently selected from the group consisting of a hydrogen atom, alkyl, cycloalkyl, heterocyclyl, aryl, and heteroaryl, wherein the alkyl, cycloalkyl, heterocyclyl, aryl, and heteroaryl are each independently and optionally substituted with one or more R⁰¹;
alternatively, R³ and R⁴, together with the nitrogen atom to which they are attached, form heterocyclyl; the heterocyclyl is optionally substituted with one or more R⁰¹;
R⁶, R⁷, and R⁸ are identical or different and are each independently selected from the group consisting of a hydrogen atom, alkyl, alkoxy, haloalkyl, haloalkoxy, hydroxyalkyl, cycloalkyl, heterocyclyl, cycloalkylalkyl, heterocyclylalkyl, aryl, and heteroaryl; the alkyl, alkoxy, cycloalkyl, heterocyclyl, cycloalkylalkyl, heterocyclylalkyl, aryl, and heteroaryl are each independently and optionally substituted with one or more substituents selected from the group consisting of halogen, hydroxy, cyano, oxo, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, and heteroaryl;
each R⁰¹ is independently selected from the group consisting of halogen, hydroxy, cyano, oxo, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, and heteroaryl;
R' is selected from the group consisting of a hydrogen atom, alkyl, haloalkyl, hydroxyalkyl, cycloalkyl, and heterocyclyl;
X is O or S;
R^{a} and R^{b} are identical or different and are each independently selected from the group consisting of a hydrogen atom, halogen, hydroxy, cyano, amino, alkyl, alkenyl, alkynyl, alkoxy, haloalkyl, haloalkoxy, hydroxyalkyl, cycloalkyl, heterocyclyl, cycloalkyloxy, and heterocyclyloxy; the cycloalkyl, heterocyclyl, cycloalkyloxy, and heterocyclyloxy are optionally substituted with one or more R⁰²;
with the proviso that R^{a} and R^{b} are not simultaneously hydrogen;
R^{c} and R^{d} are identical or different and are each independently selected from the group consisting of a hydrogen atom, halogen, hydroxy, cyano, amino, alkyl, alkenyl, alkynyl, alkoxy, haloalkyl, haloalkoxy, hydroxyalkyl, cycloalkyl, heterocyclyl, cycloalkyloxy, and heterocyclyloxy; the cycloalkyl, heterocyclyl, cycloalkyloxy, and heterocyclyloxy are optionally substituted with one or more R⁰²;
alternatively, R^{a} and R^{b}, together with the carbon atom to which they are attached, form cycloalkyl or heterocyclyl; alternatively, R^{c} and R^{d}, together with the carbon atom to which they are attached, form cycloalkyl or heterocyclyl; wherein the cycloalkyl or heterocyclyl is independently and optionally substituted with one or more R⁰²;
R^{e} is selected from the group consisting of a hydrogen atom, halogen, hydroxy, cyano, amino, alkyl, alkoxy, haloalkyl, haloalkoxy, and hydroxyalkyl;
each R⁰² is independently selected from the group consisting of halogen, hydroxy, cyano, oxo, amino, an amide group, alkyl, alkenyl, alkynyl, alkoxy, haloalkyl, haloalkoxy, hydroxyalkyl, cycloalkyl, heterocyclyl, aryl, and heteroaryl;
X¹ is CR^{X1} or N;
X² is CR^{X2} or N;
X³ is CR^{X3} or N;
X⁴ is CR^{X4} or N;
X⁵ is CR^{x5} or N;
X¹, X², X³, X⁴, and X⁵ are not simultaneously N;
R^{X1}, R^{X2}, R^{X3}, R^{X4}, and R^{X5} are identical or different and are each independently selected from the group consisting of a hydrogen atom, halogen, hydroxy, cyano, amino, an amide group, nitro, alkyl, alkenyl, alkynyl, alkoxy, haloalkyl, haloalkoxy, hydroxyalkyl, cycloalkyl, heterocyclyl, -O-(CH₂)ₙ-cycloalkyl, -O-(CH₂)ₛ-heterocyclyl, aryl, and heteroaryl, wherein the cycloalkyl, heterocyclyl, aryl, and heteroaryl are each independently and optionally substituted with one or more R⁰³;
each R⁰³ is independently selected from the group consisting of halogen, hydroxy, cyano, oxo, amino, an amide group, alkyl, alkenyl, alkynyl, alkoxy, haloalkyl, haloalkoxy, hydroxyalkyl, cycloalkyl, heterocyclyl, aryl, and heteroaryl;
v is selected from the group consisting of 0, 1, and 2;
n is selected from the group consisting of 0, 1, 2, 3, 4, and 5;
s is selected from the group consisting of 0, 1, 2, 3, 4, and 5; and
r is selected from the group consisting of 0, 1, 2, 3, 4, and 5;
with the proviso that 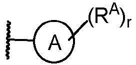 is not

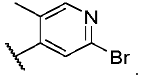

The present disclosure aims to provide a compound represented by general formula (I) or a pharmaceutically acceptable salt thereof: 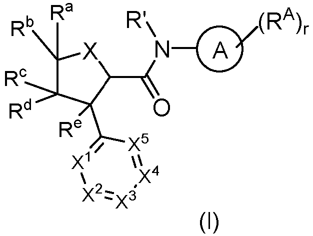 wherein:
ring A is phenyl or 6-membered heteroaryl;
each R^{A} is identical or different and is independently selected from the group consisting of halogen, hydroxy, cyano, alkyl, alkoxy, haloalkyl, haloalkoxy, hydroxyalkyl, hydroxyalkoxy, alkenyl, -NR³R⁴, -alkylene-NR³R⁴, -O-alkylene-NR³R⁴, -C(=NR⁵)R⁶,-S(O)ᵥNR³R⁴, -NR⁵S(O)ᵥR⁶, -S(O)ᵥR⁶, -S(=NR⁵)(O)R⁶, -NR⁵C(O)R⁶, -NR⁵C(O)NR³R⁴,-C(O)NR⁵-OR⁶, -P(O)R⁷R⁸, cycloalkyl, heterocyclyl, aryl, heteroaryl, -O-alkylene-heteroaryl, and -O-alkylene-heterocyclyl, wherein the alkenyl, alkylene, cycloalkyl, heterocyclyl, aryl, and heteroaryl are each independently and optionally substituted with one or more R⁰¹;
R³, R⁴, and R⁵ are identical or different and are each independently selected from the group consisting of a hydrogen atom, alkyl, cycloalkyl, heterocyclyl, aryl, and heteroaryl, wherein the alkyl, cycloalkyl, heterocyclyl, aryl, and heteroaryl are each independently and optionally substituted with one or more R⁰¹;
alternatively, R³ and R⁴, together with the nitrogen atom to which they are attached, form heterocyclyl; the heterocyclyl is optionally substituted with one or more R⁰¹;
R⁶, R⁷, and R⁸ are identical or different and are each independently selected from the group consisting of a hydrogen atom, alkyl, alkoxy, haloalkyl, haloalkoxy, hydroxyalkyl, cycloalkyl, heterocyclyl, aryl, and heteroaryl;
each R⁰¹ is independently selected from the group consisting of halogen, hydroxy, cyano, oxo, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, and heteroaryl;
R' is selected from the group consisting of a hydrogen atom, alkyl, haloalkyl, hydroxyalkyl, cycloalkyl, and heterocyclyl;
X is O or S;
R^{a} and R^{b} are identical or different and are each independently selected from the group consisting of a hydrogen atom, halogen, hydroxy, cyano, amino, alkyl, alkenyl, alkynyl, alkoxy, haloalkyl, haloalkoxy, hydroxyalkyl, cycloalkyl, heterocyclyl, cycloalkyloxy, and heterocyclyloxy; the cycloalkyl, heterocyclyl, cycloalkyloxy, and heterocyclyloxy are optionally substituted with one or more R⁰²;
with the proviso that R^{a} and R^{b} are not simultaneously hydrogen;
R^{c} and R^{d} are identical or different and are each independently selected from the group consisting of a hydrogen atom, halogen, hydroxy, cyano, amino, alkyl, alkenyl, alkynyl, alkoxy, haloalkyl, haloalkoxy, hydroxyalkyl, cycloalkyl, heterocyclyl, cycloalkyloxy, and heterocyclyloxy; the cycloalkyl, heterocyclyl, cycloalkyloxy, and heterocyclyloxy are optionally substituted with one or more R⁰²;
alternatively, R^{a} and R^{b}, together with the carbon atom to which they are attached, form cycloalkyl or heterocyclyl; alternatively, R^{c} and R^{d}, together with the carbon atom to which they are attached, form cycloalkyl or heterocyclyl; wherein the cycloalkyl or heterocyclyl is independently and optionally substituted with one or more R⁰²;
R^{e} is selected from the group consisting of a hydrogen atom, halogen, hydroxy, cyano, amino, alkyl, alkoxy, haloalkyl, haloalkoxy, and hydroxyalkyl;
each R⁰² is independently selected from the group consisting of halogen, hydroxy, cyano, oxo, amino, an amide group, alkyl, alkenyl, alkynyl, alkoxy, haloalkyl, haloalkoxy, hydroxyalkyl, cycloalkyl, heterocyclyl, aryl, and heteroaryl;
X¹ is CR^{X1} or N;
X² is CR^{X2} or N;
X³ is CR^{X3} or N;
X⁴ is CR^{X4} or N;
X⁵ is CR^{x5} or N;
X¹, X², X³, X⁴, and X⁵ are not simultaneously N;
R^{X1}, R^{X2}, R^{X3}, R^{X4}, and R^{X5} are identical or different and are each independently selected from the group consisting of a hydrogen atom, halogen, hydroxy, cyano, amino, an amide group, nitro, alkyl, alkenyl, alkynyl, alkoxy, haloalkyl, haloalkoxy, hydroxyalkyl, cycloalkyl, heterocyclyl, -O-(CH₂)ₙ-cycloalkyl, -O-(CH₂)ₛ-heterocyclyl, aryl, and heteroaryl, wherein the cycloalkyl, heterocyclyl, aryl, and heteroaryl are each independently and optionally substituted with one or more R⁰³;
each R⁰³ is independently selected from the group consisting of halogen, hydroxy, cyano, oxo, amino, an amide group, alkyl, alkenyl, alkynyl, alkoxy, haloalkyl, haloalkoxy, hydroxyalkyl, cycloalkyl, heterocyclyl, aryl, and heteroaryl;
v is selected from the group consisting of 0, 1, and 2;
n is selected from the group consisting of 0, 1, 2, 3, 4, and 5;
s is selected from the group consisting of 0, 1, 2, 3, 4, and 5; and
r is selected from the group consisting of 0, 1, 2, 3, 4, and 5;
with the proviso that 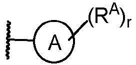 is not

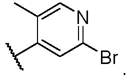

The present disclosure aims to provide a compound represented by general formula (I) or a pharmaceutically acceptable salt thereof: 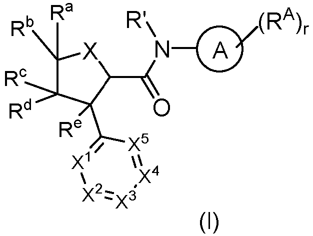 wherein:
ring A is phenyl or 6-membered heteroaryl;
each R^{A} is identical or different and is independently selected from the group consisting of halogen, hydroxy, cyano, alkyl, alkoxy, haloalkyl, haloalkoxy, hydroxyalkyl, hydroxyalkoxy, alkenyl, -NR³R⁴, -alkylene-NR³R⁴, -O-alkylene-NR³R⁴, -C(=NR⁵)R⁶,-S(O)ᵥNR³R⁴, -NR⁵S(O)ᵥR⁶, -S(O)ᵥR⁶, -S(=NR⁵)(O)R⁶, -P(O)R⁷R⁸, cycloalkyl, heterocyclyl, aryl, heteroaryl, -O-alkylene-heteroaryl, and -O-alkylene-heterocyclyl, wherein the alkenyl, alkylene, cycloalkyl, heterocyclyl, aryl, and heteroaryl are each independently and optionally substituted with one or more R⁰¹;
R³, R⁴, and R⁵ are identical or different and are each independently selected from the group consisting of a hydrogen atom, alkyl, cycloalkyl, heterocyclyl, aryl, and heteroaryl, wherein the alkyl, cycloalkyl, heterocyclyl, aryl, and heteroaryl are each independently and optionally substituted with one or more R⁰¹;
alternatively, R³ and R⁴, together with the nitrogen atom to which they are attached, form heterocyclyl; the heterocyclyl is optionally substituted with one or more R⁰¹;
R⁶, R⁷, and R⁸ are identical or different and are each independently selected from the group consisting of a hydrogen atom, alkyl, alkoxy, haloalkyl, haloalkoxy, hydroxyalkyl, cycloalkyl, heterocyclyl, aryl, and heteroaryl;
each R⁰¹ is independently selected from the group consisting of halogen, hydroxy, cyano, oxo, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, and heteroaryl; R' is selected from the group consisting of a hydrogen atom, alkyl, haloalkyl, hydroxyalkyl, cycloalkyl, and heterocyclyl;
X is O or S;
R^{a} and R^{b} are identical or different and are each independently selected from the group consisting of a hydrogen atom, halogen, hydroxy, cyano, amino, alkyl, alkenyl, alkynyl, alkoxy, haloalkyl, haloalkoxy, hydroxyalkyl, cycloalkyl, heterocyclyl, cycloalkyloxy, and heterocyclyloxy; the cycloalkyl, heterocyclyl, cycloalkyloxy, and heterocyclyloxy are optionally substituted with one or more R⁰²;
with the proviso that R^{a} and R^{b} are not simultaneously hydrogen;
R^{c} and R^{d} are identical or different and are each independently selected from the group consisting of a hydrogen atom, halogen, hydroxy, cyano, amino, alkyl, alkenyl, alkynyl, alkoxy, haloalkyl, haloalkoxy, hydroxyalkyl, cycloalkyl, heterocyclyl, cycloalkyloxy, and heterocyclyloxy; the cycloalkyl, heterocyclyl, cycloalkyloxy, and heterocyclyloxy are optionally substituted with one or more R⁰²;
alternatively, R^{a} and R^{b}, together with the carbon atom to which they are attached, form cycloalkyl or heterocyclyl; alternatively, R^{c} and R^{d}, together with the carbon atom to which they are attached, form cycloalkyl or heterocyclyl; wherein the cycloalkyl or heterocyclyl is independently and optionally substituted with one or more R⁰²;
R^{e} is selected from the group consisting of a hydrogen atom, halogen, hydroxy, cyano, amino, alkyl, alkoxy, haloalkyl, haloalkoxy, and hydroxyalkyl;
each R⁰² is independently selected from the group consisting of halogen, hydroxy, cyano, oxo, amino, an amide group, alkyl, alkenyl, alkynyl, alkoxy, haloalkyl, haloalkoxy, hydroxyalkyl, cycloalkyl, heterocyclyl, aryl, and heteroaryl;
X¹ is CR^{X1} or N;
X² is CR^{X2} or N;
X³ is CR^{X3} or N;
X⁴ is CR^{X4} or N;
X⁵ is CR^{x5} or N;
X¹, X², X³, X⁴, and X⁵ are not simultaneously N;
R^{X1}, R^{X2}, R^{X3}, R^{X4}, and R^{X5} are identical or different and are each independently selected from the group consisting of a hydrogen atom, halogen, hydroxy, cyano, amino, an amide group, nitro, alkyl, alkenyl, alkynyl, alkoxy, haloalkyl, haloalkoxy, hydroxyalkyl, cycloalkyl, heterocyclyl, -O-(CH₂)ₙ-cycloalkyl, -O-(CH₂)ₛ-heterocyclyl, aryl, and heteroaryl, wherein the cycloalkyl, heterocyclyl, aryl, and heteroaryl are each independently and optionally substituted with one or more R⁰³;
each R⁰³ is independently selected from the group consisting of halogen, hydroxy, cyano, oxo, amino, an amide group, alkyl, alkenyl, alkynyl, alkoxy, haloalkyl, haloalkoxy, hydroxyalkyl, cycloalkyl, heterocyclyl, aryl, and heteroaryl;
v is selected from the group consisting of 0, 1, and 2;
n is selected from the group consisting of 0, 1, 2, 3, 4, and 5;
s is selected from the group consisting of 0, 1, 2, 3, 4, and 5; and
r is selected from the group consisting of 0, 1, 2, 3, 4, and 5;
with the proviso that 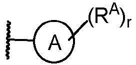 is not

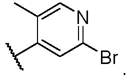

In some embodiments of the present disclosure, provided is the compound represented by general formula (I) or the pharmaceutically acceptable salt thereof, wherein R^{e} is selected from the group consisting of a hydrogen atom, halogen, hydroxy, cyano, and C₁₋₆ alkyl; preferably, R^{e} is a hydrogen atom.

In some embodiments of the present disclosure, provided is the compound represented by general formula (I) or the pharmaceutically acceptable salt thereof, wherein X¹ is N, X² is CR^{X2}, X³ is CR^{X3}, X⁴ is CR^{X4} , and X⁵ is CR^{X5}; alternatively, X² is N, X¹ is CR^{X1}, X³ is CR^{X3}, X⁴ is CR^{X4} , and X⁵ is CR^{X5}; alternatively, X³ is N, X¹ is CR^{X1}, X² is CR^{X2}, X⁴ is CR^{X4} , and X⁵ is CR^{X5}; alternatively, X¹ is N, X³ is N, X² is CR^{X2}, X⁴ is CR^{X4} , and X⁵ is CR^{X5}; alternatively, X² is N, X⁴ is N, X¹ is CR^{X1}, X³ is CR^{X3}, and X⁵ is CR^{X5}; alternatively, X¹ is CR^{X1}, X² is CR^{X2}, X³ is CR^{X3}, X⁴ is CR^{X4} , and X⁵ is CR^{X5}; R^{X1}, R^{X2}, R^{X3}, R^{X4}, and R^{X5} are as defined in general formula (I); preferably, X¹ is CR^{X1}, X² is CR^{X2}, X³ is CR^{X3}, X⁴ is CR^{X4}, and X⁵ is CR^{X5}; R^{X1}, R^{X2}, R^{X3}, R^{X4}, and R^{X5} are as defined in general formula (I); more preferably, X¹ is CR^{X1}, X² is CR^{X2}, X³ is CR^{X3}, X⁴ is CH, and X⁵ is CH; R^{X1}, R^{X2}, and R^{X3} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, C₁₋₆ hydroxyalkyl, 3- to 8-membered cycloalkyl, 4- to 7-membered heterocyclyl, 3- to 8-membered cycloalkyloxy, and 4- to 7-membered heterocyclyloxy; the 3- to 8-membered cycloalkyl, 4- to 7-membered heterocyclyl, 3- to 8-membered cycloalkyloxy, and 4- to 7-membered heterocyclyloxy are each independently and optionally substituted with one or more R⁰³; each R⁰³ is independently selected from the group consisting of halogen, hydroxy, cyano, oxo, amino, C₁₋₆ alkyl, C₁₋₆ haloalkyl, C₁₋₆ haloalkoxy, C₁₋₆ hydroxyalkyl, 3- to 10-membered cycloalkyl, 3- to 10-membered heterocyclyl, 6- to 10-membered aryl, and 5- to 14-membered heteroaryl; most preferably, X¹ is CR^{X1}, X² is CR^{X2}, X³ is CR^{X3}, X⁴ is CH, and X⁵ is CH; R^{X1}, R^{X2}, and R^{X3} are identical or different and are each independently selected from the group consisting of a hydrogen atom, halogen, C₁₋₆ alkyl, C₁₋₆ alkoxy, C₁₋₆ haloalkyl, and C₁₋₆ haloalkoxy.

In some embodiments of the present disclosure, provided is the compound represented by general formula (I) or the pharmaceutically acceptable salt thereof, wherein 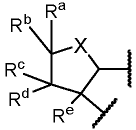 is selected from the group consisting of 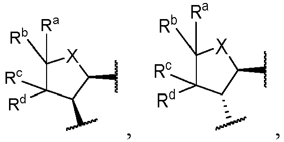 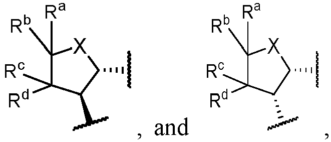 and R^{a}, R^{b}, R^{c}, R^{d}, and X are as defined in general formula (I); preferably, 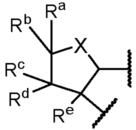 is selected from the group consisting of 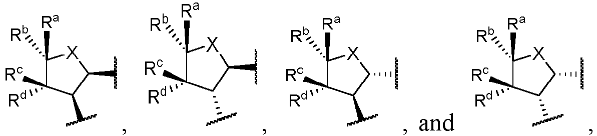 X is O or S, R^{a} and R^{b} are different and are each independently selected from the group consisting of a hydrogen atom, halogen, C₁₋₆ alkyl, C₁₋₆ alkoxy, C₁₋₆ haloalkyl, C₁₋₆ haloalkoxy, and 3- to 10-membered cycloalkyl, and R^{c} and R^{d} are different and are each independently selected from the group consisting of a hydrogen atom, halogen, C₁₋₆ alkyl, C₁₋₆ alkoxy, C₁₋₆ haloalkyl, C₁₋₆ haloalkoxy, and 3- to 10-membered cycloalkyl; more preferably, 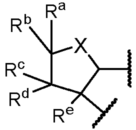 is 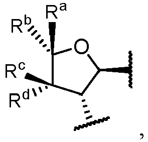 R^{a} and R^{b} are different and are each independently selected from the group consisting of a hydrogen atom, C₁₋₆ alkyl, and C₁₋₆ haloalkyl, and R^{c} and R^{d} are different and are each independently selected from the group consisting of a hydrogen atom, C₁₋₆ alkyl, and C₁₋₆ haloalkyl.

In some embodiments of the present disclosure, provided is the compound represented by general formula (I) or the pharmaceutically acceptable salt thereof, wherein 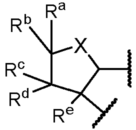 is 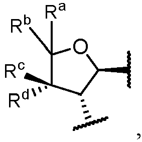 and R^{a} and R^{b} are identical or different and are each independently selected from the group consisting of a hydrogen atom, halogen, C₁₋₆ alkyl, C₁₋₆ haloalkyl, and 3- to 10-membered cycloalkyl; R^{c} and R^{d} are different and are each independently selected from the group consisting of a hydrogen atom, halogen, C₁₋₆ alkyl, C₁₋₆ haloalkyl, and 3- to 10-membered cycloalkyl.

In some embodiments, provided is the compound represented by general formula (I) or the pharmaceutically acceptable salt thereof, wherein 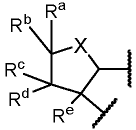 is 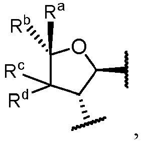 and R^{a} and R^{b} are different and are each independently selected from the group consisting of a hydrogen atom, halogen, C₁₋₆ alkyl, C₁₋₆ haloalkyl, and 3- to 10-membered cycloalkyl; R^{c} and R^{d} are identical or different and are each independently selected from the group consisting of a hydrogen atom, halogen, C₁₋₆ alkyl, C₁₋₆ haloalkyl, and 3- to 10-membered cycloalkyl.

In some embodiments of the present disclosure, the compound represented by general formula (I) or the pharmaceutically acceptable salt thereof is a compound represented by general formula (II) or a pharmaceutically acceptable salt thereof: 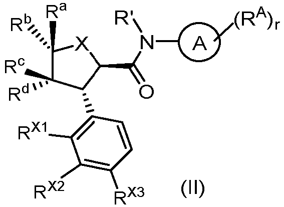 wherein:
R^{a} and R^{b} are different and are each independently selected from the group consisting of a hydrogen atom, halogen, hydroxy, cyano, amino, C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, 3- to 10-membered cycloalkyl, 3- to 10-membered heterocyclyl, 3- to 10-membered cycloalkyloxy, and 3- to 10-membered heterocyclyloxy; the 3- to 10-membered cycloalkyl, 3- to 10-membered heterocyclyl, 3- to 10-membered cycloalkyloxy, and 3- to 10-membered heterocyclyloxy are optionally substituted with one or more R¹²;
R^{c} and R^{d} are different and are each independently selected from the group consisting of a hydrogen atom, halogen, hydroxy, cyano, amino, C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, 3- to 10-membered cycloalkyl, 3- to 10-membered heterocyclyl, 3- to 10-membered cycloalkyloxy, and 3- to 10-membered heterocyclyloxy; the 3- to 10-membered cycloalkyl, 3- to 10-membered heterocyclyl, 3- to 10-membered cycloalkyloxy, and 3- to 10-membered heterocyclyloxy are optionally substituted with one or more R¹²;
ring A, R^{A}, R', X, R^{X1}, R^{X2}, R^{X3} R⁰², and r are as defined in general formula (I).

In some embodiments of the present disclosure, the compound represented by general formula (I) or general formula (II) or the pharmaceutically acceptable salt thereof is a compound represented by general formula (II') or a pharmaceutically acceptable salt thereof: 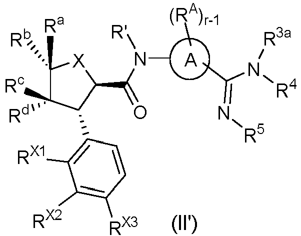
wherein R^{3a} is selected from the group consisting of a hydrogen atom, alkyl, haloalkyl, hydroxyalkyl, OR⁶, cycloalkyl, and heterocyclyl; the alkyl, cycloalkyl, and heterocyclyl are each independently and optionally substituted with one or more substituents selected from the group consisting of halogen, hydroxy, cyano, oxo, amino, alkyl, alkenyl, alkynyl, alkoxy, haloalkyl, haloalkoxy, hydroxyalkyl, cycloalkyl, and heterocyclyl;
r-1 is 0, 1, 2, 3, or 4;
ring A, R^{A}, R', X, R^{a}, R^{b}, R^{c}, R^{d}, R^{X1}, R^{X2}, R^{X3}, R⁴, R⁵, and R⁶ are as defined in general formula (II).

In some embodiments of the present disclosure, the compound represented by general formula (I) or general formula (II) or the pharmaceutically acceptable salt thereof is a compound represented by general formula (II-1) or a pharmaceutically acceptable salt thereof: 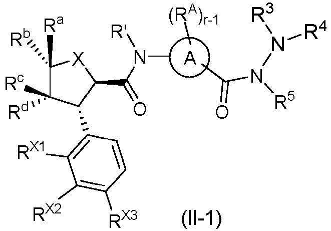
wherein r-1 is 0, 1, 2, 3, or 4;
ring A, R^{A}, R', X, R^{a}, R^{b}, R^{c}, R^{d}, R^{X1}, R^{X2}, R^{X3}, R³, R⁴, and R⁵ are as defined in general formula (II).

In some embodiments of the present disclosure, the compound represented by general formula (I) or general formula (II) or the pharmaceutically acceptable salt thereof is a compound represented by general formula (III) or a pharmaceutically acceptable salt thereof: 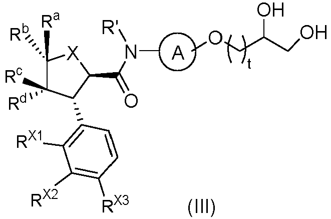
wherein t is selected from the group consisting of 0, 1, 2, 3, and 4;
ring A, R', R^{a}, R^{b}, R^{c}, R^{d}, X, R^{X1}, R^{X2}, and R^{X3} are as defined in general formula (II).

In some embodiments of the present disclosure, the compound represented by general formula (I), (II), or (III) or the pharmaceutically acceptable salt thereof is a compound represented by general formula (III-1) or a pharmaceutically acceptable salt thereof: 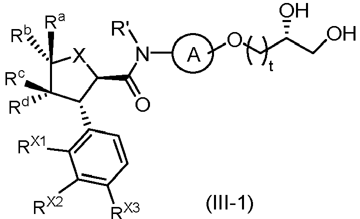
wherein t is selected from the group consisting of 0, 1, 2, 3, and 4;
ring A, R', R^{a}, R^{b}, R^{c}, R^{d}, X, R^{X1}, R^{X2}, and R^{X3} are as defined in general formula (II).

In some embodiments of the present disclosure, the compound represented by general formula (I) or (II) or the pharmaceutically acceptable salt thereof is a compound represented by general formula (IV) or a pharmaceutically acceptable salt thereof: 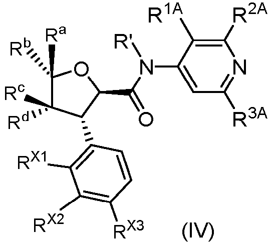
wherein R^{1A} and R^{2A} are identical or different and are each independently a hydrogen atom or R^{A};
R^{3A} is R^{A};
R^{a}, R^{b}, R^{c}, R^{d}, R^{X1}, R^{X2}, R^{X3}, R', and R^{A} are as defined in general formula (II).

In some embodiments of the present disclosure, the compound represented by general formula (I) or (II) or the pharmaceutically acceptable salt thereof is a compound represented by general formula (IV) or a pharmaceutically acceptable salt thereof: 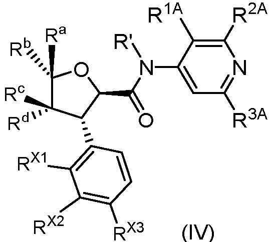
wherein R^{1A} and R^{2A} are identical or different and are each independently a hydrogen atom or R^{A}
R^{3A} is selected from the group consisting of F, Cl, hydroxy, cyano, alkyl, alkoxy, haloalkyl, haloalkoxy, hydroxyalkyl, hydroxyalkoxy, alkenyl, alkynyl, -NR³R⁴,-alkylene-NR³R⁴, -O-alkylene-NR³R⁴, -C(=NR⁵)R⁶, -S(O)ᵥNR³R⁴, -NR⁵S(O)ᵥR⁶,-S(O)ᵥR⁶, -S(=NR⁵)(O)R⁶, -NR⁵C(O)R⁶, -NR⁵C(O)NR³R⁴, -C(O)NR⁵-OR⁶, -P(O)R⁷R⁸,-C(O)NR⁵-NR³R⁴, -C(=NR⁵)NR⁵-OR⁶, -C(O)NR⁵-alkylene-Cy, -C(=NR⁵)NR³R⁴, -C(O)-C(O)-NR³R⁴, -S(=NR⁵)NR³R⁴, cycloalkyl, heterocyclyl, aryl, heteroaryl, -O-alkylene-heteroaryl, and -O-alkylene-heterocyclyl, wherein the alkenyl, alkynyl, alkylene, cycloalkyl, heterocyclyl, aryl, and heteroaryl are each independently and optionally substituted with one or more R⁰¹;
R^{a}, R^{b}, R^{c}, R^{d} , R^{X1}, R^{X2}, R^{X3}, R', R^{A}, R³, R⁴, R⁵*,* R⁶, R⁷, R⁸, R⁰¹, and v are as defined in general formula (II).

In some embodiments of the present disclosure, the compound represented by general formula (I) or (II) or the pharmaceutically acceptable salt thereof is a compound represented by general formula (IV) or a pharmaceutically acceptable salt thereof: 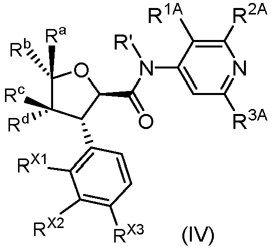
wherein R^{1A} and R^{2A} are identical or different and are each independently selected from the group consisting of a hydrogen atom, halogen, hydroxy, cyano, alkyl, alkoxy, haloalkyl, haloalkoxy, hydroxyalkyl, hydroxyalkoxy, alkenyl, -NR³R⁴, -alkylene-NR³R⁴, -O-alkylene-NR³R⁴, -C(=NR⁵)R⁶, -S(O)ᵥNR³R⁴, -NR⁵S(O)ᵥR⁶, -S(O)ᵥR⁶,-S(=NR⁵)(O)R⁶, -NR⁵C(O)R⁶, -NR⁵C(O)NR³R⁴, -P(O)R⁷R⁸, cycloalkyl, heterocyclyl, aryl, heteroaryl, -O-alkylene-heteroaryl, and -O-alkylene-heterocyclyl, wherein the alkenyl, alkylene, cycloalkyl, heterocyclyl, aryl, and heteroaryl are each independently and optionally substituted with one or more R⁰¹;
R^{3A} is selected from the group consisting of halogen, hydroxy, cyano, alkyl, alkoxy, haloalkyl, haloalkoxy, hydroxyalkyl, hydroxyalkoxy, alkenyl, -NR³R⁴, -alkylene-NR³R⁴, -O-alkylene-NR³R⁴, -C(=NR⁵)R⁶, -S(O)ᵥNR³R⁴, -NR⁵S(O)ᵥR⁶, -S(O)ᵥR⁶,-S(=NR⁵)(O)R⁶, -NR⁵C(O)R⁶, -NR⁵C(O)NR³R⁴, -C(O)NR⁵-OR⁶, -P(O)R⁷R⁸, -C(O)NR⁵-NR³R⁴, -C(=NR⁵)NR⁵-OR⁶, -C(O)NR⁵-alkylene-Cy, -C(=NR⁵)NR³R⁴, -C(O)-C(O)-NR³R⁴, cycloalkyl, heterocyclyl, aryl, heteroaryl, -O-alkylene-heteroaryl, and -O-alkylene-heterocyclyl, wherein the alkenyl, alkylene, cycloalkyl, heterocyclyl, aryl, and heteroaryl are each independently and optionally substituted with one or more R⁰¹;
R^{a}, R^{b}, R^{c}, R^{d}, R^{X1}, R^{X2}, R^{X3}, R', R³, R⁴, R⁵, R⁶, R⁷, R⁸, R⁰¹, and v are as defined in general formula (I); preferably, R^{a}, R^{b}, R^{c}, and R^{d} are as defined in general formula (II).

In some embodiments of the present disclosure, the compound represented by general formula (I) or (II) or the pharmaceutically acceptable salt thereof is a compound represented by general formula (IV) or a pharmaceutically acceptable salt thereof: 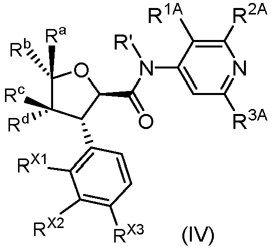
wherein R^{1A} and R^{2A} are identical or different and are each independently selected from the group consisting of a hydrogen atom, halogen, hydroxy, cyano, alkyl, alkoxy, haloalkyl, haloalkoxy, hydroxyalkyl, hydroxyalkoxy, alkenyl, -NR³R⁴, -alkylene-NR³R⁴, -O-alkylene-NR³R⁴, -C(=NR⁵)R⁶, -S(O)ᵥNR³R⁴, -NR⁵S(O)ᵥR⁶, -S(O)ᵥR⁶,-S(=NR⁵)(O)R⁶, -NR⁵C(O)R⁶, -NR⁵C(O)NR³R⁴, -P(O)R⁷R⁸, cycloalkyl, heterocyclyl, aryl, heteroaryl, -O-alkylene-heteroaryl, and -O-alkylene-heterocyclyl, wherein the alkenyl, alkylene, cycloalkyl, heterocyclyl, aryl, and heteroaryl are each independently and optionally substituted with one or more R⁰¹;
R^{3A} is selected from the group consisting of halogen, hydroxy, cyano, alkyl, alkoxy, haloalkyl, haloalkoxy, hydroxyalkyl, hydroxyalkoxy, alkenyl, -NR³R⁴, -alkylene-NR³R⁴, -O-alkylene-NR³R⁴, -C(=NR⁵)R⁶, -S(O)ᵥNR³R⁴, -NR⁵S(O)ᵥR⁶, -S(O)ᵥR⁶,-S(=NR⁵)(O)R⁶, -NR⁵C(O)R⁶, -NR⁵C(O)NR³R⁴, -C(O)NR⁵-OR⁶, -P(O)R⁷R⁸, cycloalkyl, heterocyclyl, aryl, heteroaryl, -O-alkylene-heteroaryl, and -O-alkylene-heterocyclyl, wherein the alkenyl, alkylene, cycloalkyl, heterocyclyl, aryl, and heteroaryl are each independently and optionally substituted with one or more R⁰¹;
R^{a}, R^{b}, R^{c}, R^{d}, R^{X1}, R^{X2}, R^{X3}, R', R³, R⁴, R⁵, R⁶, R⁷, R⁸, R⁰¹, and v are as defined in general formula (I); preferably, R^{a}, R^{b}, R^{c}, and R^{d} are as defined in general formula (II).

In some embodiments of the present disclosure, provided is the compound represented by general formula (IV) or the pharmaceutically acceptable salt thereof, wherein R^{3A} is selected from the group consisting of hydroxy, amino, C₁₋₆ alkyl, C₁₋₆ alkoxy, C₁₋₆ hydroxyalkyl, C₁₋₆ hydroxyalkoxy, -C₁₋₆ alkylene-NH₂, -O-C₁₋₆ alkylene-NH₂,-NR⁵C(O)NR³R⁴, -C(O)NR⁵-OR⁶, 3- to 8-membered cycloalkyl, 3- to 8-membered heterocyclyl, phenyl, 5- or 6-membered heteroaryl, and -O-C₁₋₆ alkylene-5- or 6-membered heteroaryl, and the C₁₋₆ alkylene, 3- to 8-membered cycloalkyl, 3- to 8-membered heterocyclyl, phenyl, and 5- or 6-membered heteroaryl are each independently and optionally substituted with one or more R⁰¹; each R⁰¹ is independently selected from the group consisting of halogen, hydroxy, cyano, oxo, amino, -NHC₁₋₆ alkyl, -N(C₁₋₆ alkyl)₂, C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, 3- to 10-membered cycloalkyl, 3- to 10-membered heterocyclyl, 6- to 10-membered aryl, and 5- to 14-membered heteroaryl; R³, R⁴, R⁵, and R⁶ are as defined in general formula (I).

In some embodiments, R^{3A} is selected from the group consisting of amino, C₁₋₆ hydroxyalkyl, C₁₋₆ hydroxyalkoxy, -C₁₋₆ alkylene-NH₂, -NR⁵C(O)NR³ R⁴, -C(O)NR⁵-OR⁶, 3- to 8-membered cycloalkyl, and 5- or 6-membered heteroaryl; the C₁₋₆ alkylene, 3- to 8-membered cycloalkyl, and 5- or 6-membered heteroaryl are each independently and optionally substituted with one or more R⁰¹; each R⁰¹ is independently selected from the group consisting of halogen, hydroxy, cyano, oxo, amino, -NHC₁₋₆ alkyl, -N(C₁₋₆ alkyl)₂, C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, 3- to 10-membered cycloalkyl, 3- to 10-membered heterocyclyl, 6- to 10-membered aryl, and 5- to 14-membered heteroaryl; R³, R⁴, R⁵, and R⁶ are as defined in general formula (I).

In some embodiments, R^{3A} is selected from the group consisting of amino, C₁₋₆ hydroxyalkyl, C₁₋₆ hydroxyalkoxy, -C₁₋₆ alkylene-NH₂, -NR⁵C(O)NR³ R⁴, -C(O)NR⁵-OR⁶, 3- to 6-membered cycloalkyl, and 5- or 6-membered heteroaryl; the C₁₋₆ alkylene, 3- to 6-membered cycloalkyl, and 5- or 6-membered heteroaryl are each independently and optionally substituted with one or more R⁰¹; each R⁰¹ is independently selected from the group consisting of hydroxy, cyano, amino, C₁₋₆ alkyl, and C₁₋₆ hydroxyalkyl; R³, R⁴, R⁵*,* and R⁶ are identical or different and are each independently a hydrogen atom or C₁₋₆ alkyl; in some embodiments, R^{3A} is selected from the group consisting of amino, C₁₋₆ hydroxyalkyl, 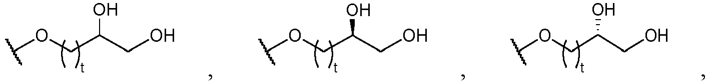 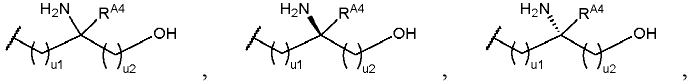 NR⁵C(O)NR³R⁴, 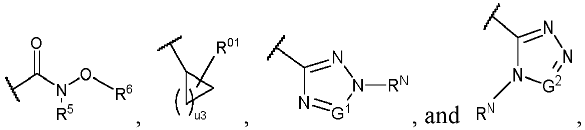 wherein G¹ is a nitrogen atom or CR^{G}; G² is a nitrogen atom or CR^{G}; R^{A4} is selected from the group consisting of a hydrogen atom, hydroxy, C₁₋₆ alkyl, and C₁₋₆ hydroxyalkyl; R³, R⁴, R⁵*,* and R⁶ are identical or different and are each independently a hydrogen atom or C₁₋₆ alkyl; R⁰¹ is selected from the group consisting of hydroxy, cyano, amino, and C₁₋₆ hydroxyalkyl; each R^{G} and R^{N} is identical or different and is independently a hydrogen atom or C₁₋₆ alkyl; t is selected from the group consisting of 0, 1, 2, and 3; u1 is selected from the group consisting of 0, 1, and 2; u2 is selected from the group consisting of 0, 1, 2, and 3; u3 is selected from the group consisting of 1, 2, and 3.

In some embodiments, R^{3A} is selected from the group consisting of amino, 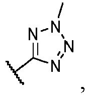 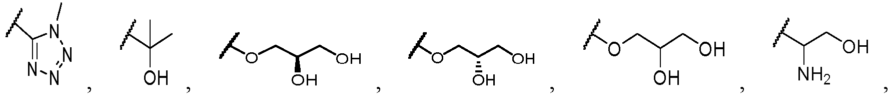 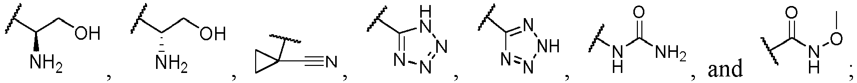 in some embodiments, R^{3A} is selected from the group consisting of 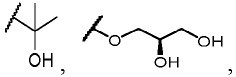 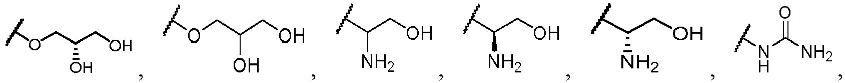 and

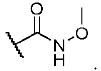

In an embodiment, R^{3A} is selected from the group consisting of amino,

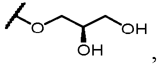

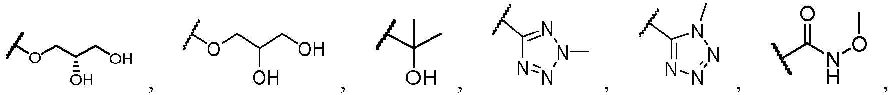

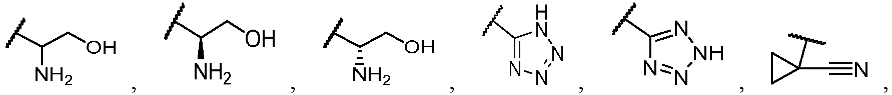

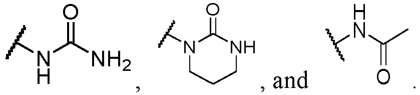

In some embodiments of the present disclosure, provided is the compound represented by general formula (IV) or the pharmaceutically acceptable salt thereof, wherein R^{3A} is selected from the group consisting of C₁₋₆ hydroxyalkyl, C₁₋₆ hydroxyalkoxy, -NR³R⁴, - C₁₋₆ alkylene-NR³R⁴, *-*NR⁵C(O)R⁶*,* -NR^{S}C(O)NR³R⁴*,* -C(O)NR⁵-OR⁶, 3- to 10-membered cycloalkyl, 3- to 10-membered heterocyclyl, and 5- or 6-membered heteroaryl; the C₁₋₆ alkylene, 3- to 10-membered cycloalkyl, 3- to 10-membered heterocyclyl, and 5- or 6-membered heteroaryl are each independently and optionally substituted with one or more R⁰¹; each R⁰¹ is independently selected from the group consisting of hydroxy, cyano, C₁₋₆ alkyl, and C₁₋₆ hydroxyalkyl; R³ and R⁴ are identical or different and are each independently a hydrogen atom or C₁₋₆ alkyl; R⁵ and R⁶ are identical or different and are each independently a hydrogen atom or C₁₋₆ alkyl.

In some embodiments, R^{3A} is selected from the group consisting of C₁₋₆ hydroxyalkyl, C₁₋₆ hydroxyalkoxy, -C₁₋₆ alkylene-NH₂, -C(O)NR⁵-OR⁶, 3- to 6-membered cycloalkyl, and 5- or 6-membered heteroaryl; the C₁₋₆ alkylene, 3- to 6-membered cycloalkyl, and 5- or 6-membered heteroaryl are each independently and optionally substituted with one or more R⁰¹; each R⁰¹ is independently selected from the group consisting of hydroxy, cyano, C₁₋₆ alkyl, and C₁₋₆ hydroxyalkyl; R⁵ and R⁶ are identical or different and are each independently a hydrogen atom or C₁₋₆ alkyl; in some embodiments, R^{3A} is -C₁₋₆ alkylene-NH₂ or -C(O)NR⁵-OR⁶; the C₁₋₆ alkylene is optionally substituted with one or more R⁰¹; each R⁰¹ is independently selected from the group consisting of hydroxy and C₁₋₆ hydroxyalkyl; R⁵ and R⁶ are identical or different and are each independently a hydrogen atom or C₁₋₆ alkyl.

In some embodiments of the present disclosure, the compound represented by general formula (I), (II), or (IV) or the pharmaceutically acceptable salt thereof is a compound represented by general formula (V) or a pharmaceutically acceptable salt thereof: 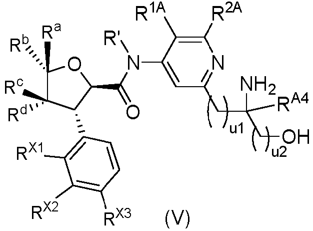 wherein
R^{1A} and R^{2A} are identical or different and are each independently selected from the group consisting of a hydrogen atom, halogen, hydroxy, cyano, alkyl, alkoxy, haloalkyl, haloalkoxy, hydroxyalkyl, hydroxyalkoxy, alkenyl, -NR³R⁴, -alkylene-NR³R⁴, -O-alkylene-NR³R⁴, -C(=NR⁵)R⁶, -S(O)ᵥNR³R⁴, -NR⁵S(O)ᵥR⁶, -S(O)ᵥR⁶, -S(=NR⁵)(O)R⁶, - P(O)R⁷R⁸, -NR⁵C(O)R⁶, -NR⁵C(O)NR³R⁴, cycloalkyl, heterocyclyl, aryl, heteroaryl, -O-alkylene-heteroaryl, and -O-alkylene-heterocyclyl, wherein the alkenyl, alkylene, cycloalkyl, heterocyclyl, aryl, and heteroaryl are each independently and optionally substituted with one or more R⁰¹;
R^{A4} is selected from the group consisting of a hydrogen atom, hydroxy, alkyl, and hydroxyalkyl; u1 is selected from the group consisting of 0, 1, 2, and 3; u2 is selected from the group consisting of 0, 1, 2, and 3;
R^{a}, R^{b}, R^{c}, R^{d}, R^{X1}, R^{X2}, R^{X3}, R', R³, R⁴, R⁵*,* R⁶, R⁷, R⁸, R⁰¹, and v are as defined in general formula (I); preferably, R^{a}, R^{b}, R^{c}, and R^{d} are as defined in general formula (II).

In some embodiments of the present disclosure, the compound represented by general formula (I), (II), (IV), or (V) or the pharmaceutically acceptable salt thereof is a compound represented by general formula (V-1) or (V-2) or a pharmaceutically acceptable salt thereof: 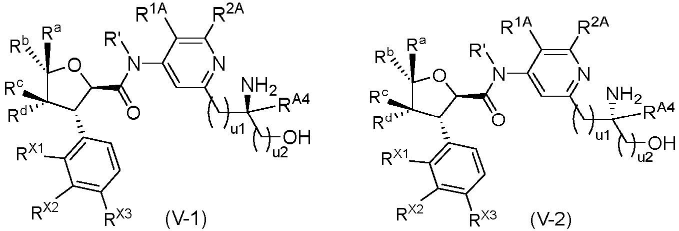 wherein R^{a}, R^{b}, R^{c}, R^{d}, R^{X1}, R^{X2}, R^{X3}, R', R^{1A}, R^{2A}, R^{A4}, u1, and u2 are as defined in general formula (V).

In some embodiments of the present disclosure, provided is the compound represented by general formula (V), (V-1), or (V-2) or the pharmaceutically acceptable salt thereof, wherein R^{A4} is selected from the group consisting of a hydrogen atom, C₁₋₆ alkyl, and C₁₋₆ hydroxyalkyl; preferably, R^{A4} is a hydrogen atom.

In some embodiments of the present disclosure, provided is the compound represented by general formula (V), (V-1), or (V-2) or the pharmaceutically acceptable salt thereof, wherein u1 is selected from the group consisting of 0, 1, and 2, and u2 is selected from the group consisting of 0, 1, and 2; preferably, u1 is 0 or 1, and u2 is 0 or 1; more preferably, u1 is 0, and u2 is 1.

In some embodiments of the present disclosure, provided is the compound represented by general formula (V), (V-1), or (V-2) or the pharmaceutically acceptable salt thereof, wherein R^{A4} is a hydrogen atom; u1 is selected from the group consisting of 0, 1, and 2, and u2 is selected from the group consisting of 0, 1, and 2.

In some embodiments of the present disclosure, the compound represented by general formula (I) or (II) or the pharmaceutically acceptable salt thereof is a compound represented by general formula (VI') or a pharmaceutically acceptable salt thereof: 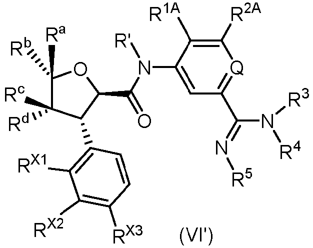
wherein Q is selected from the group consisting of CR^{5A}, N, and N⁺-O⁻;
R^{5A} is R^{1A};
R^{1A}, R^{2A}, R', R^{a}, R^{b}, R^{c}, R^{d}, R^{X1}, R^{X2}, R^{X3}, R³, R⁴, and R⁵ are as defined in general formula (IV).

In some embodiments of the present disclosure, the compound represented by general formula (I), (II), or (VI') or the pharmaceutically acceptable salt thereof is a compound represented by general formula (VI'-1) or a pharmaceutically acceptable salt thereof: 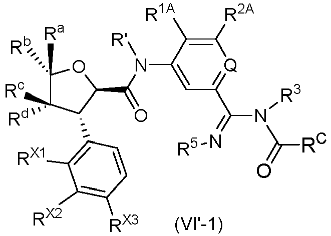 wherein
Q is selected from the group consisting of CR^{5A}, N, and N⁺-O⁻;
R^{5A} is R^{1A};
R^{C} is R²³ or OR²³;
R^{1A}, R^{2A}, R', R^{a}, R^{b}, R^{c}, R^{d}, R^{X1}, R^{X2}, R^{X3}, R³, R⁵*,* and R²³ are as defined in general formula (IV).

In some embodiments of the present disclosure, provided is the compound represented by general formula (VI'-1) or the pharmaceutically acceptable salt thereof, wherein R^{C} is selected from the group consisting of C₁₋₈ alkyl, C₁₋₈ alkoxy, and 3- to 10-membered cycloalkyloxy; in some embodiments, R^{C} is C₁₋₈ alkoxy; in some embodiments, R^{C} is isopropoxy or n-hexyloxy.

In some embodiments of the present disclosure, the compound represented by general formula (I), (II), (II'), (IV), or (VI') or the pharmaceutically acceptable salt thereof is a compound represented by general formula (VI) or a pharmaceutically acceptable salt thereof: 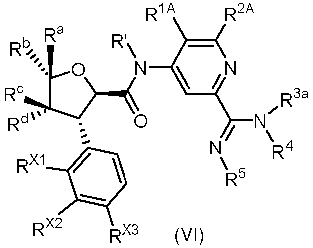
wherein R^{3a} is selected from the group consisting of a hydrogen atom, alkyl, haloalkyl, hydroxyalkyl, OR⁶, cycloalkyl, and heterocyclyl; the alkyl, cycloalkyl, and heterocyclyl are each independently and optionally substituted with one or more substituents selected from the group consisting of halogen, hydroxy, cyano, oxo, amino, alkyl, alkenyl, alkynyl, alkoxy, haloalkyl, haloalkoxy, hydroxyalkyl, cycloalkyl, and heterocyclyl;
R^{1A}, R^{2A}, R', R^{a}, R^{b}, R^{c}, R^{d}, R^{X1}, R^{X2}, R^{X3}, R⁴, R⁵*,* and R⁶ are as defined in general formula (IV).

In some embodiments of the present disclosure, provided is the compound represented by general formula (II') or (VI) or the pharmaceutically acceptable salt thereof, wherein R^{3a} is selected from the group consisting of a hydrogen atom, C₁₋₆ alkyl, C₁₋₆ haloalkyl, C₁₋₆ hydroxyalkyl, OR⁶, 3- to 6-membered cycloalkyl, and 3- to 6-membered cycloalkyl C₁₋₆ alkyl; R⁶ is selected from the group consisting of a hydrogen atom, C₁₋₆ alkyl, 3- to 6-membered cycloalkyl, and 3- to 6-membered cycloalkyl C₁₋₆ alkyl.

In some embodiments, R^{3a} is selected from the group consisting of a hydrogen atom, C₁₋₆ alkyl, C₁₋₆ alkoxy, and 3- to 6-membered cycloalkyl; in some embodiments, R^{3a} is selected from the group consisting of a hydrogen atom, C₁₋₆ alkyl, and C₁₋₆ alkoxy; in some embodiments, R^{3a} is selected from the group consisting of a hydrogen atom, C₁₋₆ alkyl, and 3- to 6-membered cycloalkyl; in some embodiments, R^{3a} is a hydrogen atom or C₁₋₆ alkyl.

In some embodiments, R^{3a} is selected from the group consisting of a hydrogen atom, methyl, ethyl, methoxy, and cyclopropyl; in some embodiments, R^{3a} is selected from the group consisting of a hydrogen atom, methyl, ethyl, and cyclopropyl; in some embodiments, R^{3a} is a hydrogen atom; in some embodiments, R^{3a} is selected from the group consisting of a hydrogen atom, methyl, ethyl, hydroxy, methoxy, deuterated methoxy, cyclopropyl,

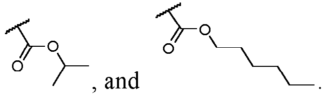

In some embodiments of the present disclosure, the compound represented by general formula (I), (II), or (II-1) or the pharmaceutically acceptable salt thereof is a compound represented by general formula (VII') or a pharmaceutically acceptable salt thereof: 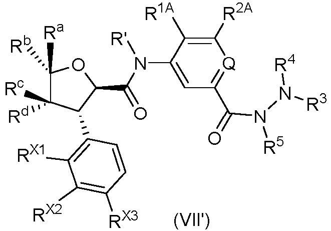
wherein Q is selected from the group consisting of CR^{5A}, N, and N⁺-O⁻;
R^{5A} is R^{1A};
R^{1A}, R^{2A}, R', R^{a}, R^{b}, R^{c}, R^{d}, R^{X1}, R^{X2}, R^{X3}, R³, R⁴, and R⁵ are as defined in general formula (IV).

In some embodiments of the present disclosure, the compound represented by general formula (I), (II), (II-1), (IV), or (VII') or the pharmaceutically acceptable salt thereof is a compound represented by general formula (VII) or a pharmaceutically acceptable salt thereof: 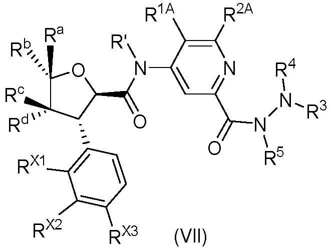 wherein R^{1A}, R^{2A}, R', R^{a}, R^{b}, R^{c}, R^{d}, R^{X1}, R^{X2}, R^{X3}, R³, R⁴, and R⁵ are as defined in general formula (IV).

In some embodiments of the present disclosure, the compound represented by general formula (I) or (II) or the pharmaceutically acceptable salt thereof is a compound represented by general formula (VIII) or a pharmaceutically acceptable salt thereof: 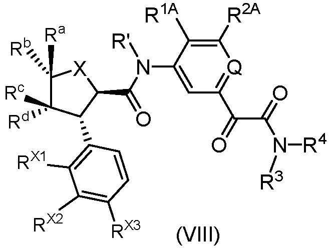
wherein Q is selected from the group consisting of CR^{5A}, N, and N⁺-O⁻;
R^{5A} is R^{1A};
R^{1A}, R^{2A}, R', X, R^{a}, R^{b}, R^{c}, R^{d}, R^{X1}, R^{X2}, R^{X3}, R³, and R⁴ are as defined in general formula (IV).

In some embodiments of the present disclosure, provided is the compound represented by general formula (VI'), (VI'-1), (VII'), or (VIII) or the pharmaceutically acceptable salt thereof, wherein Q is N or CR^{5A}, R^{5A} is R^{1A}, and R^{1A} is as defined in general formula (IV); in some embodiments, Q is N; in some embodiments, Q is CR^{5A}, R^{5A} is R^{1A}, and R^{1A} is as defined in general formula (IV); in some embodiments, Q is N or CF.

In some embodiments of the present disclosure, provided is the compound represented by general formula (VI'), (VI'-1), (VII'), or (VIII) or the pharmaceutically acceptable salt thereof, wherein R^{5A} is selected from the group consisting of a hydrogen atom, halogen, C₁₋₆ alkyl, C₁₋₆ alkoxy, C₁₋₆ haloalkyl, C₁₋₆ haloalkoxy, C₁₋₆ hydroxyalkyl, 3- to 6-membered cycloalkyl, and 3- to 6-membered heterocyclyl; in some embodiments, R^{5A} is selected from the group consisting of a hydrogen atom, halogen, C₁₋₆ alkyl, and C₁₋₆ haloalkyl; in some embodiments, R^{5A} is halogen; in some embodiments, R^{5A} is F.

In some embodiments of the present disclosure, provided is the compound represented by general formula (IV), (V), (V-1), (V-2), (VI), (VII), (VI'), (VI'-1), (VII'), or (VIII) or the pharmaceutically acceptable salt thereof, wherein R^{1A} and R^{2A} are identical or different and are each independently selected from the group consisting of a hydrogen atom, halogen, hydroxy, amino, C₁₋₆ alkyl, C₁₋₆ alkoxy, C₁₋₆ haloalkyl, C₁₋₆ haloalkoxy, C₁₋₆ hydroxyalkyl, and C₁₋₆ hydroxyalkoxy; preferably, R^{1A} and R^{2A} are identical or different and are each independently selected from the group consisting of a hydrogen atom, halogen, C₁₋₆ alkyl, and C₁₋₆ hydroxyalkyl; more preferably, R^{1A} and R^{2A} are both hydrogen atoms.

In some embodiments of the present disclosure, provided is the compound represented by general formula (I), (II), (II'), (II-1), (III), (III-1), (IV), (V), (V-1), (V-2), (VI), (VII), (VI'), (VI'-1), (VII'), or (VIII) or the pharmaceutically acceptable salt thereof, wherein
R^{a} is selected from the group consisting of a hydrogen atom, halogen, C₁₋₆ alkyl, C₁₋₆ alkoxy, C₁₋₆ haloalkyl, C₁₋₆ haloalkoxy, C₁₋₆ hydroxyalkyl, 3- to 10-membered cycloalkyl, and 3- to 10-membered heterocyclyl; in some embodiments, R^{a} is selected from the group consisting of a hydrogen atom, halogen, C₁₋₆ alkyl, and C₁₋₆ haloalkyl; in some embodiments, R^{a} is C₁₋₆ alkyl or C₁₋₆ haloalkyl; in some embodiments, R^{a} is CH₃ or CF₃. In some embodiments, R^{a} is C₁₋₆ alkyl; in some embodiments, R^{a} is selected from the group consisting of CH₃, deuterated methyl, and CF₃; in some embodiments, R^{a} is CH₃ or deuterated methyl; in some embodiments, R^{a} is CH₃.

In some embodiments of the present disclosure, provided is the compound represented by general formula (I), (II), (II'), (II-1), (III), (III-1), (IV), (V), (V-1), (V-2), (VI), (VII), (VI'), (VI'-1), (VII'), or (VIII) or the pharmaceutically acceptable salt thereof, wherein R^{b} is selected from the group consisting of a hydrogen atom, halogen, C₁₋₆ alkyl, C₁₋₆ alkoxy, C₁₋₆ haloalkyl, C₁₋₆ haloalkoxy, C₁₋₆ hydroxyalkyl, 3- to 10-membered cycloalkyl, and 3-to 10-membered heterocyclyl; in some embodiments, R^{b} is selected from the group consisting of a hydrogen atom, halogen, C₁₋₆ alkyl, and C₁₋₆ haloalkyl; in some embodiments, R^{b} is C₁₋₆ alkyl or C₁₋₆ haloalkyl; in some embodiments, R^{b} is selected from the group consisting of CH₃, deuterated methyl, and CF₃; in some embodiments, R^{b} is CH₃ or CF₃; in some embodiments, R^{b} is C₁₋₆ haloalkyl; in some embodiments, R^{b} is CF₃. In some embodiments of the present disclosure, provided is the compound represented by general formula (I), (II), (II'), (II-1), (III), (III-1), (IV), (V), (V-1), (V-2), (VI), (VII), (VI'), (VI'-1), (VII'), or (VIII) or the pharmaceutically acceptable salt thereof, wherein R^{c} is selected from the group consisting of a hydrogen atom, halogen, C₁₋₆ alkyl, C₁₋₆ alkoxy, C₁₋₆ haloalkyl, C₁₋₆ haloalkoxy, C₁₋₆ hydroxyalkyl, 3- to 10-membered cycloalkyl, and 3-to 10-membered heterocyclyl; preferably, R^{c} is selected from the group consisting of a hydrogen atom, halogen, C₁₋₆ alkyl, and C₁₋₆ haloalkyl; more preferably, R^{c} is a hydrogen atom or C₁₋₆ alkyl; most preferably, R^{c} is a hydrogen atom or CH₃; in some embodiments, R^{c} is selected from the group consisting of a hydrogen atom, CH₃, deuterated methyl, and CF₃.

In some embodiments of the present disclosure, provided is the compound represented by general formula (I), (II), (II'), (II-1), (III), (III-1), (IV), (V), (V-1), (V-2), (VI), (VII), (VI'), (VI'-1), (VII'), or (VIII) or the pharmaceutically acceptable salt thereof, wherein R^{d} is selected from the group consisting of a hydrogen atom, halogen, C₁₋₆ alkyl, C₁₋₆ alkoxy, C₁₋₆ haloalkyl, C₁₋₆ haloalkoxy, C₁₋₆ hydroxyalkyl, 3- to 10-membered cycloalkyl, and 3-to 10-membered heterocyclyl; preferably, R^{d} is selected from the group consisting of a hydrogen atom, halogen, C₁₋₆ alkyl, and C₁₋₆ haloalkyl; more preferably, R^{d} is a hydrogen atom or C₁₋₆ alkyl; more preferably, R^{d} is a hydrogen atom or CH₃; in some embodiments, R^{d} is selected from the group consisting of a hydrogen atom, CH₃, deuterated methyl, and CF₃; in some embodiments, R^{d} is selected from the group consisting of a hydrogen atom, CH₃, and CD₃.

In some embodiments of the present disclosure, provided is the compound represented by general formula (I) or the pharmaceutically acceptable salt thereof, wherein R^{a} and R^{b} are identical or different and are each independently selected from the group consisting of a hydrogen atom, C₁₋₆ alkyl, and C₁₋₆ haloalkyl; in some embodiments, R^{a} and R^{b} are identical or different and are each independently C₁₋₆ alkyl or C₁₋₆ haloalkyl; in some embodiments, R^{a} is C₁₋₆ alkyl, and R^{b} is C₁₋₆ haloalkyl; in some embodiments, R^{a} is CH₃, and R^{b} is CF₃.

In some embodiments, R^{a} and R^{b} are identical or different and are each independently selected from the group consisting of a hydrogen atom, CH₃, deuterated methyl, and CF₃; in some embodiments, R^{a} and R^{b} are identical or different and are each independently selected from the group consisting of a hydrogen atom, CH₃, and CF₃.

In some embodiments, R^{a} and R^{b} are identical or different and are each independently C₁₋₆ alkyl; in some embodiments, R^{a} and R^{b} are identical or different and are each independently C₁₋₆ haloalkyl.

In some embodiments of the present disclosure, provided is the compound represented by general formula (II), (II'), (II-1), (III), (III-1), (IV), (V), (V-1), (V-2), (VI), (VII), (VII), (VI'), (VI'-1), (VII'), or (VIII) or the pharmaceutically acceptable salt thereof, wherein R^{a} and R^{b} are different and are each independently selected from the group consisting of a hydrogen atom, C₁₋₆ alkyl, and C₁₋₆ haloalkyl; preferably, R^{a} and R^{b} are different and are each independently C₁₋₆ alkyl or C₁₋₆ haloalkyl; more preferably, R^{a} is C₁₋₆ alkyl, and R^{b} is C₁₋₆ haloalkyl; most preferably, R^{a} is CH₃, and R^{b} is CF₃.

In some embodiments, R^{a} and R^{b} are different and are each independently selected from the group consisting of a hydrogen atom, CH₃, deuterated methyl, and CF₃; in some embodiments, R^{a} and R^{b} are different and are each independently selected from the group consisting of a hydrogen atom, CH₃, and CF₃.

In some embodiments, R^{a} and R^{b} are different and are each independently C₁₋₆ alkyl; in some embodiments, R^{a} and R^{b} are different and are each independently C₁₋₆ haloalkyl.

In some embodiments of the present disclosure, provided is the compound represented by general formula (I), (II), (II'), (II-1), (III), (III-1), (IV), (V), (V-1), (V-2), (VI), (VII), (VI'), (VI'-1), (VII'), or (VIII) or the pharmaceutically acceptable salt thereof, wherein R^{a} is C₁₋₆ haloalkyl, and R^{b} is a hydrogen atom; in some embodiments, R^{a} is C₁₋₆ haloalkyl, and R^{b} is C₁₋₆ alkyl; in some embodiments, R^{a} is CF₃, and R^{b} is CH₃.

In some embodiments, R^{a} is a hydrogen atom, and R^{b} is C₁₋₆ haloalkyl; in some embodiments, R^{a} is a hydrogen atom, and R^{b} is C₁₋₆ alkyl; in some embodiments, R^{a} is C₁₋₆ alkyl, and R^{b} is a hydrogen atom.

In some embodiments of the present disclosure, provided is the compound represented by general formula (I) or the pharmaceutically acceptable salt thereof, wherein R^{c} and R^{d} are identical or different and are each independently selected from the group consisting of a hydrogen atom, C₁₋₆ alkyl, and 3- to 6-membered cycloalkyl; in some embodiments, R^{c} and R^{d} are identical or different and are each independently a hydrogen atom or C₁₋₆ alkyl; in some embodiments, R^{c} is a hydrogen atom, and R^{d} is C₁₋₆ alkyl; in some embodiments, R^{c} is a hydrogen atom, and R^{d} is CH₃.

In some embodiments, R^{c} and R^{d} are identical or different and are each independently selected from the group consisting of a hydrogen atom, CH₃, and deuterated methyl; in some embodiments, R^{c} and R^{d} are identical or different and are each independently a hydrogen atom or CH₃; in some embodiments, R^{c} and R^{d} are both hydrogen atoms.

In some embodiments of the present disclosure, provided is the compound represented by general formula (II), (II'), (II-1), (III), (III-1), (IV), (V), (V-1), (V-2), (VI), (VII), (VI'), (VI'-1), (VII'), or (VIII) or the pharmaceutically acceptable salt thereof, wherein R^{c} and R^{d} are different and are each independently selected from the group consisting of a hydrogen atom, C₁₋₆ alkyl, and 3- to 6-membered cycloalkyl; in some embodiments, R^{c} and R^{d} are different and are each independently a hydrogen atom or C₁₋₆ alkyl; in some embodiments, R^{c} is a hydrogen atom, and R^{d} is C₁₋₆ alkyl; in some embodiments, R^{c} is a hydrogen atom, and R^{d} is CH₃.

In some embodiments, R^{c} and R^{d} are different and are each independently selected from the group consisting of a hydrogen atom, CH₃, and deuterated methyl; in some embodiments, R^{c} and R^{d} are different and are each independently a hydrogen atom or CH₃.

In some embodiments of the present disclosure, provided is the compound represented by general formula (I), (II), (II'), (II-1), (III), (III-1), (IV), (V), (V-1), (V-2), (VI), (VII), (VI'), (VI'-1), (VII'), or (VIII) or the pharmaceutically acceptable salt thereof, wherein R^{c} is C₁₋₆ alkyl, and R^{d} is a hydrogen atom; in some embodiments, R^{c} is CH₃, and R^{d} is a hydrogen atom; in some embodiments, R^{c} is 3- to 10-membered cycloalkyl (preferably cyclopropyl), and R^{d} is a hydrogen atom; in some embodiments, R^{c} is a hydrogen atom, and R^{d} is 3- to 10-membered cycloalkyl (preferably cyclopropyl).

In some embodiments of the present disclosure, provided is the compound represented by general formula (I), (II), (II'), (II-1), (III), (III-1), (IV), (V), (V-1), (V-2), (VI), (VII), (VI'), (VI'-1), (VII'), or (VIII) or the pharmaceutically acceptable salt thereof, wherein R^{a} is C₁₋₆ alkyl, and/or R^{b} is C₁₋₆ haloalkyl, and/or R^{c} is a hydrogen atom, and/or R^{d} is C₁₋₆ alkyl; in some embodiments, R^{a} is CH₃, and/or R^{b} is CF₃, and/or R^{c} is a hydrogen atom, and/or R^{d} is CH₃.

In some embodiments of the present disclosure, provided is the compound represented by general formula (I), (II), (II'), (II-1), (III), (III-1), or (VIII) or the pharmaceutically acceptable salt thereof, wherein X is O.

In some embodiments of the present disclosure, provided is the compound represented by general formula (I), (II), (II'), (II-1), (III), (III-1), (IV), (V), (V-1), (V-2), (VI), (VII), (VI'), (VI'-1), (VII'), or (VIII) or the pharmaceutically acceptable salt thereof, wherein R' is a hydrogen atom or C₁₋₆ alkyl; preferably, R' is a hydrogen atom.

In some embodiments of the present disclosure, provided is the compound represented by general formula (I), (II), (II'), (II-1), (III), (III-1), (IV), (V), (V-1), (V-2), (VI), (VII), (VI'), (VI'-1), (VII'), or (VIII) or the pharmaceutically acceptable salt thereof, wherein X is O, and/or R' is a hydrogen atom.

In some embodiments of the present disclosure, provided is the compound represented by general formula (I), (II), (II'), (II-1), (III), or (III-1) or the pharmaceutically acceptable salt thereof, wherein ring A is 6-membered heteroaryl; preferably, ring A is selected from the group consisting of pyridinyl, pyrimidinyl, pyrazinyl, pyridazinyl, and triazinyl; more preferably, ring A is pyridinyl or pyrimidinyl; yet more preferably, ring A is 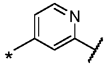 or 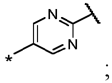 most preferably, ring A is 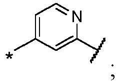 wherein the * end is attached to - NR', and the 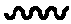 end is attached to R^{A}.

In some embodiments, ring A is selected from the group consisting of phenyl, pyridinyl, and pyrimidinyl; in some embodiments, ring A is selected from the group consisting of 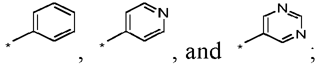 in some embodiments, ring A is selected from the group consisting of 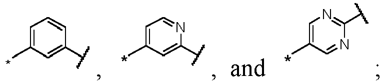 in some embodiments, ring A is selected from the group consisting of 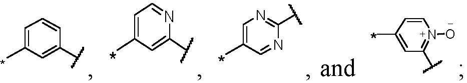 in some embodiments, ring A is selected from the group consisting of 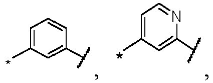 and 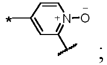 in some embodiments, ring A is 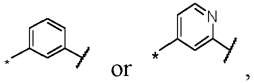 in some embodiments, ring A is 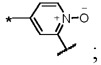 wherein the * end is attached to -NR', and the 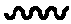 end is attached to R^{A}; in some embodiments, ring A is selected from the group consisting of 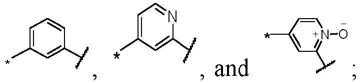 the * end is attached to -NR', and the 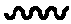 end is attached to one R^{A}.

In some embodiments of the present disclosure, provided is the compound represented by general formula (I) or (II) or the pharmaceutically acceptable salt thereof, wherein 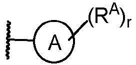 is selected from the group consisting of 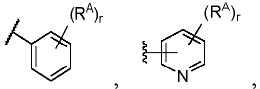 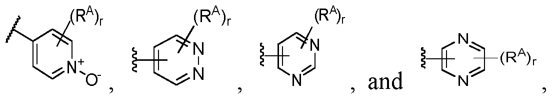 and R^{A} and r are as defined in general formula (I); preferably, 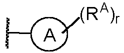 is selected from the group consisting of 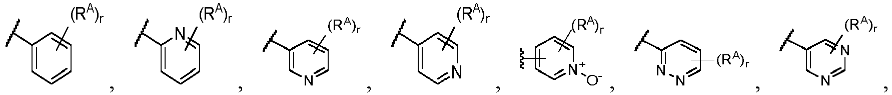 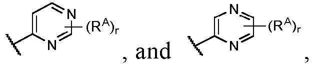 and R^{A} and r are as defined in general formula (I); more preferably, 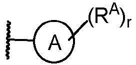 is selected from the group consisting of 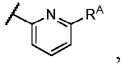 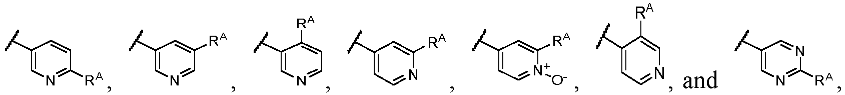 and R^{A} is as defined in general formula (I); yet more preferably, 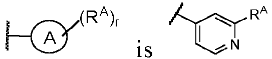 or 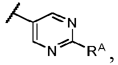 and R^{A} is as defined in general formula (I); most preferably, 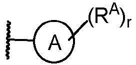 is 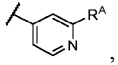 and R^{A} is as defined in general formula (I).

In some embodiments of the present disclosure, provided is the compound represented by general formula (I), (II), (II'), (II-1), or (IV) or the pharmaceutically acceptable salt thereof, wherein R^{A} is selected from the group consisting of C₁₋₆ hydroxyalkyl, C₁₋₆ hydroxyalkoxy, -NR³R⁴, -C₁₋₆ alkylene-NR³R⁴, -NR⁵C(O)R⁶, -NR⁵C(O)NR³R⁴, - C(O)NR⁵-OR⁶, 3- to 10-membered cycloalkyl, 3- to 10-membered heterocyclyl, and 5- or 6-membered heteroaryl; the C₁₋₆ alkylene, 3- to 10-membered cycloalkyl, 3- to 10-membered heterocyclyl, and 5- or 6-membered heteroaryl are each independently and optionally substituted with one or more R⁰¹; each R⁰¹ is independently selected from the group consisting of halogen, hydroxy, cyano, oxo, amino, -NHC₁₋₆ alkyl, -N(C₁₋₆ alkyl)₂, C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, 3- to 10-membered cycloalkyl, 3- to 10-membered heterocyclyl, 6- to 10-membered aryl, and 5- to 14-membered heteroaryl; R³, R⁴, R⁵*,* and R⁶ are as defined in general formula (I); in some embodiments, R^{A} is selected from the group consisting of amino,

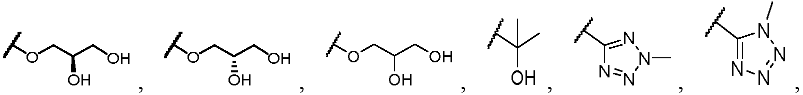

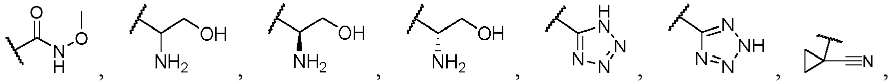

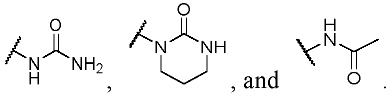

In some embodiments, R^{A} is selected from the group consisting of F, Cl, amino, cyano,

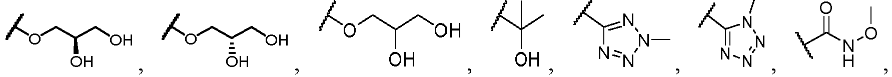

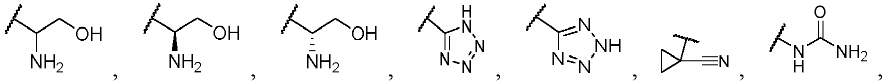

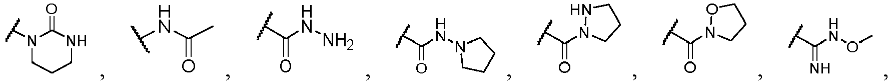

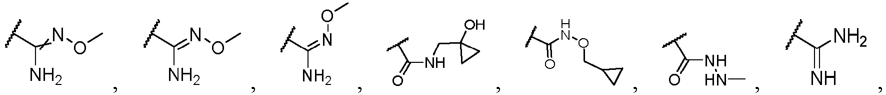

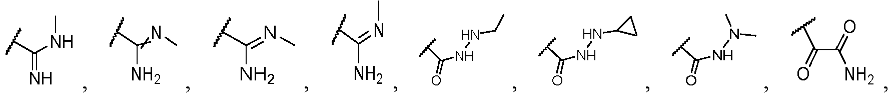

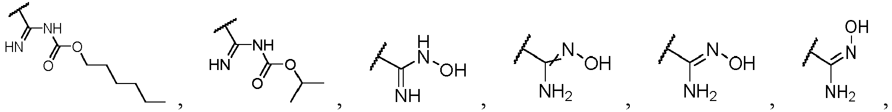

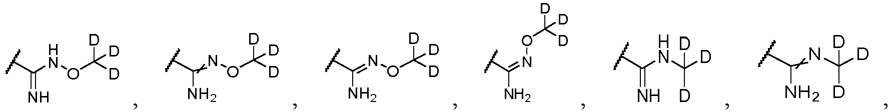

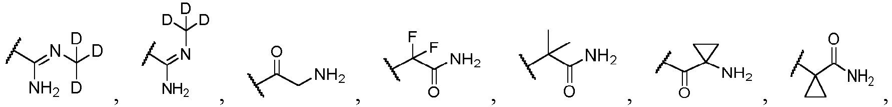

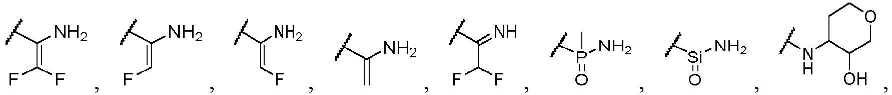

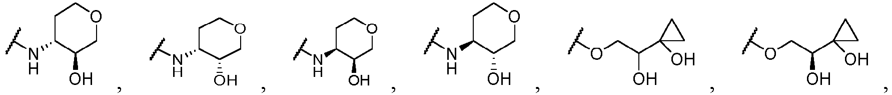

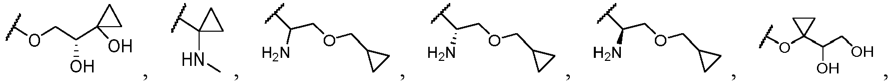

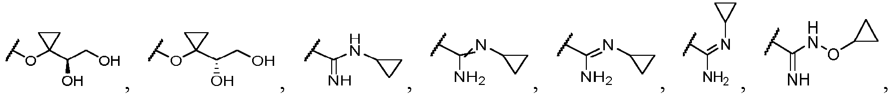

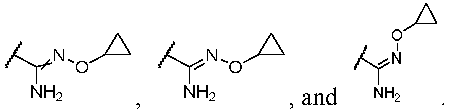

In some embodiments, R^{A} is selected from the group consisting of F, Cl, amino, cyano,

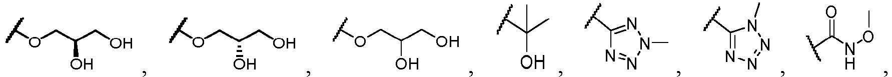

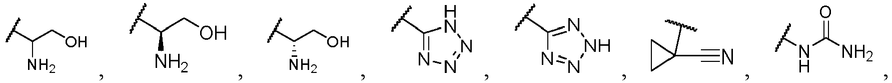

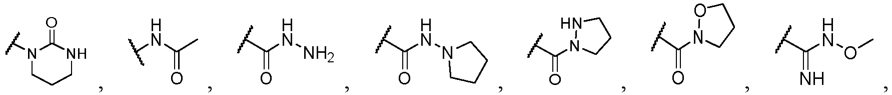

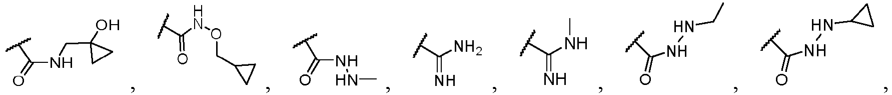

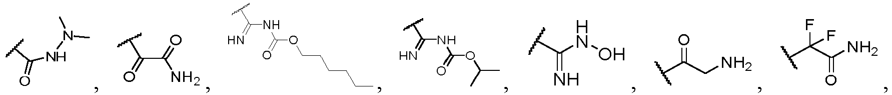

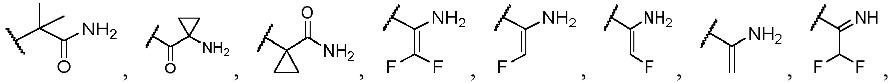

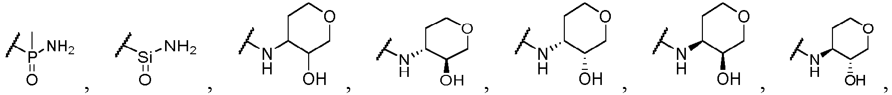

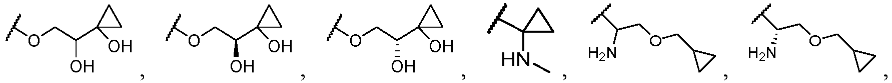

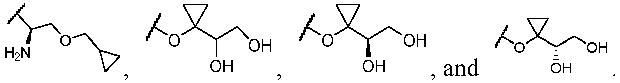

In some embodiments, R^{A} is selected from the group consisting of F, Cl, amino,

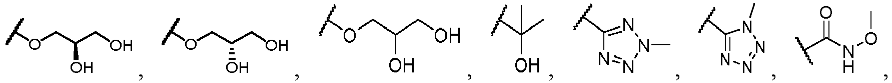

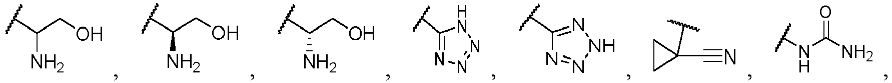

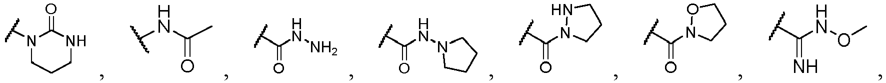

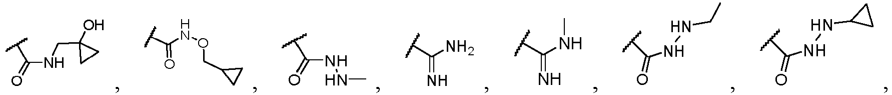

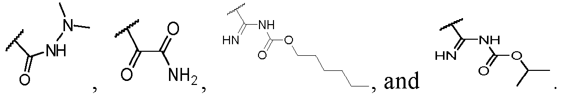

In some embodiments, R^{A} is selected from the group consisting of amino, 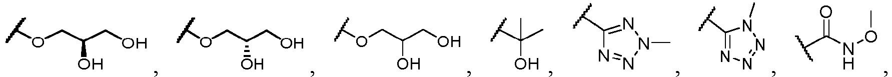 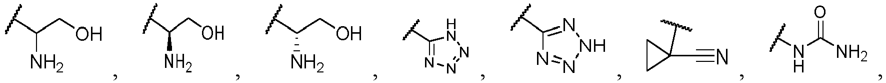 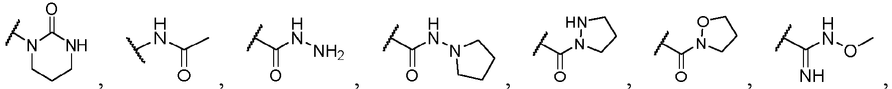 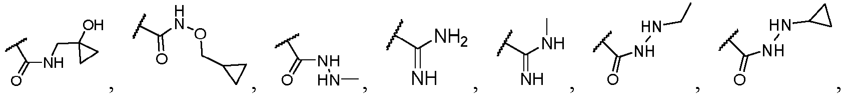 and

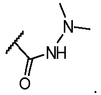

In some embodiments, R^{A} is selected from the group consisting of amino, 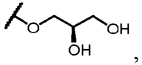 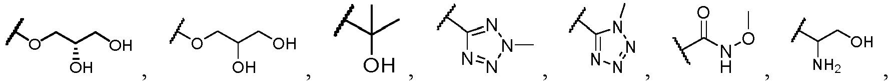 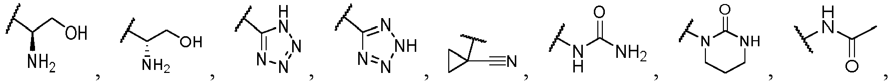 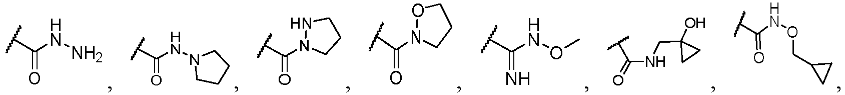 and

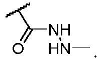

In some embodiments, R^{A} is selected from the group consisting of

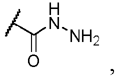

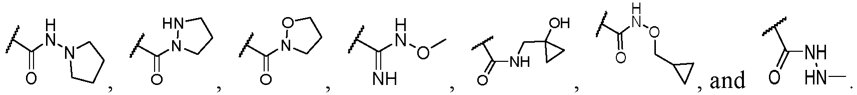

In some embodiments, R^{A} is selected from the group consisting of

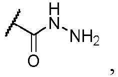

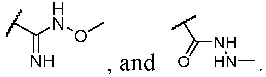

In some embodiments, R^{A} is selected from the group consisting of 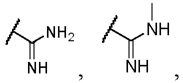 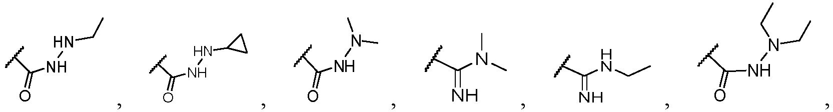 and 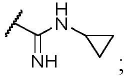 in some embodiments, R^{A} is selected from the group consisting of

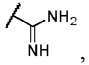

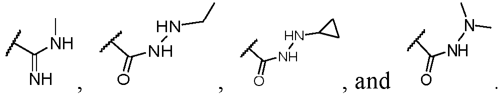

In some embodiments, R^{A} is selected from the group consisting of

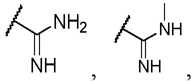

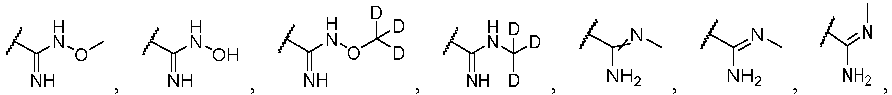

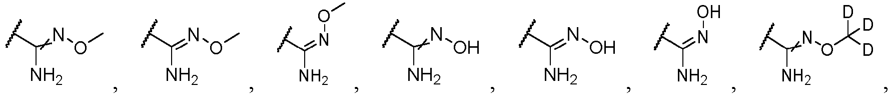

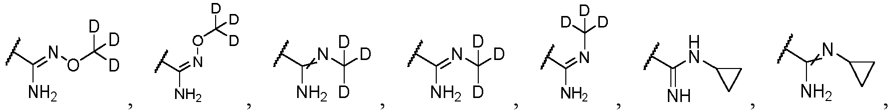

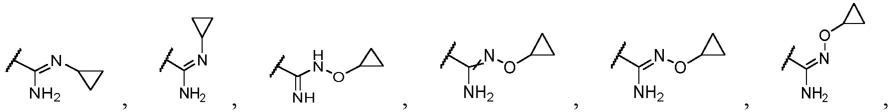

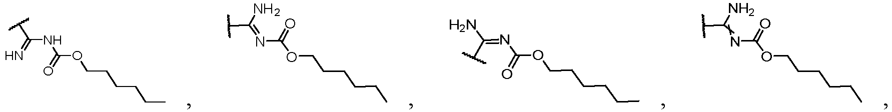

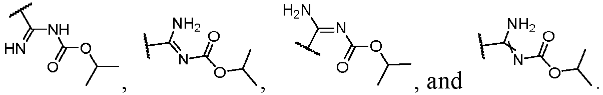

In some embodiments, R^{A} is selected from the group consisting of

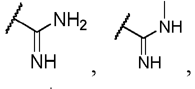

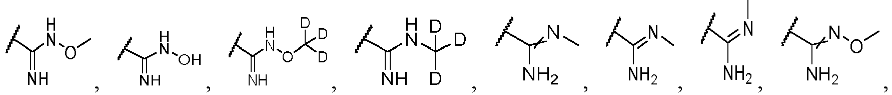

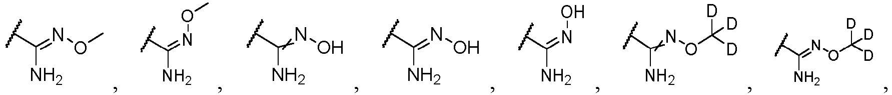

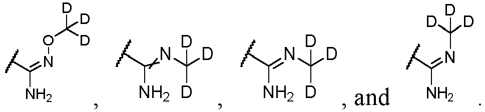

In some embodiments, R^{A} is selected from the group consisting of 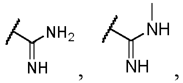 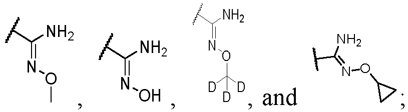 in some embodiments, R^{A} is selected from the group consisting of 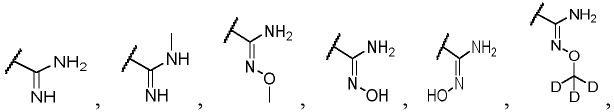 and 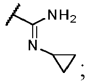 in some embodiments, R^{A} is 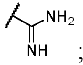 in some embodiments, R^{A} is 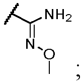 in some embodiments, R^{A} is 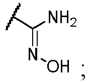 in some embodiments, R^{A} is 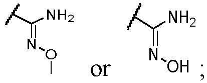 in some embodiments, R^{A} is

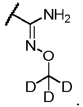

In some embodiments, R^{A} is selected from the group consisting of 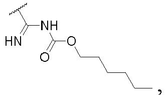 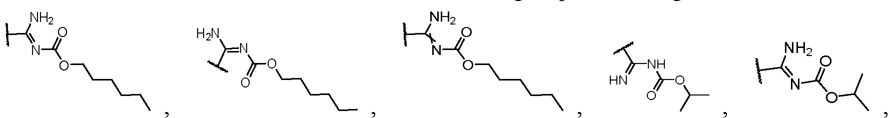 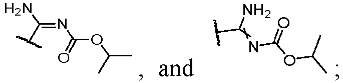 in some embodiments, R^{A} is 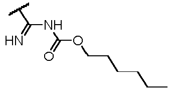 or 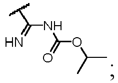 in some embodiments, R^{A} is 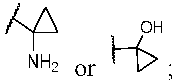 in some embodiments, R^{A} is 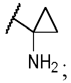 in some embodiments, R^{A} is

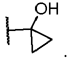

In some embodiments, R^{A} is selected from the group consisting of -C(O)NR⁵-OR⁶, - C(O)NR⁵-NR³R⁴, -C(=NR⁵)NR⁵-OR⁶, -C(O)NR⁵-alkylene-Cy, -C(=NR⁵)NR³R⁴, and - C(O)-C(O)-NR³R⁴; the alkylene is optionally substituted with one or more R⁰¹; Cy, R³, R⁴, R⁵*,* R⁶, and R⁰¹ are as defined in general formula (I); in some embodiments, R^{A} is selected from the group consisting of -C(O)NR⁵-NR³R⁴, -C(=NR⁵)NR⁵-OR⁶, -C(O)NR⁵-alkylene-Cy, -C(O)NR⁵-O-alkylene-cycloalkyl, -C(=NR⁵)NR³R⁴, and -C(O)-C(O)-NR³R⁴; the alkylene and cycloalkyl are optionally substituted with one or more R⁰¹; Cy, R³, R⁴, R⁵*,* R⁶, and R⁰¹ are as defined in general formula (I); in some embodiments, R^{A} is -C(O)NR⁵-NR³R⁴ or -C(=NR⁵)NR³-OR⁶, and R³, R⁴, R⁵*,* and R⁶ are as defined in general formula (I); in some embodiments, R^{A} is -C(O)NR⁵-NR³R⁴, and R³, R⁴, and R⁵ are as defined in general formula (I); in some embodiments, R^{A} is -C(=NR⁵)NR³-OR⁶, and R³, R⁵*,* and R⁶ are as defined in general formula (I); in some embodiments, R^{A} is - C(=NR⁵)NR³R⁴, and R³, R⁴, and R⁵ are as defined in general formula (I); in some embodiments, R^{A} is -C(=NR⁵)NR³R⁴, and R³, R⁴, and R⁵ are identical or different and are each independently selected from the group consisting of a hydrogen atom, C₁₋₆ alkyl, and 3- to 6-membered cycloalkyl.

In some embodiments, R^{A} is -C(O)-C(O)-NR³R⁴, and R³ and R⁴ are as defined in general formula (I); in some embodiments, R^{A} is -C(O)-C(O)-NR³R⁴, and R³ and R⁴ are identical or different and are each independently a hydrogen atom or C₁₋₆ alkyl.

In some embodiments, R^{A} is -C(O)NR⁵-NR³R⁴ or -C(=NR⁵)NR³-OR⁶, and R³, R⁴, R⁵*,* and R⁶ are identical or different and are each independently a hydrogen atom or C₁₋₆ alkyl.

In some embodiments of the present disclosure, provided is the compound represented by general formula (I), (II), (II'), or (II-1) or the pharmaceutically acceptable salt thereof, wherein R^{A} is selected from the group consisting of amino, C₁₋₆ hydroxyalkyl, C₁₋₆ hydroxyalkoxy, -C₁₋₆ alkylene-NH₂, -NR⁵C(O)NR³R⁴, -C(O)NR⁵-OR⁶, 3- to 8-membered cycloalkyl, and 5- or 6-membered heteroaryl; the C₁₋₆ alkylene, 3- to 8-membered cycloalkyl, and 5- or 6-membered heteroaryl are each independently and optionally substituted with one or more R⁰¹; each R⁰¹ is independently selected from the group consisting of halogen, hydroxy, cyano, oxo, amino, -NHC₁₋₆ alkyl, -N(C₁₋₆ alkyl)₂, C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, 3- to 10-membered cycloalkyl, 3- to 10-membered heterocyclyl, 6- to 10-membered aryl, and 5- to 14-membered heteroaryl; R³, R⁴, R⁵*,* and R⁶ are as defined in general formula (I).

In some embodiments, R^{A} is selected from the group consisting of amino, C₁₋₆ hydroxyalkyl, C₁₋₆ hydroxyalkoxy, -C₁₋₆ alkylene-NH₂, -NR⁵C(O)NR³R⁴, -C(O)NR⁵-OR⁶, 3- to 6-membered cycloalkyl, and 5- or 6-membered heteroaryl; the C₁₋₆ alkylene, 3- to 6-membered cycloalkyl, and 5- or 6-membered heteroaryl are each independently and optionally substituted with one or more R⁰¹; each R⁰¹ is independently selected from the group consisting of hydroxy, cyano, amino, C₁₋₆ alkyl, and C₁₋₆ hydroxyalkyl; R³, R⁴, R⁵*,* and R⁶ are identical or different and are each independently a hydrogen atom or C₁₋₆ alkyl.

In some embodiments, R^{A} is selected from the group consisting of amino, C₁₋₆ hydroxyalkyl, 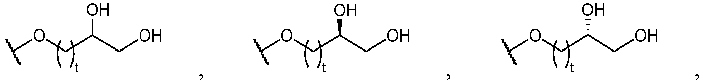 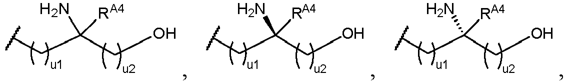 NR⁵C(O)NR³R⁴, 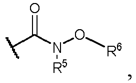 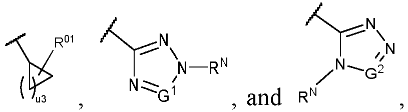 wherein G¹ is a nitrogen atom or CR^{G}; G² is a nitrogen atom or CR^{G}; R^{A4} is a hydrogen atom, hydroxy, C₁₋₆ alkyl, and C₁₋₆ hydroxyalkyl; R³, R⁴, R⁵*,* and R⁶ are identical or different and are each independently a hydrogen atom or C₁₋₆ alkyl; each R⁰¹ is independently selected from the group consisting of hydroxy, cyano, amino, and C₁₋₆ hydroxyalkyl; each R^{G} and R^{N} is identical or different and is independently a hydrogen atom or C₁₋₆ alkyl; t is selected from the group consisting of 0, 1, 2, and 3; u1 is selected from the group consisting of 0, 1, 2, and 3; u2 is selected from the group consisting of 0, 1, 2, and 3; u3 is selected from the group consisting of 1, 2, and 3.

In some embodiments, R^{A} is selected from the group consisting of amino,

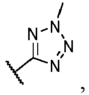

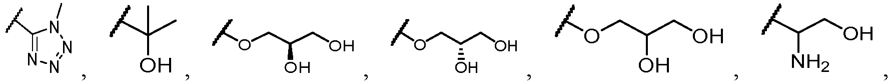

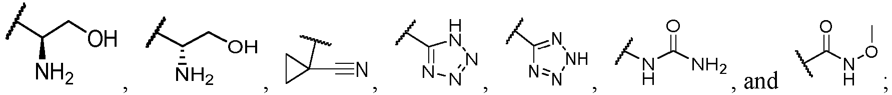

in some embodiments, R^{A} is selected from the group consisting of

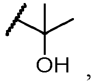

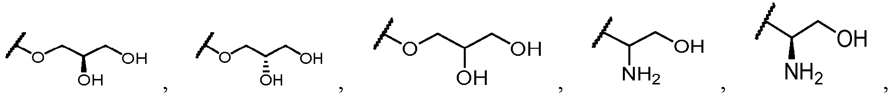

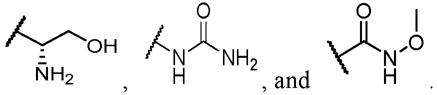

In some embodiments, R^{A} is selected from the group consisting of C₁₋₆ hydroxyalkyl, C₁₋₆ hydroxyalkoxy, -C₁₋₆ alkylene-NH₂, -C(O)NR⁵-OR⁶, 3- to 6-membered cycloalkyl, and 5- or 6-membered heteroaryl; the C₁₋₆ alkylene, 3- to 6-membered cycloalkyl, and 5- or 6-membered heteroaryl are optionally substituted with one or more R⁰¹; each R⁰¹ is independently selected from the group consisting of hydroxy, cyano, C₁₋₆ alkyl, and C₁₋₆ hydroxyalkyl; R⁵and R⁶ are identical or different and are each independently a hydrogen atom or C₁₋₆ alkyl; in some embodiments, R^{A} is -C₁₋₆ alkylene-NH₂ or -C(O)NR⁵-OR⁶; the C₁₋₆ alkylene is optionally substituted with one or more R⁰¹; each R⁰¹ is independently selected from the group consisting of hydroxy and C₁₋₆ hydroxyalkyl; R⁵ and R⁶ are identical or different and are each independently a hydrogen atom or C₁₋₆ alkyl; in some embodiments, R^{A} is selected from the group consisting of

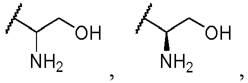

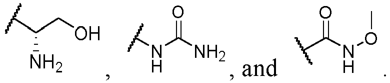

In some embodiments, R^{A} is 3- to 6-membered cycloalkyl or 5- or 6-membered heteroaryl; the 3- to 6-membered cycloalkyl and 5- or 6-membered heteroaryl are each independently and optionally substituted with one or more R⁰¹; each R⁰¹ is independently selected from the group consisting of hydroxy, cyano, amino, C₁₋₆ alkyl, and C₁₋₆ hydroxyalkyl; in some embodiments, R^{A} is 3- to 6-membered cycloalkyl or 5-membered heteroaryl; the 3- to 6-membered cycloalkyl and 5-membered heteroaryl are each independently and optionally substituted with one or more R⁰¹; each R⁰¹ is independently selected from the group consisting of hydroxy, cyano, amino, C₁₋₆ alkyl, and C₁₋₆ hydroxyalkyl; in some embodiments, R^{A} is selected from the group consisting of 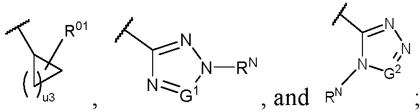 G¹ is a nitrogen atom or CR^{G}; G² is a nitrogen atom or CR^{G}; R⁰¹ is selected from the group consisting of hydroxy, cyano, amino, and C₁₋₆ hydroxyalkyl; each R^{G} and R^{N} is identical or different and is independently a hydrogen atom or C₁₋₆ alkyl; u3 is 1 or 2.

In some embodiments, each R^{A} is identical or different and is independently selected from the group consisting of a fluorine atom, a chlorine atom, hydroxy, cyano, C₁₋₆ alkyl, C₁₋₆ alkoxy, C₁₋₆ haloalkyl, C₁₋₆ haloalkoxy, C₁₋₆ hydroxyalkyl, C₁₋₆ hydroxyalkoxy, -NR³R⁴, -C₁₋₆ alkylene-NR³R⁴, -O-C₁₋₆ alkylene-NR³R⁴, -C(=NR⁵)R⁶, -S(O)ᵥNR³R⁴, - NR⁵S(O)ᵥR⁶, -S(=NR⁵)(O)R⁶, 3- to 10-membered cycloalkyl, 3- to 10-membered heterocyclyl, 6- to 10-membered aryl, 5- to 14-membered heteroaryl, -O-C₁₋₆ alkylene-5 to 14 heteroaryl, and -O-C₁₋₆ alkylene-3- to 10-membered heterocyclyl, wherein the C₁₋₆ alkylene, 3- to 10-membered cycloalkyl, 3- to 10-membered heterocyclyl, 6- to 10-membered aryl, and 5- to 14-membered heteroaryl are each independently and optionally substituted with one or more R⁰¹; each R⁰¹ is independently selected from the group consisting of halogen, hydroxy, cyano, oxo, amino, -NHC₁₋₆ alkyl, -N(C₁₋₆ alkyl)₂, acetyl, C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, 3- to 10-membered cycloalkyl, 3- to 10-membered heterocyclyl, 6- to 10-membered aryl, and 5- to 14-membered heteroaryl; R³, R⁴, R⁵*,* R⁶, and v are as defined in general formula (I).

In some embodiments, each R^{A} is identical or different and is independently selected from the group consisting of hydroxy, amino, -C₁₋₆ alkylene-NH₂, -O-C₁₋₆ alkylene-NH₂, C₁₋₆ alkoxy, C₁₋₆ hydroxyalkyl, C₁₋₆ hydroxyalkoxy, 3- to 8-membered cycloalkyl, 4- to 7-membered heterocyclyl, phenyl, 5- or 6-membered heteroaryl, and -O-C₁₋₆ alkylene-5- or 6-membered heteroaryl, wherein the C₁₋₆ alkylene, 3- to 8-membered cycloalkyl, 4- to 7-membered heterocyclyl, phenyl, and 5- or 6-membered heteroaryl are each independently and optionally substituted with one or more R⁰¹; each R⁰¹ is independently selected from the group consisting of halogen, hydroxy, cyano, oxo, amino, -NHC₁₋₆ alkyl, -N(C₁₋₆ alkyl)₂, C₁₋₆ alkyl, C₂₋₆ alkenyl, C₂₋₆ alkynyl, C₁₋₆ alkoxy, C₁₋₆ haloalkoxy, C₁₋₆ haloalkyl, C₁₋₆ haloalkoxy, C₁₋₆ hydroxyalkyl, 3- to 10-membered cycloalkyl, 3- to 10-membered heterocyclyl, 6- to 10-membered aryl, and 5- to 14-membered heteroaryl.

In some embodiments, each R^{A} is identical or different and is independently selected from the group consisting of amino, C₁₋₆ hydroxyalkyl, C₁₋₆ hydroxyalkoxy, phenyl, and 5- or 6-membered heteroaryl; the phenyl and 5- or 6-membered heteroaryl are each independently and optionally substituted with one or more R⁰¹; each R⁰¹ is independently selected from the group consisting of halogen, hydroxy, cyano, oxo, amino, -NHC₁₋₆ alkyl, -N(C₁₋₆ alkyl)₂, C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, 3- to 10-membered cycloalkyl, 3- to 10-membered heterocyclyl, 6- to 10-membered aryl, and 5- to 14-membered heteroaryl; in some embodiments, each R^{A} is identical or different and is independently selected from the group consisting of amino, 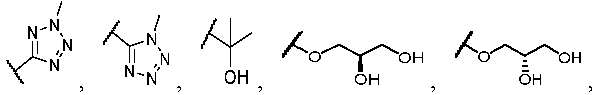 and 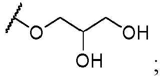 in some embodiments, R^{A} is

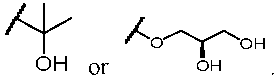

In some embodiments of the present disclosure, provided is the compound represented by general formula (I) or (II) or the pharmaceutically acceptable salt thereof, wherein 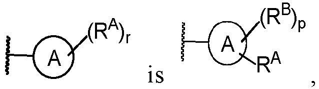 and each R^{B} is identical or different and is independently selected from the group consisting of halogen, hydroxy, cyano, amino, alkyl, alkoxy, haloalkyl, haloalkoxy, cycloalkyl, and heterocyclyl; the alkyl, alkoxy, cycloalkyl, and heterocyclyl are each independently and optionally substituted with one or more groups selected from the group consisting of halogen, hydroxy, cyano, amino, alkyl, alkoxy, haloalkyl, haloalkoxy, cycloalkyl, and heterocyclyl; p is 0, 1, 2, 3, or 4; ring A and R^{A} are as defined in general formula (I).

Preferably, 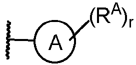 is 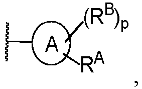 and R^{B} is selected from the group consisting of halogen, hydroxy, C₁₋₆ alkyl, C₁₋₆ alkoxy, C₁₋₆ haloalkyl, and C₁₋₆ haloalkoxy; p is selected from the group consisting of 0, 1, and 2, ring A is selected from the group consisting of phenyl, pyridinyl, pyrimidinyl, pyrazinyl, pyridazinyl, and 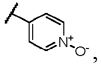 and R^{A} is as defined in general formula (I); more preferably, 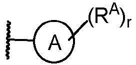 is 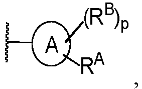 and R^{B} is selected from the group consisting of halogen, hydroxy, C₁₋₆ alkyl, and C₁₋₆ haloalkyl; p is 0 or 1, and ring A is selected from the group consisting of phenyl, pyridinyl, and pyrimidinyl; R^{A} is selected from the group consisting of amino, C₁₋₆ hydroxyalkyl, C₁₋₆ hydroxyalkoxy, phenyl, and 5- or 6-membered heteroaryl; the phenyl and 5- or 6-membered heteroaryl are each independently and optionally substituted with one or more R⁰¹, and each R⁰¹ is as defined in general formula (I).

In some embodiments of the present disclosure, each R^{B} is identical or different and is independently halogen; in some embodiments, R^{B} is F.

In some embodiments of the present disclosure, each R^{B} is identical or different and is independently selected from the group consisting of halogen, C₁₋₆ alkyl, and C₁₋₆ alkoxy; and/or p is 0 or 1; in some embodiments, each R^{B} is identical or different and is independently halogen, and/or p is 0 or 1.

In some embodiments of the present disclosure, p is 0 or 1; in some embodiments, p is 0; in some embodiments, p is 1.

In some embodiments of the present disclosure, provided is the compound represented by general formula (I) or (II) or the pharmaceutically acceptable salt thereof, wherein 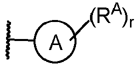 is 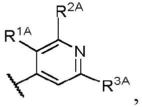 and R^{1A}, R^{2A}, and R^{3A} are identical or different and are each independently selected from the group consisting of a hydrogen atom, a fluorine atom, a chlorine atom, hydroxy, cyano, C₁₋₆ alkyl, C₁₋₆ alkoxy, C₁₋₆ haloalkyl, C₁₋₆ haloalkoxy, C₁₋₆ hydroxyalkyl, C₁₋₆ hydroxyalkoxy, -NR³R⁴, -C₁₋₆ alkylene-NR³R⁴, -O-C₁₋₆ alkylene-NR³R⁴, -C(=NR⁵)R⁶, -S(O)ᵥNR³R⁴, -NR⁵S(O)ᵥR⁶, -S(O)ᵥR⁶, - S(=NR⁵)(O)R⁶, 3- to 10-membered cycloalkyl, 3- to 10-membered heterocyclyl, 6- to 10-membered aryl, 5- to 14-membered heteroaryl, -O-C₁₋₆ alkylene-5 to 14 heteroaryl, and - O-C₁₋₆ alkylene-3- to 10-membered heterocyclyl, wherein the C₁₋₆ alkylene, 3- to 10-membered cycloalkyl, 3- to 10-membered heterocyclyl, 6- to 10-membered aryl, and 5-to 14-membered heteroaryl are each independently and optionally substituted with one or more R⁰¹; R⁰¹, R³, R⁴, R⁵*,* R⁶, and v are as defined in general formula (I).

In some embodiments of the present disclosure, provided is the compound represented by general formula (I), (II), (II'), or (II-1) or the pharmaceutically acceptable salt thereof, wherein R^{A} is selected from the group consisting of halogen, hydroxy, C₁₋₆ alkyl, C₁₋₆ alkoxy, C₁₋₆ haloalkyl, and C₁₋₆ haloalkoxy.

In some embodiments of the present disclosure, provided is the compound represented by general formula (II') or (II-1) or the pharmaceutically acceptable salt thereof, wherein r-1 is 0, 1, or 2; in some embodiments, r-1 is 0; in some embodiments, r-1 is 1.

In some embodiments of the present disclosure, provided is the compound represented by general formula (I) or (II) or the pharmaceutically acceptable salt thereof, wherein 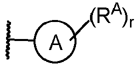 is 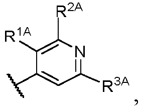 and R^{1A} and R^{2A} are identical or different and are each independently selected from the group consisting of a hydrogen atom, halogen, hydroxy, amino, C₁₋₆ alkyl, C₁₋₆ alkoxy, C₁₋₆ haloalkyl, C₁₋₆ haloalkoxy, C₁₋₆ hydroxyalkyl, and C₁₋₆ hydroxyalkoxy; R^{3A} is selected from the group consisting of halogen, hydroxy, cyano, alkyl, alkoxy, haloalkyl, haloalkoxy, hydroxyalkyl, hydroxyalkoxy, alkenyl, -NR³R⁴, - alkylene-NR³R⁴, -O-alkylene-NR³R⁴, -C(=NR⁵)R⁶, -S(O)ᵥNR³R⁴, -NR⁵S(O)ᵥR⁶, - S(O)ᵥR⁶, -S(=NR⁵)(O)R⁶, -NR⁵C(O)R⁶, -NR⁵C(O)NR³R⁴, -C(O)NR⁵-OR⁶, -P(O)R⁷R⁸, - C(O)NR⁵-NR³R⁴, -C(=NR⁵)NR⁵-OR⁶, -C(O)NR⁵-alkylene-Cy, -C(=NR⁵)NR³R⁴, -C(O)-C(O)-NR³R⁴, cycloalkyl, heterocyclyl, aryl, heteroaryl, -O-alkylene-heteroaryl, and -O-alkylene-heterocyclyl, wherein the alkenyl, alkylene, cycloalkyl, heterocyclyl, aryl, and heteroaryl are each independently and optionally substituted with one or more R⁰¹; R⁰¹, R³, R⁴, R⁵*,* R⁶, and v are as defined in general formula (I).

In some embodiments, R^{1A} and R^{2A} are identical or different and are each independently selected from the group consisting of a hydrogen atom, halogen, hydroxy, amino, C₁₋₆ alkyl, C₁₋₆ alkoxy, C₁₋₆ haloalkyl, C₁₋₆ haloalkoxy, C₁₋₆ hydroxyalkyl, and C₁₋₆ hydroxyalkoxy; R^{3A} is selected from the group consisting of hydroxy, amino, C₁₋₆ alkyl, C₁₋₆ alkoxy, C₁₋₆ hydroxyalkyl, C₁₋₆ hydroxyalkoxy, -C₁₋₆ alkylene-NH₂, -O-C₁₋₆ alkylene-NH₂, phenyl, 5- or 6-membered heteroaryl, and -O-C₁₋₆ alkylene-5- or 6-membered heteroaryl, and the C₁₋₆ alkylene, phenyl, and 5- or 6-membered heteroaryl are each independently and optionally substituted with one or more R⁰¹; each R⁰¹ is independently selected from the group consisting of halogen, hydroxy, cyano, oxo, amino, -NHC₁₋₆ alkyl, -N(C₁₋₆ alkyl)₂, C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, 3- to 10-membered cycloalkyl, 3- to 10-membered heterocyclyl, 6- to 10-membered aryl, and 5- to 14-membered heteroaryl.

Preferably, R^{1A} and R^{2A} are identical or different and are each independently selected from the group consisting of a hydrogen atom, halogen, C₁₋₆ alkyl, and C₁₋₆ haloalkyl; R^{3A} is selected from the group consisting of hydroxy, amino, C₁₋₆ alkoxy, C₁₋₆ hydroxyalkyl, C₁₋₆ hydroxyalkoxy, -C₁₋₆ alkylene-NH₂, -O-C₁₋₆ alkylene-NH₂, phenyl, 5- or 6-membered heteroaryl, and -O-C₁₋₆ alkylene-5- or 6-membered heteroaryl, and the C₁₋₆ alkylene, phenyl, and 5- or 6-membered heteroaryl are each independently and optionally substituted with one or more R⁰¹; each R⁰¹ is independently selected from the group consisting of halogen, hydroxy, cyano, oxo, amino, -NHC₁₋₆ alkyl, -N(C₁₋₆ alkyl)₂, C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, 3- to 10-membered cycloalkyl, 3- to 10-membered heterocyclyl, 6- to 10-membered aryl, and 5- to 14-membered heteroaryl.

In some embodiments of the present disclosure, provided is the compound represented by general formula (I), (II), or (IV) or the pharmaceutically acceptable salt thereof, wherein R^{3A} is selected from the group consisting of F, Cl, amino,

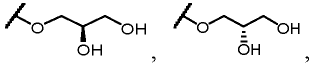

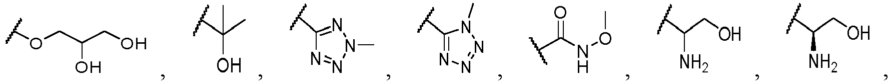

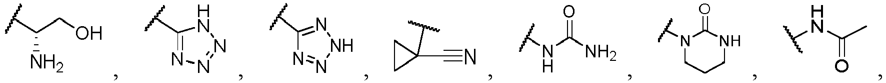

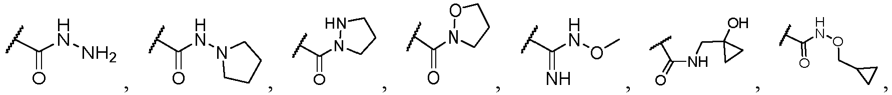

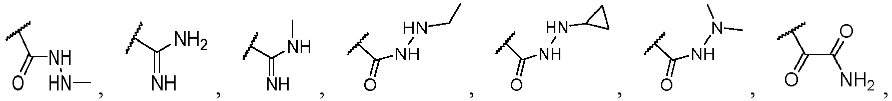

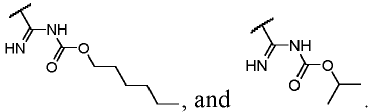

In some embodiments, R^{3A} is selected from the group consisting of amino, 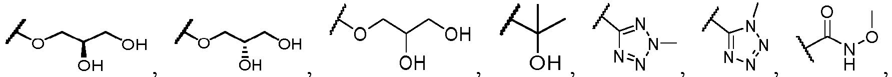 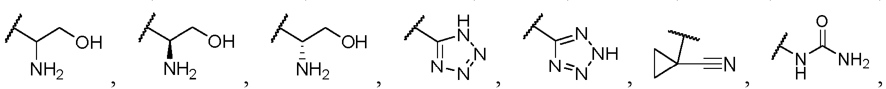 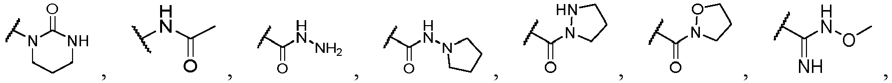 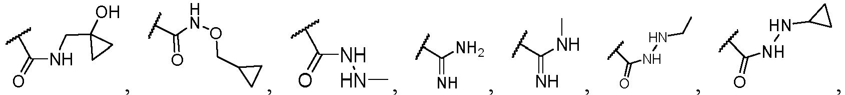 and

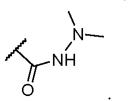

In some embodiments, R^{3A} is selected from the group consisting of amino, 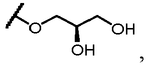 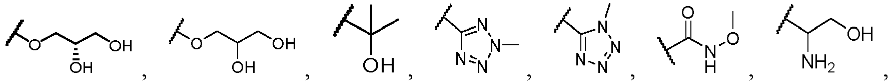 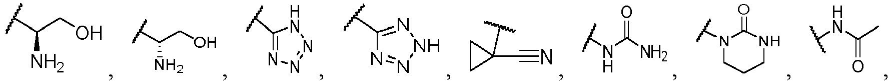 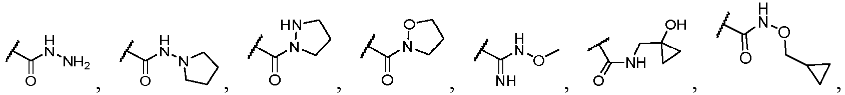 and

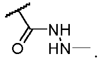

In some embodiments, R^{3A} is selected from the group consisting of

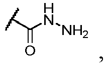

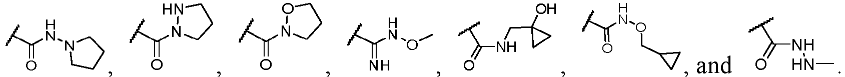

In some embodiments, R^{3A} is selected from the group consisting of 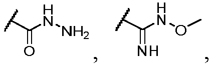 and

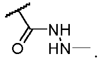

In some embodiments, R^{3A} is selected from the group consisting of

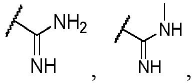

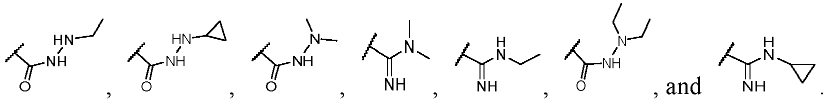

In some embodiments, R^{3A} is selected from the group consisting of

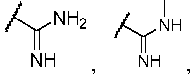

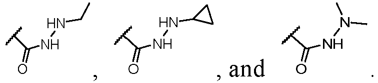

In some embodiments, R^{3A} is selected from the group consisting of

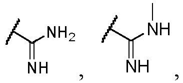

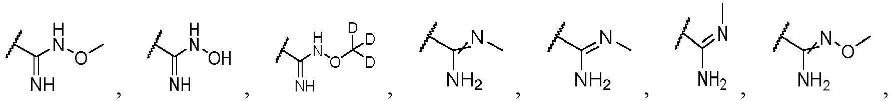

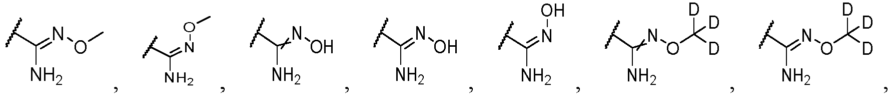

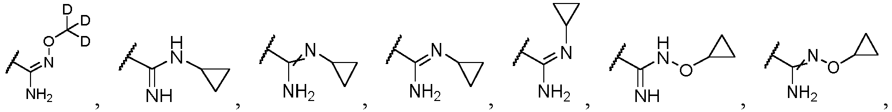

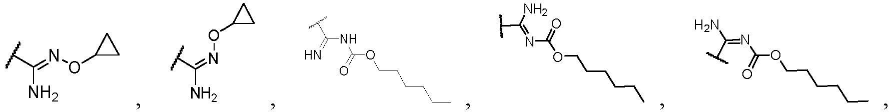

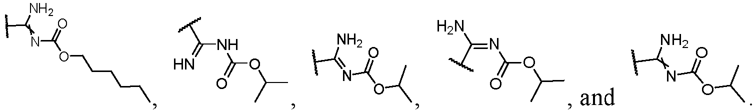

In some embodiments, R^{3A} is selected from the group consisting of 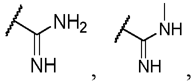 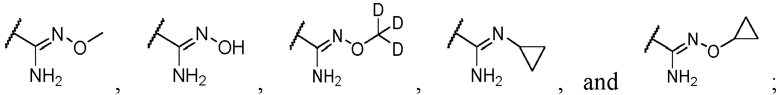 in some embodiments, R^{3A} is 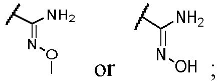 in some embodiments, R^{3A} is 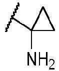 or 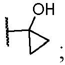 in some embodiments, R^{3A} is 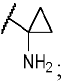 in some embodiments, R^{3A} is

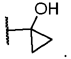

In some embodiments, R^{3A} is selected from the group consisting of -C(O)NR⁵-OR⁶, - C(O)NR⁵-NR³R⁴, -C(=NR⁵)NR⁵-OR⁶, -C(O)NR⁵-alkylene-Cy, -C(=NR⁵)NR³R⁴, and - C(O)-C(O)-NR³R⁴; the alkylene is optionally substituted with one or more R⁰¹; Cy, R³, R⁴, R⁵, R⁶, and R⁰¹ are as defined in general formula (I); in some embodiments, R^{3A} is selected from the group consisting of -C(O)NR⁵-NR³R⁴, -C(=NR⁵)NR⁵-OR⁶, -C(O)NR⁵-alkylene-Cy, -C(O)NR⁵-O-alkylene-cycloalkyl, -C(=NR⁵)NR³R⁴, and -C(O)-C(O)-NR³R⁴; the alkylene is optionally substituted with one or more R⁰¹; Cy, R³, R⁴, R⁵, R⁶, and R⁰¹ are as defined in general formula (I); in some embodiments, R^{3A} is -C(O)NR⁵-NR³R⁴ or -C(=NR⁵)NR³-OR⁶, and R³, R⁴, R⁵, and R⁶ are as defined in general formula (I); in some embodiments, R^{3A} is -C(O)NR⁵-NR³R⁴, and R³, R⁴, and R⁵ are as defined in general formula (I); in some embodiments, R^{3A} is -C(=NR⁵)NR³-OR⁶, and R³, R⁵, and R⁶ are as defined in general formula (I).

In some embodiments, R^{3A} is -C(=NR⁵)NR³R⁴, and R³, R⁴, and R⁵ are as defined in general formula (I); in some embodiments, R^{3A} is -C(=NR⁵)NR³R⁴, and R³, R⁴, and R⁵ are identical or different and are each independently selected from the group consisting of a hydrogen atom, C₁₋₆ alkyl, and 3- to 6-membered cycloalkyl.

In some embodiments, R^{3A} is -C(O)-C(O)-NR³R⁴, and R³ and R⁴ are as defined in general formula (I); in some embodiments, R^{3A} is -C(O)-C(O)-NR³R⁴, and R³ and R⁴ are identical or different and are each independently a hydrogen atom or C₁₋₆ alkyl.

In some embodiments, R^{3A} is -C(O)NR⁵-NR³R⁴ or -C(=NR⁵)NR³-OR⁶, and R³, R⁴, R⁵, and R⁶ are identical or different and are each independently a hydrogen atom or C₁₋₆ alkyl. In some embodiments of the present disclosure, provided is the compound represented by general formula (I), (II), (II'), (II-1), or (IV) or the pharmaceutically acceptable salt thereof, wherein Cy is 3- to 6-membered cycloalkyl, and the 3- to 6-membered cycloalkyl is optionally substituted with one or more substituents selected from the group consisting of halogen, C₁₋₆ alkyl, C₁₋₆ haloalkyl, C₁₋₆ alkoxy, C₁₋₆ haloalkoxy, hydroxy, amino, cyano, and 3- to 6-membered cycloalkyl; in some embodiments, Cy is cyclopropyl, and the cyclopropyl is optionally substituted with one or more substituents selected from the group consisting of halogen, C₁₋₆ alkyl, C₁₋₆ haloalkyl, C₁₋₆ alkoxy, C₁₋₆ haloalkoxy, hydroxy, cyano, and 3- to 6-membered cycloalkyl; in some embodiments, Cy is 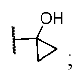 in some embodiments, Cy is selected from the group consisting of cyclopropyl, cyclobutyl, and cyclopentyl, and the cyclopropyl, cyclobutyl, and cyclopentyl are optionally substituted with one or more substituents selected from the group consisting of halogen, C₁₋₆ alkyl, C₁₋₆ haloalkyl, C₁₋₆ alkoxy, C₁₋₆ haloalkoxy, and hydroxy.

In some embodiments of the present disclosure, provided is the compound represented by general formula (I), (II), (II'), (II-1), (IV), (V), (V-1), (V-2), (VI), (VII), (VI'), (VI'-1), (VII'), or (VIII) or the pharmaceutically acceptable salt thereof, wherein R³ and R⁴ are identical or different and are each independently selected from the group consisting of a hydrogen atom, C₁₋₆ alkyl, C₁₋₆ haloalkyl, and C₁₋₆ hydroxyalkyl; preferably, R³ and R⁴ are identical or different and are each independently a hydrogen atom or C₁₋₆ alkyl; more preferably, R³ and R⁴ are both hydrogen atoms.

In some embodiments of the present disclosure, provided is the compound represented by general formula (I), (II), (II'), (II-1), (IV), (V), (V-1), (V-2), (VI), (VII), (VI'), (VI'-1), (VII'), or (VIII) or the pharmaceutically acceptable salt thereof, wherein R³ is selected from the group consisting of a hydrogen atom, C₁₋₆ alkyl, C₁₋₆ haloalkyl, C₁₋₆ hydroxyalkyl, 3- to 6-membered cycloalkyl, and 3- to 6-membered cycloalkyl C₁₋₆ alkyl; the C₁₋₆ alkyl and 3- to 6-membered cycloalkyl are each independently and optionally substituted with one or more groups selected from the group consisting of halogen, hydroxy, oxo, C₁₋₆ alkyl, and C₁₋₆ alkoxy.

In some embodiments, R³ is selected from the group consisting of a hydrogen atom, C₁₋₆ alkyl, C₁₋₆ haloalkyl, C₁₋₆ hydroxyalkyl, 3- to 6-membered cycloalkyl, and 3- to 6-membered cycloalkyl C₁₋₆ alkyl; in some embodiments, R³ is selected from the group consisting of a hydrogen atom, C₁₋₆ alkyl, and 3- to 6-membered cycloalkyl; in some embodiments, R³ is selected from the group consisting of a hydrogen atom, methyl, ethyl, and cyclopropyl; in some embodiments, R³ is ethyl or cyclopropyl.

In some embodiments, R³ is 3- to 6-membered cycloalkyl; in some embodiments, R³ is a hydrogen atom or C₁₋₆ alkyl; in some embodiments, R³ is selected from the group consisting of a hydrogen atom, C₁₋₆ alkyl, OR²³, 3- to 6-membered cycloalkyl, and C(O)OR²³, and R²³ is as defined in general formula (I); in some embodiments, R³ is selected from the group consisting of a hydrogen atom, C₁₋₆ alkyl, OR²³, 3- to 6-membered cycloalkyl, and C(O)OR²³, and R²³ is a hydrogen atom or C₁₋₈ alkyl.

In some embodiments, R³ is selected from the group consisting of a hydrogen atom, C₁₋₆ alkyl, C₁₋₆ alkoxy, 3- to 6-membered cycloalkyl, and C(O)OR²³, and R²³ is as defined in general formula (I).

In some embodiments, R³ is selected from the group consisting of a hydrogen atom, C₁₋₆ alkyl, C₁₋₆ alkoxy, 3- to 6-membered cycloalkyl, and C(O)OR²³, and R²³ is a hydrogen atom or C₁₋₈ alkyl.

In some embodiments, R³ is selected from the group consisting of a hydrogen atom, methyl, ethyl, hydroxy, methoxy, cyclopropyl, 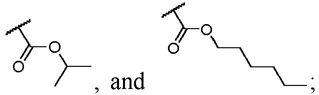 in some embodiments, R³ is selected from the group consisting of a hydrogen atom, methyl, ethyl, methoxy, cyclopropyl, 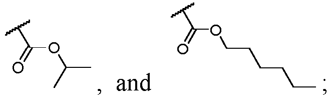 in some embodiments, R³ is selected from the group consisting of a hydrogen atom, methyl, ethyl, hydroxy, methoxy, deuterated methoxy, cyclopropyl,

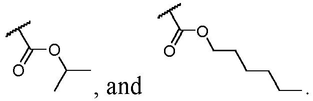

In some embodiments, R³ is a hydrogen atom; in some embodiments, R³ is methyl; in some embodiments, R³ is ethyl; in some embodiments, R³ is hydroxy; in some embodiments, R³ is methoxy; in some embodiments, R³ is cyclopropyl; in some embodiments, R³ is C(O)OR²³, and R²³ is C₁₋₈ alkyl.

In some embodiments of the present disclosure, provided is the compound represented by general formula (I), (II), (II'), (II-1), (IV), (V), (V-1), (V-2), (VI), (VII), (VI'), (VI'-1), (VII'), or (VIII) or the pharmaceutically acceptable salt thereof, wherein R⁴ is a hydrogen atom or C₁₋₆ alkyl; in some embodiments, R⁴ is a hydrogen atom or methyl; in some embodiments, R⁴ is a hydrogen atom; in some embodiments, R⁴ is C₁₋₆ alkyl; in some embodiments, R⁴ is methyl or ethyl.

In some embodiments of the present disclosure, provided is the compound represented by general formula (I), (II), (II'), (II-1), (IV), (V), (V-1), (V-2), (VI), (VII), (VI'), (VI'-1), (VII'), or (VIII) or the pharmaceutically acceptable salt thereof, wherein R³ is selected from the group consisting of a hydrogen atom, C₁₋₆ alkyl, OR²³, and C(O)OR²³, and R²³ is as defined in general formula (I); and/or R⁴ is a hydrogen atom; in some embodiments, R³ is selected from the group consisting of a hydrogen atom, hydroxy, C₁₋₆ alkyl, C₁₋₆ alkoxy, deuterated C₁₋₆ alkoxy, -O-3- to 6-membered cycloalkyl, and C(O)OR²³, and R²³ is C₁₋₈ alkyl; and/or R⁴ is a hydrogen atom; in some embodiments, R³ is selected from the group consisting of a hydrogen atom, methyl, ethyl, hydroxy, methoxy, cyclopropyl, 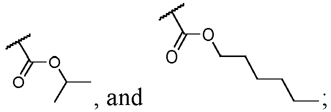 and/or R⁴ is a hydrogen atom.

In some embodiments of the present disclosure, provided is the compound represented by general formula (I), (II), (II'), (II-1), (IV), (V), (V-1), (V-2), (VI), (VII), (VI'), (VI'-1), (VII'), or (VIII) or the pharmaceutically acceptable salt thereof, wherein R²⁰ and R²¹ are identical or different and are each independently selected from the group consisting of a hydrogen atom, C₁₋₈ alkyl, C₁₋₈ haloalkyl, and C₁₋₈ hydroxyalkyl; in some embodiments, R²⁰ and R²¹ are identical or different and are each independently a hydrogen atom or C₁₋₈ alkyl; in some embodiments, R²⁰ is a hydrogen atom, and R²¹ is C₁₋₈ alkyl; in some embodiments, R²⁰ is a hydrogen atom, and R²¹ is n-hexyl.

In some embodiments of the present disclosure, provided is the compound represented by general formula (I), (II), (II'), (II-1), (IV), (V), (V-1), (V-2), (VI), (VII), (VI'), (VI'-1), (VII'), or (VIII) or the pharmaceutically acceptable salt thereof, wherein R²³ is selected from the group consisting of a hydrogen atom, C₁₋₈ alkyl, deuterated C₁₋₆ alkyl, C₁₋₈ haloalkyl, and C₁₋₈ hydroxyalkyl; in some embodiments, R²³ is selected from the group consisting of a hydrogen atom, C₁₋₈ alkyl, C₁₋₈ haloalkyl, and C₁₋₈ hydroxyalkyl; in some embodiments, R²³ is selected from the group consisting of a hydrogen atom, C₁₋₈ alkyl, and deuterated C₁₋₆ alkyl; in some embodiments, R²³ is selected from the group consisting of a hydrogen atom, methyl, deuterated methyl, cyclopropyl, isopropyl, and n-hexyl; in some embodiments, R²³ is C₁₋₈ alkyl; in some embodiments, R²³ is a hydrogen atom or methyl; in some embodiments, R²³ is selected from the group consisting of a hydrogen atom, methyl, and deuterated methyl; in some embodiments, R²³ is isopropyl or n-hexyl. In some embodiments of the present disclosure, provided is the compound represented by general formula (I), (II), (II'), (II-1), (IV), (V), (V-1), (V-2), (VI), (VII), (VI'), (VI'-1), (VII'), or (VIII) or the pharmaceutically acceptable salt thereof, wherein R²² is a hydrogen atom or C₁₋₆ alkyl; in some embodiments, R²² is a hydrogen atom.

In some embodiments of the present disclosure, provided is the compound represented by general formula (I), (II), (II'), (II-1), (III), (III-1), (IV), (V), (V-1), (V-2), (VI), (VII), (VI'), (VI'-1), (VII'), or (VIII) or the pharmaceutically acceptable salt thereof, wherein R⁵ is selected from the group consisting of a hydrogen atom, C₁₋₆ alkyl, C₁₋₆ haloalkyl, and C₁₋₆ hydroxyalkyl; preferably, R⁵ is a hydrogen atom or C₁₋₆ alkyl; in some embodiments, R⁵ is a hydrogen atom.

In some embodiments, R⁵ is selected from the group consisting of a hydrogen atom, C₁₋₆ alkyl, OR²³, 3- to 6-membered cycloalkyl, and C(O)OR²³, and R²³ is as defined in general formula (I).

In some embodiments, R⁵ is selected from the group consisting of a hydrogen atom, a deuterium atom, C₁₋₆ alkyl, deuterated C₁₋₆ alkyl, C₁₋₆ alkoxy, deuterated C₁₋₆ alkoxy, 3-to 6-membered cycloalkyl, -O-3- to 6-membered cycloalkyl, and C(O)OR²³, and R²³ is a hydrogen atom or C₁₋₈ alkyl; in some embodiments, R⁵ is selected from the group consisting of a hydrogen atom, hydroxy, C₁₋₆ alkyl, C₁₋₆ alkoxy, deuterated C₁₋₆ alkoxy, - O-3- to 6-membered cycloalkyl, and C(O)OR²³, and R²³ is C₁₋₈ alkyl; in some embodiments, R⁵ is selected from the group consisting of a hydrogen atom, C₁₋₆ alkyl, C₁₋₆ alkoxy, deuterated C₁₋₆ alkoxy, -O-3- to 6-membered cycloalkyl, and C(O)OR²³, and R²³ is C₁₋₈ alkyl; in some embodiments, R⁵ is hydroxy or C₁₋₆ alkoxy.

In some embodiments, R⁵ is selected from the group consisting of a hydrogen atom, a deuterium atom, methyl, deuterated methyl, hydroxy, methoxy, deuterated methoxy, cyclopropyl, -O-cyclopropyl, 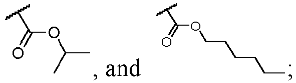 in some embodiments, R⁵ is selected from the group consisting of a hydrogen atom, methyl, hydroxy, methoxy, OCD₃, 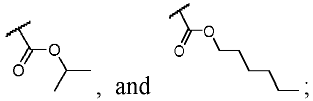 in some embodiments, R⁵ is selected from the group consisting of a hydrogen atom, methyl, hydroxy, methoxy, deuterated methoxy, -O-cyclopropyl, 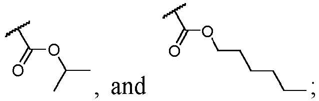 in some embodiments, R⁵ is hydroxy or methoxy.

In some embodiments, R⁵ is methyl; in some embodiments, R⁵ is deuterated methyl; in some embodiments, R⁵ is hydroxy; in some embodiments, R⁵ is methoxy; in some embodiments, R⁵ is deuterated methoxy; in some embodiments, R⁵ is OCD₃; in some embodiments, R⁵ is cyclopropyl or -O-cyclopropyl; in some embodiments, R⁵ is 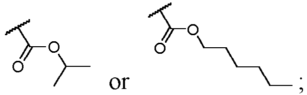 in some embodiments, R⁵ is selected from the group consisting of a hydrogen atom, methyl, hydroxy, methoxy, deuterated methoxy, and -O-cyclopropyl; in some embodiments, R⁵ is selected from the group consisting of a hydrogen atom, methyl, hydroxy, and methoxy.

In some embodiments of the present disclosure, provided is the compound represented by general formula (I), (II), (II'), (II-1), (IV), (VI), (VII), (VI'), (VI'-1), or (VII') or the pharmaceutically acceptable salt thereof, wherein R³ and R⁴ are both hydrogen atoms, and R⁵ is selected from the group consisting of a hydrogen atom, a deuterium atom, methyl, deuterated methyl, hydroxy, methoxy, deuterated methoxy, cyclopropyl, -O-cyclopropyl, 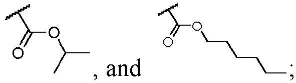 alternatively, R⁵ is a hydrogen atom, R⁴ is a hydrogen atom, and R³ is selected from the group consisting of a hydrogen atom, methyl, ethyl, hydroxy, methoxy, deuterated methoxy, cyclopropyl, 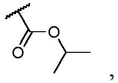 and 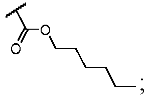 in some embodiments, R³ and R⁴ are both hydrogen atoms, and R⁵ is selected from the group consisting of a hydrogen atom, a deuterium atom, methyl, deuterated methyl, hydroxy, methoxy, deuterated methoxy, cyclopropyl, -O-cyclopropyl,

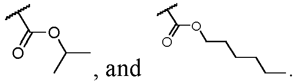

In some embodiments of the present disclosure, provided is the compound represented by general formula (I), (II), (II'), (IV), or (VI) or the pharmaceutically acceptable salt thereof, wherein R^{3a} and R⁴ are both hydrogen atoms, and R⁵ is selected from the group consisting of a hydrogen atom, a deuterium atom, methyl, deuterated methyl, hydroxy, methoxy, deuterated methoxy, cyclopropyl, -O-cyclopropyl, 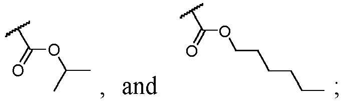 alternatively, R⁵ is a hydrogen atom, R⁴ is a hydrogen atom, and R^{3a} is selected from the group consisting of a hydrogen atom, methyl, ethyl, hydroxy, methoxy, deuterated methoxy, cyclopropyl, 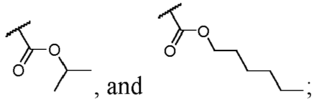 in some embodiments, R^{3a} and R⁴ are both hydrogen atoms, and R⁵ is selected from the group consisting of a hydrogen atom, a deuterium atom, methyl, deuterated methyl, hydroxy, methoxy, deuterated methoxy, cyclopropyl, -O-cyclopropyl,

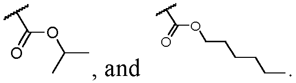

In some embodiments of the present disclosure, provided is the compound represented by general formula (I), (II), (II'), (II-1), (III), (III-I), (IV), (V), (V-1), (V-2), (VI), (VII), (VI'), (VI'-1), (VII'), or (VIII) or the pharmaceutically acceptable salt thereof, wherein R⁶ is selected from the group consisting of a hydrogen atom, C₁₋₆ alkyl, C₁₋₆ haloalkyl, and C₁₋₆ hydroxyalkyl; preferably, R⁶ is a hydrogen atom or C₁₋₆ alkyl; more preferably, R⁶ is C₁₋₆ alkyl; most preferably, R⁶ is CH₃.

In some embodiments, R⁶ is selected from the group consisting of a hydrogen atom, C₁₋₆ alkyl, C₁₋₆ haloalkyl, C₁₋₆ alkoxy, C₁₋₆ haloalkoxy, C₁₋₆ hydroxyalkyl, 3- to 6-membered cycloalkyl, and 3- to 6-membered cycloalkyl C₁₋₆ alkyl; in some embodiments, R⁶ is selected from the group consisting of C₁₋₆ alkyl, 3- to 6-membered cycloalkyl, and 3- to 6-membered cycloalkyl C₁₋₆ alkyl; in some embodiments, R⁶ is C₁₋₆ alkyl or 3- to 6-membered cycloalkyl C₁₋₆ alkyl; in some embodiments, R⁶ is CH₃ or 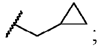 in some embodiments, R⁶ is a hydrogen atom.

In some embodiments, R⁶ is selected from the group consisting of a hydrogen atom, C₁₋₆ alkyl, deuterated C₁₋₆ alkyl, and 3- to 6-membered cycloalkyl; in some embodiments, R⁶ is selected from the group consisting of a hydrogen atom, methyl, deuterated methyl, and cyclopropyl; in some embodiments, R⁶ is selected from the group consisting of a hydrogen atom, methyl, and deuterated methyl; in some embodiments, R⁶ is a hydrogen atom or methyl.

In some embodiments of the present disclosure, provided is the compound represented by general formula (I), (II), (II'), (II-1), (IV), (V), (V-1), (V-2), (VI), (VII), (VI'), (VI'-1), (VII'), or (VIII) or the pharmaceutically acceptable salt thereof, wherein R⁷ and R⁸ are identical or different and are each independently selected from the group consisting of a hydrogen atom, C₁₋₆ alkyl, C₁₋₆ haloalkyl, and C₁₋₆ hydroxyalkyl; preferably, R⁷ and R⁸ are each independently a hydrogen atom or C₁₋₆ alkyl; more preferably, R⁷ and R⁸ are each independently C₁₋₆ alkyl; most preferably, R⁷ and R⁸ are both CH₃; in some embodiments, R⁷ and R⁸ are identical or different and are each independently C₁₋₆ alkyl or amino; in some embodiments, R⁷ and R⁸ are identical or different and are each independently methyl or amino; in some embodiments, R⁷ is methyl, and R⁸ is amino. In some embodiments of the present disclosure, provided is the compound represented by general formula (I), (II), (II'), (II-1), (IV), (V), (V-1), (V-2), (VI), (VII), (VI'), (VI'-1), (VII'), or (VIII) or the pharmaceutically acceptable salt thereof, wherein each R⁰¹ is independently selected from the group consisting of halogen, hydroxy, cyano, oxo, amino, -NH alkyl, -N(alkyl)₂, C₁₋₆ alkyl, C₁₋₆ alkoxy, C₁₋₆ haloalkyl, C₁₋₆ haloalkoxy, C₁₋₆ hydroxyalkyl, 3- to 6-membered cycloalkyl, and 3- to 6-membered heterocyclyl; in some embodiments, each R⁰¹ is independently selected from the group consisting of halogen, hydroxy, cyano, oxo, C₁₋₆ alkyl, C₁₋₆ alkoxy, C₁₋₆ haloalkyl, and C₁₋₆ haloalkoxy; in some embodiments, each R⁰¹ is independently selected from the group consisting of halogen, hydroxy, oxo, C₁₋₆ alkyl, and C₁₋₆ alkoxy; in some embodiments, R⁰¹ is a deuterium atom. In some embodiments of the present disclosure, provided is the compound represented by general formula (I), (II), (II'), (II-1), (III), (III-I), (IV), (V), (V-1), (V-2), (VI), (VII), (VI'), (VI'-1), (VII'), or (VIII) or the pharmaceutically acceptable salt thereof, wherein R^{X1} is selected from the group consisting of a hydrogen atom, halogen, hydroxy, C₁₋₆ alkyl, C₁₋₆ alkoxy, C₁₋₆ haloalkyl, C₁₋₆ haloalkoxy, 3- to 6-membered cycloalkyl, 4- to 7-membered heterocyclyl, 3- to 6-membered cycloalkyloxy, and 4- to 7-membered heterocyclyloxy; the 3- to 6-membered cycloalkyl, 4- to 7-membered heterocyclyl, 3- to 6-membered cycloalkyloxy, and 4- to 7-membered heterocyclyloxy are each independently and optionally substituted with one or more R⁰³; each R⁰³ is independently selected from the group consisting of halogen, C₁₋₆ alkyl, and C₁₋₆ haloalkyl; preferably, R^{X1} is selected from the group consisting of hydroxy, C₁₋₆ alkoxy, and C₁₋₆ haloalkoxy; more preferably, R^{X1} is C₁₋₆ alkoxy; most preferably, R^{X1} is methoxy.

In some embodiments, R^{X1} is selected from the group consisting of hydroxy, C₁₋₆ alkoxy, and 3- to 6-membered cycloalkyloxy; in some embodiments, R^{X1} is selected from the group consisting of hydroxy, methoxy, and 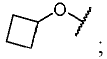 in some embodiments, R^{X1} is hydroxy; in some embodiments, R^{X1} is 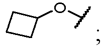 in some embodiments, R^{X1} is selected from the group consisting of hydroxy, methoxy, deuterated methoxy, and 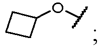 in some embodiments, R^{X1} is selected from the group consisting of hydroxy, methoxy, OCD₃, and 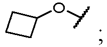 in some embodiments, R^{X1} is deuterated methoxy; in some embodiments, R^{X1} is OCD₃.

In some embodiments of the present disclosure, provided is the compound represented by general formula (I), (II), (II'), (II-1), (III), (III-I), (IV), (V), (V-1), (V-2), (VI), (VII), (VI'), (VI'-1), (VII'), or (VIII) or the pharmaceutically acceptable salt thereof, wherein R^{X2} is selected from the group consisting of a hydrogen atom, halogen, C₁₋₆ alkyl, C₁₋₆ alkoxy, C₁₋₆ haloalkyl, C₁₋₆ haloalkoxy, 3- to 6-membered cycloalkyl, and 4- to 7-membered heterocyclyl; preferably, R^{X2} is selected from the group consisting of a hydrogen atom, halogen, C₁₋₆ alkyl, and C₁₋₆ haloalkyl; more preferably, R^{X2} is halogen; most preferably, R^{X2} is a fluorine atom.

In some embodiments of the present disclosure, provided is the compound represented by general formula (I), (II), (II'), (II-1), (III), (III-I), (IV), (V), (V-1), (V-2), (VI), (VII), (VI'), (VI'-1), (VII'), or (VIII) or the pharmaceutically acceptable salt thereof, wherein R^{X3} is selected from the group consisting of a hydrogen atom, halogen, C₁₋₆ alkyl, C₁₋₆ alkoxy, C₁₋₆ haloalkyl, C₁₋₆ haloalkoxy, 3- to 6-membered cycloalkyl, and 4- to 7-membered heterocyclyl; preferably, R^{X3} is selected from the group consisting of a hydrogen atom, halogen, C₁₋₆ alkyl, and C₁₋₆ haloalkyl; more preferably, R^{X3} is halogen; most preferably, R^{X3} is a fluorine atom.

In some embodiments of the present disclosure, provided is the compound represented by general formula (I), (II), (II'), (II-1), (III), (III-I), (IV), (V), (V-1), (V-2), (VI), (VII), (VI'), (VI'-1), (VII'), or (VIII) or the pharmaceutically acceptable salt thereof, wherein R^{X1}, R^{X2}, and R^{X3} are identical or different and are each independently selected from the group consisting of a hydrogen atom, halogen, hydroxy, C₁₋₆ alkyl, C₁₋₆ alkoxy, C₁₋₆ haloalkyl, C₁₋₆ haloalkoxy, 3- to 6-membered cycloalkyloxy, and 3- to 6-membered heterocyclyloxy; in some embodiments, R^{X1}, R^{X2}, and R^{X3} are identical or different and are each independently selected from the group consisting of halogen, hydroxy, C₁₋₆ alkoxy, and 3- to 6-membered cycloalkyloxy; in some embodiments, R^{X1}, R^{X2}, and R^{X3} are identical or different and are each independently selected from the group consisting of F, hydroxy, methoxy, and

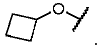

In some embodiments of the present disclosure, provided is the compound represented by general formula (I), (II), (II'), (II-1), (III), (III-I), (IV), (V), (V-1), (V-2), (VI), (VII), (VI'), (VI'-1), (VII'), or (VIII) or the pharmaceutically acceptable salt thereof, wherein R^{X1} is selected from the group consisting of hydroxy, C₁₋₆ alkoxy, and 3- to 6-membered cycloalkyloxy, and/or R^{X2} is halogen, and/or R^{X3} is halogen; in some embodiments, R^{X1} is selected from the group consisting of hydroxy, methoxy, and 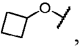 and/or R^{X2} is F, and/or R^{X3} is F.

In some embodiments of the present disclosure, provided is the compound represented by general formula (I) or the pharmaceutically acceptable salt thereof, wherein R^{X4} and R^{X5} are each independently selected from the group consisting of a hydrogen atom, halogen, C₁₋₆ alkyl, C₁₋₆ alkoxy, C₁₋₆ haloalkyl, and C₁₋₆ haloalkoxy; preferably, R^{X4} and R^{X5} are both hydrogen atoms.

In some embodiments of the present disclosure, provided is the compound represented by general formula (I), (II), (II'), (II-1), (III), (III-I), (IV), (V), (V-1), (V-2), (VI), (VII), (VI'), (VI'-1), (VII'), or (VIII) or the pharmaceutically acceptable salt thereof, wherein R^{7a} and R^{8a} are identical or different and are each independently selected from the group consisting of a hydrogen atom, C₁₋₆ alkyl, and halogen; in some embodiments, R^{7a} and R^{8a} are identical or different and are each independently a hydrogen atom or F.

In some embodiments of the present disclosure, provided is the compound represented by general formula (I), (II), (II'), (II-1), (III), (III-I), (IV), (V), (V-1), (V-2), (VI), (VII), (VI'), (VI'-1), (VII'), or (VIII) or the pharmaceutically acceptable salt thereof, wherein n is selected from the group consisting of 0, 1, 2, and 3; preferably, n is 0 or 1; more preferably, n is 0.

In some embodiments of the present disclosure, provided is the compound represented by general formula (I), (II), (II'), (II-1), (III), (III-I), (IV), (V), (V-1), (V-2), (VI), (VII), (VI'), (VI'-1), (VII'), or (VIII) or the pharmaceutically acceptable salt thereof, wherein s is selected from the group consisting of 0, 1, 2, and 3; preferably, s is 0 or 1; more preferably, s is 0.

In some embodiments of the present disclosure, provided is the compound represented by general formula (I) or (II) or the pharmaceutically acceptable salt thereof, wherein r is selected from the group consisting of 0, 1, and 2; preferably, r is 1; in some embodiments, r is 2.

In some embodiments of the present disclosure, provided is the compound represented by general formula (I), (II), (II'), (II-1), (IV), (V), (V-1), (V-2), (VI), (VII), (VI'), (VI'-1), (VII'), or (VIII) or the pharmaceutically acceptable salt thereof, wherein v is 1 or 2; preferably, v is 2.

In some embodiments of the present disclosure, provided is the compound represented by general formula (II) or the pharmaceutically acceptable salt thereof, wherein R^{a} and R^{b} are different and are each independently C₁₋₆ alkyl or C₁₋₆ haloalkyl; R^{c} and R^{d} are different and are each independently a hydrogen atom or C₁₋₆ alkyl; X is O; R' is a hydrogen atom or C₁₋₆ alkyl; ring A is pyridinyl or pyrimidinyl; R^{A} is selected from the group consisting of amino, 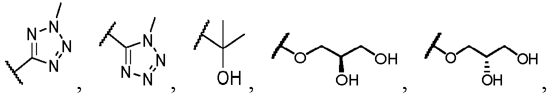 and 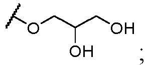 R^{X1} is C₁₋₆ alkoxy; R^{X2} is halogen; R^{X3} is halogen; and r is 1.

In some embodiments of the present disclosure, provided is the compound represented by general formula (II) or the pharmaceutically acceptable salt thereof, wherein R^{a} is C₁₋₆ alkyl, R^{b} is C₁₋₆ haloalkyl, R^{c} is a hydrogen atom, and R^{d} is C₁₋₆ alkyl; R' is a hydrogen atom, and X is O; 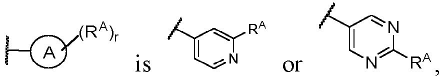 and R^{A} is selected from the group consisting of amino, 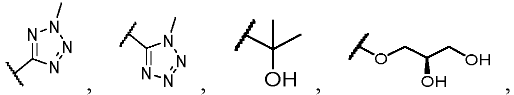 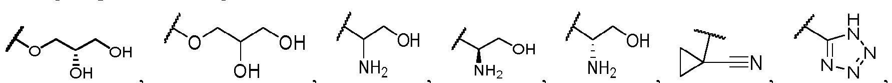 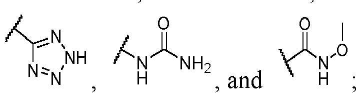 R^{X1} is C₁₋₆ alkoxy; R^{X2} is halogen; R^{X3} is halogen.

In some embodiments of the present disclosure, provided is the compound represented by general formula (II') or the pharmaceutically acceptable salt thereof, wherein R^{a} is C₁₋₆ alkyl, R^{b} is C₁₋₆ haloalkyl, R^{c} is a hydrogen atom, and R^{d} is C₁₋₆ alkyl; R' is a hydrogen atom, and X is O; ring A is pyridinyl or pyrimidinyl; r-1 is 0; R⁵ is a hydrogen atom; R^{3a} is selected from the group consisting of a hydrogen atom, C₁₋₆ alkyl, and C₁₋₆ alkoxy; R⁴ is a hydrogen atom or methyl; R^{X1} is C₁₋₆ alkoxy; R^{X2} is halogen; R^{X3} is halogen.

In some embodiments of the present disclosure, provided is the compound represented by general formula (II-1) or the pharmaceutically acceptable salt thereof, wherein R^{a} is C₁₋₆ alkyl, R^{b} is C₁₋₆ haloalkyl, R^{c} is a hydrogen atom, and R^{d} is C₁₋₆ alkyl; R' is a hydrogen atom, and X is O; ring A is pyridinyl or pyrimidinyl; r-1 is 0; R⁵ is a hydrogen atom; R³ is selected from the group consisting of a hydrogen atom, C₁₋₆ alkyl, and 3- to 6-membered cycloalkyl; R⁴ is a hydrogen atom or methyl; R^{X1} is C₁₋₆ alkoxy; R^{X2} is halogen; R^{X3} is halogen.

In some embodiments of the present disclosure, provided is the compound represented by general formula (IV) or the pharmaceutically acceptable salt thereof, wherein R^{a} is CH₃, R^{b} is CF₃, R^{c} is a hydrogen atom, and R^{d} is CH₃; R' is a hydrogen atom, and R^{1A} and R^{2A} are both hydrogen atoms; R^{3A} is selected from the group consisting of 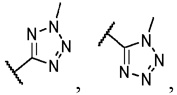 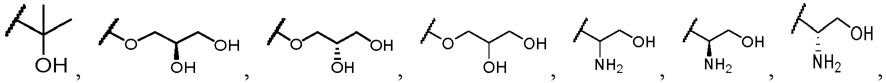 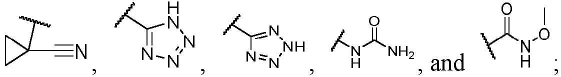 R^{X1} is methoxy; R^{X2} is a fluorine atom; R^{X3} is a fluorine atom.

In some embodiments of the present disclosure, provided is the compound represented by general formula (IV) or the pharmaceutically acceptable salt thereof, wherein R^{a} is C₁₋₆ alkyl, R^{b} is C₁₋₆ haloalkyl, R^{c} is a hydrogen atom, and R^{d} is C₁₋₆ alkyl; R' is a hydrogen atom, and R^{1A} and R^{2A} are both hydrogen atoms; R^{3A} is selected from the group consisting of 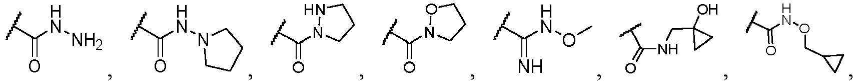 and 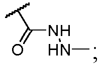 R^{X1} is C₁₋₆ alkoxy; R^{X2} is halogen; R^{X3} is halogen.

In some embodiments of the present disclosure, provided is the compound represented by general formula (IV) or the pharmaceutically acceptable salt thereof, wherein R^{a} is C₁₋₆ alkyl, R^{b} is C₁₋₆ haloalkyl, R^{c} is a hydrogen atom, and R^{d} is C₁₋₆ alkyl; R' is a hydrogen atom, and R^{1A} and R^{2A} are both hydrogen atoms; R^{3A} is -C(O)NR⁵-NR³R⁴ or - C(=NR⁵)NR³-OR⁶, and R³, R⁴, R⁵, and R⁶ are identical or different and are each independently a hydrogen atom or C₁₋₆ alkyl; R^{X1} is C₁₋₆ alkoxy; R^{X2} is halogen; R^{X3} is halogen.

In some embodiments of the present disclosure, provided is the compound represented by general formula (IV) or the pharmaceutically acceptable salt thereof, wherein R^{a} is C₁₋₆ alkyl, R^{b} is C₁₋₆ haloalkyl, R^{c} is a hydrogen atom, and R^{d} is C₁₋₆ alkyl; R' is a hydrogen atom, and R^{1A} and R^{2A} are both hydrogen atoms; R^{3A} is -C(=NR⁵)NR³R⁴, and R³, R⁴, and R⁵ are identical or different and are each independently selected from the group consisting of a hydrogen atom, C₁₋₆ alkyl, and 3- to 6-membered cycloalkyl; R^{X1} is C₁₋₆ alkoxy; R^{X2} is halogen; R^{X3} is halogen.

In some embodiments of the present disclosure, provided is the compound represented by general formula (IV) or the pharmaceutically acceptable salt thereof, wherein R^{a} is C₁₋₆ alkyl, R^{b} is C₁₋₆ haloalkyl, R^{c} is a hydrogen atom, and R^{d} is C₁₋₆ alkyl; R' is a hydrogen atom, and R^{1A} and R^{2A} are both hydrogen atoms; R^{3A} is selected from the group consisting of 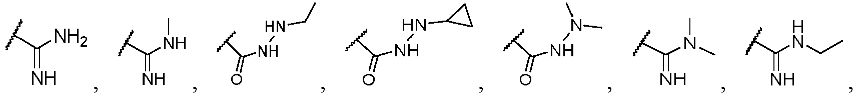 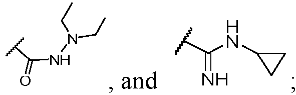 R^{X1} is C₁₋₆ alkoxy; R^{X2} is halogen; R^{X3} is halogen.

In some embodiments of the present disclosure, provided is the compound represented by general formula (VI) or the pharmaceutically acceptable salt thereof, wherein R^{a} is C₁₋₆ alkyl, R^{b} is C₁₋₆ haloalkyl, R^{c} is a hydrogen atom, and R^{d} is C₁₋₆ alkyl; R' is a hydrogen atom, and R^{1A} and R^{2A} are both hydrogen atoms; R⁵ is a hydrogen atom; R^{3a} is selected from the group consisting of a hydrogen atom, C₁₋₆ alkyl, and C₁₋₆ alkoxy; R⁴ is a hydrogen atom or methyl; R^{X1} is C₁₋₆ alkoxy; R^{X2} is halogen; R^{X3} is halogen.

In some embodiments of the present disclosure, provided is the compound represented by general formula (VII) or the pharmaceutically acceptable salt thereof, wherein R^{a} is C₁₋₆ alkyl, R^{b} is C₁₋₆ haloalkyl, R^{c} is a hydrogen atom, and R^{d} is C₁₋₆ alkyl; R' is a hydrogen atom, and R^{1A} and R^{2A} are both hydrogen atoms; R⁵ is a hydrogen atom; R³ is selected from the group consisting of a hydrogen atom, C₁₋₆ alkyl, and 3- to 6-membered cycloalkyl; R⁴ is a hydrogen atom or methyl; R^{X1} is C₁₋₆ alkoxy; R^{X2} is halogen; R^{X3} is halogen.

In some embodiments of the present disclosure, provided is the compound represented by general formula (VI') or the pharmaceutically acceptable salt thereof, wherein R^{a} is C₁₋₆ alkyl, R^{b} is C₁₋₆ haloalkyl, R^{c} is a hydrogen atom, and R^{d} is C₁₋₆ alkyl; R' is a hydrogen atom, and R^{X1} is C₁₋₆ alkoxy; R^{X2} is halogen; R^{X3} is halogen; R^{1A} and R^{2A} are both hydrogen atoms; Q is selected from the group consisting of CR^{5A}, N, and N⁺-O⁻; R^{5A} is halogen; R³ is selected from the group consisting of a hydrogen atom, methyl, ethyl, methoxy, cyclopropyl, 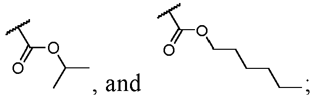 R⁴ is a hydrogen atom or methyl; R⁵ is a hydrogen atom.

In some embodiments of the present disclosure, provided is the compound represented by general formula (VIII) or the pharmaceutically acceptable salt thereof, wherein X is O; R^{a} is C₁₋₆ alkyl, R^{b} is C₁₋₆ haloalkyl, R^{c} is a hydrogen atom, and R^{d} is C₁₋₆ alkyl; R' is a hydrogen atom, and R^{X1} is C₁₋₆ alkoxy; R^{X2} is halogen; R^{X3} is halogen; R^{1A} and R^{2A} are both hydrogen atoms; Q is selected from the group consisting of CR^{5A}, N, and N⁺-O⁻; R^{5A} is halogen; R³ is selected from the group consisting of a hydrogen atom, methyl, ethyl, methoxy, cyclopropyl, 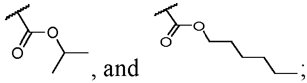 R⁴ is a hydrogen atom or methyl.

In some embodiments of the present disclosure, provided is the compound represented by general formula (II) or the pharmaceutically acceptable salt thereof, wherein R^{a} is CH₃, R^{b} is CF₃, R^{c} is a hydrogen atom, and R^{d} is CH₃; R^{X1} is selected from the group consisting of hydroxy, methoxy, and 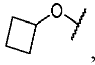 R^{X2} is F, and R^{X3} is F; X is O, and R' is a hydrogen atom; 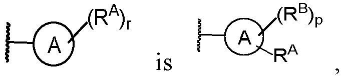 each R^{B} is identical or different and is independently halogen, and p is 0 or 1; ring A is selected from the group consisting of 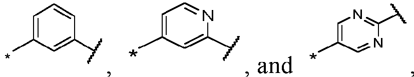 wherein the * end is attached to -NR', and the 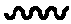 end is attached to R^{A};
R^{A} is selected from the group consisting of F, Cl, amino, cyano,

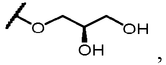

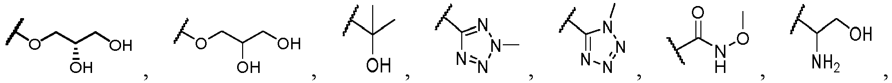

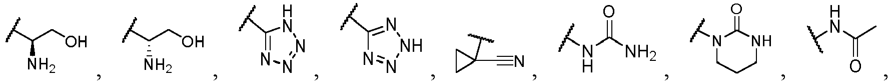

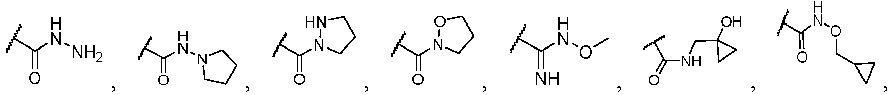

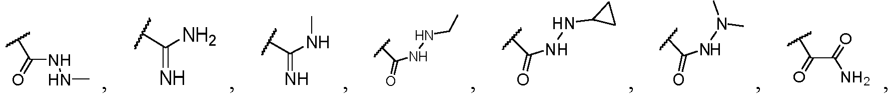

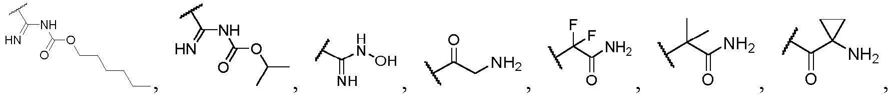

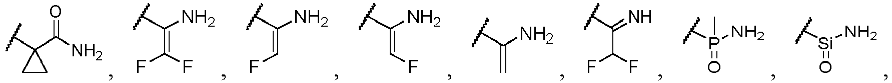

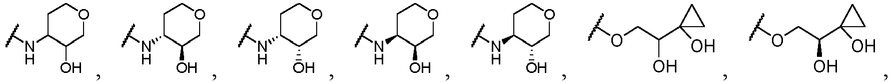

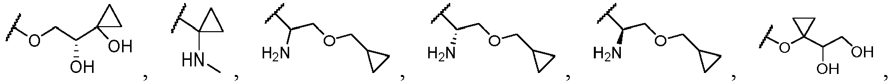

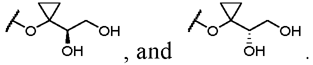

In some embodiments of the present disclosure, provided is the compound represented by general formula (II) or the pharmaceutically acceptable salt thereof, wherein R^{a} is CH₃, R^{b} is CF₃, R^{c} is a hydrogen atom, and R^{d} is CH₃; R^{X1} is selected from the group consisting of hydroxy, methoxy, deuterated methoxy, and 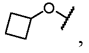 R^{X2} is F, and R^{X3} is F; X is O, and R' is a hydrogen atom; 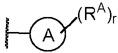 is 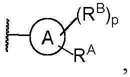 R^{B} is F, and p is 0 or 1; ring A is 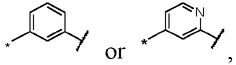 wherein the * end is attached to -NR', and the 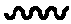 end is attached to R^{A}; R^{A} is selected from the group consisting of

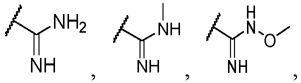

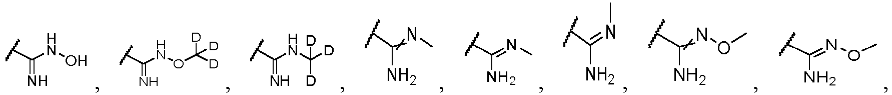

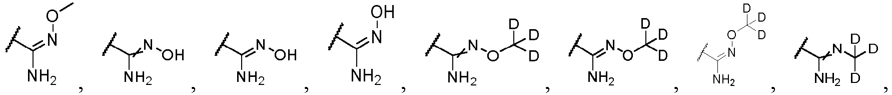

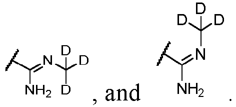

In some embodiments of the present disclosure, provided is the compound represented by general formula (VI') or the pharmaceutically acceptable salt thereof, wherein R^{a} is CH₃, R^{b} is CF₃, R^{c} is a hydrogen atom, and R^{d} is CH₃; R^{X1} is selected from the group consisting of hydroxy, methoxy, and 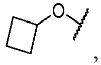 R^{X2} is F, and R^{X3} is F; R' is a hydrogen atom; R^{1A} and R^{2A} are both hydrogen atoms; Q is selected from the group consisting of CR^{5A}, N, and N⁺-O⁻; R^{5A} is halogen; R³ is selected from the group consisting of a hydrogen atom, methyl, ethyl, hydroxy, methoxy, cyclopropyl, 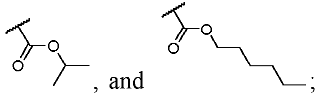 R⁴ is a hydrogen atom or methyl; R⁵ is a hydrogen atom.

In some embodiments of the present disclosure, provided is the compound represented by general formula (VI') or the pharmaceutically acceptable salt thereof, wherein R^{a} is CH₃, R^{b} is CF₃, R^{c} is a hydrogen atom, and R^{d} is CH₃; R^{X1} is selected from the group consisting of hydroxy, methoxy, deuterated methoxy, and 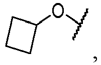 R^{X2} is F, and R^{X3} is F; R' is a hydrogen atom; R^{1A} and R^{2A} are both hydrogen atoms; Q is N or CR^{5A}; R^{5A} is F; R³ is a hydrogen atom, and R⁴ is a hydrogen atom; R⁵ is selected from the group consisting of a hydrogen atom, a deuterium atom, methyl, deuterated methyl, hydroxy, methoxy, deuterated methoxy, cyclopropyl, -O-cyclopropyl,

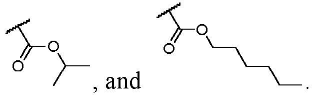

In some embodiments of the present disclosure, provided is the compound represented by general formula (VI') or the pharmaceutically acceptable salt thereof, wherein R^{a} is CH₃, R^{b} is CF₃, R^{c} is a hydrogen atom, and R^{d} is CH₃; R^{X1} is selected from the group consisting of hydroxy, methoxy, and 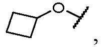 R^{X2} is F, and R^{X3} is F; R' is a hydrogen atom; R^{1A} and R^{2A} are both hydrogen atoms; Q is selected from the group consisting of CR^{5A}, N, and N⁺-O⁻; R^{5A} is F; R³ is a hydrogen atom, and R⁴ is a hydrogen atom; R⁵ is selected from the group consisting of a hydrogen atom, methyl, hydroxy, methoxy, deuterated methoxy, -O-cyclopropyl,

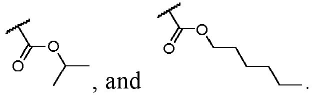

In some embodiments of the present disclosure, provided is the compound represented by general formula (IV) or the pharmaceutically acceptable salt thereof, wherein R^{a} is CH₃, R^{b} is CF₃, R^{c} is a hydrogen atom, and R^{d} is CH₃; R^{X1} is selected from the group consisting of hydroxy, methoxy, and 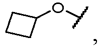 R^{X2} is F, and R^{X3} is F; R' is a hydrogen atom; R^{1A} and R^{2A} are both hydrogen atoms; R^{3A} is

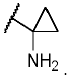

In some embodiments of the present disclosure, provided is the compound represented by general formula (IV) or the pharmaceutically acceptable salt thereof, wherein R^{a} is CH₃, R^{b} is CF₃, R^{c} is a hydrogen atom, and R^{d} is CH₃; R^{X1} is selected from the group consisting of hydroxy, methoxy, and 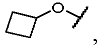 R^{X2} is F, and R^{X3} is F; R' is a hydrogen atom; R^{1A} and R^{2A} are both hydrogen atoms; R^{A} is

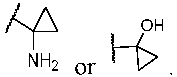

**Table A. Typical compounds of the present disclosure include, but are not limited to:**

| Compound No. | Compound structure | Name |
|---|---|---|
| **1** | 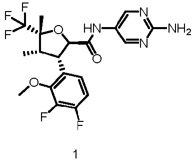 | (2*R*,3*S*,4*S*,5*R*)-*N*-(2-Aminopyrimidin-5-y1)-3-(3,4-difluoro-2-methoxyphenyl)-4,5-dimethyl-5-(trifluoromethyl)tetrahydrofuran-2-carboxamide **1** |
| | 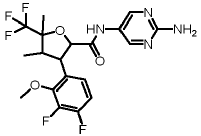 | *N*-(2-Aminopyrimidin-5-yl)-3-(3,4-difluoro-2-methoxyphenyl)-4,5-dimethyl-5-(trifluoromethyl)tetrahydrofuran-2-carboxamide |
| | 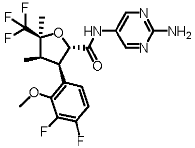 | (2*S*,3*R*,4*R*,5*S*)-*N*-(2-Aminopyrimidin-5-yl)-3-(3,4-difluoro-2-methoxyphenyl)-4,5-dimethyl-5-(trifluoromethyl)tetrahydrofuran-2-carboxamide |
| **2** | 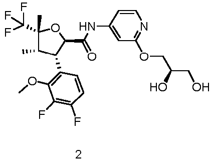 | (2*R,*3*S,*4*S*,5*R*)-3-(3,4-Difluoro-2-methoxyphenyl)-*N*-(2-((*R*)-2,3-dihydroxypropoxy)pyridin-4-yl)-4,5-dimethyl-5-(trifluoromethyl)tetrahydrofuran-2-carboxamide **2** |
| | 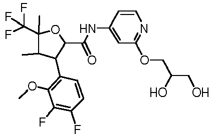 | 3-(3,4-Difluoro-2-methoxyphenyl)-*N*-(2-(2,3-dihydroxypropoxy)pyridin-4-yl)-4,5-dimethyl-5-(trifluoromethyl)tetrahydrofuran-2-carboxamide |
| | 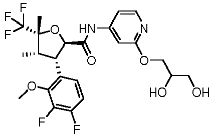 | (2*R*,3*S*,4*S*,5*R*)-3-(3,4-Difluoro-2-methoxyphenyl)-*N*-(2-(2,3-dihydroxypropoxy)pyridin-4-yl)-4,5-dimethyl-5-(trifluoromethyl)tetrahydrofuran-2-carboxamide |
| | 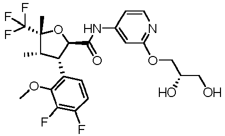 | (2*R*,3*S*,4*S*,5*R*)-3-(3,4-Difluoro-2-methoxyphenyl)-*N*-(2-((*S*)-2,3-dihydroxypropoxy)pyridin-4-yl)-4,5-dimethyl-5-(trifluoromethyl)tetrahydrofuran-2-carboxamide |
| | 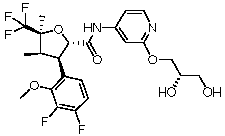 | (2*S*,3*R*,4*R*,5*S*)-3-(3,4-Difluoro-2-methoxyphenyl)-*N*-(2-((*S*)-2,3-dihydroxypropoxy)pyridin-4-yl)-4,5-dimethyl-5-(trifluoromethyl)tetrahydrofuran-2-carboxamide |
| | 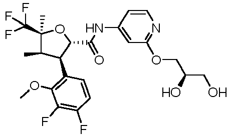 | (2*S*,3*R*,4*R*,5*S*)-3-(3,4-Difluoro-2-methoxyphenyl)-*N*-(2-((*R*)-2,3-dihydroxypropoxy)pyridin-4-yl)-4,5-dimethyl-5-(trifluoromethyl)tetrahydrofuran-2-carboxamide |
| **3** | 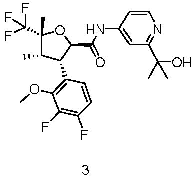 | (2*R*,3*S*,4*S*,5*R*)-3-(3,4-Difluoro-2-methoxyphenyl)-*N*-(2-(2-hydroxypropan-2-yl)pyridin-4-yl)-4,5-dimethyl-5-(trifluoromethyl)tetrahydrofuran-2-carboxamide **3** |
| | 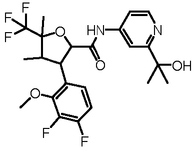 | 3-(3,4-Difluoro-2-methoxyphenyl)-*N*-(2-(2-hydroxypropan-2-yl)pyridin-4-yl)-4,5-dimethyl-5-(trifluoromethyl)tetrahydrofuran-2-carboxamide |
| | 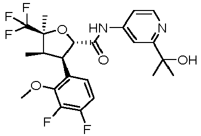 | (2*S*,3*R*,4*R*,5*S*)-3-(3,4-Difluoro-2-methoxyphenyl)-*N*-(2-(2-hydroxypropan-2-yl)pyridin-4-yl)-4,5-dimethyl-5-(trifluoromethyl)tetrahydrofuran-2-carboxamide |
| **4-1** | 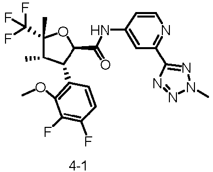 | (2*R*,3*S*,4*S*,5*R*)-3-(3,4-Difluoro-2-methoxyphenyl)-4,5-dimethyl-*N*-(2-(2-methyl-2*H*-tetrazol-5-yl)pyridin-4-yl)-5-(trifluoromethyl)tetrahydrofuran-2-carboxamide **4-1** |
| | 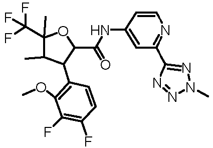 | 3-(3,4-Difluoro-2-methoxyphenyl)-4,5-dimethyl-*N*-(2-(2-methyl-2*H*-tetrazol-5-yl)pyridin-4-yl)-5-(trifluoromethyl)tetrahydrofuran-2-carboxamide |
| | 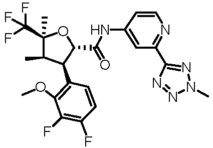 | (2*S*,3*R*,4*R*,5*S*)-3-(3,4-Difluoro-2-methoxyphenyl)-4,5-dimethyl-*N*-(2-(2-methyl-2*H*-tetrazol-5-yl)pyridin-4-yl)-5-(trifluoromethyl)tetrahydrofuran-2-carboxamide |
| **4-2** | 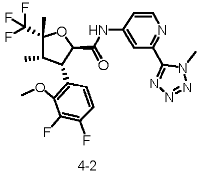 | (2*R*,3*S*,4*S*,5*R*)-3-(3,4-Difluoro-2-methoxyphenyl)-4,5-dimethyl-*N*-(2-(1-methyl-1*H*-tetrazol-5-yl)pyridin-4-yl)-5-(trifluoromethyl)tetrahydrofuran-2-carboxamide **4-2** |
| | 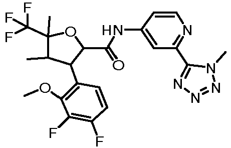 | 3-(3,4-Difluoro-2-methoxyphenyl)-4,5-dimethyl-*N*-(2-(1-methyl-1*H*-tetrazol-5-yl)pyridin-4-yl)-5-(trifluoromethyl)tetrahydrofuran-2-carboxamide |
| | 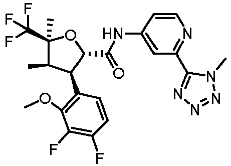 | (2*S*,3*R*,4*R*,5*S*)-3-(3,4-Difluoro-2-methoxyphenyl)-4,5-dimethyl-*N*-(2-(1-methyl-1*H*-tetrazol-5-yl)pyridin-4-yl)-5-(trifluoromethyl)tetrahydrofuran-2-carboxamide |
| **5** | 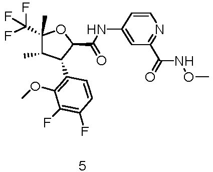 | 4-((2*R*,3*S*,4*S*,5*R*)-3-(3,4-Difluoro-2-methoxyphenyl)-4,5-dimethyl-5-(trifluoromethyl)tetrahydrofuran-2-carboxamido)-*N*-methoxypicolinamide **5** |
| | 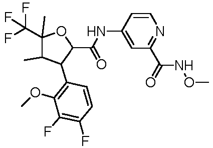 | 4-(3-(3,4-Difluoro-2-methoxyphenyl)-4,5-dimethyl-5-(trifluoromethyl)tetrahydrofuran-2-carboxamido)-N-methoxypicolinamide |
| | 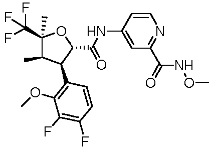 | 4-((2*S*,3*R*,4*R*,5*S*)-3-(3,4-Difluoro-2-methoxyphenyl)-4,5-dimethyl-5-(trifluoromethyl)tetrahydrofuran-2-carboxamido)-N-methoxypicolinamide |
| **6** | 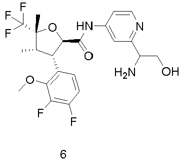 | (2*R*,3*S*,4*S*,5*R*)-*N*-(2-(1-Amino-2-hydroxyethyl)pyridin-4-yl)-3-(3,4-difluoro-2-methoxyphenyl)-4,5-dimethyl-5-(trifluoromethyl)tetrahydrofuran-2-carboxamide **6** |
| | 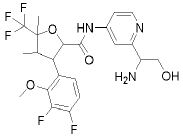 | *N*-(2-(1-Amino-2-hydroxyethyl)pyridin-4-yl)-3-(3,4-difluoro-2-methoxyphenyl)-4,5-dimethyl-5-(trifluoromethyl)tetrahydrofuran-2-carboxamide |
| | 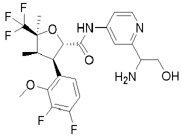 | (2*S*,3*R*,4*R*,5*S*)-*N*-(2-(1-Amino-2-hydroxyethyl)pyridin-4-yl)-3-(3,4-difluoro-2-methoxyphenyl)-4,5-dimethyl-5-(trifluoromethyl)tetrahydrofuran-2-carboxamide |
| **6-1** | 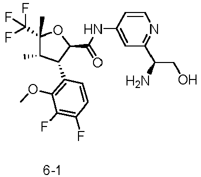 | (2*R*,3*S*,4*S*,5*R*)-*N*-(2-((*S*)-1-Amino-2-hydroxyethyl)pyridin-4-yl)-3-(3,4-difluoro-2-methoxyphenyl)-4,5-dimethyl-5-(trifluoromethyl)tetrahydrofuran-2-carboxamide **6-1** |
| | 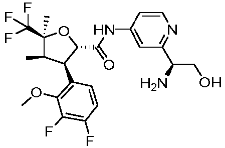 | (2*S*,3*R*,4*R*,5*S*)-*N*-(2-((*S*)-1-Amino-2-hydroxyethyl)pyridin-4-yl)-3-(3,4-difluoro-2-methoxyphenyl)-4,5-dimethyl-5-(trifluoromethyl)tetrahydrofuran-2-carboxamide |
| **6-2** | 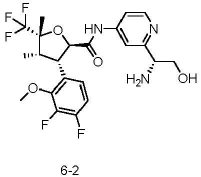 | (2*R*,3*S*,4*S*,5*R*)-*N*-(2-((*R*)-1-Amino-2-hydroxyethyl)pyridin-4-yl)-3-(3,4-difluoro-2-methoxyphenyl)-4,5-dimethyl-5-(trifluoromethyl)tetrahydrofuran-2-carboxamide **6-2** |
| | 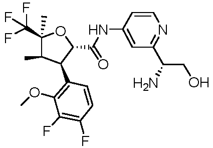 | (2*S*,3*R*,4*R*,5*S*)-*N*-(2-((*R*)-1-Amino-2-hydroxyethyl)pyridin-4-yl)-3-(3,4-difluoro-2-methoxyphenyl)-4,5-dimethyl-5-(trifluoromethyl)tetrahydrofuran-2-carboxamide |
| **7** | 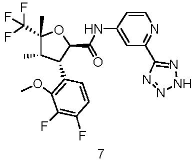 | (2*R*,3*S*,4*S*,5*R*)-*N*-(2-(2*H-*Tetrazol-5-yl)pyridin-4-yl)-3-(3,4-difluoro-2-methoxyphenyl)-4,5-dimethyl-5-(trifluoromethyl)tetrahydrofuran-2-carboxamide 7 |
| | 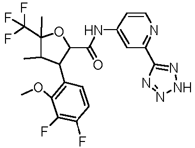 | *N*-(2-(2*H*-Tetrazol-5-yl)pyridin-4-yl)-3-(3,4-difluoro-2-methoxyphenyl)-4,5-dimethyl-5-(trifluoromethyl)tetrahydrofuran-2-carboxamide |
| | 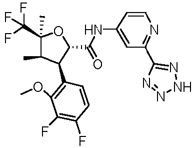 | (2*S*,3*R*,4*R*,5*S*)-*N*-(2-(2*H*-Tetrazol-5-yl)pyridin-4-yl)-3-(3,4-difluoro-2-methoxyphenyl)-4,5-dimethyl-5-(trifluoromethyl)tetrahydrofuran-2-carboxamide |
| 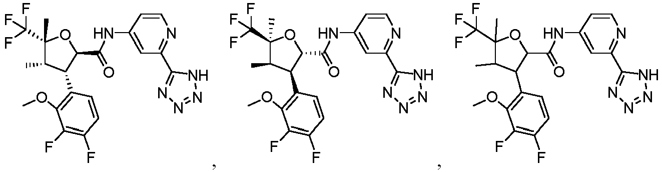 | | |
| **8** | 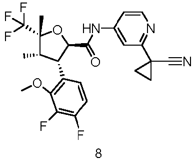 | (2*R*,3*S*,4*S,*5*R*)-*N-*(2-(1-Cyanocyclopropyl)pyridin-4-yl)-3-(3,4-difluoro-2-methoxyphenyl)-4,5-dimethyl-5-(trifluoromethyl)tetrahydrofuran-2-carboxamide **8** |
| | 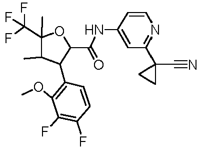 | *N*-(2-(1-Cyanocyclopropyl)pyridin-4-yl)-3-(3,4-difluoro-2-methoxyphenyl)-4,5-dimethyl-5-(trifluoromethyl)tetrahydrofuran-2-carboxamide |
| | 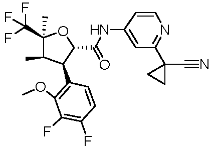 | (2*S*,3*R*,4*R*,5*S*)-*N*-(2-(1-Cyanocyclopropyl)pyridin-4-yl)-3-(3,4-difluoro-2-methoxyphenyl)-4,5-dimethyl-5-(trifluoromethyl)tetrahydrofuran-2-carboxamide |
| **9** | 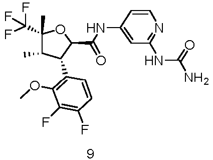 | (2*R*,3*S*,4*S*,5*R*)-3-(3,4-Difluoro-2-methoxyphenyl)-4,5-dimethyl-5-(trifluoromethyl)-*N*-(2-ureidopyridin-4-yl)tetrahydrofuran-2-carboxamide **9** |
| | 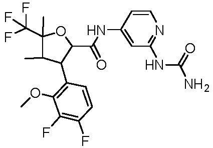 | 3-(3,4-Difluoro-2-methoxyphenyl)-4,5-dimethyl-5-(trifluoromethyl)-*N*-(2-ureidopyridin-4-yl)tetrahydrofuran-2-carboxamide |
| | 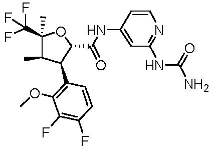 | (2*S*,3*R*,4*R*,5*S*)-3-(3,4-Difluoro-2-methoxyphenyl)-4,5-dimethyl-5-(trifluoromethyl)-*N*-(2-ureidopyridin-4-yl)tetrahydrofuran-2-carboxamide |
| **10** | 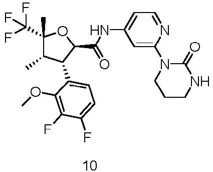 | (2*R*,3*S*,4*S*,5*R*)-3-(3,4-Difluoro-2-methoxyphenyl)-4,5-dimethyl-*N*-(2-(2-oxotetrahydropyrimidin-1(2*H*)-yl)pyridin-4-yl)-5-(trifluoromethyl)tetrahydrofuran-2-carboxamide **10** |
| | 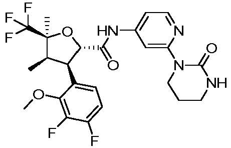 | (2*S*,3*R*,4*R*,5*S*)-3-(3,4-Difluoro-2-methoxyphenyl)-4,5-dimethyl-*N*-(2-(2-oxotetrahydropyrimidin-1(2*H*)-yl)pyridin-4-yl)-5-(trifluoromethyl)tetrahydrofuran-2-carboxamide |
| | 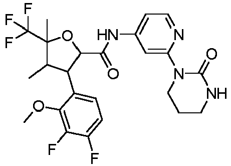 | 3-(3,4-Difluoro-2-methoxyphenyl)-4,5-dimethyl-*N*-(2-(2-oxotetrahydropyrimidin-1(2*H*)-yl)pyridin-4-yl)-5-(trifluoromethyl)tetrahydrofuran-2-carboxamide |
| **11** | 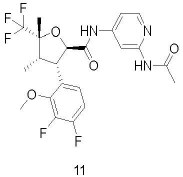 | (2*R*,3*S*,4*S*,5*R*)-*N*-(2-Acetamidopyridin-4-yl)-3-(3,4-difluoro-2-methoxyphenyl)-4,5-dimethyl-5-(trifluoromethyl)tetrahydrofuran-2-carboxamide **11** |
| | 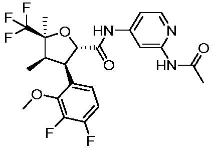 | (2*S*,3*R*,4*R*,5*S*)-*N*-(2-Acetamidopyridin-4-yl)-3-(3,4-difluoro-2-methoxyphenyl)-4,5-dimethyl-5-(trifluoromethyl)tetrahydrofuran-2-carboxamide |
| | 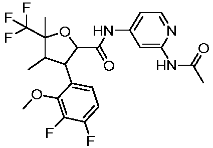 | *N*-(2-Acetamidopyridin-4-yl)-3-(3,4-difluoro-2-methoxyphenyl)-4,5-dimethyl-5-(trifluoromethyl)tetrahydrofuran-2-carboxamide |
| **12** | 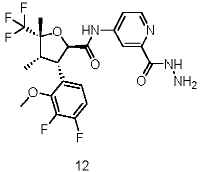 | (2*R*,3*S*,4*S*,5*R*)-3-(3,4-Difluoro-2-methoxyphenyl)-*N*-(2-(hydrazinecarbonyl)pyridin-4-yl)-4,5-dimethyl-5-(trifluoromethyl)tetrahydrofuran-2-carboxamide **12** |
| 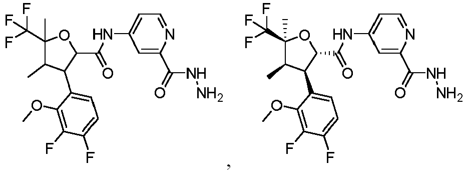 | | |
| **13** | 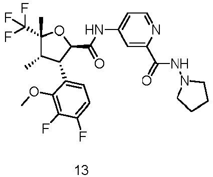 | 4-((2*R*,3*S*,4*S*,5*R*)-3-(3,4-Difluoro-2-methoxyphenyl)-4,5-dimethyl-5-(trifluoromethyl)tetrahydrofuran-2-carboxamido)-*N*-(pyrrolidin-1-yl)picolinamide **13** |
| 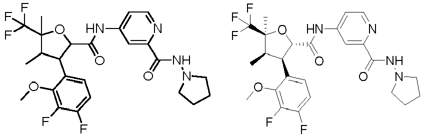 | | |
| **14** | 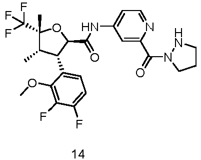 | (2*R*,3*S*,4*S*,5*R*)-3-(3,4-Difluoro-2-methoxyphenyl)-4,5-dimethyl-*N*-(2-(pyrazolidine-1-carbonyl)pyridin-4-yl)-5-(trifluoromethyl)tetrahydrofuran-2-carboxamide **14** |
| 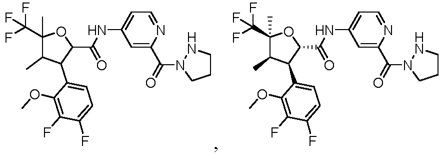 | | |
| **15** | 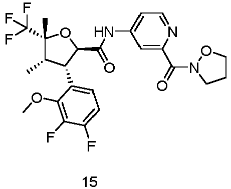 | (2*R*,3*S*,4*S*,5*R*)-3-(3,4-Difluoro-2-methoxyphenyl)-*N*-(2-(isoxazolidine-2-carbonyl)pyridin-4-yl)-4,5-dimethyl-5-(trifluoromethyl)tetrahydrofuran-2-carboxamide **15** |
| 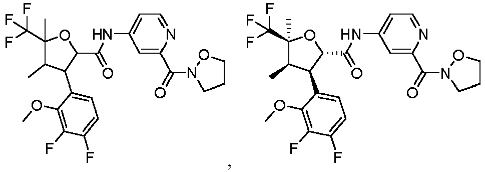 | | |
| **16** | 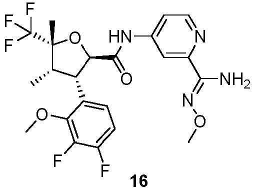 | (2*R*,3*S*,4S*,*5*R*)-3-(3,4-Difluoro-2-methoxyphenyl)-*N*-(2-((Z)-(*N*'-methoxycarbamimidoyl)pyridin-4-yl)-4,5-dimethyl-5-(trifluoromethyl)tetrahydrofuran-2-carboxamide **16** |
| 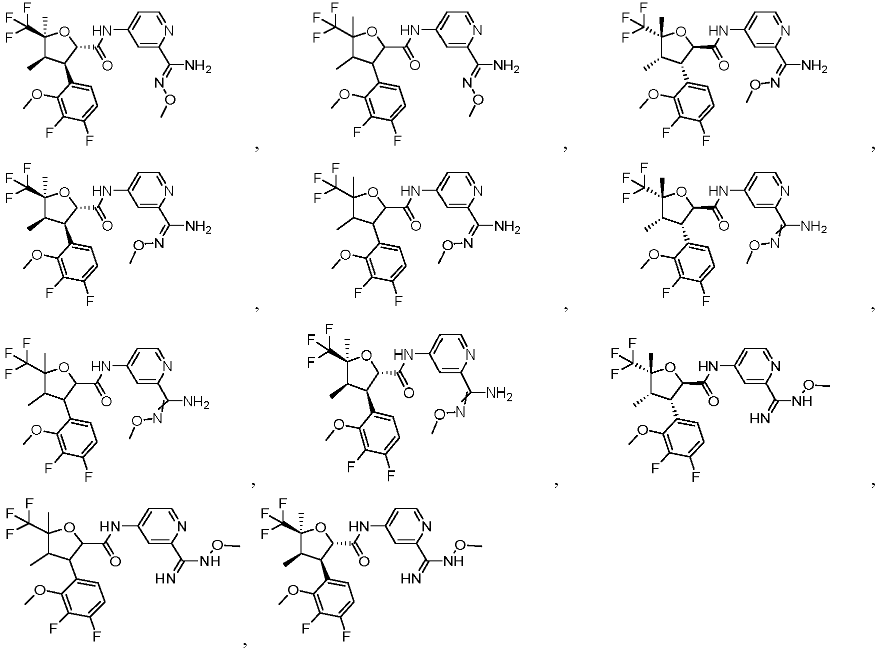 | | |
| **17** | 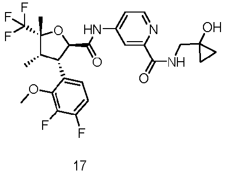 | 4-(2*R*,3*S*,4*S*,5*R*)-3-(3,4-Difluoro-2-methoxyphenyl)-4,5-dimethyl-5-(trifluoromethyl)tetrahydrofuran-2-carboxamido-*N*-((1-hydroxycyclopropyl)methyl)picolinamide **17** |
| 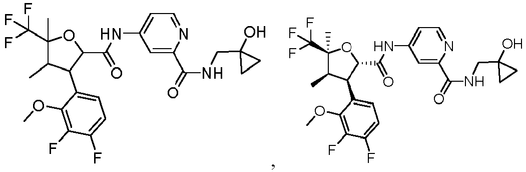 | | |
| **18** | 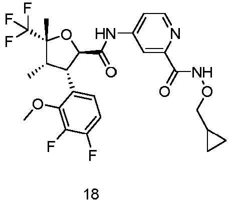 | *N*-(Cyclopropylmethoxy)-4-((2*R*,3*S*,4*S*,5*R*)-3-(3,4-difluoro-2-methoxyphenyl)-4,5-dimethyl-5-(trifluoromethyl)tetrahydrofuran-2-carboxamido)picolinamide **18** |
| 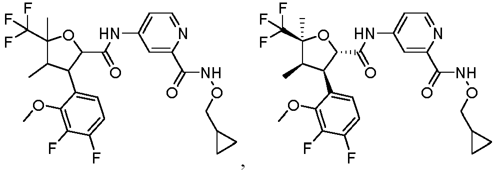 | | |
| **19** | 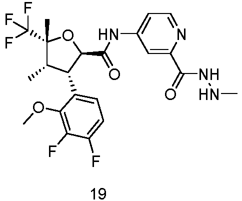 | (2*R*,3*S*,4*S*,5*R*)-3-(3,4-Difluoro-2-methoxyphenyl)-4,5-dimethyl-*N*-(2-(2-methylhydrazine-1-carbonyl)pyridin-4-yl)-5-(trifluoromethyl)tetrahydrofuran-2-carboxamide **19** |
| 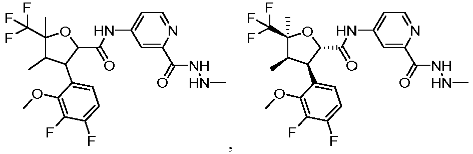 | | |
| **20** | 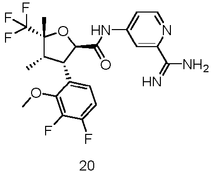 | (2*R*,3*S*,4*S*,5*R*)-*N*(2-Carbamimidoylpyridin-4-yl)-3-(3,4-difluoro-2-methoxyphenyl)-4,5-dimethyl-5-(trifluoromethyl)tetrahydrofuran-2-carboxamide **20** |
| 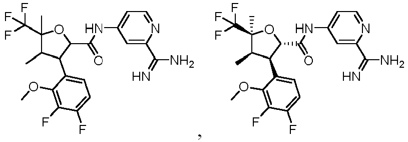 | | |
| **21** | 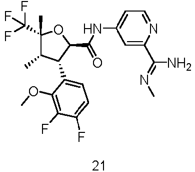 | (2*R*,3*S*,4*S*,5*R*)-3-(3,4-Difluoro-2-methoxyphenyl)-4,5-dimethyl-*N*-(2-((*Z*)-*N*'-methylcarbamimidoyl)pyridin-4-yl)-5-(trifluoromethyl)tetrahydrofuran-2-carboxamide **21** |
| 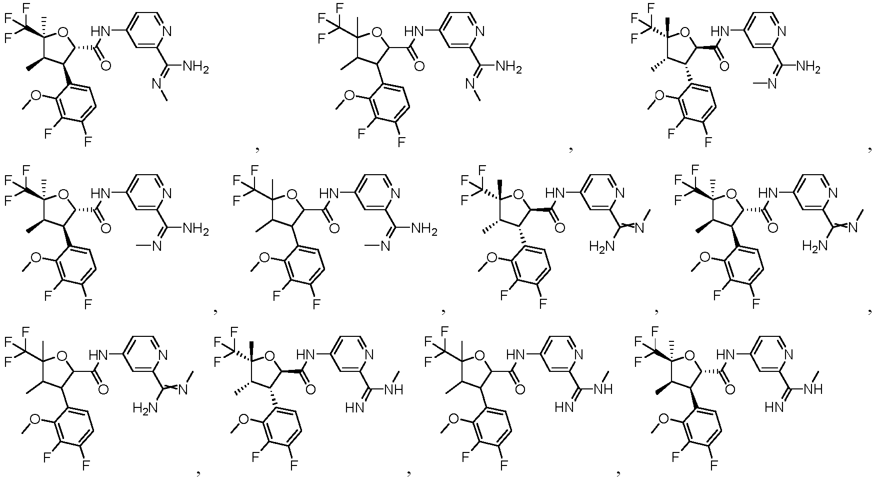 | | |
| **22** | 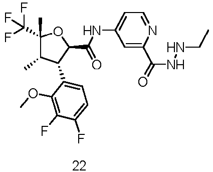 | (2*R*,3*S*,4*S*,5*R*)-3-(3,4-Difluoro-2-methoxyphenyl)-*N*-(2-(2-ethylhydrazine-1-carbonyl)pyridin-4-yl)-4,5-dimethyl-5-(trifluoromethyl)tetrahydrofuran-2-carboxamide **22** |
| 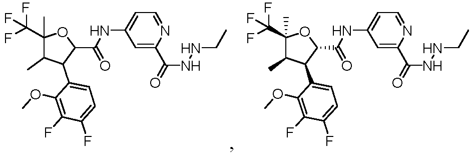 | | |
| **23** | 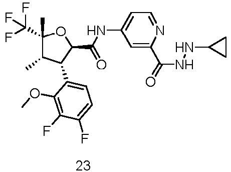 | (2*R*,3*S*,4*S*,5*R*)-*N*-(2-(2-Cyclopropylhydrazine-1-carbonyl)pyridin-4-yl)-3-(3,4-difluoro-2-methoxyphenyl)-4,5-dimethyl-5-(trifluoromethyl)tetrahydrofuran-2-carboxamide **23** |
| 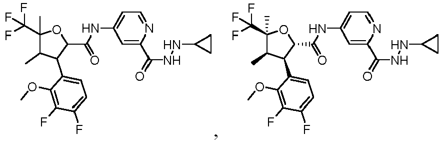 | | |
| **24** | 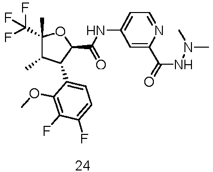 | (2*R*,3*S*,4*S*,5*R*)-3-(3,4-Difluoro-2-methoxyphenyl)-*N*-(2-(2,2-dimethylhydrazine-1-carbonyl)pyridin-4-yl)-4,5-dimethyl-5-(trifluoromethyl)tetrahydrofuran-2-carboxamide **24** |
| 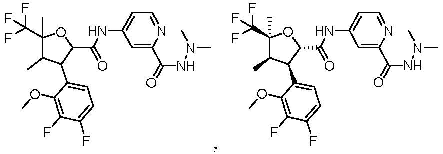 | | |
| **25** | 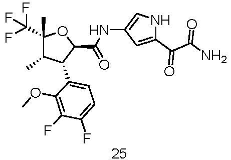 | (2*R*,3*S*,4*S*,5*R*)-*N*-(5-(2-Amino-2-oxoacetyl)-1*H-*pyrrol-3-yl)-3-(3,4-difluoro-2-methoxyphenyl)-4,5-dimethyl-5-(trifluoromethyl)tetrahydrofuran-2-carboxamide **25** |
| 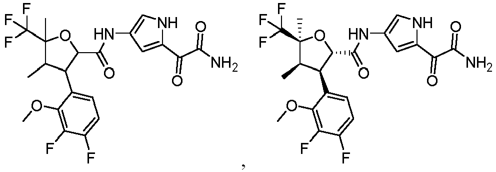 | | |
| **26** | 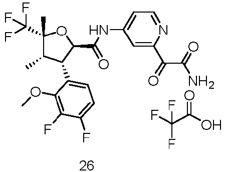 | (2*R*,3*S*,4*S*,5*R*)-*N*-(2*-*(2-Amino-2-oxoacetyl)pyridin-4-yl)-3-(3,4-difluoro-2-methoxyphenyl)-4,5-dimethyl-5-(trifluoromethyl)tetrahydrofuran-2-carboxamide trifluoroacetate **26** |
| 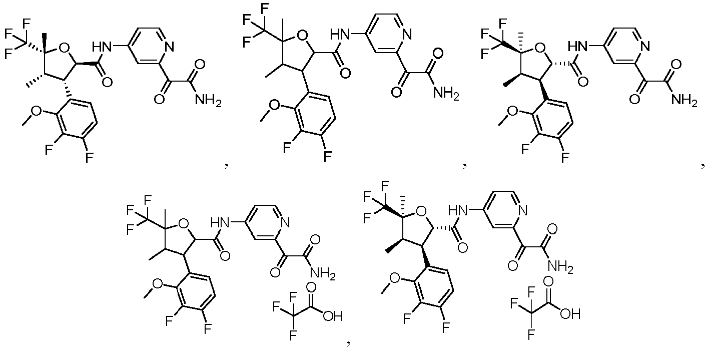 | | |
| **27** | 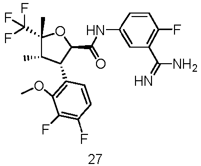 | (2*R*,3*S*,4*S*,5*R*)-*N*-(3-Carbamimidoyl-4-fluorophenyl)-3-(3,4-difluoro-2-methoxyphenyl)-4,5-dimethyl-5-(trifluoromethyl)tetrahydrofuran-2-carboxamide **27** |
| 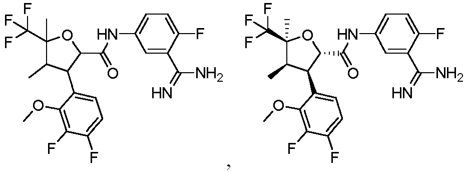 | | |
| **28** | 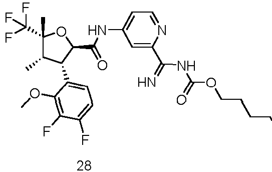 | Hexyl ((4-((2*R*,3*S*,4*S*,5*R*)-3-(3,4-difluoro-2-methoxyphenyl)-4,5-dimethyl-5-(trifluoromethyl)tetrahydrofuran-2-carboxamido)pyridin-2-yl)(imino)methyl)carbamate **28** |
| 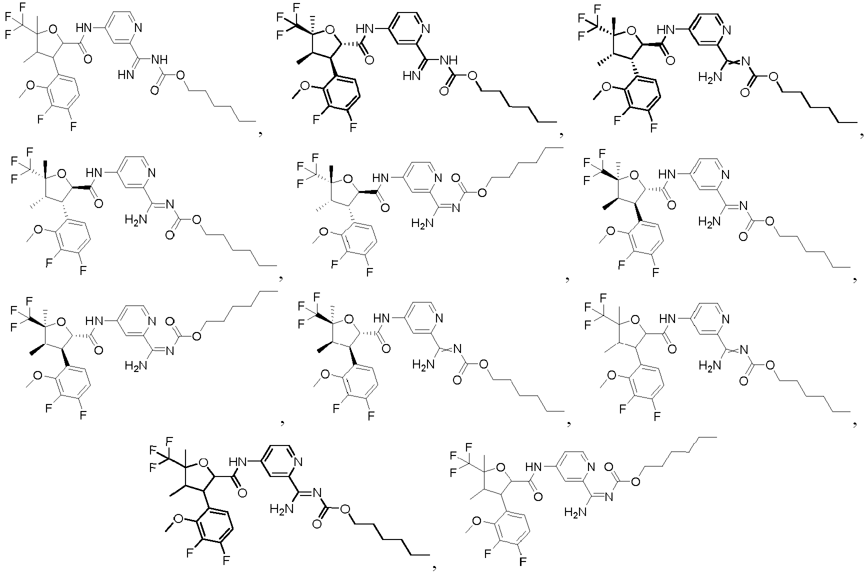 | | |
| **28a** | 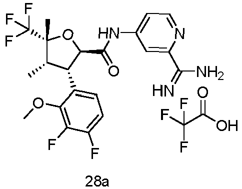 | (2*R*,3*S*,4*S*,5*R*)-*N-*(2-Carbamimidoylpyridin-4-yl)-3-(3,4-difluoro-2-methoxyphenyl)-4,5-dimethyl-5-(trifluoromethyl)tetrahydrofuran-2-carboxamide 2,2,2-trifluoroacetate **28a** |
| 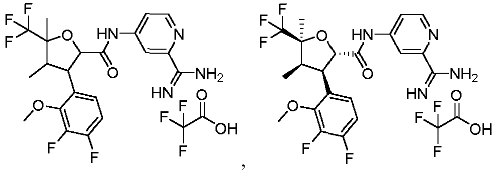 | | |
| **29** | 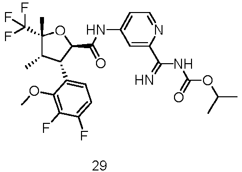 | Isopropyl ((4-((2*R*,3*S*,4*S*,5*R*)-3-(3,4-difluoro-2-methoxyphenyl)-4,5-dimethyl-5-(trifluoromethyl)tetrahydrofuran-2-carboxamido)pyridin-2-yl)(imino)methyl)carbamate **29** |
| 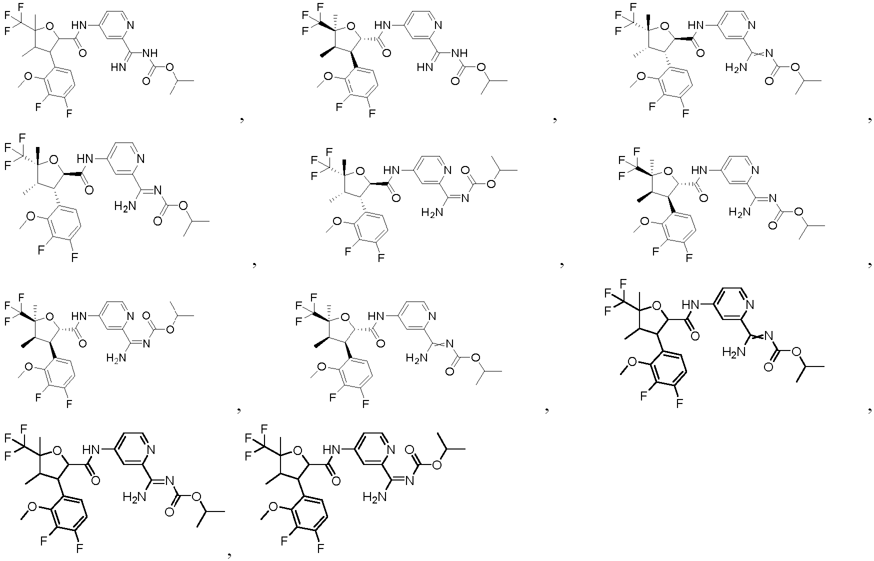 | | |
| **30** | 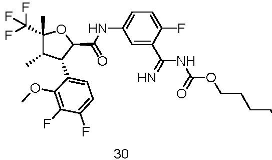 | Hexyl ((5-((2*R*,3*S*,4*S*,5*R*)-3-(3,4-difluoro-2-methoxyphenyl)-4,5-dimethyl-5-(trifluoromethyl)tetrahydrofuran-2-carboxamido)-2-fluorophenyl)(imino)methyl)carbamate **30** |
| 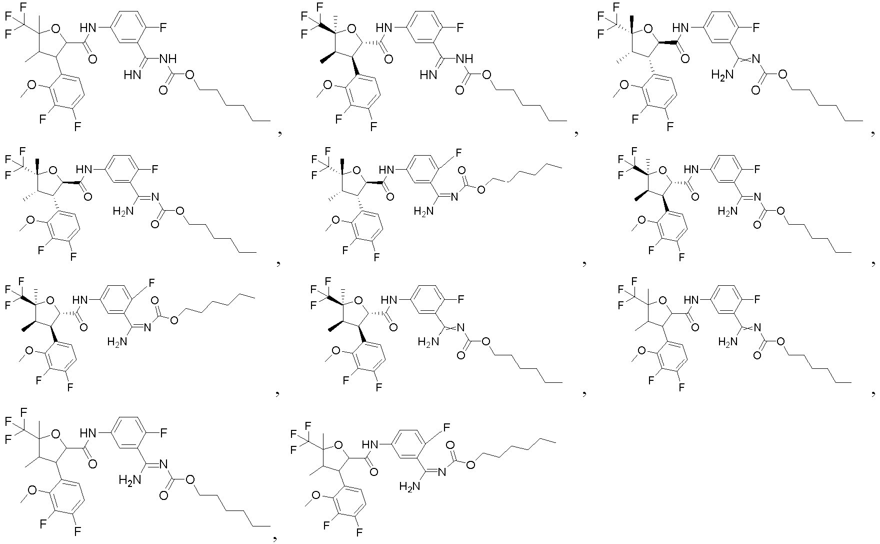 | | |
| **31** | 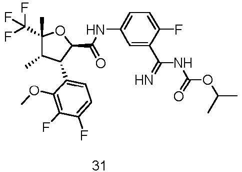 | Isopropyl ((5-((2*R*,3*S*,4*S*,5*R*)-3-(3,4-difluoro-2-methoxyphenyl)-4,5-dimethyl-5-(trifluoromethyl)tetrahydrofuran-2-carboxamido)-2-fluorophenyl)(imino)methyl)carbamate **31** |
| 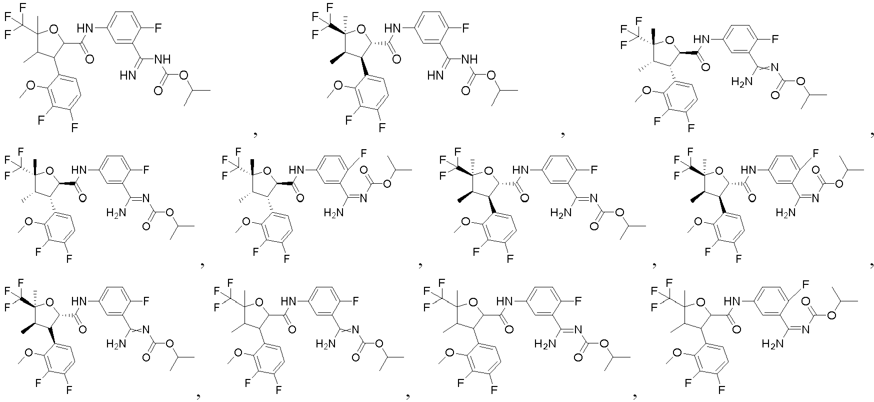 | | |
| **32** | 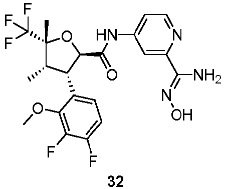 | (2*R*,3*S*,4*S*,5*R*)-3-(3,4-Difluoro-2-methoxyphenyl)-*N*-(2-((*Z*)-(*N*'-hydroxycarbamimidoyl)pyridin-4-yl)-4,5-dimethyl-5-(trifluoromethyl)tetrahydrofuran-2-carboxamide **32** |
| 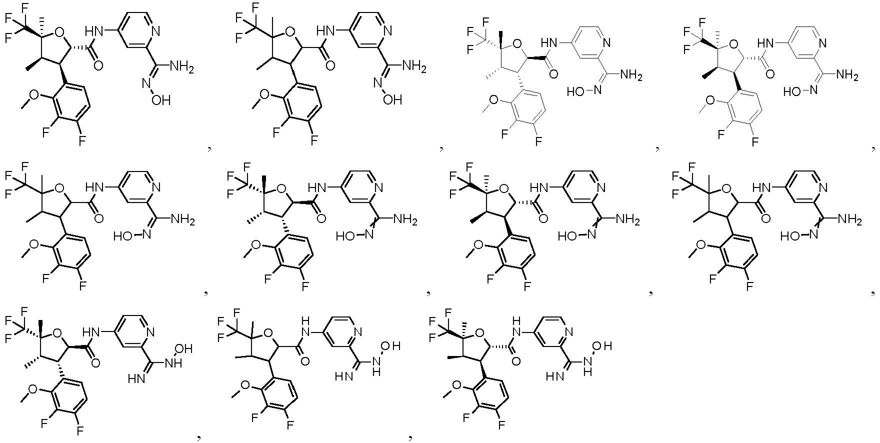 | | |
| **33** | 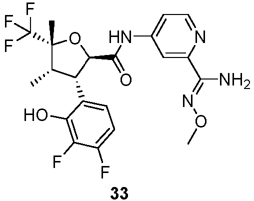 | (2*R*,3*S*,4*S*,5*R*)-3-(3,4-Difluoro-2-hydroxyphenyl)-*N*-(2-((*Z*)-*N*'-methoxycarbamimidoyl)pyridin-4-yl)-4,5-dimethyl-5-(trifluoromethyl)tetrahydrofuran-2-carboxamide **33** |
| 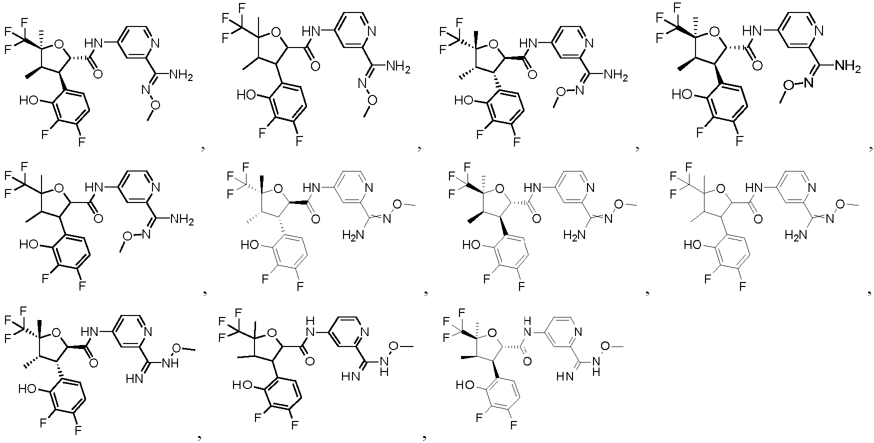 | | |
| **34** | 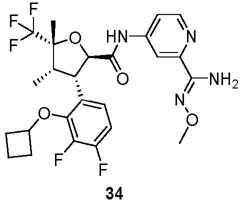 | (2*R*,3*S*,4*S*,5*R*)-3-(2-Cyclobutoxy-3,4-difluorophenyl)-*N*-(2-((*Z*)-(*N*'-methoxycarbamimidoyl)pyridin-4-yl)-4,5-dimethyl-5-(trifluoromethyl)tetrahydrofuran-2-carboxamide **34** |
| 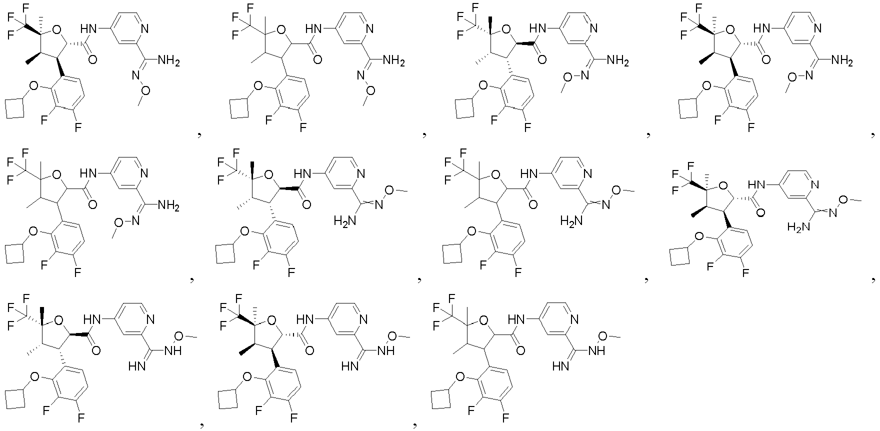 | | |
| 35 | 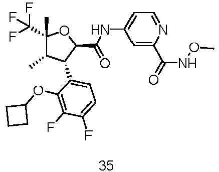 | 4-((2*R*,3*S*,4*S*,5*R*)-3-(2-Cyclobutoxy-3,4-difluorophenyl)-4,5-dimethyl-5-(trifluoromethyl)tetrahydrofuran-2-carboxamido)-N-methoxypicolinamide 35 |
| 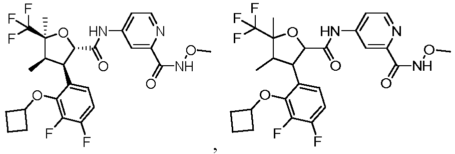 | | |
| 36 | 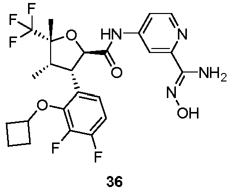 | (2*R*,3*S*,4*S*,5*R*)-3-(2-Cyclobutoxy-3,4-difluorophenyl)-*N*-(2-((*Z*)-(*N'-*hydroxycarbamimidoyl)pyridin-4-yl)-4,5-dimethyl-5-(trifluoromethyl)tetrahydrofuran-2-carboxamide 36 |
| 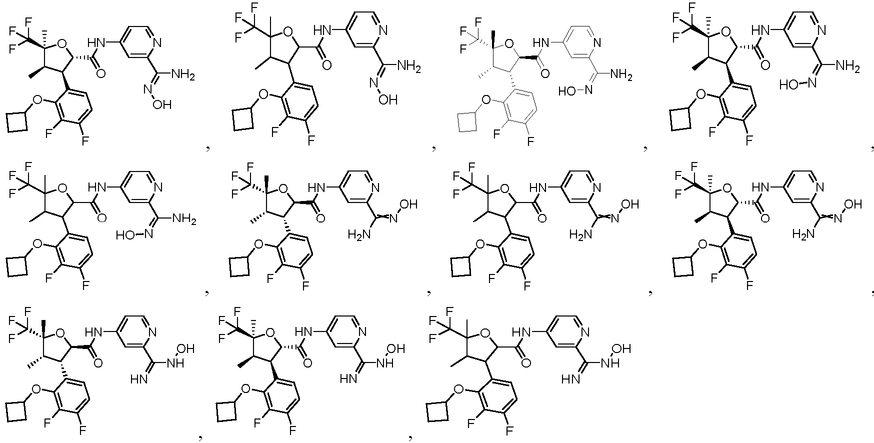 | | |
| **37** | 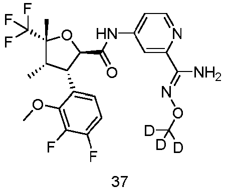 | (2*R*,3*S*,4*S*,5*R*)-3-(3,4-Difluoro-2-methoxyphenyl)-*N*-(2-((*Z*)-(*N*'-methoxy-d₃)carbamimidoyl)pyridin-4-yl)-4,5-dimethyl-5-(trifluoromethyl)tetrahydrofuran-2-carboxamide **37** |
| 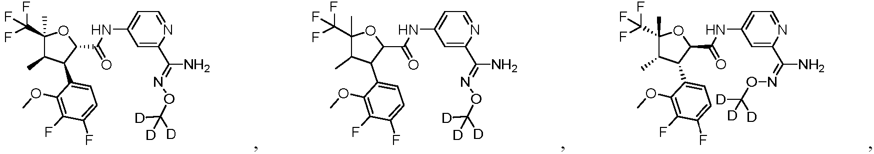 | | |
| 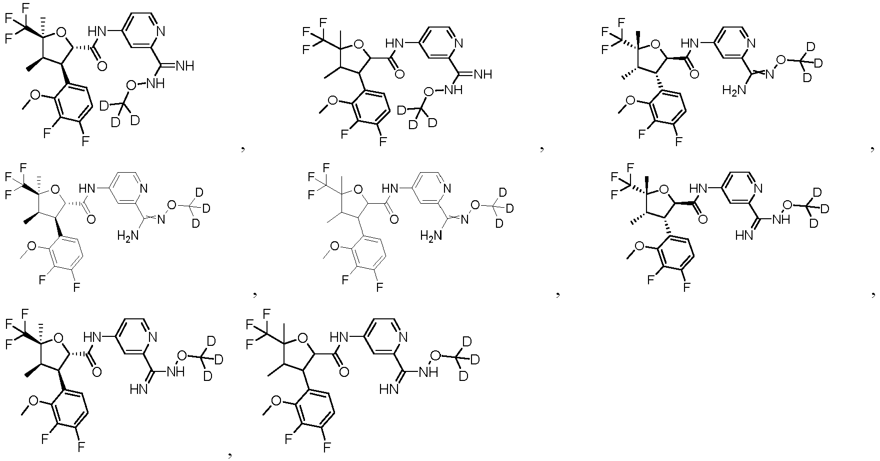 | | |
| | 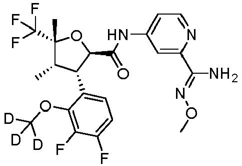 | |
| 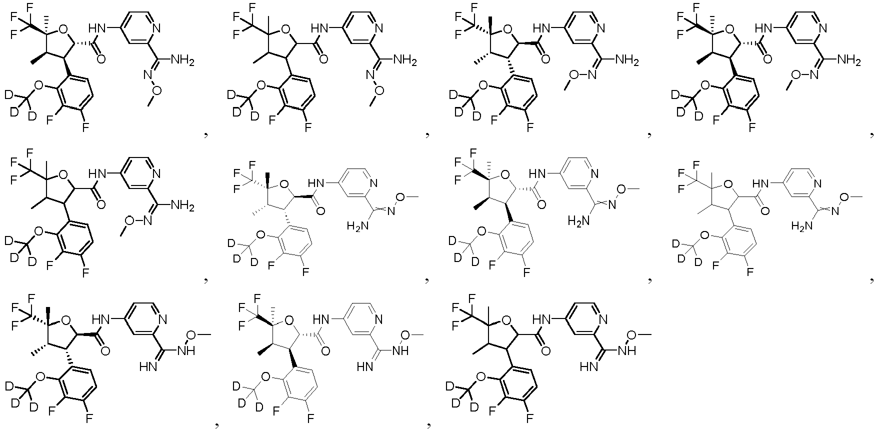 | | |
| | 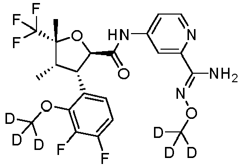 | |
| 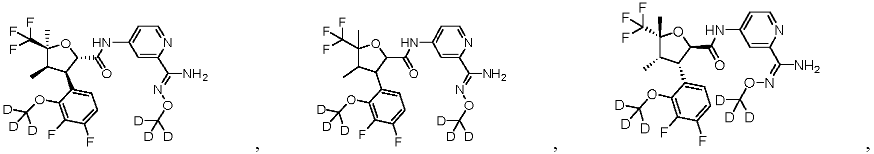 | | |
| 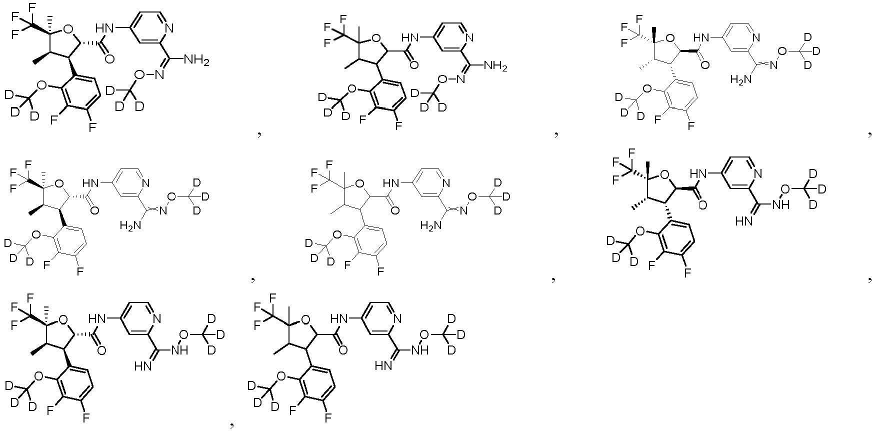 | | |
| **38** | 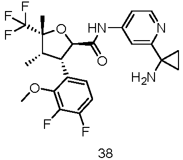 | (2*R*,3*S,*4*S*,5*R*)-*N*-(2-(1-Aminocyclopropyl)pyridin-4-yl)-3-(3,4-difluoro-2-methoxyphenyl)-4,5-dimethyl-5-(trifluoromethyl)tetrahydrofuran-2-carboxamide **38** |
| 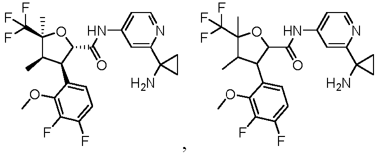 | | |
| **39** | 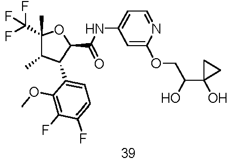 | (2*R*,3*S*,4*S*,5*R*)-3-(3,4-Difluoro-2-methoxyphenyl)-*N*-(2-(2-hydroxy-2-(1-hydroxycyclopropyl)ethoxy)pyridin-4-yl)-4,5-dimethyl-5-(trifluoromethyl)tetrahydrofuran-2-carboxamide **39** (a mixture of diastereomers) |
| 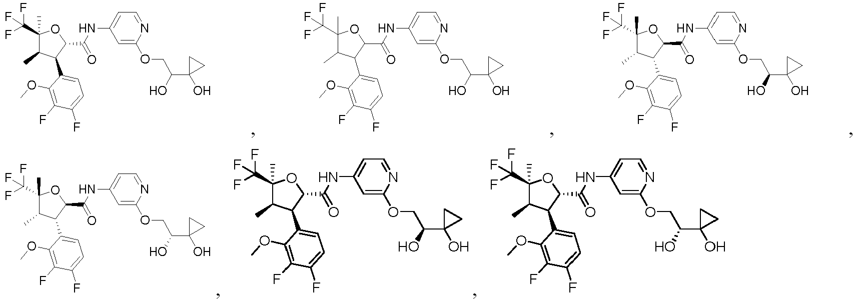 | | |
| **40** | 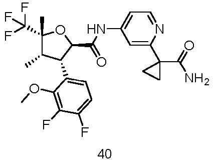 | (2*R*,3*S*,4*S*,5*R*)-*N*-(2-(1-Carboxamidocyclopropyl)pyridin-4-yl)-3-(3,4-difluoro-2-methoxyphenyl)-4,5-dimethyl-5-(trifluoromethyl)tetrahydrofuran-2-carboxamide **40** |
| 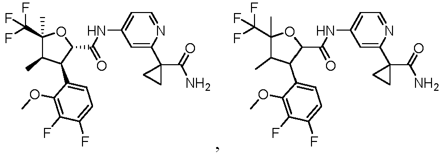 | | |
| **41** | 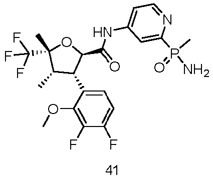 | (2*R*,3*S*,4*S*,5*R*)*-N*-(2-(Amino(methyl)phosphoryl)pyridin-4-yl)-3-(3,4-difluoro-2-methoxyphenyl)-4,5-dimethyl-5-(trifluoromethyl)tetrahydrofuran-2-carboxamide **41** (a mixture of diastereomers) |
| 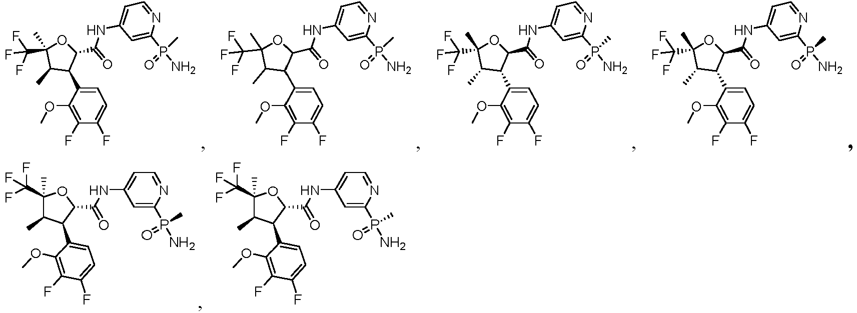 | | |
| **42** | 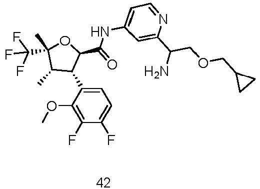 | (2*R*,3*S*,4*S*,5*R*))-*N*-(2-(1-Amino-2-(cyclopropylmethoxy)ethyl)pyridin-4-yl)-3-(3,4-difluoro-2-methoxyphenyl)-4,5-dimethyl-5-(trifluoromethyl)tetrahydrofuran-2-carboxamide **42** (a mixture of diastereomers) |
| 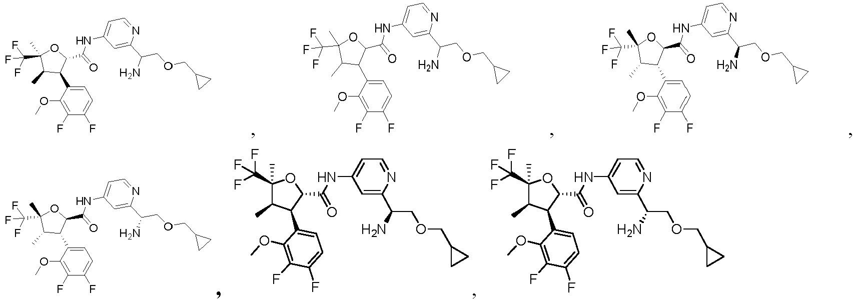 | | |
| **43** | 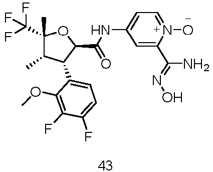 | 4-((2*R*,3*S*,4*S*,5*R*)-3-(3,4-Difluoro-2-methoxyphenyl)-4,5-dimethyl-5-(trifluoromethyl)tetrahydrofuran-2-carboxamido)-2-((*Z*)-*N*'-hydroxycarbamimidoyl)pyridine 1-oxide **43** |
| 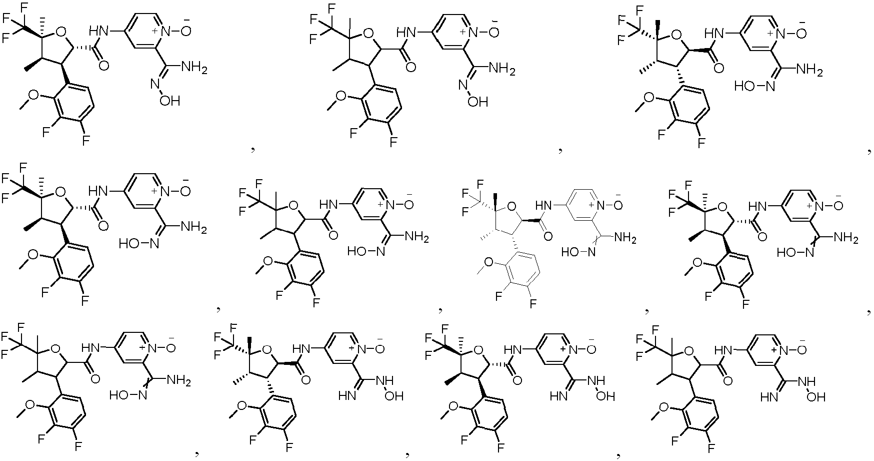 | | |
| **44** | 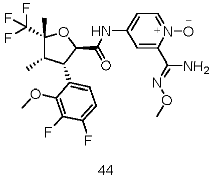 | 4-((2*R*,3*S*,4*S*,5*R*)-3-(3,4-Difluoro-2-methoxyphenyl)-4,5-dimethyl-5-(trifluoromethyl)tetrahydrofuran-2-carboxamido)-2-((*Z*)-*N*'-methoxycarbamimidoyl)pyridine 1-oxide 44 |
| 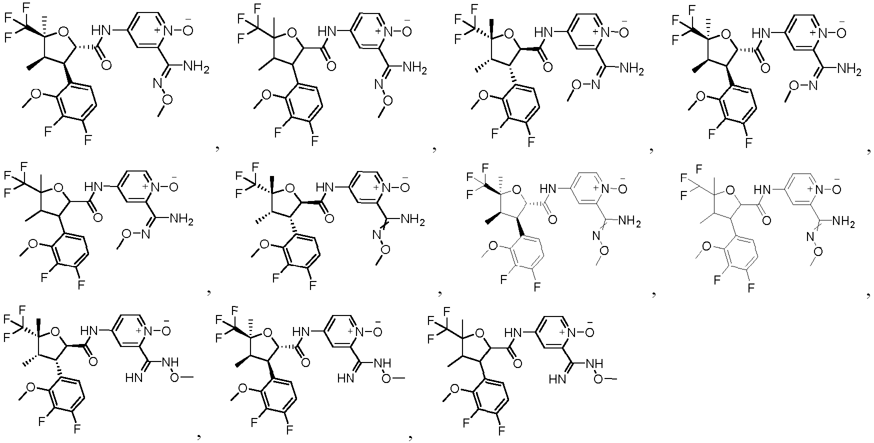 | | |
| **45** | 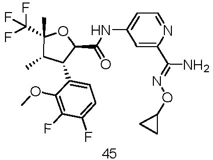 | (2*R*,3*S*,4*S*,5*R*)-*N*-(2-((*Z*)-(*N*'-Cyclopropoxycarbamimidoyl)pyridin-4-yl)-3-(3,4-difluoro-2-methoxyphenyl)-4,5-dimethyl-5-(trifluoromethyl)tetrahydrofuran-2-carboxamide 45 |
| 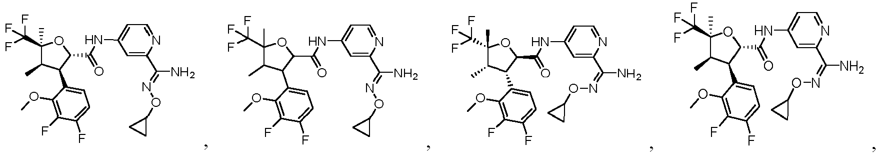 | | |
| 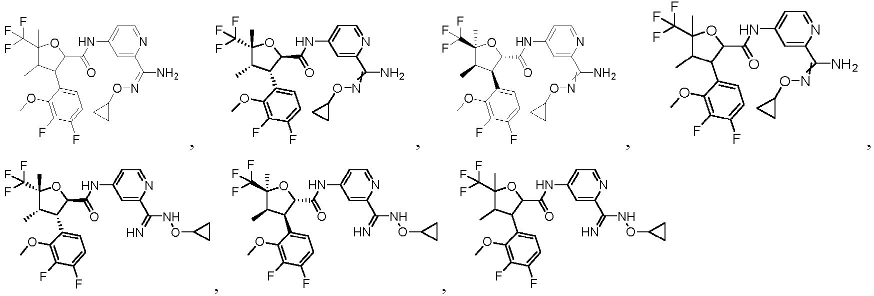 | | |
| **46** | 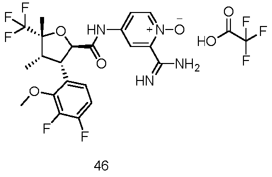 | 2-Carbamimidoyl-4-((2*R*,3*S*,4*S*,5*R*)-3-(3,4-difluoro-2-methoxyphenyl)-4,5-dimethyl-5-(trifluoromethyl)tetrahydrofuran-2-carboxamido)pyridine 1-oxide 2,2,2-trifluoroacetate **46** |
| 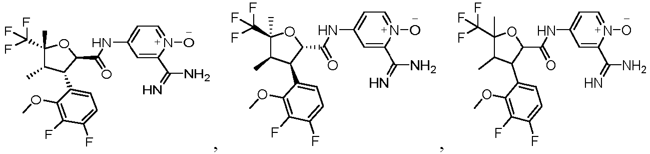 | | |
| **47** | 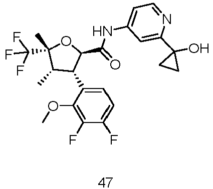 | (2*R*,3*S*,4*S*,5*R*)-3-(3,4-Difluoro-2-methoxyphenyl)-*N*-(2-(1-hydroxycyclopropyl)pyridin-4-yl)-4,5-dimethyl-5-(trifluoromethyl)tetrahydrofuran-2-carboxamide **47** |
| 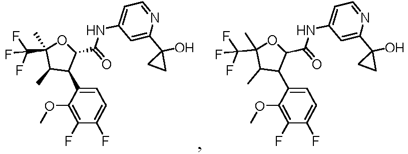 | | |
| | 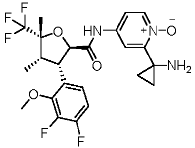 | 2-(1-Aminocyclopropyl)-4-((2*R*,3*S*,4*S*,5*R*)-3-(3,4-difluoro-2-methoxyphenyl)-4,5-dimethyl-5-(trifluoromethyl)tetrahydrofuran-2-carboxamido)pyridine 1-oxide |
| 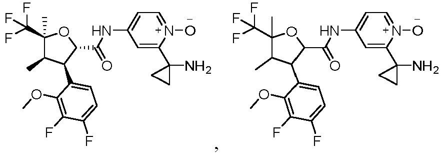 | | |

Another aspect of the present disclosure relates to a compound represented by general formula (IIA') or a salt thereof, 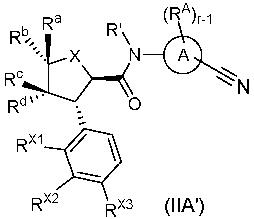 wherein ring A, R^{A}, R', X, R^{a}, R^{b}, R^{c}, R^{d}, R^{X1}, R^{X2}, R^{X3}, and r-1 are as defined in general formula (II').

Another aspect of the present disclosure relates to a compound represented by general formula (IIa') or a salt thereof, 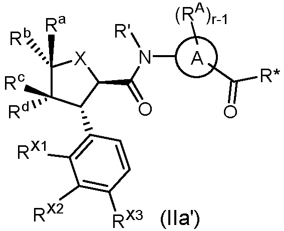
wherein R* is selected from the group consisting of hydroxy, alkoxy, and halogen;
ring A, R^{A}, R', X, R^{a}, R^{b}, R^{c}, R^{d}, R^{X1}, R^{X2}, R^{X3}, and r-1 are as defined in general formula (II').

Another aspect of the present disclosure relates to a compound represented by general formula (IIIA) or a salt thereof, 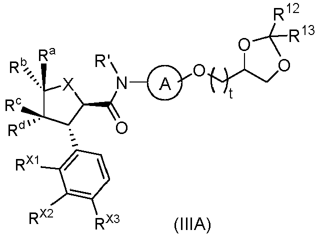
wherein t is selected from the group consisting of 0, 1, 2, 3, and 4;
R¹² and R¹³ are identical or different and are each independently selected from the group consisting of a hydrogen atom, alkyl, cycloalkyl, and phenyl; preferably, R¹² and R¹³ are identical or different and are each independently selected from the group consisting of a hydrogen atom, C₁₋₆ alkyl, and 5- or 6-membered cycloalkyl; most preferably, R¹² and R¹³ are both CH₃;
ring A, R', R^{a}, R^{b}, R^{c}, R^{d}, X, R^{X1}, R^{X2}, and R^{X3} are as defined in general formula (III).

Another aspect of the present disclosure relates to a compound represented by general formula (IIIA-1) or a salt thereof, 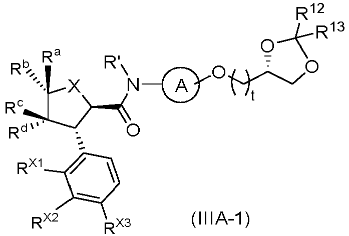
wherein t is selected from the group consisting of 0, 1, 2, 3, and 4;
R¹² and R¹³ are identical or different and are each independently selected from the group consisting of a hydrogen atom, alkyl, cycloalkyl, and phenyl; preferably, R¹² and R¹³ are identical or different and are each independently selected from the group consisting of a hydrogen atom, C₁₋₆ alkyl, and 5- or 6-membered cycloalkyl; most preferably, R¹² and
R¹³ are both CH₃;
ring A, R', R^{a}, R^{b}, R^{c}, R^{d}, X, R^{X1}, R^{X2}, and R^{X3} are as defined in general formula (III-1).

Another aspect of the present disclosure relates to a compound represented by general formula (IVA') or a salt thereof, 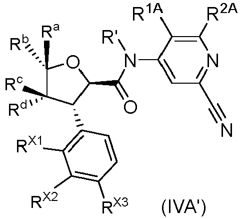 wherein R^{1A}, R^{2A}, R', R^{a}, R^{b}, R^{c}, R^{d}, R^{X1}, R^{X2}, and R^{X3} are as defined in general formula (IV).

Another aspect of the present disclosure relates to a compound represented by general formula (IVa') or a salt thereof, 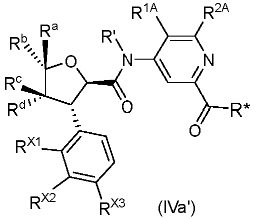
wherein R* is selected from the group consisting of hydroxy, alkoxy, and halogen;
R^{1A}, R^{2A}, R', R^{a}, R^{b}, R^{c}, R^{d}, R^{X1}, R^{X2}, and R^{X3} are as defined in general formula (IV).

Another aspect of the present disclosure relates to a compound represented by general formula (VI'A) or a salt thereof, 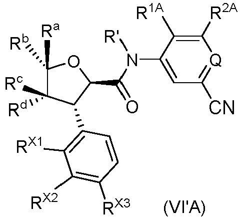 wherein Q, R^{1A}, R^{2A}, R', R^{a}, R^{b}, R^{c}, R^{d}, R^{X1}, R^{X2}, and R^{X3} are as defined in general formula (VI').

Another aspect of the present disclosure relates to a compound represented by general formula (VII'A) or a salt thereof, 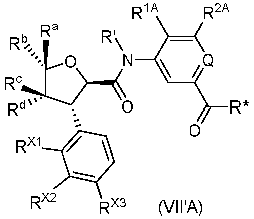
wherein R* is selected from the group consisting of hydroxy, alkoxy, and halogen;
Q, R^{1A}, R^{2A}, R', R^{a}, R^{b}, R^{c}, R^{d}, R^{X1}, R^{X2}, and R^{X3} are as defined in general formula (VII').

Another aspect of the present disclosure relates to a compound represented by general formula (VIIIA) or a salt thereof, 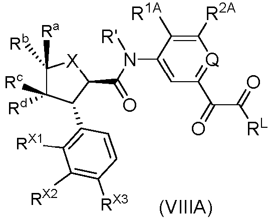
wherein R^{L} is selected from the group consisting of halogen, hydroxy, and alkoxy;
Q, R^{1A}, R^{2A}, R', X, R^{a}, R^{b}, R^{c}, R^{d}, R^{X1}, R^{X2}, and R^{X3} are as defined in general formula (VIII).

**Table B. Typical intermediate compounds of the present disclosure include, but are not limited to:**

| Compound No. | Compound structure | Name |
|---|---|---|
| **2d** | 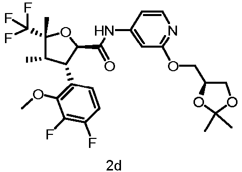 | (2*R*,3*S*,4*S*,5*R*)-3-(3,4-Difluoro-2-methoxyphenyl)-*N*-(2-((*S*)-2,2-dimethyl-1,3-dioxolan-4-yl)methoxy)pyridin-4-yl)-4,5-dimethyl-5-(trifluoromethyl)tetrahydrofuran-2-carboxamide **2d** |
| | 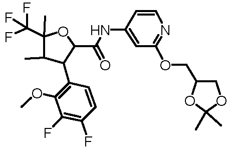 | 3-(3,4-Difluoro-2-methoxyphenyl)-*N*-(2-(2,2-dimethyl-1,3-dioxolan-4-yl)methoxy)pyridin-4-yl)-4,5-dimethyl-5-(trifluoromethyl)tetrahydrofuran-2-carboxamide |
| | 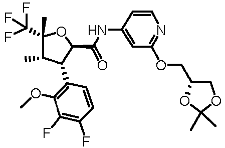 | (2*R*,3*S*,4*S*,5*R*)-3-(3,4-Difluoro-2-methoxyphenyl)-*N*-(2-((*R*)-2,2-dimethyl-1,3-dioxolan-4-yl)methoxy)pyridin-4-yl)-4,5-dimethyl-5-(trifluoromethyl)tetrahydrofuran-2-carboxamide |
| | 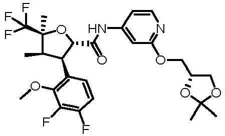 | (2*S*,3*R*,4*R*,5*S*)-3-(3,4-Difluoro-2-methoxyphenyl)-*N*-(2-((*R*)-2,2-dimethyl-1,3-dioxolan-4-yl)methoxy)pyridin-4-yl)-4,5-dimethyl-5-(trifluoromethyl)tetrahydrofuran-2-carboxamide |
| | 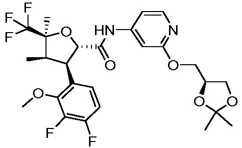 | (2*S*,3*R*,4*R*,5*S*)-3-(3,4-Difluoro-2-methoxyphenyl)-*N*-(2-((*S*)-2,2-dimethyl-1,3-dioxolan-4-yl)methoxy)pyridin-4-yl)-4,5-dimethyl-5-(trifluoromethyl)tetrahydrofuran-2-carboxamide |
| **5a** | 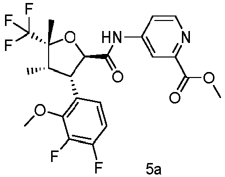 | Methyl 4-((2*R*,3*S*,4*S*,5*R*)-3-(3,4-difluoro-2-methoxyphenyl)-4,5-dimethyl-5-(trifluoromethyl)tetrahydrofuran-2-carboxamido)picolinate **5a** |
| | 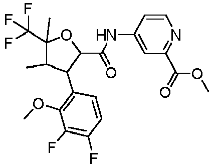 | Methyl 4-(3-(3,4-difluoro-2-methoxyphenyl)-4,5-dimethyl-5-(trifluoromethyl)tetrahydrofuran-2-carboxamido)picolinate |
| | 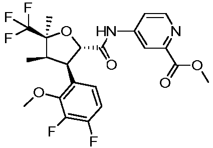 | Methyl 4-((2*S*,3*R*,4*R*,5*S*)-3-(3,4-difluoro-2-methoxyphenyl)-4,5-dimethyl-5-(trifluoromethyl)tetrahydrofuran-2-carboxamido)picolinate |
| **6e** | 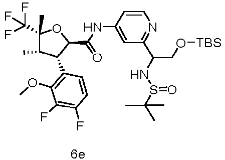 | (2*R*,3*S*,4*S*,5*R*)-*N*-(2-(2-((tert-Butyldimethylsilyl)oxy)-1-((tert-butylsulfinyl)amino)ethyl)pyridin-4-yl)-3-(3,4-difluoro-2-methoxyphenyl)-4,5-dimethyl-5-(trifluoromethyl)tetrahydrofuran-2-carboxamide **6e** |
| | 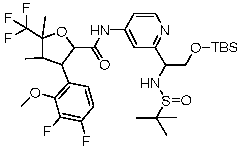 | *N*-(2-(2-((tert-Butyldimethylsilyl)oxy)-1-((tert-butylsulfinyl)amino)ethyl)pyridin-4-yl)-3-(3,4-difluoro-2-methoxyphenyl)-4,5-dimethyl-5-(trifluoromethyl)tetrahydrofuran-2-carboxamide |
| | 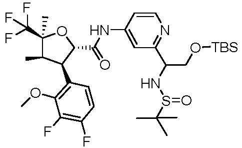 | (2*S*,3*R*,4*R*,5*S*)-*N*-(2-(2-((tert-Butyldimethylsilyl)oxy)-1-((tert-butylsulfinyl)amino)ethyl)pyridin-4-yl)-3-(3,4-difluoro-2-methoxyphenyl)-4,5-dimethyl-5-(trifluoromethyl)tetrahydrofuran-2-carboxamide |
| | 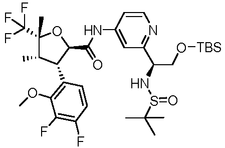 | (2*R*,3*S*,4*S*,5*R*)-*N*-(2-((1*S*)-2-((tert-Butyldimethylsilyl)oxy)-1-((tert-butylsulfinyl)amino)ethyl)pyridin-4-yl)-3-(3,4-difluoro-2-methoxyphenyl)-4,5-dimethyl-5-(trifluoromethyl)tetrahydrofuran-2-carboxamide |
| | 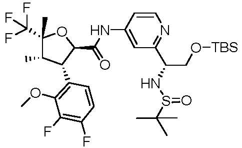 | (2*R*,3*S*,4*S*,5*R*)-*N*-(2-((1*R*)-2-((tert-Butyldimethylsilyl)oxy)-1-((tert-butylsulfinyl)amino)ethyl)pyridin-4-yl)-3-(3,4-difluoro-2-methoxyphenyl)-4,5-dimethyl-5-(trifluoromethyl)tetrahydrofuran-2-carboxamide |
| **6f-1** | 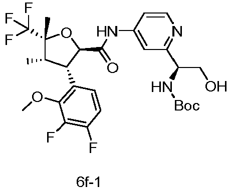 | tert-Butyl ((*S*)-(1-(4-((2*R*,3*S*,4*S*,5*R*)-3-(3,4-difluoro-2-methoxyphenyl)-4,5-dimethyl-5-(trifluoromethyl)tetrahydrofuran-2-carboxamido)pyridin-2-yl)-2-hydroxyethyl)carbamate **6f-1** |
| **6f** | 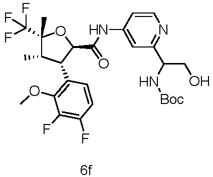 | tert-Butyl (1-(4-((2*R*,3*S*,4*S*,5*R*)-3-(3,4-difluoro-2-methoxyphenyl)-4,5-dimethyl-5-(trifluoromethyl)tetrahydrofuran-2-carboxamido)pyridin-2-yl)-2-hydroxyethyl)carbamate **6f** |
| **6f-2** | 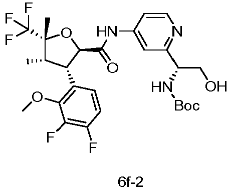 | tert-Butyl ((*R*)-(1-(4-((2*R*,3*S*,4*S*,5*R*)-3-(3,4-difluoro-2-methoxyphenyl)-4,5-dimethyl-5-(trifluoromethyl)tetrahydrofuran-2-carboxamido)pyridin-2-yl)-2-hydroxyethyl)carbamate **6f-2** |
| 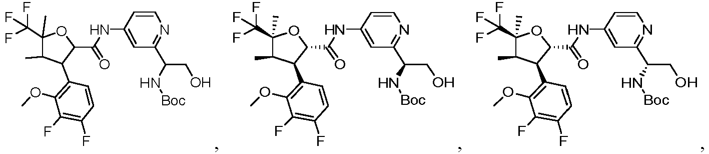 | | |
| 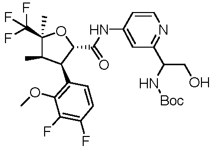 | | |
| **7b** | 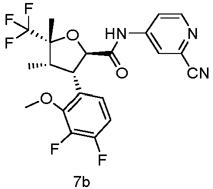 | (2*R*,3*S*,4*S*,5*R*)-*N*-(2-Cyanopyridin-4-yl)-3-(3,4-difluoro-2-methoxyphenyl)-4,5-dimethyl-5-(trifluoromethyl)tetrahydrofuran-2-carboxamide **7b** |
| | 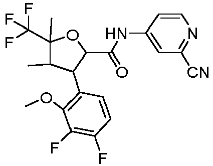 | *N*-(2-Cyanopyridin-4-yl)-3-(3,4-difluoro-2-methoxyphenyl)-4,5-dimethyl-5-(trifluoromethyl)tetrahydrofuran-2-carboxamide |
| | 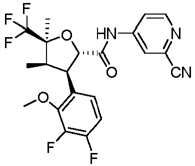 | (2*S*,3*R*,4*R*,5*S*)-*N*-(2-Cyanopyridin-4-y1)-3-(3,4-difluoro-2-methoxyphenyl)-4,5-dimethyl-5-(trifluoromethyl)tetrahydrofuran-2-carboxamide |
| **9a** | 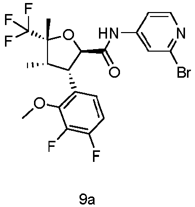 | (2*R*,3*S*,4*S*,5*R*)-*N*-(2-Bromopyridin-4-yl)-3-(3,4-difluoro-2-methoxyphenyl)-4,5-dimethyl-5-(trifluoromethyl)tetrahydrofuran-2-carboxamide **9a** |
| | 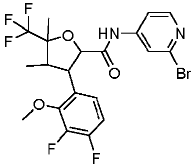 | *N*-(2-Bromopyridin-4-yl)-3-(3,4-difluoro-2-methoxyphenyl)-4,5-dimethyl-5-(trifluoromethyl)tetrahydrofuran-2-carboxamide |
| | 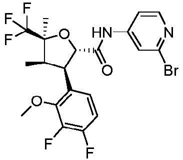 | (2*S*,3*R*,4*R*,5*S*)-*N*-(2-Bromopyridin-4-yl)-3-(3,4-difluoro-2-methoxyphenyl)-4,5-dimethyl-5-(trifluoromethyl)tetrahydrofuran-2-carboxamide |
| **9b** | 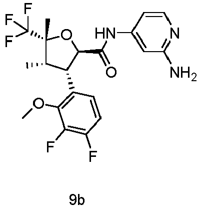 | (2*R*,3*S*,4*S*,5*R*)-*N*-(2-Aminopyridin-4-yl)-3-(3,4-difluoro-2-methoxyphenyl)-4,5-dimethyl-5-(trifluoromethyl)tetrahydrofuran-2-carboxamide **9b** |
| | 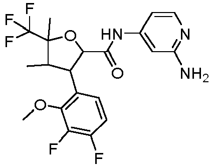 | *N*-(2-Aminopyridin-4-yl)-3-(3,4-difluoro-2-methoxyphenyl)-4,5-dimethyl-5-(trifluoromethyl)tetrahydrofuran-2-carboxamide |
| | 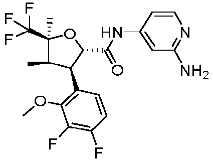 | (2*S*,3*R,*4*R*,5*S*)-*N*-(2-Aminopyridin-4-yl)-3-(3,4-difluoro-2-methoxyphenyl)-4,5-dimethyl-5-(trifluoromethyl)tetrahydrofuran-2-carboxamide |
| **9c** | 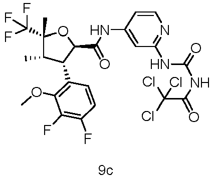 | (2*R*,3*S*,4*S*,5*R*)-3-(3,4-Difluoro-2-methoxyphenyl)-4,5-dimethyl-*N*-(2-(3-(2,2,2-trichloroacetyl)ureido)pyridin-4-yl)-5-(trifluoromethyl)tetrahydrofuran-2-carboxamide **9c** |
| | 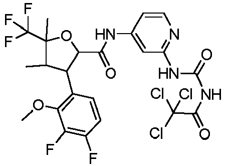 | 3-(3,4-Difluoro-2-methoxyphenyl)-4,5-dimethyl-*N*-(2-(3 -(2,2,2-trichloroacetyl)ureido)pyridin-4-yl)-5-(trifluoromethyl)tetrahydrofuran-2-carboxamide |
| | 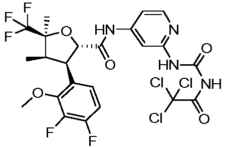 | (2*S*,3*R*,4*R*,5*S*)-3-(3,4-Difluoro-2-methoxyphenyl)-4,5-dimethyl-*N*-(2-(3-(2,2,2-trichloroacetyl)ureido)pyridin-4-yl)-5-(trifluoromethyl)tetrahydrofuran-2-carboxamide |
| **12b** | 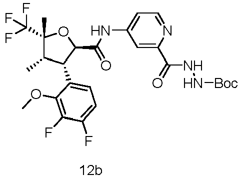 | tert-Butyl 2-(4-((2*R*,3*S*,4*S*,5*R*)-3-(3,4-difluoro-2-methoxyphenyl)-4,5-dimethyl-5-(trifluoromethyl)tetrahydrofuran-2-carboxamido)picolinoyl)hydrazine-1-carboxylate **12b** |
| 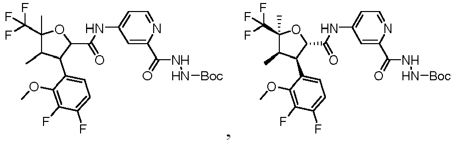 | | |
| **19a** | 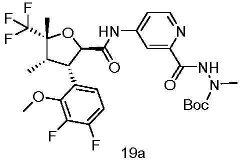 | tert-Butyl 2-(4-((2*R*,3*S*,4*S*,5*R*)-3-(3,4-difluoro-2-methoxyphenyl)-4,5-dimethyl-5-(trifluoromethyl)tetrahydrofuran-2-carboxamido)picolinoyl)-1-methylhydrazine-1-carboxylate **19a** |
| 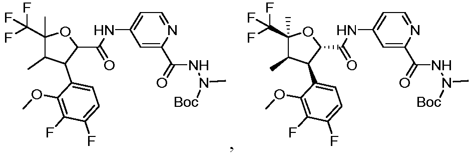 | | |
| **26b** | 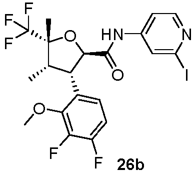 | (2*R*,3*S*,4*S*,5*R*)-3-(3,4-Difluoro-2-methoxyphenyl)-*N*-(2-iodopyridin-4-yl)-4,5-dimethyl-5-(trifluoromethyl)tetrahydrofuran-2-carboxamide **26b** |
| | 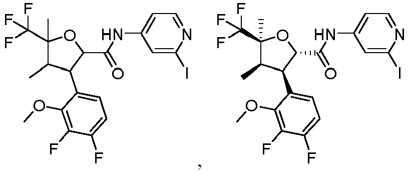 | |
| **26c** | 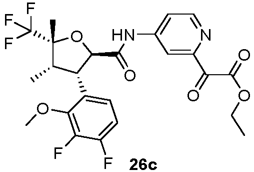 | Ethyl 2-(4-((2*R*,3*S*,4*S*,5*R*)-3-(3,4-difluoro-2-methoxyphenyl)-4,5-dimethyl-5-(trifluoromethyl)tetrahydrofuran-2-carboxamido)pyridin-2-yl)-2-oxoacetate **26c** |
| 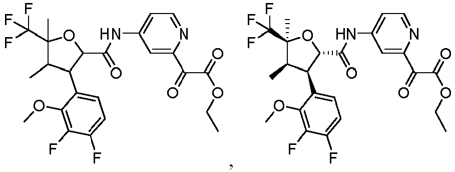 | | |
| **27b** | 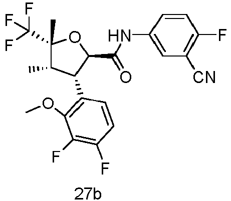 | (2*R*,3*S*,4*S*,5*R*)-*N*-(3-Cyano-4-fluorophenyl)-3-(3,4-difluoro-2-methoxyphenyl)-4,5-dimethyl-5-(trifluoromethyl)tetrahydrofuran-2-carboxamide **27b** |
| 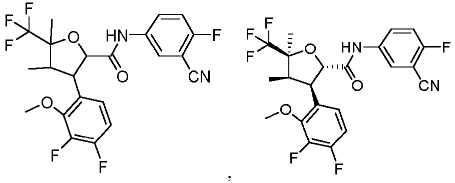 | | |
| **34d** | 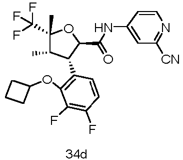 | (2*R*,3*S*,4*S*,5*R*)-*N*-(2-Cyanopyridin-4-yl)-3-(2-cyclobutoxy-3,4-difluorophenyl)-4,5-dimethyl-5-(trifluoromethyl)tetrahydrofuran-2-carboxamide **34d** |
| 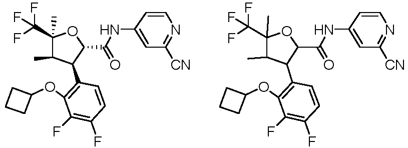 | | |
| **39g** | 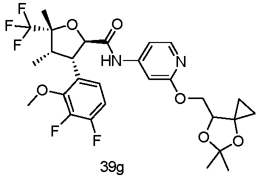 | (2*R*,3*S*,4*S*,5*R*)-3-(3,4-Difluoro-2-methoxyphenyl)-*N*-(2-((5,5-dimethyl-4,6-dioxaspiro [2.4]hept-7-yl)methoxy)pyridin-4-yl)-4,5-dimethyl-5-(trifluoromethyl)tetrahydrofuran-2-carboxamide **39g** (a mixture of diastereomers) |
| 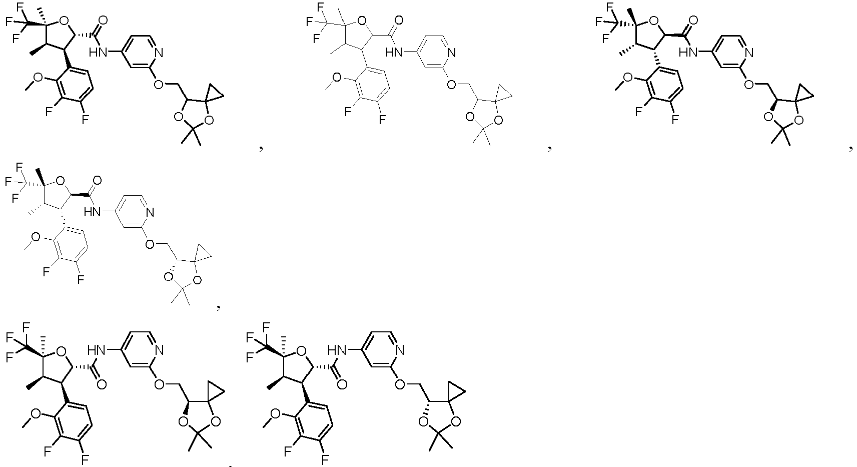 | | |
| **41a** | 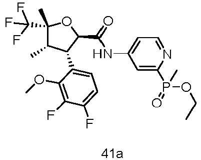 | Ethyl (4-((2*R*,3*S*,4*S*,5*R*)-3-(3,4-difluoro-2-methoxyphenyl)-4,5-dimethyl-5-(trifluoromethyl)tetrahydrofuran-2-carboxamido)pyridin-2-yl)(methyl)phosphinate **41a** (a mixture of diastereomers) |
| 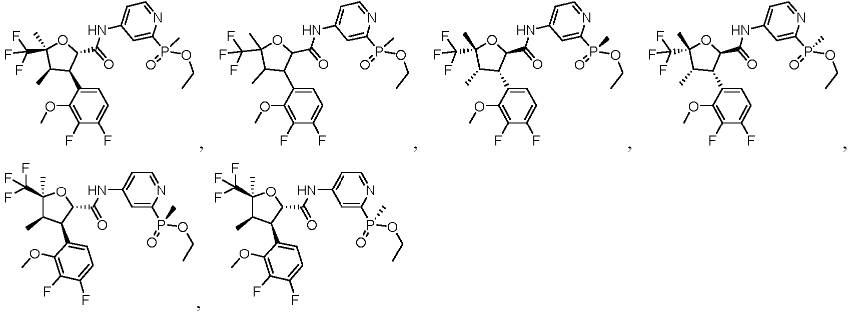 | | |
| **42j** | 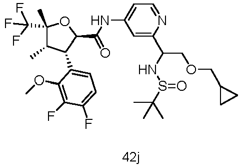 | (2*R*,3*S*,4*S*,5*R*)-*N-*(2-(1-((tert-Butylsulfinyl)amino)-2-(cyclopropylmethoxy)ethyl)pyridin-4-yl)-3-(3,4-difluoro-2-methoxyphenyl)-4,5-dimethyl-5-(trifluoromethyl)tetrahydrofuran-2-carboxamide **42j** (a mixture of diastereomers) |
| 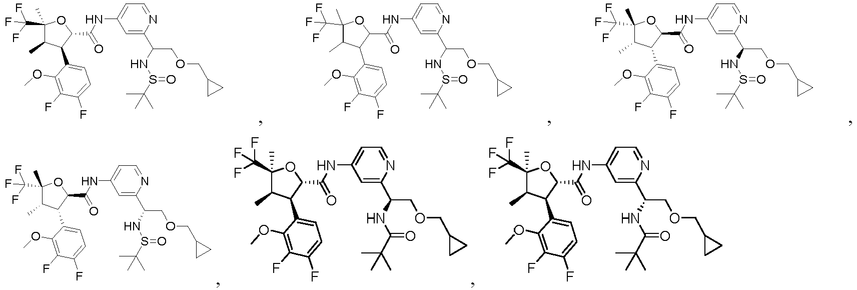 | | |
| **43a** | 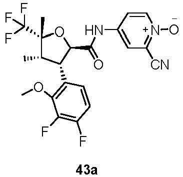 | 2-Cyano-4-((2*R*,3*S*,4*S*,5*R*)-)-3-(3,4-difluoro-2-methoxyphenyl)-4,5-dimethyl-5-(trifluoromethyl)tetrahydrofuran-2-carboxamido)pyridine 1-oxide **43a** |
| 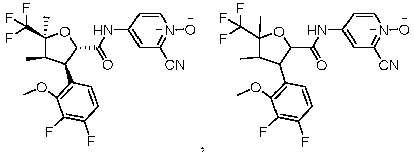 | | |
| **47b** | 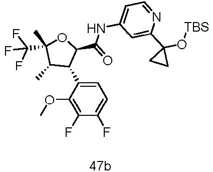 | (2*R*,3*S*,4*S*,5*R*)-*N*-(2-(1-((tert-Butyldimethylsilyl)oxy)cyclopropyl)pyridin-4-yl)-3-(3,4-difluoro-2-methoxyphenyl)-4,5-dimethyl-5-(trifluoromethyl)tetrahydrofuran-2-carboxamide **47b** |
| 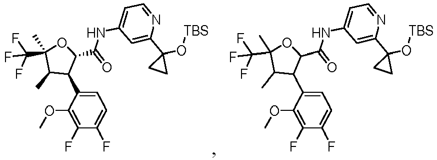 | | |

Another aspect of the present disclosure relates to a method for preparing the compound represented by general formula (I) or the pharmaceutically acceptable salt thereof described above, the method comprising: 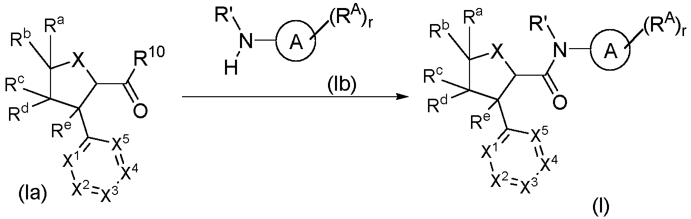 conducting a condensation reaction of a compound represented by general formula (Ia) or a salt thereof with a compound represented by general formula (Ib) or a salt thereof to give the compound represented by general formula (I) or the pharmaceutically acceptable salt thereof, wherein:
R¹⁰ is halogen (preferably a chlorine atom) or OH;
ring A, R^{A}, R', R^{a}, R^{b}, R^{c}, R^{d}, R^{e}, X, X¹, X², X³, X⁴, X⁵, and r are as defined in general formula (I).

Another aspect of the present disclosure relates to a method for preparing the compound represented by general formula (I) or the pharmaceutically acceptable salt thereof described above, the method comprising: 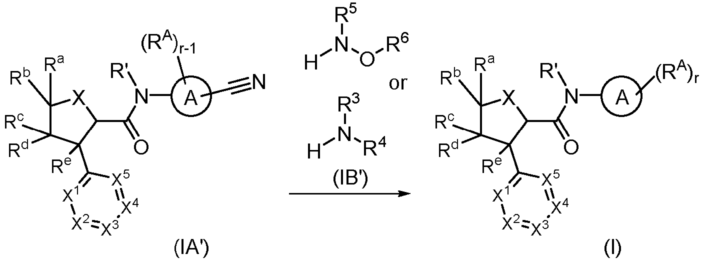 reacting a compound represented by general formula (IA') or a salt thereof with a compound represented by general formula (IB') or a salt thereof (preferably hydrochloride) to give the compound represented by general formula (I) or the pharmaceutically acceptable salt thereof, wherein:
at least one R^{A} in general formula (I) is -C(=NH)NR⁵-OR⁶ or -C(=NH)NR³R⁴;
r is 1, 2, 3, 4, or 5; r-1 is 0, 1, 2, 3, or 4;
the remaining R^{A}s are as defined in general formula (I); ring A, R', R^{a}, R^{b}, R^{c}, R^{d}, R^{e}, X, X¹, X², X³, X⁴, X⁵, R³, R⁴, R⁵*,* and R⁶ are as defined in general formula (I).

Another aspect of the present disclosure relates to a method for preparing the compound represented by general formula (II) or the pharmaceutically acceptable salt thereof described above, the method comprising: 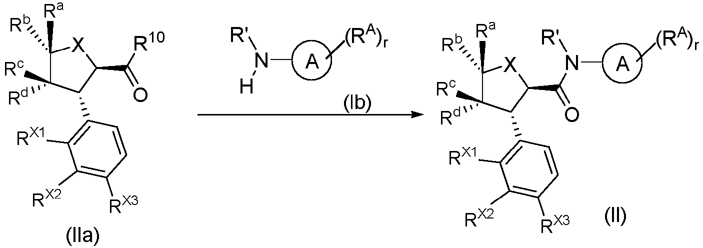 conducting a condensation reaction of a compound represented by general formula (IIa) or a salt thereof with a compound represented by general formula (Ib) or a salt thereof to give the compound represented by general formula (II) or the pharmaceutically acceptable salt thereof, wherein:
R¹⁰ is halogen (preferably a chlorine atom) or OH;
ring A, R^{A}, R', R^{a}, R^{b}, R^{c}, R^{d}, X, R^{X1}, R^{X2}, R^{X3}, and r are as defined in general formula (II).

Another aspect of the present disclosure relates to a method for preparing the compound represented by general formula (II) or the pharmaceutically acceptable salt thereof described above, the method comprising: 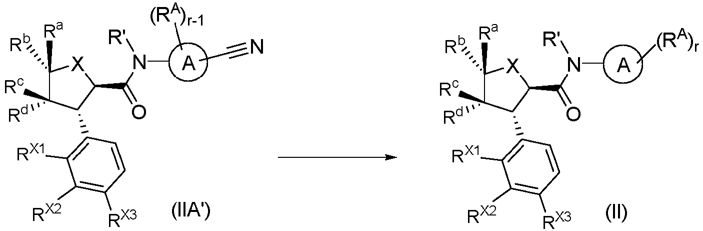 reacting a compound represented by general formula (IIA') or a salt thereof with NH₂-R⁵ or a salt thereof (preferably hydrochloride) to give the compound represented by general formula (II) or the pharmaceutically acceptable salt thereof, wherein:
at least one R^{A} in general formula (II) is -C(=NR⁵)NR³R⁴;
r is 1, 2, 3, 4, or 5; r-1 is 0, 1, 2, 3, or 4;
R³ and R⁴ are both hydrogen atoms; the remaining R^{A}s are as defined in general formula (II);
ring A, R', R^{a}, R^{b}, R^{c}, R^{d}, X, R^{X1}, R^{X2}, R^{X3,} and R⁵ are as defined in general formula (II).

Another aspect of the present disclosure relates to a method for preparing the compound represented by general formula (II) or the pharmaceutically acceptable salt thereof described above, the method comprising: 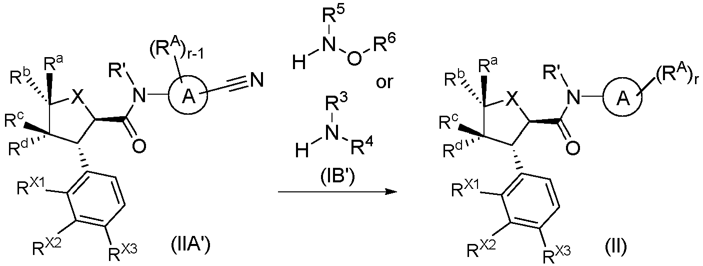 reacting a compound represented by general formula (IIA') or a salt thereof with a compound represented by general formula (IB') or a salt thereof (preferably hydrochloride) to give the compound represented by general formula (II) or the pharmaceutically acceptable salt thereof, wherein:
at least one R^{A} in general formula (II) is -C(=NH)NR⁵-OR⁶ or -C(=NH)NR³R⁴;
r is 1, 2, 3, 4, or 5; r-1 is 0, 1, 2, 3, or 4;
the remaining R^{A}_{S} are as defined in general formula (II);
ring A, R', R^{a}, R^{b}, R^{c}, R^{d}, X, R^{X1}, R^{X2}, R^{X3} R³, R⁴, R⁵, and R⁶ are as defined in general formula (II).

Another aspect of the present disclosure relates to a method for preparing the compound represented by general formula (II) or the pharmaceutically acceptable salt thereof described above, the method comprising: 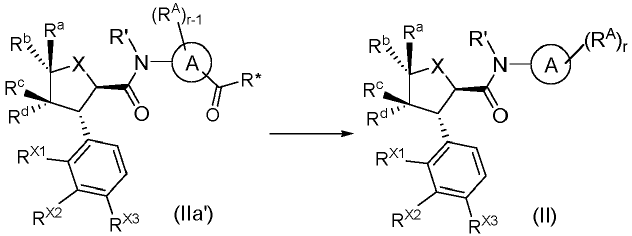 conducting an amidation reaction of a compound represented by general formula (IIa') or a salt thereof with an amine or a salt thereof (preferably hydrochloride) to give the compound represented by general formula (II) or the pharmaceutically acceptable salt thereof, wherein:
the amine described above is selected from the group consisting of HNR⁵-OR⁶, HNR⁵-NR³R⁴, and HNR⁵-alkylene-Cy;
R* is selected from the group consisting of hydroxy, alkoxy, and halogen;
at least one R^{A} in general formula (II) is selected from the group consisting of -C(O)NR⁵-OR⁶, -C(O)NR⁵-NR³R⁴, and -C(O)NR⁵-alkylene-Cy; the remaining R^{A}_{S} are as defined in general formula (II);
r is 1, 2, 3, 4, or 5; r-1 is 0, 1, 2, 3, or 4;
when an amino group comprises a protecting group, the method further comprises a step of removing the protecting group; the amino protecting group is preferably Boc;
ring A, R', X, R^{a}, R^{b}, R^{c}, R^{d}, R^{X1}, R^{X2}, R^{X3} R³, R⁴, R⁵, R⁶, and Cy are as defined in general formula (II).

Another aspect of the present disclosure relates to a method for preparing the compound represented by general formula (II') or the pharmaceutically acceptable salt thereof described above, the method comprising: 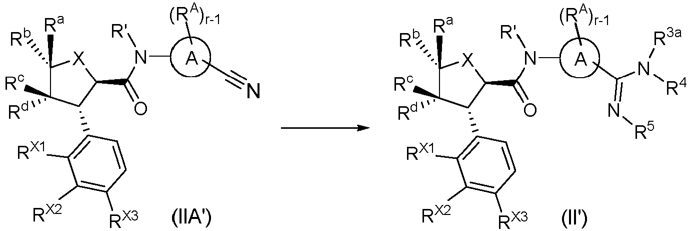 reacting a compound represented by general formula (IIA') or a salt thereof with NH₂-R⁵ or a salt thereof (preferably hydrochloride) to give the compound represented by general formula (II') or the pharmaceutically acceptable salt thereof, wherein:
R^{3a} and R⁴ are both hydrogen atoms;
ring A, R^{A}, R', X, R^{a}, R^{b}, R^{c}, R^{d}, R^{X1}, R^{X2}, R^{X3} R⁵, and r-1 are as defined in general formula (II').

Another aspect of the present disclosure relates to a method for preparing the compound represented by general formula (II') or the pharmaceutically acceptable salt thereof described above, the method comprising: 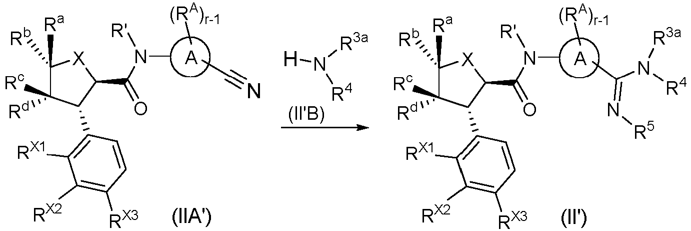 reacting a compound represented by general formula (IIA') or a salt thereof with a compound represented by general formula (II'B) or a salt thereof (preferably hydrochloride) to give the compound represented by general formula (II') or the pharmaceutically acceptable salt thereof, wherein:
R⁵ is a hydrogen atom;
ring A, R^{A}, R', X, R^{a}, R^{b}, R°, R^{d}, R^{X1}, R^{X2}, R^{X3,} R^{3a}, R⁴, and r-1 are as defined in general formula (II').

Another aspect of the present disclosure relates to a method for preparing the compound represented by general formula (II') or the pharmaceutically acceptable salt thereof described above, the method comprising: 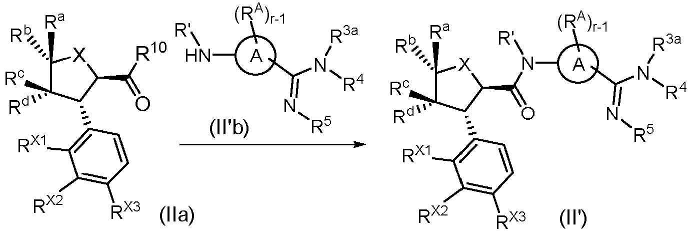 conducting a condensation reaction of a compound represented by general formula (IIa) or a salt thereof with a compound represented by general formula (II'b) or a salt thereof (preferably hydrochloride) to give the compound represented by general formula (II') or the pharmaceutically acceptable salt thereof, wherein:
R¹⁰ is halogen (preferably a chlorine atom) or OH;
ring A, R^{A}, R', X, R^{a}, R^{b}, R^{c}, R^{d}, R^{X1}, R^{X2}, R^{X3} R^{3a}, R⁴, R⁵, and r-1 are as defined in general formula (II').

Another aspect of the present disclosure relates to a method for preparing the compound represented by general formula (II-1) or the pharmaceutically acceptable salt thereof described above, the method comprising: 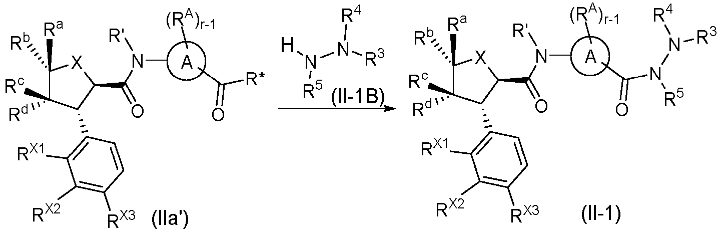 reacting a compound represented by general formula (IIa') or a salt thereof with a compound represented by general formula (II-1B) or a salt thereof (preferably hydrochloride) to give the compound represented by general formula (II-1) or the pharmaceutically acceptable salt thereof, wherein:
R* is selected from the group consisting of hydroxy, alkoxy, and halogen;
when an amino group comprises a protecting group, the method further comprises a step of removing the protecting group; the amino protecting group is preferably Boc;
ring A, R^{A}, R', X, R^{a}, R^{b}, R^{c}, R^{d}, R^{X1}, R^{X2}, R^{X3} R³, R⁴, R⁵, and r-1 are as defined in general formula (II-1).

Another aspect of the present disclosure relates to a method for preparing the compound represented by general formula (II-1) or the pharmaceutically acceptable salt thereof described above, the method comprising: 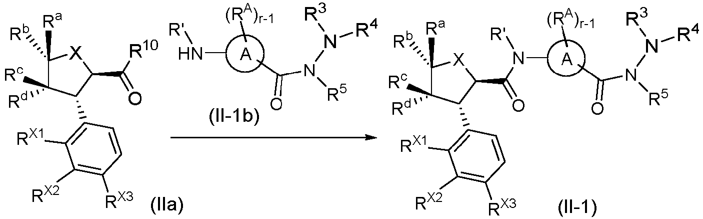 conducting a condensation reaction of a compound represented by general formula (IIa) or a salt thereof with a compound represented by general formula (II-1b) or a salt thereof (preferably hydrochloride) to give the compound represented by general formula (II-1) or the pharmaceutically acceptable salt thereof, wherein:
R¹⁰ is halogen (preferably a chlorine atom) or OH;
ring A, R^{A}, R', X, R^{a}, R^{b}, R^{c}, R^{d}, R^{X1}, R^{X2}, R^{X3} R³, R⁴, R⁵*,* and r-1 are as defined in general formula (II-1).

Another aspect of the present disclosure relates to a method for preparing the compound represented by general formula (III) or the pharmaceutically acceptable salt thereof described above, the method comprising: 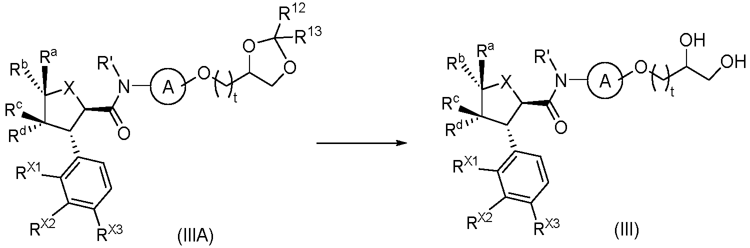 conducting a hydrolysis reaction of a compound represented by general formula (IIIA) or a salt thereof to give the compound represented by general formula (III) or the pharmaceutically acceptable salt thereof, wherein:
R¹² and R¹³ are identical or different and are each independently selected from the group consisting of a hydrogen atom, alkyl, cycloalkyl, and phenyl; preferably, R¹² and R¹³ are identical or different and are each independently selected from the group consisting of a hydrogen atom, C₁₋₆ alkyl, and 5- or 6-membered cycloalkyl; most preferably, R¹² and R¹³ are both CH₃;
ring A, R', X, R^{a}, R^{b}, R^{c}, R^{d}, R^{X1}, R^{X2}, R^{X3}, and t are as defined in general formula (III).

Another aspect of the present disclosure relates to a method for preparing the compound represented by general formula (III-1) or the pharmaceutically acceptable salt thereof described above, the method comprising: 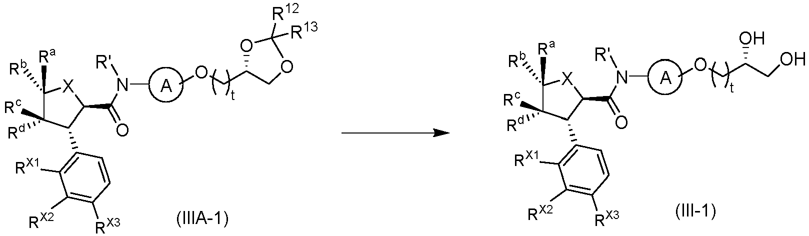 conducting a hydrolysis reaction of a compound represented by general formula (IIIA-1) or a salt thereof to give the compound represented by general formula (III-1) or the pharmaceutically acceptable salt thereof, wherein:
R¹² and R¹³ are identical or different and are each independently selected from the group consisting of a hydrogen atom, alkyl, cycloalkyl, and phenyl; preferably, R¹² and R¹³ are identical or different and are each independently selected from the group consisting of a hydrogen atom, C₁₋₆ alkyl, and 5- or 6-membered cycloalkyl; most preferably, R¹² and R¹³ are both CH₃;
ring A, R', X, R^{a}, R^{b}, R^{c}, R^{d}, R^{X1}, R^{X2}, R^{X3}, and t are as defined in general formula (III-1).

Another aspect of the present disclosure relates to a method for preparing the compound represented by general formula (IV) or the pharmaceutically acceptable salt thereof described above, the method comprising: 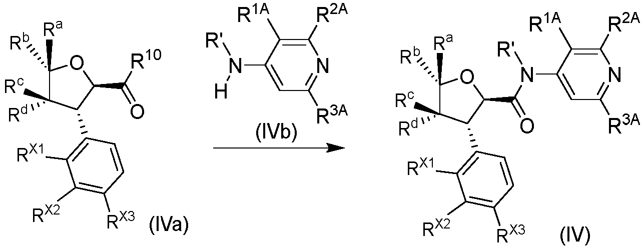 conducting a condensation reaction of a compound represented by general formula (IVa) or a salt thereof with a compound represented by general formula (IVb) or a salt thereof to give the compound represented by general formula (IV) or the pharmaceutically acceptable salt thereof, wherein:
R¹⁰ is halogen (preferably a chlorine atom) or OH;
R^{a}, R^{b}, R^{c}, R^{d}, R^{X1}, R^{X2}, R^{X3}, R', R^{1A}, R^{2A}, and R^{3A} are as defined in general formula (IV).

Another aspect of the present disclosure relates to a method for preparing the compound represented by general formula (IV) or the pharmaceutically acceptable salt thereof described above, the method comprising: 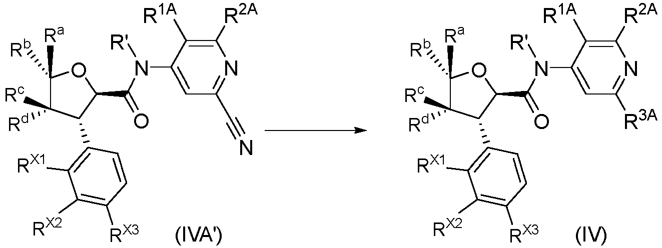 reacting a compound represented by general formula (IVA') or a salt thereof with NH₂-R⁵ or a salt thereof (preferably hydrochloride) to give the compound represented by general formula (IV) or the pharmaceutically acceptable salt thereof, wherein:
R^{3A} is -C(=NR⁵)NR³R ⁴;
R³ and R⁴ are both hydrogen atoms;
R^{1A}, R^{2A}, R', R^{a}, R^{b}, R^{c}, R^{d}, R^{X1}, R^{X2}, R^{X3}, and R⁵ are as defined in general formula (IV).

Another aspect of the present disclosure relates to a method for preparing the compound represented by general formula (IV) or the pharmaceutically acceptable salt thereof described above, the method comprising: 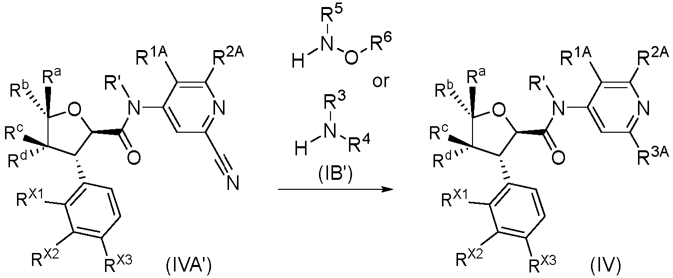 reacting a compound represented by general formula (IVA') or a salt thereof with a compound represented by general formula (IB') or a salt thereof (preferably hydrochloride) to give the compound represented by general formula (IV) or the pharmaceutically acceptable salt thereof, wherein:
R^{3A} is -C(=NH)NR⁵-OR⁶ or -C(=NH)NR³R⁴;
R^{1A}, R^{2A}, R', R^{a}, R^{b}, R^{c}, R^{d}, R^{X1}, R^{X2}, R^{X3}, R³, R⁴, R⁵*,* and R⁶ are as defined in general formula (IV).

Another aspect of the present disclosure relates to a method for preparing the compound represented by general formula (IV) or the pharmaceutically acceptable salt thereof described above, the method comprising: 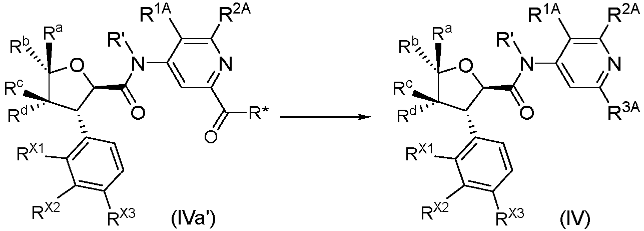 conducting an amidation reaction of a compound represented by general formula (IVa') or a salt thereof with an amine or a salt thereof (preferably hydrochloride) to give the compound represented by general formula (IV) or the pharmaceutically acceptable salt thereof, wherein:
the amine described above is selected from the group consisting of HNR⁵-OR⁶, HNR⁵-NR³R⁴, and HNR⁵-alkylene-Cy;
R* is selected from the group consisting of hydroxy, alkoxy, and halogen;
R^{3A} is selected from the group consisting of -C(O)NR⁵-OR⁶, -C(O)NR⁵-NR³R⁴, and - C(O)NR⁵-alkylene-Cy;
when an amino group comprises a protecting group, the method further comprises a step of removing the protecting group; the amino protecting group is preferably Boc;
R^{1A}, R^{2A}, R', R^{a}, R^{b}, R^{c}, R^{d}, R^{X1}, R^{X2}, R^{X3}, R³, R⁴, R⁵*,* R⁶, and Cy are as defined in general formula (IV).

Another aspect of the present disclosure relates to a method for preparing the compound represented by general formula (V) or the pharmaceutically acceptable salt thereof described above, the method comprising: 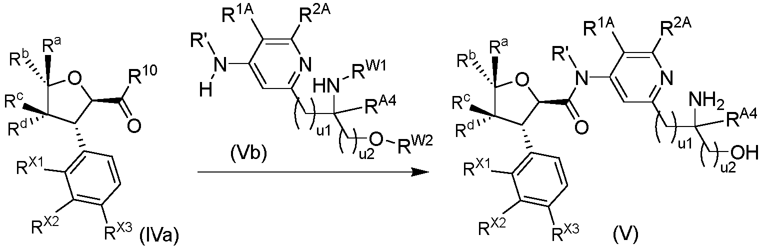 conducting a condensation reaction of a compound represented by general formula (IVa) or a salt thereof with a compound represented by general formula (Vb) or a salt thereof to give the compound represented by general formula (V) or the pharmaceutically acceptable salt thereof, wherein:
R^{W1} is a hydrogen atom or an amino protecting group (preferably tert-butylsulfinyl), and
R^{W2} is a hydrogen atom or a hydroxy protecting group (preferably TBS);
when R^{W1} is an amino protecting group and/or R^{W2} is a hydroxy protecting group, the method further comprises a step of removing the protecting group(s);
R¹⁰ is halogen (preferably a chlorine atom) or OH;
R^{a}, R^{b}, R^{c}, R^{d}, R^{X1}, R^{X2}, R^{X3}, R', R^{1A}, R^{2A}, R^{A4}, u1, and u2 are as defined in general formula (V).

Another aspect of the present disclosure relates to a method for preparing the compound represented by general formula (V-1) or (V-2) or the pharmaceutically acceptable salt thereof described above, the method comprising: 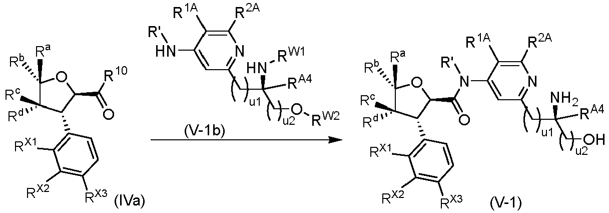 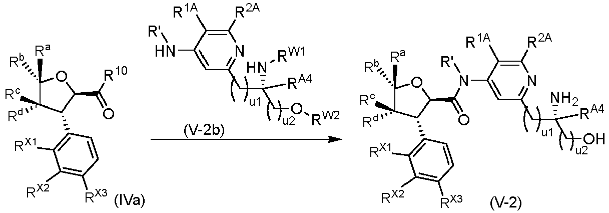
conducting a condensation reaction of a compound represented by general formula (IVa) or a salt thereof with a compound represented by general formula (V-1b) or a salt thereof to give the compound represented by general formula (V-1) or the pharmaceutically acceptable salt thereof, or
conducting a condensation reaction of a compound represented by general formula (IVa) or a salt thereof with a compound represented by general formula (V-2b) or a salt thereof to give the compound represented by general formula (V-2) or the pharmaceutically acceptable salt thereof, wherein:
   R^{W1} is a hydrogen atom or an amino protecting group (preferably tert-butylsulfinyl), and
   R^{W2} is a hydrogen atom or a hydroxy protecting group (preferably TBS);
   when R^{W1} is an amino protecting group and/or R^{W2} is a hydroxy protecting group, the method further comprises a step of removing the protecting group(s);
   R¹⁰ is halogen (preferably a chlorine atom) or OH;
   R^{a}, R^{b}, R^{c}, R^{d}, R^{X1}, R^{X2}, R^{X3}, R', R^{1A}, R^{2A}, R^{A4}, u1, and u2 are as defined in general formula (V-1).

Another aspect of the present disclosure relates to a method for preparing the compound represented by general formula (V-1) or (V-2) or the pharmaceutically acceptable salt thereof described above, the method comprising: 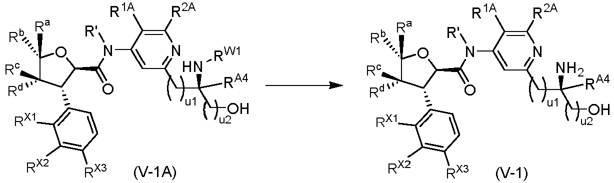 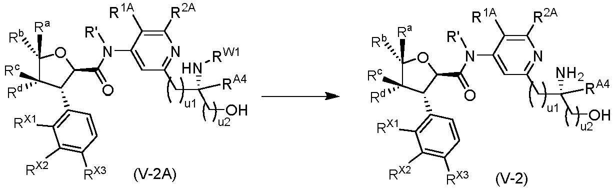
conducting a deprotection reaction of a compound represented by general formula (V-1A) or a salt thereof to give the compound represented by general formula (V-1) or the pharmaceutically acceptable salt thereof, or
conducting a deprotection reaction of a compound represented by general formula (V-2A) or a salt thereof to give the compound represented by general formula (V-2) or the pharmaceutically acceptable salt thereof, wherein:
   R^{W1} is an amino protecting group, and the amino protecting group is preferably Boc;
   R^{a}, R^{b}, R^{c}, R^{d}, R^{X1}, R^{X2}, R^{X3}, R', R^{1A}, R^{2A}, R^{A4}, u1, and u2 are as defined in general formula (V-1).

Another aspect of the present disclosure relates to a method for preparing the compound represented by general formula (VI') or the pharmaceutically acceptable salt thereof described above, the method comprising: 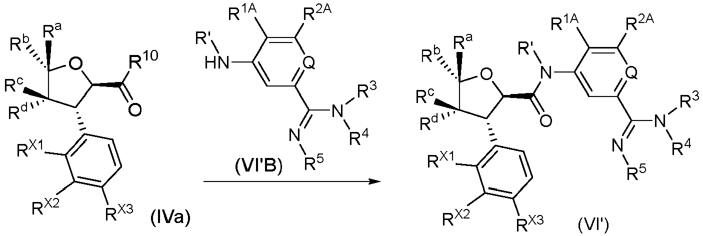 conducting a condensation reaction of a compound represented by general formula (IVa) or a salt thereof with a compound represented by general formula (VI'B) or a salt thereof (preferably hydrochloride) to give the compound represented by general formula (VI') or the pharmaceutically acceptable salt thereof, wherein:
R¹⁰ is halogen (preferably a chlorine atom) or OH;
R', R^{a}, R^{b}, R^{c}, R^{d}, R^{X1}, R^{X2}, R^{X3}, R^{1A}, R^{2A}, Q, R³, R⁴, and R⁵ are as defined in general formula (VI').

Another aspect of the present disclosure relates to a method for preparing the compound represented by general formula (VI') or the pharmaceutically acceptable salt thereof described above, the method comprising: 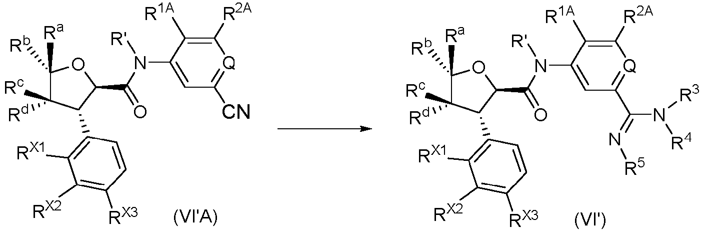 reacting a compound represented by general formula (VI'A) or a salt thereof with NH₂-R⁵ or a salt thereof (preferably hydrochloride) to give the compound represented by general formula (VI') or the pharmaceutically acceptable salt thereof, wherein:
R³ and R⁴ are both hydrogen atoms;
R', R^{a}, R^{b}, R^{c}, R^{d}, R^{X1}, R^{X2}, R^{X3}, R^{1A}, R^{2A}, Q, and R⁵ are as defined in general formula (VI').

Another aspect of the present disclosure relates to a method for preparing the compound represented by general formula (VI') or the pharmaceutically acceptable salt thereof described above, the method comprising: 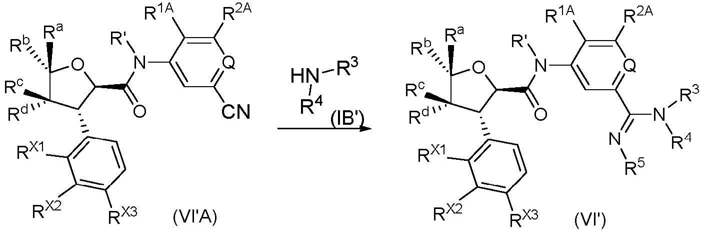 reacting a compound represented by general formula (VI'A) or a salt thereof with a compound represented by general formula (IB') or a salt thereof (preferably hydrochloride) to give the compound represented by general formula (VI') or the pharmaceutically acceptable salt thereof, wherein:
R⁵ is a hydrogen atom;
R', R^{a}, R^{b}, R^{c}, R^{d}, R^{X1}, R^{X2}, R^{X3}, R^{1A}, R^{2A}, Q, R³, and R⁴ are as defined in general formula (VI').

Another aspect of the present disclosure relates to a method for preparing the compound represented by general formula (VI'-1) or the pharmaceutically acceptable salt thereof described above, the method comprising: 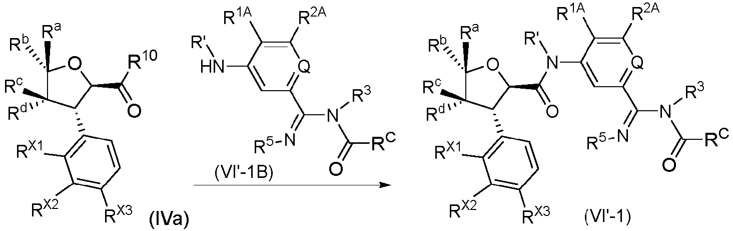 conducting a condensation reaction of a compound represented by general formula (IVa) or a salt thereof with a compound represented by general formula (VI'-1B) or a salt thereof (preferably hydrochloride) to give the compound represented by general formula (VI'-1) or the pharmaceutically acceptable salt thereof, wherein:
R¹⁰ is halogen (preferably a chlorine atom) or OH;
R', R^{a}, R^{b}, R^{c}, R^{d}, R^{X1}, R^{X2}, R^{X3}, R^{1A}, R^{2A}, Q, R³, R⁵*,* and R^{C} are as defined in general formula (VI'-1).

Another aspect of the present disclosure relates to a method for preparing the compound represented by general formula (VI'-1) or the pharmaceutically acceptable salt thereof described above, the method comprising: 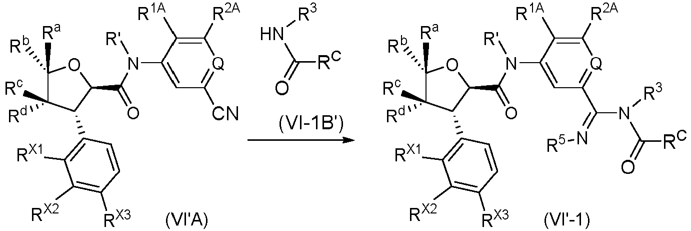 reacting a compound represented by general formula (VI'A) or a salt thereof with a compound represented by general formula (VI-1B') or a salt thereof (preferably hydrochloride) to give the compound represented by general formula (VI'-1) or the pharmaceutically acceptable salt thereof, wherein:
R⁵ is a hydrogen atom;
R', R^{a}, R^{b}, R^{c}, R^{d}, R^{X1}, R^{X2}, R^{X3}, R^{1A}, R^{2A}, Q, R³, and R^{C} are as defined in general formula (VI'-1).

Another aspect of the present disclosure relates to a method for preparing the compound represented by general formula (VI'-1) or the pharmaceutically acceptable salt thereof described above, the method comprising: 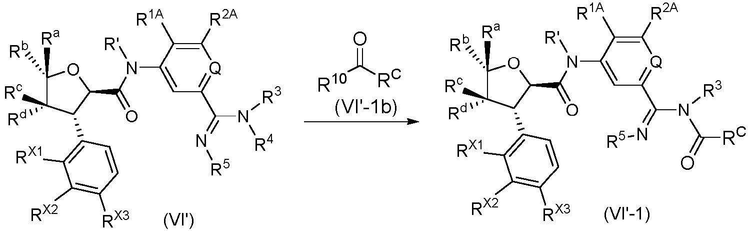 conducting an amidation reaction of a compound represented by general formula (VI') or a salt thereof (preferably trifluoroacetate) with a compound represented by general formula (VI'-1b) or a salt thereof to give the compound represented by general formula (VI'-1) or the pharmaceutically acceptable salt thereof, wherein:
R⁴ is a hydrogen atom;
R¹⁰ is halogen (preferably chlorine) or OH;
R', R^{a}, R^{b}, R^{c}, R^{d}, R^{X1}, R^{X2}, R^{X3}, R^{1A}, R^{2A}, Q, R³, R⁵*,* and R^{C} are as defined in general formula (VI'-1).

Another aspect of the present disclosure relates to a method for preparing the compound represented by general formula (VI) or the pharmaceutically acceptable salt thereof described above, the method comprising: 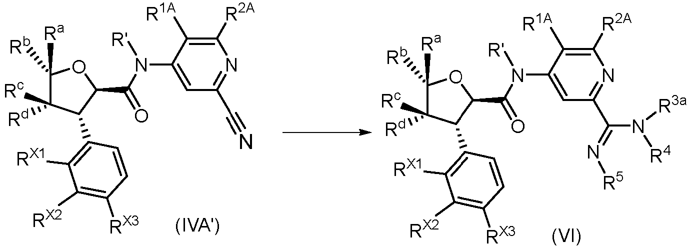 reacting a compound represented by general formula (IVA') or a salt thereof with NH₂-R⁵ or a salt thereof (preferably hydrochloride) to give the compound represented by general formula (VI) or the pharmaceutically acceptable salt thereof, wherein:
R^{3a} and R⁴ are both hydrogen atoms;
R^{1A}, R^{2A}, R', R^{a}, R^{b}, R^{c}, R^{d}, R^{X1}, R^{X2}, R^{X3}, and R⁵ are as defined in general formula (VI).

Another aspect of the present disclosure relates to a method for preparing the compound represented by general formula (VI) or the pharmaceutically acceptable salt thereof described above, the method comprising: 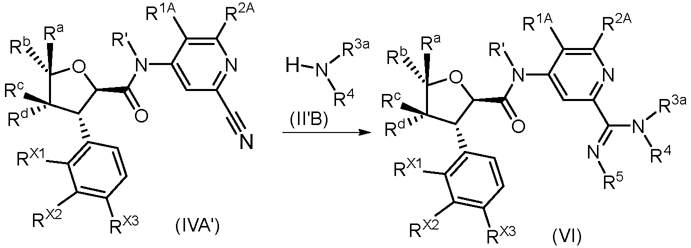 reacting a compound represented by general formula (IVA') or a salt thereof with a compound represented by general formula (II'B) or a salt thereof (preferably hydrochloride) to give the compound represented by general formula (VI) or the pharmaceutically acceptable salt thereof, wherein:
R⁵ is a hydrogen atom;
R^{1A}, R^{2A}, R', R^{a}, R^{b}, R^{c}, R^{d}, R^{X1}, R^{X2}, R^{X3}, R^{3a}, and R⁴ are as defined in general formula (VI).

Another aspect of the present disclosure relates to a method for preparing the compound represented by general formula (VI) or the pharmaceutically acceptable salt thereof described above, the method comprising: 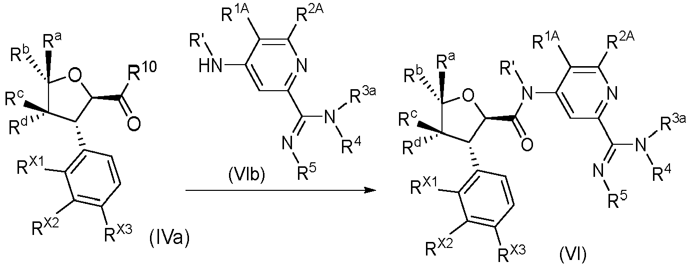 conducting a condensation reaction of a compound represented by general formula (IVa) or a salt thereof with a compound represented by general formula (VIb) or a salt thereof (preferably hydrochloride) to give the compound represented by general formula (VI) or the pharmaceutically acceptable salt thereof, wherein:
R¹⁰ is halogen (preferably a chlorine atom) or OH;
R', R^{a}, R^{b}, R^{c}, R^{d}, R^{X1}, R^{X2}, R^{X3}, R^{1A}, R^{2A}, R^{3a}, R⁴, and R⁵ are as defined in general formula (VI).

Another aspect of the present disclosure relates to a method for preparing the compound represented by general formula (VII') or the pharmaceutically acceptable salt thereof described above, the method comprising: 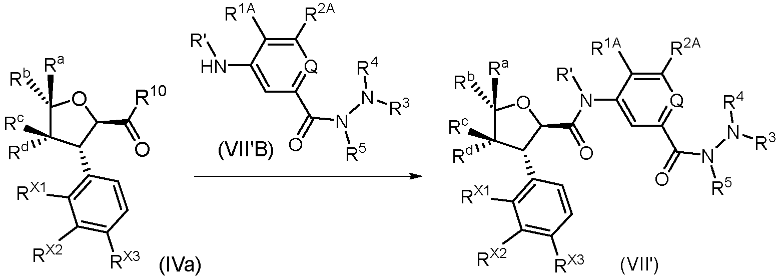 conducting a condensation reaction of a compound represented by general formula (IVa) or a salt thereof with a compound represented by general formula (VII'B) or a salt thereof (preferably hydrochloride) to give the compound represented by general formula (VII') or the pharmaceutically acceptable salt thereof, wherein:
R¹⁰ is halogen (preferably a chlorine atom) or OH;
R', R^{a}, R^{b}, R^{c}, R^{d}, R^{X1}, R^{X2}, R^{X3}, R^{1A}, R^{2A}, Q, R³, R⁴, and R⁵ are as defined in general formula (VII').

Another aspect of the present disclosure relates to a method for preparing the compound represented by general formula (VII') or the pharmaceutically acceptable salt thereof described above, the method comprising: 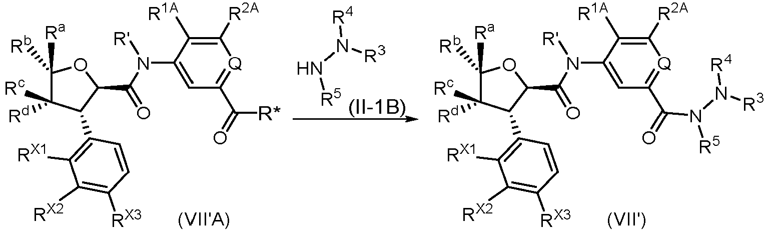 conducting an amidation reaction of a compound represented by general formula (VII') or a salt thereof with a compound represented by general formula (II-1B) or a salt thereof (preferably hydrochloride) to give the compound represented by general formula (VII') or the pharmaceutically acceptable salt thereof, wherein:
R* is selected from the group consisting of hydroxy, alkoxy, and halogen;
when an amino group comprises a protecting group, the method further comprises a step of removing the protecting group; the amino protecting group is preferably Boc;
R', R^{a}, R^{b}, R^{c}, R^{d}, R^{X1}, R^{X2}, R^{X3}, R^{1A}, R^{2A}, Q, R³, R⁴, and R⁵ are as defined in general formula (VII').

Another aspect of the present disclosure relates to a method for preparing the compound represented by general formula (VII) or the pharmaceutically acceptable salt thereof described above, the method comprising: 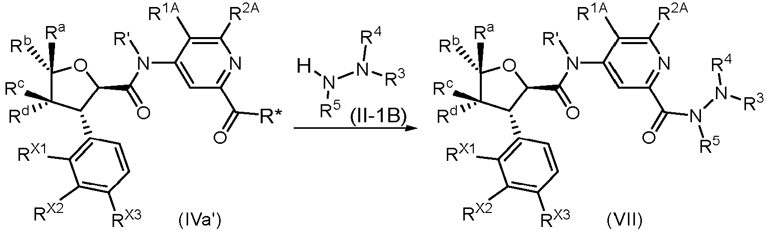 conducting an amidation reaction of a compound represented by general formula (IVa') or a salt thereof with a compound represented by general formula (II-1B) or a salt thereof (preferably hydrochloride) to give the compound represented by general formula (VII) or the pharmaceutically acceptable salt thereof, wherein:
R* is selected from the group consisting of hydroxy, alkoxy, and halogen;
when an amino group comprises a protecting group, the method further comprises a step of removing the protecting group; the amino protecting group is preferably Boc;
R^{1A}, R^{2A}, R', R^{a}, R^{b}, R^{c}, R^{d}, R^{X1}, R^{X2}, R^{X3}, R³, R⁴, and R⁵ are as defined in general formula (VII).

Another aspect of the present disclosure relates to a method for preparing the compound represented by general formula (VII) or the pharmaceutically acceptable salt thereof described above, the method comprising: 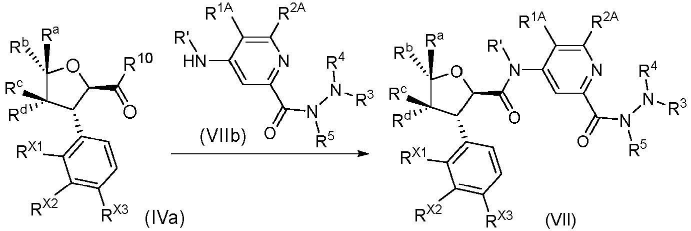 conducting a condensation reaction of a compound represented by general formula (IVa) or a salt thereof with a compound represented by general formula (VIIb) or a salt thereof (preferably hydrochloride) to give the compound represented by general formula (VII) or the pharmaceutically acceptable salt thereof, wherein:
R¹⁰ is halogen (preferably a chlorine atom) or OH;
R', R^{a}, R^{b}, R^{c}, R^{d}, R^{X1}, R^{X2}, R^{X3}, R^{1A}, R^{2A}, R³, R⁴, and R⁵ are as defined in general formula (VII).

Another aspect of the present disclosure relates to a method for preparing the compound represented by general formula (VIII) or the pharmaceutically acceptable salt thereof described above, the method comprising: 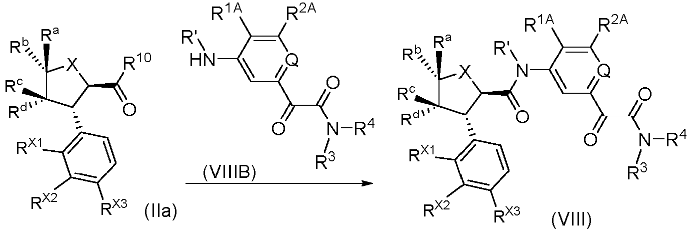 conducting a condensation reaction of a compound represented by general formula (IIa) or a salt thereof with a compound represented by general formula (VIIIB) or a salt thereof (preferably hydrochloride) to give the compound represented by general formula (VIII) or the pharmaceutically acceptable salt thereof, wherein:
R¹⁰ is halogen (preferably a chlorine atom) or OH;
R', X, R^{a}, R^{b}, R^{c}, R^{d}, R^{X1}, R^{X2}, R^{X3}, R^{1A}, R^{2A}, Q, R³, and R⁴ are as defined in general formula (VIII).

Another aspect of the present disclosure relates to a method for preparing the compound represented by general formula (VIII) or the pharmaceutically acceptable salt thereof described above, the method comprising: 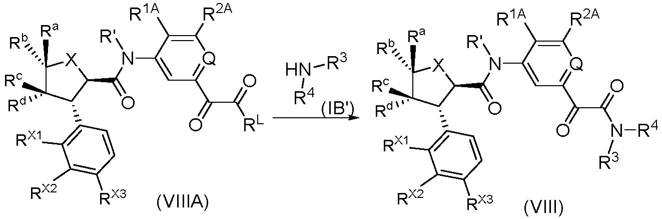 conducting a urethane exchange reaction of a compound represented by general formula (VIIIA) or a salt thereof with a compound represented by general formula (IB') or a salt thereof (preferably hydrochloride) to give the compound represented by general formula (VIII) or the pharmaceutically acceptable salt thereof, wherein:
R^{L} is alkoxy;
R', X, R^{a}, R^{b}, R^{c}, R^{d}, R^{X1}, R^{X2}, R^{X3}, R^{1A}, R^{2A}, Q, R³, and R⁴ are as defined in general formula (VIII).

In some embodiments of the present disclosure, provided is the method for preparing the compound represented by general formula (VIII) or the pharmaceutically acceptable salt thereof, wherein R^{L} is C₁₋₆ alkoxy, preferably methoxy or ethoxy.

In some embodiments of the present disclosure, provided is the method for preparing the compound represented by general formula (II), (II-1), (IV), or (VII) or the pharmaceutically acceptable salt thereof, wherein R* is selected from the group consisting of hydroxy, C₁₋₆ alkoxy, and halogen; in some embodiments, R* is selected from the group consisting of hydroxy, methoxy, ethoxy, and chlorine; in some embodiments, R* is hydroxy.

In some embodiments of the present disclosure, provided is the method for preparing the compound represented by general formula (II), (II-1), (IV), or (VII) or the pharmaceutically acceptable salt thereof, wherein R⁴ is selected from the group consisting of a hydrogen atom, C₁₋₆ alkyl, and an amino protecting group, and when R⁴ is an amino protecting group, the amidation reaction is further followed by a step of removing the amino protecting group.

Another aspect of the present disclosure relates to a method for preparing the compound represented by general formula (II), (II'), (II-1), (III), (III-1), (IV), (V), (V-1), (V-2), (VI), (VII), (VI'), (VI'-1), (VII'), or (VIII) or the pharmaceutically acceptable salt thereof described above, the method comprising: conducting an alkyl-removing or cycloalkyl-removing reaction of a compound represented by general formula (II), (II'), (II-1), (III), (III-1), (IV), (V), (V-1), (V-2), (VI), (VII), (VI'), (VI'-1), (VII'), or (VIII) in which R^{X1} is alkoxy or cycloalkoxy, or a pharmaceutically acceptable salt thereof to give a compound represented by general formula (II), (II'), (II-1), (III), (III-1), (IV), (V), (V-1), (V-2), (VI), (VII), (VI'), (VI'-1), (VII'), or (VIII) in which R^{X1} is -OH, or a pharmaceutically acceptable salt thereof.

Provided is the method for preparing the compound represented by general formula (II), (II'), (II-1), (III), (III-1), (IV), (V), (V-1), (V-2), (VI), (VII), (VI'), (VI'-1), (VII'), or (VIII) or the pharmaceutically acceptable salt thereof described above of the present disclosure, wherein R^{X1} is selected from the group consisting of methoxy, deuterated methoxy, and 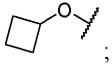 in some embodiments, R^{X1} is methoxy or

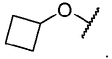

Another aspect of the present disclosure relates to a pharmaceutical composition comprising the compound represented by general formula (I), (II), (II'), (II-1), (III), (III-1), (IV), (V), (V-1), (V-2), (VI), (VII), (VI'), (VI'-1), (VII'), or (VIII) of the present disclosure or a compound shown in Table A or a pharmaceutically acceptable salt thereof, and one or more pharmaceutically acceptable carriers, diluents, or excipients.

The present disclosure further relates to use of a compound represented by general formula (I), (II), (II'), (II-1), (III), (III-1), (IV), (V), (V-1), (V-2), (VI), (VII), (VI'), (VI'-1), (VII'), or (VIII) or a compound shown in Table A or a pharmaceutically acceptable salt thereof, or a pharmaceutical composition comprising same in the preparation of a medicament for inhibiting a voltage-gated sodium channel; preferably, the voltage-gated sodium channel is Nav1.8.

The present disclosure further relates to use of a compound represented by general formula (I), (II), (II'), (II-1), (III), (III-1), (IV), (V), (V-1), (V-2), (VI), (VII), (VI'), (VI'-1), (VII'), or (VIII) or a compound shown in Table A or a pharmaceutically acceptable salt thereof, or a pharmaceutical composition comprising same in the preparation of a medicament for treating and/or preventing a disease or disorder mediated by a voltage-gated sodium channel; preferably, the voltage-gated sodium channel is Nav1.8.

The present disclosure further relates to use of a compound represented by general formula (I), (II), (II'), (II-1), (III), (III-1), (IV), (V), (V-1), (V-2), (VI), (VII), (VI'), (VI'-1), (VII'), or (VIII) or a compound shown in Table A or a pharmaceutically acceptable salt thereof, or a pharmaceutical composition comprising same in the preparation of a medicament for treating and/or alleviating pain and pain-related diseases, multiple sclerosis, Charcot-Marie-Tooth syndrome, incontinence, pathological cough, or cardiac arrhythmia; preferably, the pain is selected from the group consisting of chronic pain, acute pain, inflammatory pain, cancer pain, postsurgical pain, neuropathic pain, musculoskeletal pain, primary pain, gut pain, and idiopathic pain; the postsurgical pain is preferably selected from the group consisting of bunionectomy pain, herniorrhaphy pain, and abdominoplasty pain.

The present disclosure further relates to a method for inhibiting a voltage-gated sodium channel, the method comprising administering to a patient in need thereof a compound represented by general formula (I), (II), (II'), (II-1), (III), (III-1), (IV), (V), (V-1), (V-2), (VI), (VII), (VI'), (VI'-1), (VII'), or (VIII) or a compound shown in Table A or a pharmaceutically acceptable salt thereof, or a pharmaceutical composition comprising same; preferably, the voltage-gated sodium channel is Nav1.8.

The present disclosure further relates to a method for treating and/or preventing a disease or disorder mediated by a voltage-gated sodium channel, the method comprising administering to a patient in need thereof a compound represented by general formula (I), (II), (II'), (II-1), (III), (III-1), (IV), (V), (V-1), (V-2), (VI), (VII), (VI'), (VI'-1), (VII'), or (VIII) or a compound shown in Table A or a pharmaceutically acceptable salt thereof, or a pharmaceutical composition comprising same; preferably, the voltage-gated sodium channel is Nav1.8.

The present disclosure further relates to a method for treating and/or preventing pain and pain-related diseases, multiple sclerosis, Charcot-Marie-Tooth syndrome, incontinence, pathological cough, or cardiac arrhythmia, the method comprising administering to a patient in need thereof a compound represented by general formula (I), (II), (II'), (II-1), (III), (III-1), (IV), (V), (V-1), (V-2), (VI), (VII), (VI'), (VI'-1), (VII'), or (VIII) or a compound shown in Table A or a pharmaceutically acceptable salt thereof, or a pharmaceutical composition comprising same; preferably, the pain is selected from the group consisting of chronic pain, acute pain, inflammatory pain, cancer pain, postsurgical pain, neuropathic pain, musculoskeletal pain, primary pain, gut pain, and idiopathic pain; the postsurgical pain is preferably selected from the group consisting of bunionectomy pain, herniorrhaphy pain, and abdominoplasty pain.

The present disclosure further relates to a compound represented by general formula (I), (II), (II'), (II-1), (III), (III-1), (IV), (V), (V-1), (V-2), (VI), (VII), (VI'), (VI'-1), (VII'), or (VIII) or a compound shown in Table A or a pharmaceutically acceptable salt thereof, or a pharmaceutical composition comprising same for use as a medicament, preferably a medicament for inhibiting voltage-gated sodium channel activity.

The present disclosure further relates to a compound represented by general formula (I), (II), (II'), (II-1), (III), (III-1), (IV), (V), (V-1), (V-2), (VI), (VII), (VI'), (VI'-1), (VII'), or (VIII) or a compound shown in Table A or a pharmaceutically acceptable salt thereof, or a pharmaceutical composition comprising same for use in inhibiting a voltage-gated sodium channel; preferably, the voltage-gated sodium channel is Nav1.8.

The present disclosure further relates to a compound represented by general formula (I), (II), (II'), (II-1), (III), (III-1), (IV), (V), (V-1), (V-2), (VI), (VII), (VI'), (VI'-1), (VII'), or (VIII) or a compound shown in Table A or a pharmaceutically acceptable salt thereof, or a pharmaceutical composition comprising same for use as a voltage-gated sodium channel inhibitor.

The present disclosure further relates to a compound represented by general formula (I), (II), (II'), (II-1), (III), (III-1), (IV), (V), (V-1), (V-2), (VI), (VII), (VI'), (VI'-1), (VII'), or (VIII) or a compound shown in Table A or a pharmaceutically acceptable salt thereof, or a pharmaceutical composition comprising same for use as a medicament for treating and/or preventing a disease or disorder mediated by a voltage-gated sodium channel.

The present disclosure further relates to a compound represented by general formula (I), (II), (II'), (II-1), (III), (III-1), (IV), (V), (V-1), (V-2), (VI), (VII), (VI'), (VI'-1), (VII'), or (VIII) or a compound shown in Table A or a pharmaceutically acceptable salt thereof, or a pharmaceutical composition comprising same for use in treating and/or preventing a disease or disorder mediated by a voltage-gated sodium channel; preferably, the voltage-gated sodium channel is Nav1.8.

The present disclosure further relates to a compound represented by general formula (I), (II), (II'), (II-1), (III), (III-1), (IV), (V), (V-1), (V-2), (VI), (VII), (VI'), (VI'-1), (VII'), or (VIII) or a compound shown in Table A or a pharmaceutically acceptable salt thereof, or a pharmaceutical composition comprising same for use in treating and/or preventing pain and pain-related diseases, multiple sclerosis, Charcot-Marie-Tooth syndrome, incontinence, pathological cough, or cardiac arrhythmia; preferably, the pain is selected from the group consisting of chronic pain, acute pain, inflammatory pain, cancer pain, neuropathic pain, musculoskeletal pain, primary pain, gut pain, and idiopathic pain; the postsurgical pain is preferably selected from the group consisting of bunionectomy pain, herniorrhaphy pain, and abdominoplasty pain.

The disease or disorder described in the present disclosure is a disease or disorder that is treated and/or prevented by inhibiting a voltage-gated sodium channel; preferably, the voltage-gated sodium channel is Nav1.8.

Preferably, the disease or disorder mediated by a voltage-gated sodium channel described in the present disclosure is pain and pain-related diseases, multiple sclerosis, Charcot-Marie-Tooth syndrome, incontinence, or cardiac arrhythmia; preferably, the pain is selected from the group consisting of chronic pain, acute pain, inflammatory pain, cancer pain, neuropathic pain, musculoskeletal pain, primary pain, gut pain, and idiopathic pain. The neuropathic pain described in the present disclosure is preferably selected from the group consisting of trigeminal neuralgia, post-herpetic neuralgia, diabetic neuralgia, painful HIV-associated sensory neuralgia, burn syndrome, post-amputation pain, post-spinal cord injury pain, phantom pain, painful neuroma, traumatic neuroma, Morton's neuroma, nerve entrapment injury, spinal stenosis, carpal tunnel syndrome, radicular pain, sciatica, nerve avulsion injury, brachial plexus avulsion injury, complex regional pain syndrome, drug therapy-induced neuralgia, cancer chemotherapy-induced neuralgia, anti-retroviral therapy-induced neuralgia, primary small-fiber neuropathy, primary sensory neuralgia, and trigeminal autonomic cephalalgia; preferably, the neuropathic pain is selected from the group consisting of post-herpetic neuralgia, small-fiber neuralgia, diabetic neuralgia, and idiopathic small-fiber neuralgia.

The musculoskeletal pain described in the present disclosure is preferably selected from the group consisting of osteoarthritis pain, back pain, cold pain, burn pain, and dental pain.

The gut pain described in the present disclosure is preferably selected from the group consisting of inflammatory bowel disease pain, Crohn's disease pain, and interstitial cystitis pain.

The inflammatory pain described in the present disclosure is preferably selected from the group consisting of rheumatoid arthritis pain and vulvodynia.

The idiopathic pain described in the present disclosure includes fibromyalgia.

The acute pain described in the present disclosure includes acute postsurgical pain.

The postsurgical pain described in the present disclosure includes joint replacement pain, soft tissue surgery pain, bunionectomy pain, herniorrhaphy pain, and abdominoplasty pain and is preferably selected from the group consisting of bunionectomy pain, herniorrhaphy pain, and abdominoplasty pain.

The disease or disorder described in the present disclosure is selected from the group consisting of acute pain, chronic pain, neuropathic pain, inflammatory pain, arthritis, migraine, cluster headache, trigeminal neuralgia, herpetic neuralgia, general neuralgia, epilepsy, epileptic conditions, neurodegenerative disorders, psychiatric disorders, anxiety, depression, bipolar disorder, myotonia, cardiac arrhythmia, dyskinesia, neuroendocrine disorders, ataxia, multiple sclerosis, irritable bowel syndrome, incontinence, pathological cough, visceral pain, osteoarthritis pain, post-herpetic neuralgia, diabetic neuropathy, radicular pain, sciatica, back pain, head pain, neck pain, severe pain, intractable pain, nociceptive pain, breakthrough pain, postsurgical pain, cancer pain, stroke, cerebral ischemia, traumatic brain injury, amyotrophic lateral sclerosis, stress-induced angina pectoris, exercise-induced angina pectoris, palpitation, hypertension, and abnormal gastrointestinal motility.

The pain and pain-associated diseases described in the present disclosure are selected from the group consisting of femur cancer pain, non-malignant chronic bone pain, rheumatoid arthritis, osteoarthritis, spinal stenosis, neuropathic lower back pain, myofascial pain syndrome, fibromyalgia, temporomandibular joint pain, chronic visceral pain, abdominal pain, pancreatic pain, IBS pain, chronic and acute headaches, migraine, tension headache, cluster headache, chronic and acute neuropathic pain, post-herpetic neuralgia, diabetic neuropathy, HIV-associated neuropathy, trigeminal neuralgia, Charcot-Marie-Tooth neuropathy, hereditary sensory neuropathy, peripheral nerve injury, painful neuromas, ectopic proximal and distal discharges, radiculopathy, chemotherapy-induced neuropathic pain, radiotherapy-induced neuropathic pain, post-mastectomy pain, central pain, spinal cord injury pain, post-stroke pain, thalamic pain, complex regional pain syndrome, phantom pain, phantom limb pain, intractable pain, acute pain, acute postsurgical pain, acute musculoskeletal pain, joint pain, mechanical lower back pain, neck pain, tendonitis, injury pain, exercise pain, acute visceral pain, pyelonephritis, appendicitis, cholecystitis, intestinal obstruction, hernias, chest pain, cardiac pain, pelvic pain, renal colic pain, acute obstetric pain, labor pain, cesarean pain, acute inflammatory pain, burn pain, traumatic pain, acute intermittent pain, endometriosis, acute herpes zoster pain, sickle cell anemia, acute pancreatitis, breakthrough pain, orofacial pain, sinusitis pain, dental pain, multiple sclerosis (MS) pain, pain in depression, leprosy pain, Behcet's disease pain, adiposis dolorosa, phlebitic pain, Guillain-Barre pain, painful legs and moving toes, Haglund syndrome, erythromelalgia, Fabry's disease pain, bladder and urogenital diseases, urinary incontinence, pathological cough, hyperactivity bladder, painful bladder syndrome, interstitial cystitis (IC), prostatitis, type I complex regional pain syndrome (CRPS), type II complex regional pain syndrome (CRPS), widespread pain, paroxysmal extreme pain, pruritis, tinnitus, and angina-induced pain.

The disease or disorder described in the present disclosure is selected from the group consisting of acute pain, subacute and chronic pain, nociceptive pain, neuropathic pain, inflammatory pain, nociceptive pain, arthritis, migraine, cluster headache, trigeminal neuralgia, herpetic neuralgia, general neuralgia, epilepsy, epileptic disorders, neurodegenerative disorders, psychiatric disorders, anxiety, depression, bipolar disorder, myotonia, cardiac arrhythmia, dyskinesia, neurodegenerative diseases, endocrine disorders, ataxia, central neuropathic pain of multiple sclerosis and irritable bowel syndrome, incontinence, pathological cough, visceral pain, osteoarthritis pain, post-herpetic neuralgia, diabetic neuropathy, radicular pain, sciatica, back pain, non-specific chronic back pain, head pain, neck pain, moderate pain, severe pain, intractable pain, nociceptive pain, breakthrough pain, postsurgical pain, cancer pain (including chronic cancer pain and breakthrough cancer pain), stroke (e.g., post-stroke central neuropathic pain), diseases associated with cervical spine sprain, brittle fracture, spine fracture, ankylosing spondylitis, pemphigus, Raynaud's disease, scleroderma, systemic lupus erythematosus, epidermolysis bullosa, gout, juvenile idiopathic arthritis, polymyalgia rheumatica, pyoderma gangrenosum, chronic widespread pain, diffuse idiopathic skeletal hyperostosis, intervertebral disc degeneration/herniation pain, radiculopathy, facet joint syndrome, failed back surgery syndrome, burns, carpal tunnel syndrome, Paget's disease pain, spinal stenosis, intervertebral disc inflammation, transverse myelitis, Ehlers-Danlos syndrome, Fabry's disease, mastocytosis, neurofibromatosis, ocular neuropathic pain, sarcoidosis, spondylolysis, spondylolisthesis, chemotherapy-induced oral mucositis, Charcot's neuro-osteoarthropathy, temporomandibular joint disorder, joint replacement pain, non-cardiac chest pain, pudendal pain, renal colic pain, biliary disease, vascular leg ulcer, Parkinson's disease pain, Alzheimer's disease pain, cerebral ischemia, traumatic brain injury, amyotrophic lateral sclerosis, stress-induced angina pectoris, exercise-induced angina pectoris, palpitation, hypertension, and abnormal gastrointestinal motility. The active compound may be formulated into a form suitable for administration by any suitable route, and one or more pharmaceutically acceptable carriers are used to formulate the composition of the present disclosure by conventional methods. Thus, the active compound of the present disclosure may be formulated into a variety of dosage forms for oral administration, administration by injection (e.g., intravenous, intramuscular, or subcutaneous), or administration by inhalation or insufflation. The compounds of the present disclosure may also be formulated into a dosage form, such as tablets, hard or soft capsules, aqueous or oily suspensions, emulsions, injections, dispersible powders or granules, suppositories, lozenges or syrups.

As a general guide, the active compound of the present disclosure is preferably in the form of a unit dose, or in the form of a single dose that can be self-administered by a patient. The unit dose of the compound or composition of the present disclosure may be expressed in the form of a tablet, capsule, cachet, vial, powder, granule, lozenge, suppository, regenerating powder, or liquid formulation. A suitable unit dose may be 0.1-1000 mg.

The pharmaceutical composition of the present disclosure may comprise, in addition to the active compound, one or more auxiliary materials selected from the group consisting of a filler (diluent), a binder, a wetting agent, a disintegrant, or an excipient or the like. Depending on the method of administration, the composition may comprise 0.1 to 99 wt.% of the active compound.

In some embodiments, a unit dose of the pharmaceutical composition is 0.001 mg-1000 mg.

In certain embodiments, the pharmaceutical composition comprises 0.01-99.99% of an aforementioned compound or a pharmaceutically acceptable salt thereof or an isotopically substituted form thereof based on the total weight of the composition. In certain embodiments, the pharmaceutical composition comprises 0.1-99.9% of an aforementioned compound or a pharmaceutically acceptable salt thereof or an isotopically substituted form thereof. In certain embodiments, the pharmaceutical composition comprises 0.5%-99.5% of an aforementioned compound or a pharmaceutically acceptable salt thereof or an isotopically substituted form thereof. In certain embodiments, the pharmaceutical composition comprises 1%-99% of an aforementioned compound or a pharmaceutically acceptable salt thereof or an isotopically substituted form thereof. In certain embodiments, the pharmaceutical composition comprises 2%-98% of an aforementioned compound or a pharmaceutically acceptable salt thereof or an isotopically substituted form thereof.

In certain embodiments, the pharmaceutical composition comprises 0.01%-99.99% of a pharmaceutically acceptable excipient based on the total weight of the composition. In certain embodiments, the pharmaceutical composition comprises 0.1%-99.9% of a pharmaceutically acceptable excipient. In certain embodiments, the pharmaceutical composition comprises 0.5%-99.5% of a pharmaceutically acceptable excipient. In certain embodiments, the pharmaceutical composition comprises 1%-99% of a pharmaceutically acceptable excipient. In certain embodiments, the pharmaceutical composition comprises 2%-98% of a pharmaceutically acceptable excipient.

The pharmaceutically acceptable salts of the compounds described in the present disclosure may be selected from the group consisting of inorganic salts and organic salts. The tablet comprises the active ingredient, and non-toxic pharmaceutically acceptable excipients that are used for mixing and are suitable for the preparation of the tablet. These excipients may be an inert excipient, a granulating agent, a disintegrant, a binder, and a lubricant. These tablets may be uncoated or coated using known techniques that mask the taste of the drug or delay disintegration and absorption in the gastrointestinal tract, thereby providing a sustained-release effect over an extended period of time.

An oral formulation may also be provided in the form of a soft gelatin capsule in which the active ingredient is mixed with an inert solid diluent or with a water-soluble carrier or oil vehicle.

An aqueous suspension comprises the active substance and an excipient that is used for mixing and is suitable for the preparation of the aqueous suspension. Such an excipient is a suspending agent, a dispersant, or a wetting agent. The aqueous suspension may also comprise one or more preservatives, one or more colorants, one or more corrigents, and one or more sweeteners.

An oil suspension may be formulated by suspending the active ingredient in a vegetable oil, or in a mineral oil. The oil suspension may comprise a thickening agent. The sweeteners and corrigents described above may be added to provide a palatable formulation. Antioxidants may also be added to preserve the compositions.

The pharmaceutical composition of the present disclosure may also be in the form of an oil-in-water emulsion. The oil phase may be a vegetable oil or a mineral oil, or a mixture thereof. Suitable emulsifiers may be naturally occurring phospholipids, and the emulsion may also comprise a sweetener, a corrigent, a preservative, and an antioxidant. Such a formulation may also comprise a palliative, a preservative, a colorant, and an antioxidant. The pharmaceutical composition of the present disclosure may be in the form of a sterile injectable aqueous solution. Acceptable vehicles or solvents that can be used include water, Ringer's solution, and isotonic sodium chloride solution. A sterile injectable formulation may be a sterile injectable oil-in-water microemulsion in which an active ingredient is dissolved in an oil phase. The injection or microemulsion can be locally injected into the bloodstream of a patient in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition of the present disclosure may be in the form of a sterile injectable aqueous or oil suspension for intramuscular and subcutaneous administration. The suspension can be prepared according to the prior art using those suitable dispersants or wetting agents and suspending agents as described above. The sterile injectable formulation may also be a sterile injection or suspension prepared in a parenterally acceptable non-toxic diluent or solvent. In addition, a sterile fixed oil may be conventionally used as a solvent or a suspending medium. For this purpose, any blend fixed oil may be used. In addition, fatty acids may also be used to prepare injections.

The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug.

The compound of the present disclosure can be administered in the form of dispersible powders and granules that are formulated into aqueous suspensions by adding water. Such a pharmaceutical composition can be prepared by mixing the active ingredient with a dispersant or a wetting agent, a suspending agent, or one or more preservatives.

As is well known to those skilled in the art, the dose of the drug administered depends on a variety of factors, including but not limited to, the activity of the particular compound employed, the severity of the disease, the age of the patient, the weight of the patient, the health condition of the patient, the behavior of the patient, the diet of the patient, the time of administration, the route of administration, the rate of excretion, the combination of drugs, and the like. In addition, the optimal treatment regimen, such as the mode of administration, the daily dose of the compound, or the type of pharmaceutically acceptable salts, can be verified according to conventional treatment regimens.

### Terminology

Unless otherwise stated, the terms used in the specification and claims have the following meanings.

The term "alkyl" refers to a saturated straight-chain or branched-chain aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkyl). Preferably, the alkyl is an alkyl group having 1 to 12 carbon atoms (i.e., C₁₋₁₂ alkyl); more preferably, the alkyl is an alkyl group having 1 to 6 carbon atoms (i.e., C₁₋₆ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched-chain isomers thereof, and the like. Alkyl may be substituted or unsubstituted, and when it is substituted, it may be substituted at any accessible point of attachment, and the substituent is preferably selected from the group consisting of one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkylene" refers to a divalent alkyl group, wherein the alkyl is as defined above; the alkylene has 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkylene). Preferably, the alkylene is an alkylene group having 1 to 12 carbon atoms (i.e., C₁₋₁₂ alkylene); more preferably, the alkylene is an alkylene group having 1 to 6 carbon atoms (i.e., C₁₋₆ alkylene). Non-limiting examples include: -CH₂-, -CH(CH₃)-, -C(CH₃)₂-, -CH₂CH₂-, -CH(CH₂CH₃)-, -CH₂CH(CH₃)-, - CH₂C(CH₃)₂-, -CH₂CH₂CH₂-, -CH₂CH₂CH₂CH₂-, and the like. Alkylene may be substituted or unsubstituted, and when it is substituted, it may be substituted at any accessible point of attachment, and the substituent is preferably selected from the group consisting of one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkenyl" refers to an alkyl group containing at least one carbon-carbon double bond in the molecule, wherein the alkyl group is as defined above; the alkenyl has 2 to 12 (e.g., 2, 3, 4, 5, 6, 7, 8, 9, 10, 11, or 12) carbon atoms (i.e., C₂₋₁₂ alkenyl). Preferably, the alkenyl is an alkenyl group having 2 to 6 carbon atoms (i.e., C₂₋₆ alkenyl). Non-limiting examples include: ethenyl, propenyl, isopropenyl, butenyl, and the like. Alkenyl may be substituted or unsubstituted, and when it is substituted, it may be substituted at any accessible point of attachment, and the substituent is preferably selected from the group consisting of one or more of a D atom, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkynyl" refers to an alkyl group containing at least one carbon-carbon triple bond in the molecule, wherein the alkyl group is as defined above; the alkynyl has 2 to 12 (e.g., 2, 3, 4, 5, 6, 7, 8, 9, 10, 11, or 12) carbon atoms (i.e., C₂₋₁₂ alkynyl). Preferably, the alkynyl is an alkynyl group having 2 to 6 carbon atoms (i.e., C₂₋₆ alkynyl). Non-limiting examples include: ethynyl, propynyl, butynyl, pentynyl, hexynyl, and the like. Alkynyl may be substituted or unsubstituted, and when it is substituted, it may be substituted at any accessible point of attachment, and the substituent is preferably selected from the group consisting of one or more of a D atom, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples include: methoxy, ethoxy, propoxy, butoxy, and the like. Alkoxy may be substituted or unsubstituted, and when it is substituted, it may be substituted at any accessible point of attachment, and the substituent is preferably selected from the group consisting of one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "cycloalkyl" refers to a saturated or partially unsaturated monocyclic all-carbon ring (i.e., monocyclic cycloalkyl) or polycyclic system (i.e., polycyclic cycloalkyl); the cycloalkyl has 3 to 20 (e.g., 3, 4, 5, 6, 7, 8, 9, 10, 11, 12, 13, 14, 15, 16, 17, 18, 19, or 20) ring atoms (i.e., 3- to 20-membered cycloalkyl). Preferably, the cycloalkyl is a cycloalkyl group having 3 to 12 ring atoms (i.e., 3- to 12-membered cycloalkyl) and a cycloalkyl group having 3 to 10 ring atoms (i.e., 3- to 10-membered cycloalkyl); more preferably, the cycloalkyl is a cycloalkyl group having 3 to 8 ring atoms (i.e., 3- to 8-membered cycloalkyl); most preferably, the cycloalkyl is a cycloalkyl group having 3 to 6 ring atoms (i.e., 3- to 6-membered cycloalkyl).

Non-limiting examples of the monocyclic cycloalkyl include: cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like.

The polycyclic cycloalkyl includes: spirocycloalkyl, fused cycloalkyl, and bridged cycloalkyl.

The term "spirocycloalkyl" refers to a polycyclic system in which a carbon atom (referred to as a spiro atom) is shared between rings; the spirocycloalkyl may contain in the rings one or more double bonds or may contain in the rings one or more heteroatoms selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded), with the proviso that at least one all-carbon ring is contained and the point of attachment is on the all-carbon ring; the spirocycloalkyl has 5 to 20 (e.g., 5, 6, 7, 8, 9, 10, 11, 12, 13, 14, 15, 16, 17, 18, 19, or 20) ring atoms (i.e., 5- to 20-membered spirocycloalkyl). Preferably, the spirocycloalkyl is a spirocycloalkyl group having 6 to 14 ring atoms (i.e., 6- to 14-membered spirocycloalkyl); more preferably, the spirocycloalkyl is a spirocycloalkyl group having 7 to 10 ring atoms (i.e., 7- to 10-membered spirocycloalkyl). The spirocycloalkyl includes monospirocycloalkyl and polyspirocycloalkyl (e.g., bispirocycloalkyl); monospirocycloalkyl or bispirocycloalkyl is preferred, and 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospirocycloalkyl is more preferred. Non-limiting examples include: 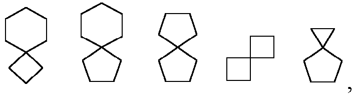 wherein the point of attachment may be at any position; 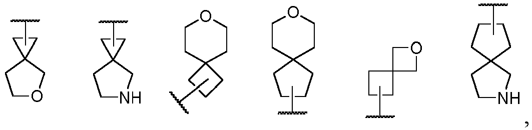 and the like.

The term "fused cycloalkyl" refers to a polycyclic system in which two adjacent carbon atoms are shared between rings; the fused cycloalkyl is formed by fusing a monocyclic cycloalkyl group with one or more monocyclic cycloalkyl groups, or fusing a monocyclic cycloalkyl group with one or more of a heterocyclyl group, an aryl group, or a heteroaryl group, wherein the point of attachment is on a monocyclic cycloalkyl group; the fused cycloalkyl may contain one or more double bonds in the rings and has 5 to 20 (e.g., 5, 6, 7, 8, 9, 10, 11, 12, 13, 14, 15, 16, 17, 18, 19, or 20) ring atoms (i.e., 5- to 20-membered fused cycloalkyl). Preferably, the fused cycloalkyl is a fused cycloalkyl group having 6 to 14 ring atoms (i.e., 6- to 14-membered fused cycloalkyl); more preferably, the fused cycloalkyl is a fused cycloalkyl group having 7 to 10 ring atoms (i.e., 7- to 10-membered fused cycloalkyl). The fused cycloalkyl includes bicyclic fused cycloalkyl and polycyclic fused cycloalkyl (e.g., tricyclic fused cycloalkyl and tetracyclic fused cycloalkyl); bicyclic fused cycloalkyl or tricyclic fused cycloalkyl is preferred, and 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused cycloalkyl is more preferred. Non-limiting examples include: 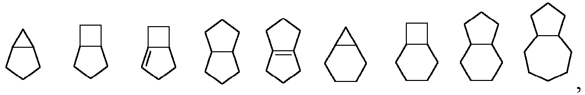 wherein the point of attachment may be at any position; 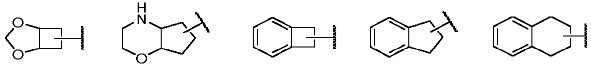 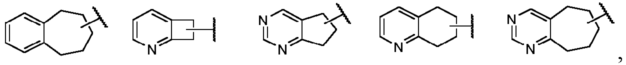 and the like.

The term "bridged cycloalkyl" refers to an all-carbon polycyclic system in which two carbon atoms that are not directly connected are shared between rings; the bridged cycloalkyl may contain one or more double bonds in the rings and has 5 to 20 (e.g., 5, 6, 7, 8, 9, 10, 11, 12, 13, 14, 15, 16, 17, 18, 19, or 20) carbon atoms (i.e., 5- to 20-membered bridged cycloalkyl). Preferably, the bridged cycloalkyl is a bridged cycloalkyl group having 6 to 14 carbon atoms (i.e., 6- to 14-membered bridged cycloalkyl); more preferably, the bridged cycloalkyl is a bridged cycloalkyl group having 7 to 10 carbon atoms (i.e., 7- to 10-membered bridged cycloalkyl). The bridged cycloalkyl includes bicyclic bridged cycloalkyl and polycyclic bridged cycloalkyl (e.g., tricyclic bridged cycloalkyl and tetracyclic bridged cycloalkyl); bicyclic bridged cycloalkyl or tricyclic bridged cycloalkyl is preferred. Non-limiting examples include: 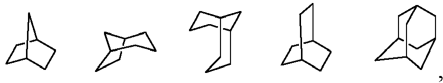 wherein the point of attachment may be at any position.

Cycloalkyl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heterocyclyl" refers to a saturated or partially unsaturated monocyclic heterocyclic (i.e., monocyclic heterocyclyl) or polycyclic heterocyclic system (i.e., polycyclic heterocyclyl); the heterocyclyl contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded) and has 3 to 20 (e.g., 3, 4, 5, 6, 7, 8, 9, 10, 11, 12, 13, 14, 15, 16, 17, 18, 19, or 20) ring atoms (i.e., 3- to 20-membered heterocyclyl). Preferably, the heterocyclyl is a heterocyclyl group having 3 to 12 ring atoms (i.e., 3- to 12-membered heterocyclyl); more preferably, the heterocyclyl is a heterocyclyl group having 3 to 10 ring atoms (i.e., 3- to 10-membered heterocyclyl) and a heterocyclyl group having 3 to 8 ring atoms (i.e., 3- to 8-membered heterocyclyl); yet more preferably, the heterocyclyl is a heterocyclyl group having 4 to 7 ring atoms (i.e., 4- to 7-membered heterocyclyl); still more preferably, the heterocyclyl is a heterocyclyl group having 3 to 6 ring atoms (i.e., 3- to 6-membered heterocyclyl); most preferably, the heterocyclyl is a heterocyclyl group having 5 or 6 ring atoms (i.e., 5- or 6-membered heterocyclyl) or a heterocyclyl group having 3 to 4 ring atoms (i.e., 3- to 4-membered heterocyclyl).

Non-limiting examples of the monocyclic heterocyclyl include: pyrrolidinyl, tetrahydropyranyl, 1,2,3,6-tetrahydropyridinyl, piperidinyl, piperazinyl, morpholinyl, thiomorpholinyl, homopiperazinyl, and the like.

The polycyclic heterocyclyl includes spiroheterocyclyl, fused heterocyclyl, and bridged heterocyclyl.

The term "spiroheterocyclyl" refers to a polycyclic heterocyclic system in which an atom (referred to as a spiro atom) is shared between rings; the spiroheterocyclyl may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded), with the proviso that at least one monocyclic heterocyclyl group is contained and the point of attachment is on the monocyclic heterocyclyl group; the spiroheterocyclyl has 5 to 20 (e.g., 5, 6, 7, 8, 9, 10, 11, 12, 13, 14, 15, 16, 17, 18, 19, or 20) ring atoms (i.e., 5- to 20-membered spiroheterocyclyl). Preferably, the spiroheterocyclyl is a spiroheterocyclyl group having 6 to 14 ring atoms (i.e., 6- to 14-membered spiroheterocyclyl); more preferably, the spiroheterocyclyl is a spiroheterocyclyl group having 7 to 10 ring atoms (i.e., 7- to 10-membered spiroheterocyclyl). The spiroheterocyclyl includes monospiroheterocyclyl and polyspiroheterocyclyl (e.g., bispiroheterocyclyl); monospiroheterocyclyl or bispiroheterocyclyl is preferred, and 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospiroheterocyclyl is more preferred. Non-limiting examples include: 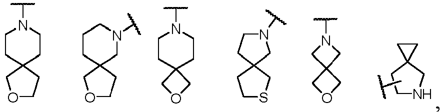 and the like.

The term "fused heterocyclyl" refers to a polycyclic heterocyclic system in which two adjacent atoms are shared between rings; the fused heterocyclyl may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded); the fused heterocyclyl is formed by fusing a monocyclic heterocyclyl group with one or more monocyclic heterocyclyl groups, or fusing a monocyclic heterocyclyl group with one or more of a cycloalkyl group, an aryl group, or a heteroaryl group, wherein the point of attachment is on a monocyclic heterocyclyl group; the fused heterocyclyl has 5 to 20 (e.g., 5, 6, 7, 8, 9, 10, 11, 12, 13, 14, 15, 16, 17, 18, 19, or 20) ring atoms (i.e., 5- to 20-membered fused heterocyclyl). Preferably, the fused heterocyclyl is a fused heterocyclyl group having 6 to 14 ring atoms (i.e., 6- to 14-membered fused heterocyclyl); more preferably, the fused heterocyclyl is a fused heterocyclyl group having 7 to 10 ring atoms (i.e., 7- to 10-membered fused heterocyclyl). The fused heterocyclyl includes bicyclic and polycyclic fused heterocyclyl (e.g., tricyclic fused heterocyclyl and tetracyclic fused heterocyclyl); bicyclic fused heterocyclyl or tricyclic fused heterocyclyl is preferred, and 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused heterocyclyl is more preferred. Non-limiting examples include: 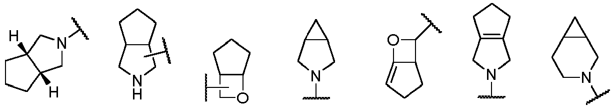 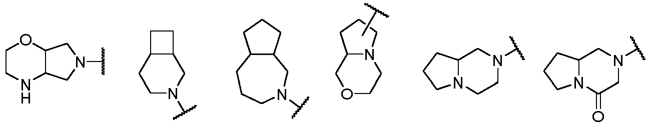 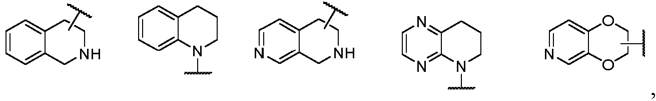 and the like.

The term "bridged heterocyclyl" refers to a polycyclic heterocyclic system in which two atoms that are not directly connected are shared between rings; the bridged heterocyclyl may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or - S-S- is excluded); the bridged heterocyclyl has 5 to 20 (e.g., 5, 6, 7, 8, 9, 10, 11, 12, 13, 14, 15, 16, 17, 18, 19, or 20) ring atoms (i.e., 5- to 20-membered bridged heterocyclyl). Preferably, the bridged heterocyclyl is a bridged heterocyclyl group having 6 to 14 ring atoms (i.e., 6- to 14-membered bridged heterocyclyl); more preferably, the bridged heterocyclyl is a bridged heterocyclyl group having 7 to 10 ring atoms (i.e., 7- to 10-membered bridged heterocyclyl). According to the number of constituent rings, the bridged heterocyclyl can be divided into bicyclic bridged heterocyclyl and polycyclic bridged heterocyclyl (e.g., tricyclic bridged heterocyclyl and tetracyclic bridged heterocyclyl); bicyclic bridged heterocyclyl or tricyclic bridged heterocyclyl is preferred. Non-limiting examples include: 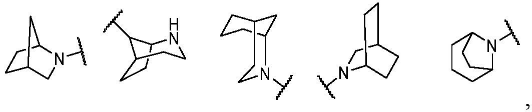 and the like.

Heterocyclyl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "aryl" refers to a monocyclic all-carbon aromatic ring (i.e., monocyclic aryl) or polycyclic aromatic ring system (i.e., polycyclic aryl) having a conjugated π-electron system; the aryl has 6 to 14 (e.g., 6, 7, 8, 9, 10, 11, 12, 13, or 14) ring atoms (i.e., 6- to 14-membered aryl). The aryl is preferably an aryl group having 6 to 10 ring atoms (i.e., 6- to 10-membered aryl). The monocyclic aryl is, for example, phenyl. Non-limiting examples of the polycyclic aryl include: naphthyl, anthryl, phenanthryl, and the like. The polycyclic aryl also includes those formed by fusing a phenyl group with one or more of a heterocyclyl group or a cycloalkyl group or fusing a naphthyl group with one or more of a heterocyclyl group or a cycloalkyl group, wherein the point of attachment is on the phenyl group or the naphthyl group, and in the circumstances the number of ring atoms continues to represent the number of ring atoms in the polycyclic aromatic ring system; non-limiting examples include: 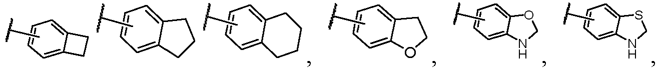 and the like.

Aryl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heteroaryl" refers to a monocyclic heteroaromatic ring (i.e., monocyclic heteroaryl) or polycyclic heteroaromatic ring system (i.e., polycyclic heteroaryl) having a conjugated π-electron system; the heteroaryl contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-Sis excluded) and has 5 to 14 (e.g., 5, 6, 7, 8, 9, 10, 11, 12, 13, or 14) ring atoms (i.e., 5- to 14-membered heteroaryl). Preferably, the heteroaryl is a heteroaryl group having 5 to 10 ring atoms (i.e., 5- to 10-membered heteroaryl); more preferably, the heteroaryl is a heteroaryl group having 5 or 6 ring atoms (i.e., 5- or 6-membered heteroaryl). Non-limiting examples of the monocyclic heteroaryl include: furanyl, thienyl, thiazolyl, isothiazolyl, oxazolyl, isoxazolyl, oxadiazolyl, thiadiazolyl, imidazolyl, pyrazolyl, triazolyl, tetrazolyl, furazanyl, pyrrolyl, N-alkylpyrrolyl, pyridinyl, pyrimidinyl, pyridonyl, N-alkylpyridinone (e.g., 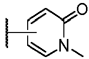 ), pyrazinyl, pyridazinyl, and the like.

Non-limiting examples of the polycyclic heteroaryl include: indolyl, indazolyl, quinolyl, isoquinolyl, quinoxalinyl, phthalazinyl, benzimidazolyl, benzothienyl, quinazolinyl, benzothiazolyl, carbazolyl, and the like. The polycyclic heteroaryl also includes those formed by fusing a monocyclic heteroaryl group with one or more aryl groups, wherein the point of attachment is on an aromatic ring, and in the circumstances the number of ring atoms continues to represent the number of ring atoms in the polycyclic heteroaromatic ring system. The polycyclic heteroaryl also includes those formed by fusing a monocyclic heteroaryl group with one or more of a cycloalkyl group or a heterocyclyl group, wherein the point of attachment is on the monocyclic heteroaromatic ring, and in the circumstances the number of ring atoms continues to represent the number of ring atoms in the polycyclic heteroaromatic ring system. Non-limiting examples include: 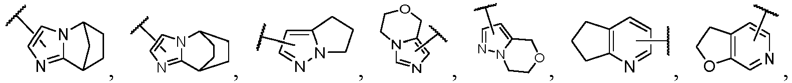 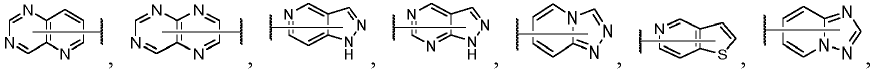 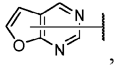 and the like.

Heteroaryl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "amino protecting group" refers to an easily removable group that is introduced onto an amino group so that the amino group can remain unchanged when reactions are taking place elsewhere in the molecule. Non-limiting examples include: (trimethylsilyl)ethoxymethyl, tetrahydropyranyl, tert-butyloxycarbonyl (Boc), benzyloxycarbonyl (Cbz), fluorenylmethoxycarbonyl (Fmoc), allyloxycarbonyl (Alloc), trimethylsilylethoxycarbonyl (Teoc), methoxycarbonyl, ethoxycarbonyl, phthaloyl (Pht), p-toluenesulfonyl (Tos), tert-butylsulfinyl, trifluoroacetyl (Tfa), trichloroacetyl, trityl (Trt), 2,4-dimethoxybenzyl (DMB), p-methoxybenzyl (PMB), acetyl, benzyl, allyl, p-methoxybenzyl, and the like.

The term "hydroxy protecting group" refers to an easily removable group that is introduced onto a hydroxy group to block or protect the hydroxy group such that reactions take place on other functional groups of the compound. Non-limiting examples include: trimethylsilyl (TMS), triethylsilyl (TES), triisopropylsilyl (TIPS), tert-butyldimethylsilyl (TBS), tert-butyldimethylsilyl (TBDMS), tert-butyldiphenylsilyl (TBDPS), methyl, tert-butyl, allyl, benzyl, methoxymethyl (MOM), ethoxyethyl, 2-tetrahydropyranyl (THP), formyl, acetyl, benzoyl, p-nitrobenzoyl, and the like.

The term "cycloalkylalkyl" refers to an alkyl group substituted with one or more cycloalkyl groups, wherein the cycloalkyl and alkyl groups are as defined above.

The term "heterocyclylalkyl" refers to an alkyl group substituted with one or more heterocyclyl groups, wherein the heterocyclyl and alkyl groups are as defined above. The term "alkoxyalkyl" refers to an alkyl group substituted with one or more alkoxy groups, wherein the alkoxy and alkyl groups are as defined above.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein the aryl is as defined above.

The term "heteroaryloxy" refers to heteroaryl-O-, wherein the heteroaryl is as defined above.

The term "alkylthio" refers to alkyl-S-, wherein the alkyl is as defined above.

The term "haloalkyl" refers to an alkyl group substituted with one or more halogens, wherein the alkyl group is as defined above.

The term "haloalkoxy" refers to an alkoxy group substituted with one or more halogens, wherein the alkoxy group is as defined above.

The term "deuterated alkyl" refers to an alkyl group substituted with one or more deuterium atoms, wherein the alkyl group is as defined above.

The term "hydroxyalkyl" refers to an alkyl group substituted with one or more hydroxy groups, wherein the alkyl group is as defined above.

The term "hydroxyalkoxy" refers to an alkoxy group substituted with one or more hydroxy groups, wherein the alkoxy group is as defined above.

The term "methylidene" refers to =CH₂.

The term "deuterated methyl" refers to a methyl group substituted with one or more deuterium atoms, e.g., CHD₂, CH₂D, and CD₃; CD₃ is preferred.

The term "deuterated methoxy" refers to -O-deuterated methyl, wherein the deuterated methyl is as defined above, e.g., OCD₃.

The term "halogen" refers to fluorine, chlorine, bromine, or iodine.

The term "hydroxy" refers to -OH.

The term "sulfhydryl" refers to -SH.

The term "amino" refers to -NH₂.

The term "cyano" refers to -CN.

The term "nitro" refers to -NO₂.

The term "oxo" refers to "=O".

The term "carbonyl" refers to C=O.

The term "acetyl" refers to -C(O)CH₃.

The term "amido" refers to -C(O)NH₂.

The term "carboxyl" refers to -C(O)OH.

The term "carboxylate group" refers to -C(O)O(alkyl), -C(O)O(cycloalkyl), (alkyl)C(O)O-, or (cycloalkyl)C(O)O-, wherein the alkyl and cycloalkyl are as defined above.

The compounds of the present disclosure may have specific stereoisomeric forms. The term "stereoisomer" refers to isomers that are structurally identical but differ in the arrangement of the atoms in space. It includes cis and trans (or *Z* and *E*) isomers, (-)- and (+)-isomers, ^{®}- and (*S*)-enantiomers, diastereomers, (*D*)- and (*L*)-isomers, tautomers, atropisomers, conformers, and mixtures thereof (e.g., mixtures of racemates and diastereomers). Additional asymmetric atoms may be present in the substituents in the compounds of the present disclosure. All such stereoisomers and mixtures thereof are included within the scope of the present disclosure. Optically active (-)- and (+)-isomers, (*R*)- and (*S*)-enantiomers, and (*D*)- and (*L*)-isomers can be prepared by chiral synthesis, chiral reagents, or other conventional techniques. One isomer of a certain compound of the present disclosure may be prepared by asymmetric synthesis or with a chiral auxiliary, or, when the molecule contains a basic functional group (e.g., amino) or an acidic functional group (e.g., carboxyl), a diastereomeric salt is formed with an appropriate optically active acid or base, followed by diastereomeric resolution by conventional methods known in the art to give the pure isomer. In addition, the separation of enantiomers and diastereomers is generally accomplished by chromatography.

In the chemical structures of the compounds of the present disclosure, a bond " 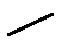 " represents an unspecified configuration; that is, if chiral isomers exist in the chemical structures, the bond " 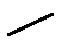 " may be " 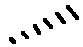 " or " 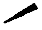 ", or both the configurations of " 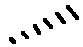 " and " 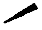 " are included simultaneously. In the chemical structures of the compounds of the present disclosure, a bond " 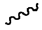 " is not specified with a configuration; that is, they may be in a Z configuration or an E configuration, or contain both configurations. For all carbon-carbon double bonds, both Z- and E-forms are included, even if only one configuration is named. The bond " 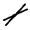 " does not specify a configuration; it may specify either the Z-configuration, the E-configuration, or both.

In the chemical structures of the compounds of the present disclosure, the bond " 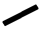 " or " 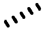 " attached to the stereogenic center of the compounds, as in 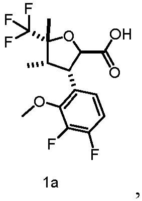 indicates the relative configuration of the stereogenic center, wherein the compound 1a of Example 1 is a mixture of 1b-1 and 1b-2.

The compounds of the present disclosure may also exist in different tautomeric forms, and all such forms are included within the scope of the present disclosure. The term "tautomer" or "tautomeric form" refers to a structural isomer that exists in equilibrium and is readily converted from one isomeric form into another. It includes all possible tautomers; that is, it is present in the form of a single isomer or in the form of a mixture of the tautomers in any ratio. Non-limiting examples include: keto-enol, imine-enamine, lactam-lactim, and the like. An example of the enamine-imine equilibrium is shown below:

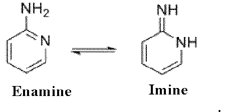

"Amidines", also known as iminoamides, are compounds in which the carbonyl oxygen atom in the amide molecule is substituted with an imino group. The imino and amino groups of amidines can be interconverted to form tautomers. The tautomers of amidines are shown below:

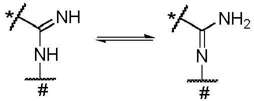

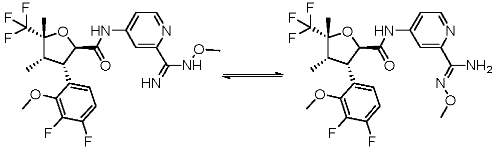

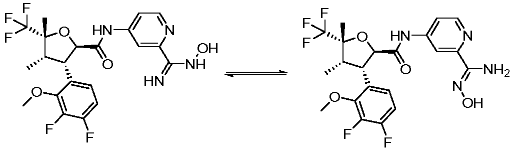

For example, reference to pyrazolyl is understood to include any one of the following two structures or a mixture of the two tautomers:

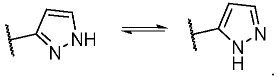

All tautomeric forms fall within the scope of the present disclosure, and the nomenclature of the compounds does not exclude any tautomer.

The compounds of the present disclosure include all suitable isotopic derivatives of the compounds thereof. The term "isotopic derivative" refers to a compound in which at least one atom is replaced with an atom having the same atomic number but a different atomic mass. Examples of isotopes that can be incorporated into the compounds of the present disclosure include stable and radioactive isotopes of hydrogen, carbon, nitrogen, oxygen, phosphorus, sulfur, fluorine, chlorine, bromine, iodine, etc., such as ²H (deuterium, D), ³H (tritium, T), ¹¹C, ¹³C, ¹⁴C, ¹⁵N, ¹⁷O, ¹⁸O, ³²p, ³³p, ³³S, ³⁴S, ³⁵S, ³⁶S, ¹⁸F, ³⁶Cl, ⁸²Br, ¹²³I, ¹²⁴I, ¹²⁵I, ¹²⁹I, and ¹³¹I, deuterium is preferred.

Compared to non-deuterated drugs, deuterated drugs have the advantages of reduced toxic and side effects, increased drug stability, enhanced efficacy, prolonged biological half-lives, and the like. All isotopic variations of the compounds of the present disclosure, whether radioactive or not, are intended to be included within the scope of the present disclosure. Each available hydrogen atom connected to a carbon atom may be independently replaced with a deuterium atom, wherein the replacement of deuterium may be partial or complete, and the replacement of partial deuterium refers to the replacement of at least one hydrogen atom with at least one deuterium atom.

In the compounds of the present disclosure, when a position is specifically assigned "deuterium" or "D", the position should be construed as the abundance of deuterium being at least 1000 times greater than the natural abundance of deuterium (which is 0.015%) (i.e., at least 15% deuterium incorporation). In some embodiments, the abundance of deuterium of each of the assigned deuterium atoms is at least 1000 times greater than the natural abundance of deuterium (i.e., at least 15% deuterium incorporation). In some embodiments, the abundance of deuterium of each of the assigned deuterium atoms is at least 2000 times greater than the natural abundance of deuterium (i.e., at least 30% deuterium incorporation). In some embodiments, the abundance of deuterium of each of the assigned deuterium atoms is at least 3000 times greater than the natural abundance of deuterium (i.e., at least 45% deuterium incorporation). In some embodiments, the abundance of deuterium of each of the assigned deuterium atoms is at least 3340 times greater than the natural abundance of deuterium (i.e., at least 50.1% deuterium incorporation). In some embodiments, the abundance of deuterium of each of the assigned deuterium atoms is at least 3500 times greater than the natural abundance of deuterium (i.e., at least 52.5% deuterium incorporation). In some embodiments, the abundance of deuterium of each of the assigned deuterium atoms is at least 4000 times greater than the natural abundance of deuterium (i.e., at least 60% deuterium incorporation). In some embodiments, the abundance of deuterium of each of the assigned deuterium atoms is at least 4500 times greater than the natural abundance of deuterium (i.e., at least 67.5% deuterium incorporation). In some embodiments, the abundance of deuterium of each of the assigned deuterium atoms is at least 5000 times greater than the natural abundance of deuterium (i.e., at least 75% deuterium incorporation). In some embodiments, the abundance of deuterium of each of the assigned deuterium atoms is at least 5500 times greater than the natural abundance of deuterium (i.e., at least 82.5% deuterium incorporation). In some embodiments, the abundance of deuterium of each of the assigned deuterium atoms is at least 6000 times greater than the natural abundance of deuterium (i.e., at least 90% deuterium incorporation). In some embodiments, the abundance of deuterium of each of the assigned deuterium atoms is at least 6333.3 times greater than the natural abundance of deuterium (i.e., at least 95% deuterium incorporation). In some embodiments, the abundance of deuterium of each of the assigned deuterium atoms is at least 6466.7 times greater than the natural abundance of deuterium (i.e., at least 97% deuterium incorporation). In some embodiments, the abundance of deuterium of each of the assigned deuterium atoms is at least 6600 times greater than the natural abundance of deuterium (i.e., at least 99% deuterium incorporation). In some embodiments, the abundance of deuterium of each of the assigned deuterium atoms is at least 6633.3 times greater than the natural abundance of deuterium (i.e., at least 99.5% deuterium incorporation).

"Optional" or "optionally" means that the event or circumstance subsequently described may, but does not necessarily, occur; this includes an instance where the event or circumstance occurs and an instance where it does not. For example, "alkyl optionally substituted with halogen or cyano" includes an instance where the alkyl is substituted with halogen or cyano and an instance where the alkyl is not substituted with halogen or cyano.

"Substitution" or "substituted" means that one or more, preferably 1-6, and more preferably 1-3, hydrogen atoms in the group are independently substituted with a corresponding number of substituents. Those skilled in the art can determine (experimentally or theoretically) possible or impossible substitutions without undue effort. For example, it may be unstable when an amino or hydroxy group having free hydrogen binds to a carbon atom having an unsaturated bond (e.g., an olefin).

"Pharmaceutical composition" refers to a mixture containing one or more of the compounds or the pharmaceutically acceptable salts thereof described herein, and other chemical components, and other components, for example, pharmaceutically acceptable carriers and excipients. The pharmaceutical composition is intended to promote administration to an organism and facilitate the absorption of the active ingredient so that it can exert its biological activity.

"Pharmaceutically acceptable salt" refers to a salt of the compound of the present disclosure, which may be selected from the group consisting of inorganic or organic salts. Such salts are safe and effective when used in the body of a mammal and possess the requisite biological activity. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

The term "pharmaceutically acceptable" as used herein means that those compounds, materials, compositions, and/or dosage forms that are, within the scope of reasonable medical judgment, suitable for use in contact with the tissues of patients without excessive toxicity, irritation, allergic reaction, or other problems or complications, and are commensurate with a reasonable benefit/risk ratio and effective for the intended use.

As used herein, the singular forms "a", "an" and "the" include plural references and vice versa, unless otherwise clearly defined in the context.

When the term "about" is applied to parameters such as pH, concentration, and temperature, it means that the parameter may vary by ±10%, and sometimes more preferably within ±5%. As will be understood by those skilled in the art, when the parameters are not critical, the numbers are generally given for illustrative purposes only and are not intended to be limiting.

### Synthesis Methods for Compounds of Present Disclosure

To achieve the purposes of the present disclosure, the following technical solutions are adopted in the present disclosure:

### Solution 1

The present disclosure provides a preparation method for a compound represented by general formula (I) or a pharmaceutically acceptable salt thereof, the method comprising: 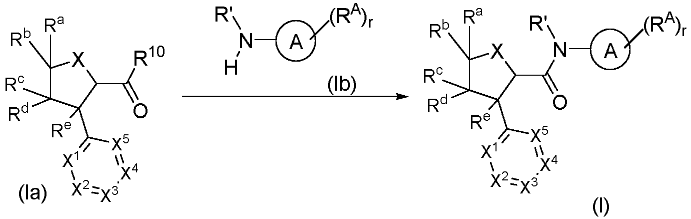 conducting a condensation reaction of a compound represented by general formula (Ia) or a salt thereof with a compound represented by general formula (Ib) or a salt thereof under alkaline conditions to give the compound represented by general formula (I) or the pharmaceutically acceptable salt thereof, wherein:
R¹⁰ is halogen (preferably a chlorine atom) or OH;
when R¹⁰ is OH, the reaction is conducted in the presence of a condensing agent;
ring A, R^{A}, R', R^{a}, R^{b}, R^{c}, R^{d}, R^{e}, X, X¹, X², X³, X⁴, X⁵, and r are as defined in general formula (I).

### Solution 1-1

The present disclosure provides a preparation method for a compound represented by general formula (I) or a pharmaceutically acceptable salt thereof, the method comprising: 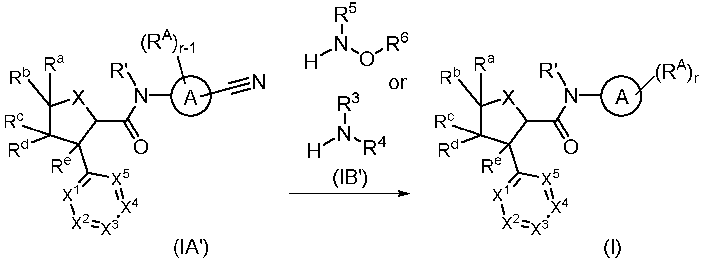 conducting a pinner reaction of a compound represented by general formula (IA') or a salt thereof with a compound represented by general formula (IB') or a salt thereof (preferably hydrochloride) in the presence of a catalyst to give the compound represented by general formula (I) or the pharmaceutically acceptable salt thereof,
preferably reacting a compound represented by general formula (IA') or a salt thereof with a compound represented by general formula (IB') or a salt thereof (preferably hydrochloride) in the presence of mercaptoacetic acid and an alkali to give the compound represented by general formula (I) or the pharmaceutically acceptable salt thereof, wherein:
at least one R^{A} in general formula (I) is -C(=NH)NR⁵-OR⁶ or -C(=NH)NR³R⁴; r is 1, 2, 3, 4, or 5; r-1 is 0, 1, 2, 3, or 4;
the remaining R^{A}s are as defined in general formula (I);
ring A, R', R^{a}, R^{b}, R^{c}, R^{d}, R^{e}, X, X¹, X², X³, X⁴, X⁵, R³, R⁴, R⁵*,* and R⁶ are as defined in general formula (I).

### Solution 2

The present disclosure provides a preparation method for a compound represented by general formula (II) or a pharmaceutically acceptable salt thereof, the method comprising: 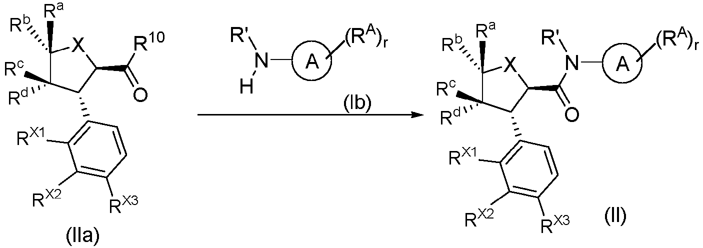 conducting a condensation reaction of a compound represented by general formula (IIa) or a salt thereof with a compound represented by general formula (Ib) or a salt thereof under alkaline conditions to give the compound represented by general formula (II) or the pharmaceutically acceptable salt thereof, wherein:
R¹⁰ is halogen (preferably a chlorine atom) or OH;
when R¹⁰ is OH, the reaction is conducted in the presence of a condensing agent or oxalyl chloride, preferably in the presence of a condensing agent;
ring A, R^{A}, R', R^{a}, R^{b}, R^{c}, R^{d}, X, R^{X1}, R^{X2}, R^{X3}, and r are as defined in general formula (II).

### Solution 2-1

The present disclosure provides a preparation method for a compound represented by general formula (II) or a pharmaceutically acceptable salt thereof, the method comprising: 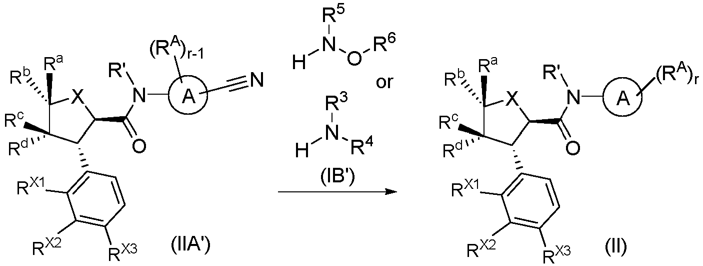
conducting a pinner reaction of a compound represented by general formula (IIA') or a salt thereof with a compound represented by general formula (IB') or a salt thereof (preferably hydrochloride) in the presence of a catalyst to give the compound represented by general formula (II) or the pharmaceutically acceptable salt thereof,
preferably reacting a compound represented by general formula (IIA') or a salt thereof with a compound represented by general formula (IB') or a salt thereof (preferably hydrochloride) in the presence of mercaptoacetic acid and an alkali to give the compound represented by general formula (II) or the pharmaceutically acceptable salt thereof, wherein:
   at least one R^{A} in general formula (II) is -C(=NH)NR⁵-OR⁶ or -C(=NH)NR³R⁴; r is 1, 2, 3, 4, or 5; r-1 is 0, 1, 2, 3, or 4;
   the remaining R^{A}s are as defined in general formula (II);
   ring A, R', R^{a}, R^{b}, R^{c}, R^{d}, X, R^{X1}, R^{X2}, R^{X3}, R³, R⁴, R⁵*,* and R⁶ are as defined in general formula (II).

### Solution 2-2

The present disclosure provides a preparation method for a compound represented by general formula (II) or a pharmaceutically acceptable salt thereof, the method comprising: 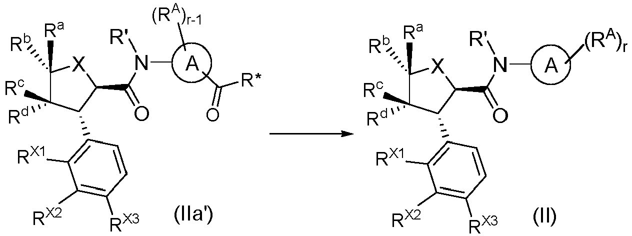 conducting an amidation reaction of a compound represented by general formula (IIa') or a salt thereof with an amine or a salt thereof (preferably hydrochloride) to give the compound represented by general formula (II) or the pharmaceutically acceptable salt thereof, wherein:
the amine described above is selected from the group consisting of HNR⁵-OR⁶, HNR⁵-NR³R⁴, and HNR⁵-alkylene-Cy;
R* is selected from the group consisting of hydroxy, alkoxy, and halogen;
at least one R^{A} in general formula (II) is selected from the group consisting of -C(O)NR⁵-OR⁶, -C(O)NR⁵-NR³R⁴, and -C(O)NR⁵-alkylene-Cy;
the remaining R^{A}s are as defined in general formula (II);
r is 1, 2, 3, 4, or 5; r-1 is 0, 1, 2, 3, or 4;
when an amino group comprises a protecting group, the method further comprises a step of removing the protecting group; the amino protecting group is preferably Boc;
ring A, R', X, R^{a}, R^{b}, R^{c}, R^{d}, R^{X1}, R^{X2}, R^{X3}, R³, R⁴, R⁵*,* R⁶, and Cy are as defined in general formula (II).

### Solution 2-3

The present disclosure provides a preparation method for a compound represented by general formula (II) or a pharmaceutically acceptable salt thereof, the method comprising: conducting an alkyl-removing or cycloalkyl-removing reaction of a compound represented by general formula (II) in which R^{X1} is alkoxy or cycloalkoxy, or a pharmaceutically acceptable salt thereof under acidic conditions to give a compound represented by general formula (II) in which R^{X1} is -OH, or a pharmaceutically acceptable salt thereof.

### Solution 2-4

The present disclosure provides a preparation method for a compound represented by general formula (II) or a pharmaceutically acceptable salt thereof, the method comprising: 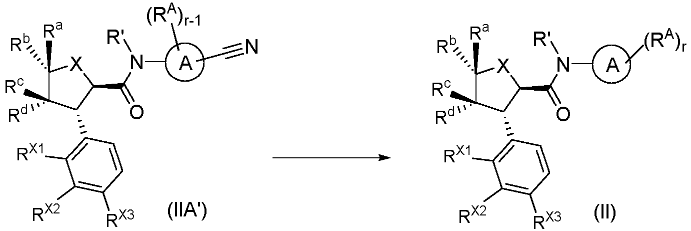 conducting a pinner reaction of a compound represented by general formula (IIA') or a salt thereof with NH₂-R⁵ or a salt thereof (preferably hydrochloride) in the presence of a catalyst to give the compound represented by general formula (II) or the pharmaceutically acceptable salt thereof, wherein:
at least one R^{A} in general formula (II) is -C(=NR⁵)NR³R⁴;
r is 1, 2, 3, 4, or 5; r-1 is 0, 1, 2, 3, or 4;
R³ and R⁴ are both hydrogen atoms;
the remaining R^{A}s are as defined in general formula (II);
ring A, R', R^{a}, R^{b}, R^{c}, R^{d}, X, R^{X1}, R^{X2}, R^{X3,} and R⁵ are as defined in general formula (II).

### Solution 2-A

The present disclosure provides a preparation method for a compound represented by general formula (II') or a pharmaceutically acceptable salt thereof, the method comprising: 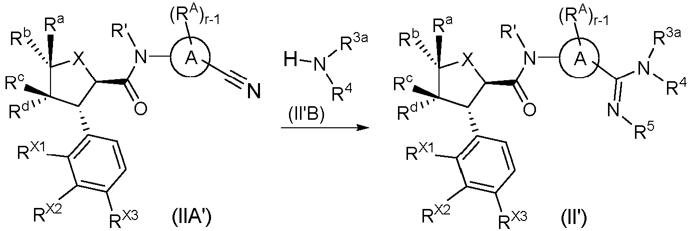 conducting a pinner reaction of a compound represented by general formula (IIA') or a salt thereof with a compound represented by general formula (II'B) or a salt thereof (preferably hydrochloride) in the presence of a catalyst to give the compound represented by general formula (II') or the pharmaceutically acceptable salt thereof, wherein:
R⁵ is a hydrogen atom;
ring A, R^{A}, R', X, R^{a}, R^{b}, R^{c}, R^{d}, R^{X1}, R^{X2}, R^{X3,} R^{3a}, R⁴, and r-1 are as defined in general formula (II').

### Solution 2-A1

The present disclosure provides a preparation method for a compound represented by general formula (II') or a pharmaceutically acceptable salt thereof, the method comprising: 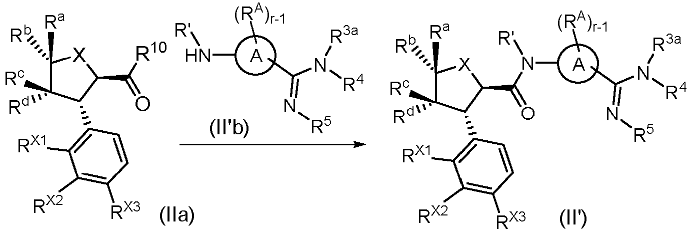 conducting a condensation reaction of a compound represented by general formula (IIa) or a salt thereof with a compound represented by general formula (II'b) or a salt thereof (preferably hydrochloride) under alkaline conditions to give the compound represented by general formula (II') or the pharmaceutically acceptable salt thereof, wherein:
R¹⁰ is halogen (preferably a chlorine atom) or OH; when R¹⁰ is OH, the reaction is conducted in the presence of a condensing agent or oxalyl chloride, preferably in the presence of a condensing agent;
ring A, R^{A}, R', X, R^{a}, R^{b}, R^{c}, R^{d}, R^{X1}, R^{X2}, R^{X3}, R^{3a}, R⁴, R⁵*,* and r-1 are as defined in general formula (II').

### Solution 2-A2

The present disclosure provides a preparation method for a compound represented by general formula (II') or a pharmaceutically acceptable salt thereof, the method comprising: conducting an alkyl-removing or cycloalkyl-removing reaction of a compound represented by general formula (II') in which R^{X1} is alkoxy or cycloalkoxy, or a pharmaceutically acceptable salt thereof under acidic conditions to give a compound represented by general formula (II') in which R^{X1} is -OH, or a pharmaceutically acceptable salt thereof.

### Solution 2-A3

The present disclosure provides a preparation method for a compound represented by general formula (II') or a pharmaceutically acceptable salt thereof, the method comprising: 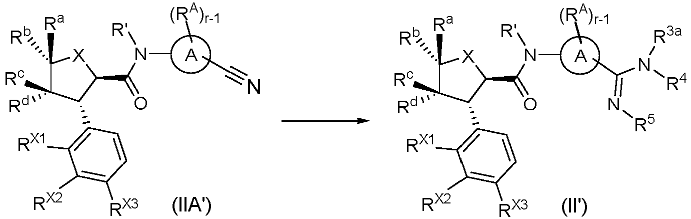 conducting a pinner reaction of a compound represented by general formula (IIA') or a salt thereof with NH₂-R⁵ or a salt thereof (preferably hydrochloride) in the presence of a catalyst to give the compound represented by general formula (II') or the pharmaceutically acceptable salt thereof, wherein:
R^{3a} and R⁴ are both hydrogen atoms;
ring A, R^{A}, R', X, R^{a}, R^{b}, R^{c}, R^{d}, R^{X1}, R^{X2}, R^{X3}, R⁵, and r-1 are as defined in general formula (II').

### Solution 2-B

The present disclosure provides a preparation method for a compound represented by general formula (II-1) or a pharmaceutically acceptable salt thereof, the method comprising: 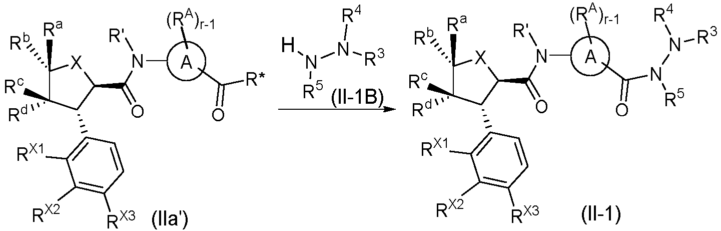 conducting an amidation reaction of a compound represented by general formula (IIa') or a salt thereof with a compound represented by general formula (II-1B) or a salt thereof (preferably hydrochloride) under alkaline conditions to give the compound represented by general formula (II-1) or the pharmaceutically acceptable salt thereof, wherein:
R* is selected from the group consisting of hydroxy, alkoxy, and halogen;
when an amino group comprises a protecting group, the method further comprises a step of removing the protecting group; the amino protecting group is preferably Boc;
ring A, R^{A}, R', X, R^{a}, R^{b}, R^{c}, R^{d}, R^{X1}, R^{X2}, R^{X3}, R³, R⁴, R⁵, and r-1 are as defined in general formula (II-1).

### Solution 2-B 1

The present disclosure provides a preparation method for a compound represented by general formula (II-1) or a pharmaceutically acceptable salt thereof, the method comprising: 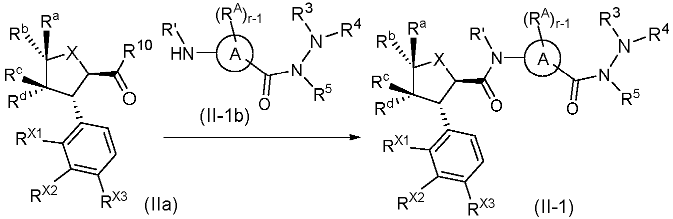 conducting a condensation reaction of a compound represented by general formula (IIa) or a salt thereof with a compound represented by general formula (II-1b) or a salt thereof (preferably hydrochloride) under alkaline conditions to give the compound represented by general formula (II-1) or the pharmaceutically acceptable salt thereof, wherein:
R¹⁰ is halogen (preferably a chlorine atom) or OH; when R¹⁰ is OH, the reaction is conducted in the presence of a condensing agent or oxalyl chloride, preferably in the presence of a condensing agent;
ring A, R^{A}, R', X, R^{a}, R^{b}, R^{c}, R^{d}, R^{X1}, R^{X2}, R^{X3}, R³, R⁴, R⁵, and r-1 are as defined in general formula (II-1).

### Solution 2-B2

The present disclosure provides a preparation method for a compound represented by general formula (II-1) or a pharmaceutically acceptable salt thereof, the method comprising: conducting an alkyl-removing or cycloalkyl-removing reaction of a compound represented by general formula (II-1) in which R^{X1} is alkoxy or cycloalkoxy, or a pharmaceutically acceptable salt thereof under acidic conditions to give a compound represented by general formula (II-1) in which R^{X1} is -OH, or a pharmaceutically acceptable salt thereof.

### Solution 3

The present disclosure provides a preparation method for a compound represented by general formula (III) or a pharmaceutically acceptable salt thereof, the method comprising: 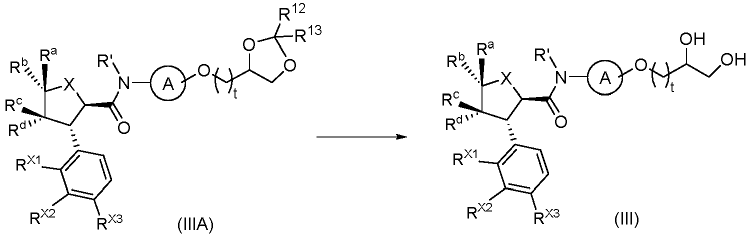 hydrolyzing a compound represented by general formula (IIIA) or a salt thereof under acidic conditions to give the compound represented by general formula (III) or the pharmaceutically acceptable salt thereof, wherein:
R¹² and R¹³ are identical or different and are each independently selected from the group consisting of a hydrogen atom, alkyl, cycloalkyl, and phenyl; preferably, R¹² and R¹³ are identical or different and are each independently selected from the group consisting of a hydrogen atom, C₁₋₆ alkyl, and 5- or 6-membered cycloalkyl; most preferably, R¹² and R¹³ are both CH₃;
ring A, R', R^{a}, R^{b}, R^{c}, R^{d}, X, R^{X1}, R^{X2}, R^{X3}, and t are as defined in general formula (III).

### Solution 3-1

The present disclosure provides a preparation method for a compound represented by general formula (III) or a pharmaceutically acceptable salt thereof, the method comprising: conducting an alkyl-removing or cycloalkyl-removing reaction of a compound represented by general formula (III) in which R^{X1} is alkoxy or cycloalkoxy, or a pharmaceutically acceptable salt thereof under acidic conditions to give a compound represented by general formula (III) in which R^{X1} is -OH, or a pharmaceutically acceptable salt thereof.

### Solution 4

The present disclosure provides a preparation method for a compound represented by general formula (III-1) or a pharmaceutically acceptable salt thereof, the method comprising: 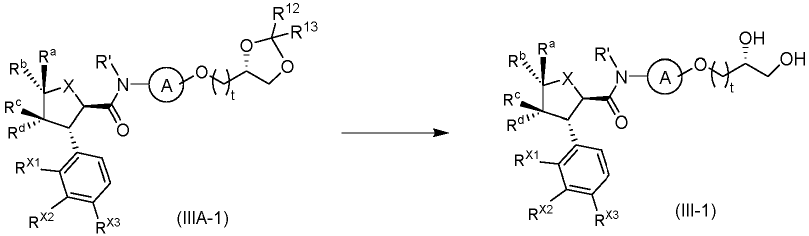 hydrolyzing a compound represented by general formula (IIIA-1) or a salt thereof under acidic conditions to give the compound represented by general formula (III-1) or the pharmaceutically acceptable salt thereof, wherein:
R¹² and R¹³ are identical or different and are each independently selected from the group consisting of a hydrogen atom, alkyl, cycloalkyl, and phenyl; preferably, R¹² and R¹³ are identical or different and are each independently selected from the group consisting of a hydrogen atom, C₁₋₆ alkyl, and 5- or 6-membered cycloalkyl; most preferably, R¹² and R¹³ are both CH₃;
ring A, R', R^{a}, R^{b}, R^{c}, R^{d}, X, R^{X1}, R^{X2}, R^{X3}, and t are as defined in general formula (III-1).

### Solution 4-1

The present disclosure provides a preparation method for a compound represented by general formula (III-1) or a pharmaceutically acceptable salt thereof, the method comprising: conducting an alkyl-removing or cycloalkyl-removing reaction of a compound represented by general formula (III-1) in which R^{X1} is alkoxy or cycloalkoxy, or a pharmaceutically acceptable salt thereof under acidic conditions to give a compound represented by general formula (III-1) in which R^{X1} is -OH, or a pharmaceutically acceptable salt thereof.

### Solution 5

The present disclosure provides a method for a compound represented by general formula (IV) or a pharmaceutically acceptable salt thereof, the method comprising: 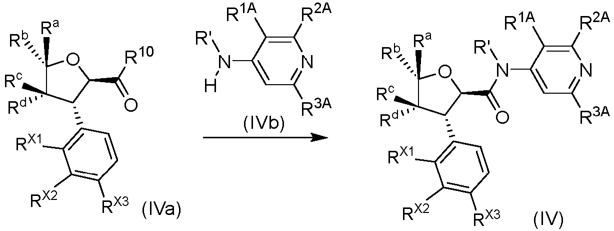 conducting a condensation reaction of a compound represented by general formula (IVa) or a salt thereof with a compound represented by general formula (IVb) or a salt thereof under alkaline conditions to give the compound represented by general formula (IV) or the pharmaceutically acceptable salt thereof, wherein:
R¹⁰ is halogen (preferably a chlorine atom) or OH;
when R¹⁰ is OH, the reaction is conducted in the presence of a condensing agent or oxalyl chloride, preferably in the presence of a condensing agent;
R^{a}, R^{b}, R^{c}, R^{d}, R^{X1}, R^{X2}, R^{X3}, R', R^{1A}, R^{2A}, and R^{3A} are as defined in general formula (IV).

### Solution 5-1

The present disclosure provides a method for a compound represented by general formula (IV) or a pharmaceutically acceptable salt thereof, the method comprising: 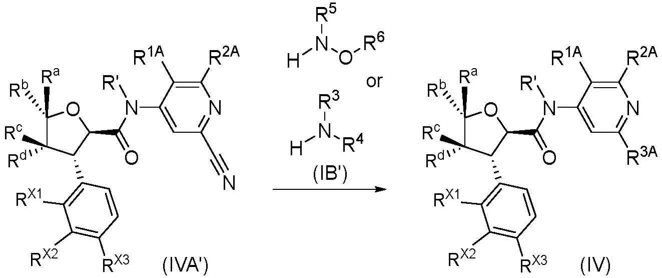
reacting a compound represented by general formula (IVA') or a salt thereof with a compound represented by general formula (IB') or a salt thereof (preferably hydrochloride) in the presence of a catalyst to give the compound represented by general formula (IV) or the pharmaceutically acceptable salt thereof,
preferably reacting a compound represented by general formula (IVA') or a salt thereof with a compound represented by general formula (IB') or a salt thereof (preferably hydrochloride) in the presence of mercaptoacetic acid and an alkali to give the compound represented by general formula (IV) or the pharmaceutically acceptable salt thereof, wherein:
   R^{3A} is -C(=NH)NR⁵-OR⁶ or -C(=NH)NR³R⁴;
   R^{1A}, R^{2A} , R', R^{a}, R^{b}, R^{c}, R^{d}, R^{X1}, R^{X2}, R^{X3}, R³, R⁴, R⁵, and R⁶ are as defined in general formula (IV).

### Solution 5-2

The present disclosure provides a method for a compound represented by general formula (IV) or a pharmaceutically acceptable salt thereof, the method comprising: 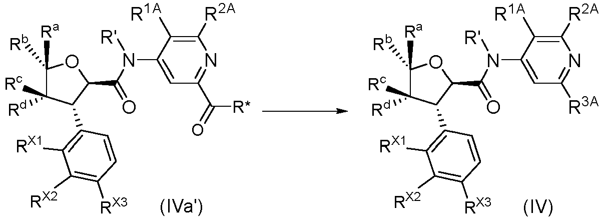 conducting an amidation reaction of a compound represented by general formula (IVa') or a salt thereof with an amine or a salt thereof (preferably hydrochloride) under alkaline conditions to give the compound represented by general formula (IV) or the pharmaceutically acceptable salt thereof, wherein:
the amine described above is selected from the group consisting of HNR⁵-OR⁶, HNR⁵-NR³R⁴, and HNR⁵-alkylene-Cy;
R* is selected from the group consisting of hydroxy, alkoxy, and halogen;
R^{3A} is selected from the group consisting of -C(O)NR⁵-OR⁶, -C(O)NR⁵-NR³R⁴, and-C(O)NR⁵-alkylene-Cy;
when an amino group comprises a protecting group, the method further comprises a step of removing the protecting group; the amino protecting group is preferably Boc;
R^{1A}, R^{2A} , R', R^{a}, R^{b}, R^{c}, R^{d}, R^{X1}, R^{X2}, R^{X3}, R³, R⁴, R⁵, R⁶, and Cy are as defined in general formula (IV).

### Solution 5-3

The present disclosure provides a method for a compound represented by general formula (IV) or a pharmaceutically acceptable salt thereof, the method comprising: conducting an alkyl-removing or cycloalkyl-removing reaction of a compound represented by general formula (IV) in which R^{X1} is alkoxy or cycloalkoxy, or a pharmaceutically acceptable salt thereof under acidic conditions to give a compound represented by general formula (IV) in which R^{X1} is -OH, or a pharmaceutically acceptable salt thereof.

### Solution 5-4

The present disclosure provides a method for a compound represented by general formula (IV) or a pharmaceutically acceptable salt thereof, the method comprising: 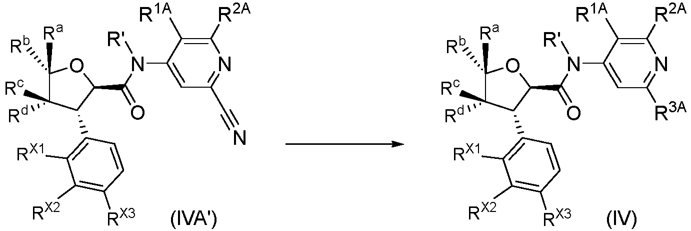 reacting a compound represented by general formula (IVA') or a salt thereof with NH₂-R⁵ or a salt thereof (preferably hydrochloride) in the presence of a catalyst to give the compound represented by general formula (IV) or the pharmaceutically acceptable salt thereof, wherein:
R^{3A} is -C(=NR⁵)NR³R⁴;
R^{1A}, R^{2A}, R', R^{a}, R^{b}, R^{c}, R^{d}, R^{X1}, R^{X2}, R^{X3}, and R⁵ are as defined in general formula (IV).

### Solution 6

The present disclosure provides a method for a compound represented by general formula (V) or a pharmaceutically acceptable salt thereof, the method comprising: 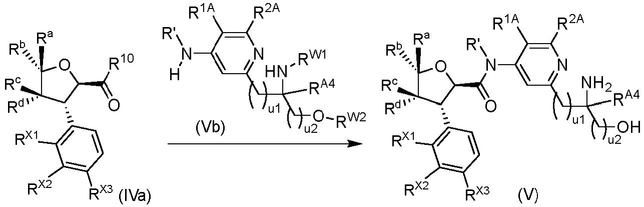 conducting a condensation reaction of a compound represented by general formula (IVa) or a salt thereof with a compound represented by general formula (Vb) or a salt thereof to give the compound represented by general formula (V) or the pharmaceutically acceptable salt thereof, wherein:
R^{W1} is a hydrogen atom or an amino protecting group (preferably tert-butylsulfinyl), and R^{w2} is a hydrogen atom or a hydroxy protecting group (preferably TBS);
when R^{W1} is an amino protecting group and/or R^{W2} is a hydroxy protecting group, the method further comprises a step of removing the protecting group(s) under acidic conditions after the condensation reaction;
R¹⁰ is halogen (preferably a chlorine atom) or OH; when R¹⁰ is OH, the reaction is conducted in the presence of a condensing agent or oxalyl chloride, preferably in the presence of a condensing agent;
R^{a}, R^{b}, R^{c}, R^{d}, R^{X1}, R^{X2}, R^{X3}, R', R^{1A}, R^{2A}, R^{A4}, u1, and u2 are as defined in general formula (V).

### Solution 6-1

The present disclosure provides a method for a compound represented by general formula (V) or a pharmaceutically acceptable salt thereof, the method comprising: conducting an alkyl-removing or cycloalkyl-removing reaction of a compound represented by general formula (V) in which R^{X1} is alkoxy or cycloalkoxy, or a pharmaceutically acceptable salt thereof under acidic conditions to give a compound represented by general formula (V) in which R^{X1} is -OH, or a pharmaceutically acceptable salt thereof.

### Solution 7

The present disclosure provides a method for a compound represented by general formula (V-1) or (V-2) or a pharmaceutically acceptable salt thereof, the method comprising: 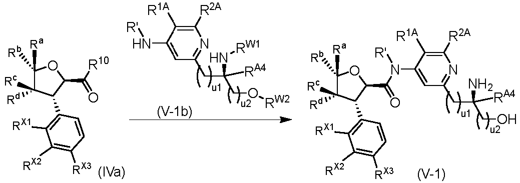 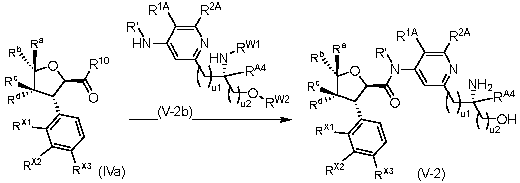
conducting a condensation reaction of a compound represented by general formula (IVa) or a salt thereof with a compound represented by general formula (V-1b) or a salt thereof under alkaline conditions to give the compound represented by general formula (V-1) or the pharmaceutically acceptable salt thereof, or
conducting a condensation reaction of a compound represented by general formula (IVa) or a salt thereof with a compound represented by general formula (V-2b) or a salt thereof under alkaline conditions to give the compound represented by general formula (V-2) or the pharmaceutically acceptable salt thereof, wherein:
   R^{W1} is a hydrogen atom or an amino protecting group (preferably tert-butylsulfinyl), and R^{W2} is a hydrogen atom or a hydroxy protecting group (preferably TBS);
   when R^{W1} is an amino protecting group and/or R^{W2} is a hydroxy protecting group, the method further comprises a step of removing the protecting group(s) under acidic conditions after the condensation reaction;
   R¹⁰ is halogen (preferably a chlorine atom) or OH; when R¹⁰ is OH, the reaction is conducted in the presence of a condensing agent or oxalyl chloride, preferably in the presence of a condensing agent;
   R^{a}, R^{b}, R^{c}, R^{d}, R^{X1}, R^{X2}, R^{X3}, R', R^{1A}, R^{2A}, R^{A4}, u1, and u2 are as defined in general formula (V-1).

### Solution 7-1

The present disclosure provides a method for a compound represented by general formula (V-1) or (V-2) or a pharmaceutically acceptable salt thereof, the method comprising: conducting an alkyl-removing or cycloalkyl-removing reaction of a compound represented by general formula (V-1) or (V-2) in which R^{X1} is alkoxy or cycloalkoxy, or a pharmaceutically acceptable salt thereof under acidic conditions to give a compound represented by general formula (V-1) or (V-2) in which R^{X1} is -OH, or a pharmaceutically acceptable salt thereof.

### Solution 8

The present disclosure provides a method for a compound represented by general formula (V-1) or (V-2) or a pharmaceutically acceptable salt thereof, the method comprising: 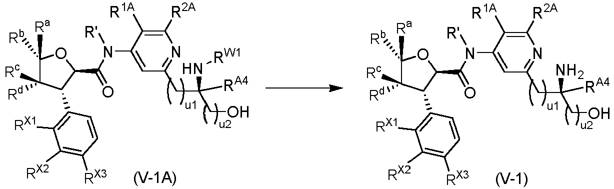 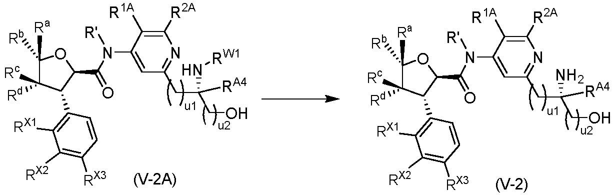
conducting a deprotection reaction of a compound represented by general formula (V-1A) or a salt thereof under acidic conditions to give the compound represented by general formula (V-1) or the pharmaceutically acceptable salt thereof, or
conducting a deprotection reaction of a compound represented by general formula (V-2A) or a salt thereof under acidic conditions to give the compound represented by general formula (V-2) or the pharmaceutically acceptable salt thereof, wherein:
   R^{W1} is an amino protecting group, and the amino protecting group is preferably Boc;
   R^{a}, R^{b}, R^{c}, R^{d}, R^{X1}, R^{X2}, R^{X3}, R', R^{1A}, R^{2A}, R^{A4}, u1, and u2 are as defined in general formula (V-1).

### Solution 9-A1

The present disclosure provides a method for a compound represented by general formula (VI') or a pharmaceutically acceptable salt thereof, the method comprising: 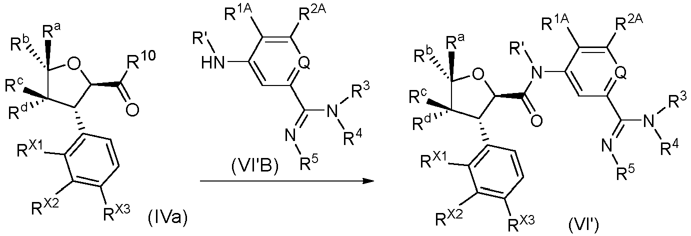 conducting a condensation reaction of a compound represented by general formula (IVa) or a salt thereof with a compound represented by general formula (VI'B) or a salt thereof (preferably hydrochloride) under alkaline conditions to give the compound represented by general formula (VI') or the pharmaceutically acceptable salt thereof, wherein:
R¹⁰ is halogen (preferably a chlorine atom) or OH; when R¹⁰ is OH, the reaction is conducted in the presence of a condensing agent or oxalyl chloride, preferably in the presence of a condensing agent;
R', R^{a}, R^{b}, R^{c}, R^{d}, R^{X1}, R^{X2}, R^{X3}, R^{1A}, R^{2A}, Q, R³, R⁴, and R⁵ are as defined in general formula (VI').

### Solution 9-A2

The present disclosure provides a method for a compound represented by general formula (VI') or a pharmaceutically acceptable salt thereof, the method comprising: 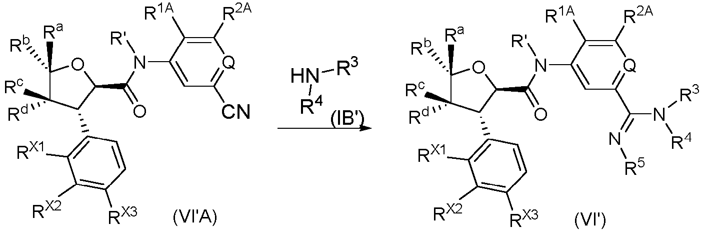 conducting a pinner reaction of a compound represented by general formula (VI'A) or a salt thereof with a compound represented by general formula (IB') or a salt thereof (preferably hydrochloride) to give the compound represented by general formula (VI') or the pharmaceutically acceptable salt thereof, wherein:
R⁵ is a hydrogen atom;
R', R^{a}, R^{b}, R^{c}, R^{d}, R^{X1}, R^{X2}, R^{X3}, R^{1A}, R^{2A}, Q, R³, and R⁴ are as defined in general formula (VI').

### Solution 9-A3

The present disclosure provides a method for a compound represented by general formula (VI') or a pharmaceutically acceptable salt thereof, the method comprising: conducting an alkyl-removing or cycloalkyl-removing reaction of a compound represented by general formula (VI') in which R^{X1} is alkoxy or cycloalkoxy, or a pharmaceutically acceptable salt thereof under acidic conditions to give a compound represented by general formula (VI') in which R^{X1} is -OH, or a pharmaceutically acceptable salt thereof.

### Solution 9-A4

The present disclosure provides a method for a compound represented by general formula (VI') or a pharmaceutically acceptable salt thereof, the method comprising: 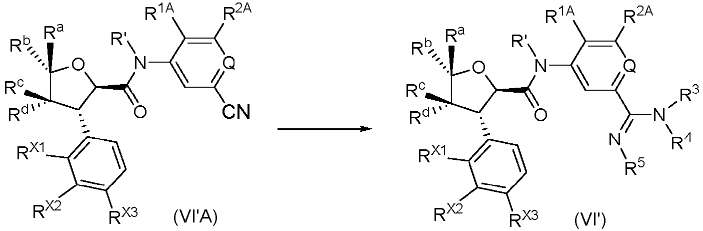 conducting a pinner reaction of a compound represented by general formula (VI'A) or a salt thereof with NH₂-R⁵ or a salt thereof (preferably hydrochloride) to give the compound represented by general formula (VI') or the pharmaceutically acceptable salt thereof, wherein:
R³ and R⁴ are both hydrogen atoms;
R', R^{a}, R^{b}, R^{c}, R^{d}, R^{X1}, R^{X2}, R^{X3}, R^{1A}, R^{2A}, Q, and R⁵ are as defined in general formula (VI').

### Solution 9-A-A1

The present disclosure provides a method for a compound represented by general formula (VI'-1) or a pharmaceutically acceptable salt thereof, the method comprising: 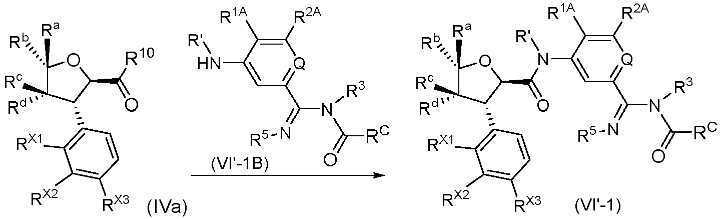 conducting a condensation reaction of a compound represented by general formula (IVa) or a salt thereof with a compound represented by general formula (VI'-1B) or a salt thereof (preferably hydrochloride) under alkaline conditions to give the compound represented by general formula (VI'-1) or the pharmaceutically acceptable salt thereof, wherein:
R¹⁰ is halogen (preferably a chlorine atom) or OH; when R¹⁰ is OH, the reaction is conducted in the presence of a condensing agent or oxalyl chloride, preferably in the presence of a condensing agent;
R', R^{a}, R^{b}, R^{c}, R^{d}, R^{X1}, R^{X2}, R^{X3}, R^{1A}, R^{2A}, Q, R³, R⁵, and R^{C} are as defined in general formula (VI'-1).

### Solution 9-A-A2

The present disclosure provides a method for a compound represented by general formula (VI'-1) or a pharmaceutically acceptable salt thereof, the method comprising: 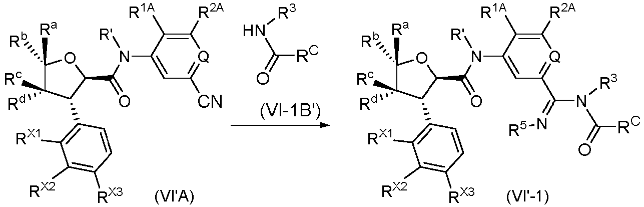 conducting a pinner reaction of a compound represented by general formula (VI'A) or a salt thereof with a compound represented by general formula (VI-1B') or a salt thereof (preferably hydrochloride) to give the compound represented by general formula (VI'-1) or the pharmaceutically acceptable salt thereof, wherein:
R⁵ is a hydrogen atom;
R', R^{a}, R^{b}, R^{c}, R^{d}, R^{X1}, R^{X2}, R^{X3}, R^{1A}, R^{2A}, Q, R³, and R^{C} are as defined in general formula (VI'-1).

### Solution 9-A-A3

The present disclosure provides a method for a compound represented by general formula (VI'-1) or a pharmaceutically acceptable salt thereof, the method comprising: 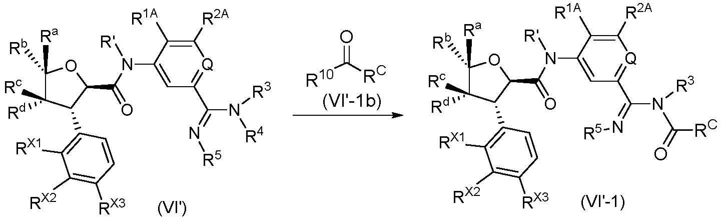 conducting an amidation reaction of a compound represented by general formula (VI') or a salt thereof (preferably trifluoroacetate) with a compound represented by general formula (VI'-1b) or a salt thereof (preferably hydrochloride) under alkaline conditions to give the compound represented by general formula (VI'-1) or the pharmaceutically acceptable salt thereof, wherein:
R⁴ is a hydrogen atom;
R¹⁰ is halogen (preferably chlorine) or OH; when R¹⁰ is OH, the reaction is conducted in the presence of a condensing agent or oxalyl chloride, preferably in the presence of a condensing agent;
R', R^{a}, R^{b}, R^{c}, R^{d}, R^{X1}, R^{X2}, R^{X3}, R^{1A}, R^{2A}, Q, R³, R⁵, and R^{C} are as defined in general formula (VI'-1).

### Solution 9-A-A4

The present disclosure provides a method for a compound represented by general formula (VI'-1) or a pharmaceutically acceptable salt thereof, the method comprising: conducting an alkyl-removing or cycloalkyl-removing reaction of a compound represented by general formula (VI'-1) in which R^{X1} is alkoxy or cycloalkoxy, or a pharmaceutically acceptable salt thereof under acidic conditions to give a compound represented by general formula (VI'-1) in which R^{X1} is -OH, or a pharmaceutically acceptable salt thereof.

### Solution 9

The present disclosure provides a method for a compound represented by general formula (VI) or a pharmaceutically acceptable salt thereof, the method comprising: 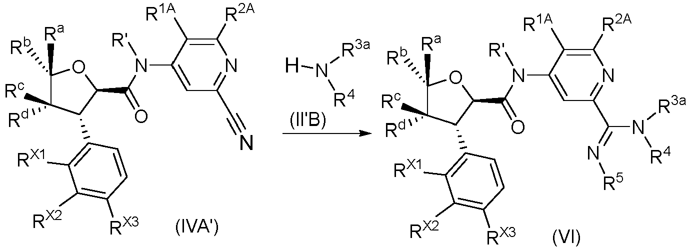 conducting a pinner reaction of a compound represented by general formula (IVA') or a salt thereof with a compound represented by general formula (II'B) or a salt thereof (preferably hydrochloride) in the presence of a catalyst to give the compound represented by general formula (VI) or the pharmaceutically acceptable salt thereof, wherein:
R⁵ is a hydrogen atom;
R^{1A}, R^{2A} , R', R^{a}, R^{b}, R^{c}, R^{d}, R^{X1}, R^{X2}, R^{X3}, R^{3a}, and R⁴ are as defined in general formula (VI).

### Solution 9-1

The present disclosure provides a method for a compound represented by general formula (VI) or a pharmaceutically acceptable salt thereof, the method comprising: 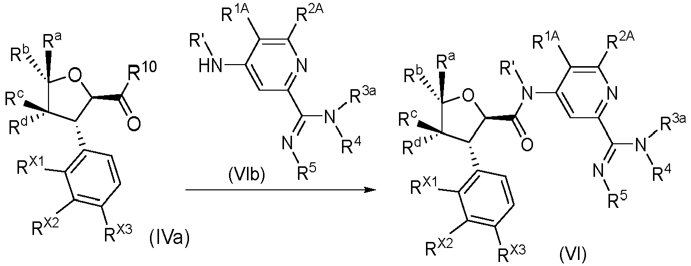 conducting a condensation reaction of a compound represented by general formula (IVa) or a salt thereof with a compound represented by general formula (VIb) or a salt thereof (preferably hydrochloride) under alkaline conditions to give the compound represented by general formula (VI) or the pharmaceutically acceptable salt thereof, wherein:
R¹⁰ is halogen (preferably a chlorine atom) or OH; when R¹⁰ is OH, the reaction is conducted in the presence of a condensing agent or oxalyl chloride, preferably in the presence of a condensing agent;
R', R^{a}, R^{b}, R^{c}, R^{d}, R^{X1}, R^{X2}, R^{X3}, R^{1A}, R^{2A}, R^{3a}, R⁴, and R⁵ are as defined in general formula (VI).

### Solution 9-2

The present disclosure provides a method for a compound represented by general formula (VI) or a pharmaceutically acceptable salt thereof, the method comprising: conducting an alkyl-removing or cycloalkyl-removing reaction of a compound represented by general formula (VI) in which R^{X1} is alkoxy or cycloalkoxy, or a pharmaceutically acceptable salt thereof under acidic conditions to give a compound represented by general formula (VI) in which R^{X1} is -OH, or a pharmaceutically acceptable salt thereof.

### Solution 9-3

The present disclosure provides a method for a compound represented by general formula (VI) or a pharmaceutically acceptable salt thereof, the method comprising: 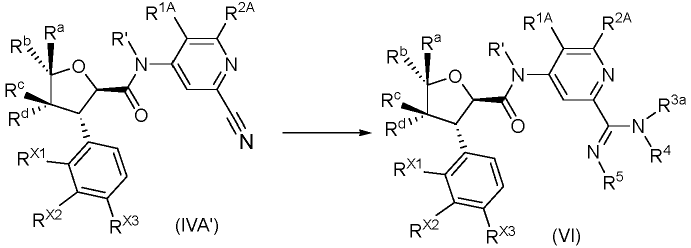 conducting a pinner reaction of a compound represented by general formula (IVA') or a salt thereof with NH₂-R⁵ or a salt thereof (preferably hydrochloride) in the presence of a catalyst to give the compound represented by general formula (VI) or the pharmaceutically acceptable salt thereof, wherein:
R^{3a} and R⁴ are both hydrogen atoms;
R^{1A}, R^{2A}, R', R^{a}, R^{b}, R^{c}, R^{d}, R^{X1}, R^{X2}, R^{X3}, and R⁵ are as defined in general formula (VI).

### Solution 10-A1

The present disclosure provides a method for a compound represented by general formula (VII') or a pharmaceutically acceptable salt thereof, the method comprising: 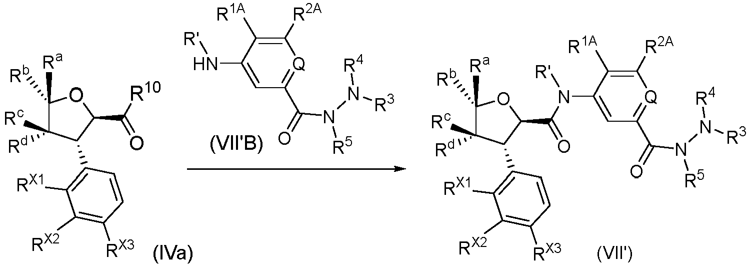 conducting a condensation reaction of a compound represented by general formula (IVa) or a salt thereof with a compound represented by general formula (VII'B) or a salt thereof (preferably hydrochloride) under alkaline conditions to give the compound represented by general formula (VII') or the pharmaceutically acceptable salt thereof, wherein:
R¹⁰ is halogen (preferably a chlorine atom) or OH; when R¹⁰ is OH, the reaction is conducted in the presence of a condensing agent or oxalyl chloride, preferably in the presence of a condensing agent;
R', R^{a}, R^{b}, R^{c}, R^{d}, R^{X1}, R^{X2}, R^{X3}, R^{1A}, R^{2A}, R³, R⁴, and R⁵ are as defined in general formula (VII').

### Solution 10-A2

The present disclosure provides a method for a compound represented by general formula (VII') or a pharmaceutically acceptable salt thereof, the method comprising: 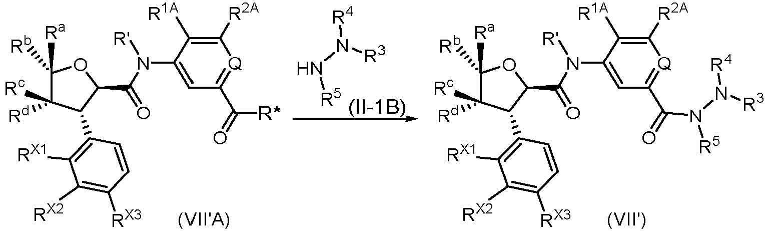 conducting a condensation reaction of a compound represented by general formula (VII') or a salt thereof with a compound represented by general formula (II-1B) or a salt thereof (preferably hydrochloride) under alkaline conditions to give the compound represented by general formula (VII') or the pharmaceutically acceptable salt thereof, wherein:
R* is selected from the group consisting of hydroxy, alkoxy, and halogen;
when an amino group comprises a protecting group, the method further comprises a step of removing the protecting group; the amino protecting group is preferably Boc;
R', R^{a}, R^{b}, R^{c}, R^{d}, R^{X1}, R^{X2}, R^{X3}, R^{1A}, R^{2A}, Q, R³, R⁴, and R⁵ are as defined in general formula (VII').

### Solution 10-A3

The present disclosure provides a method for a compound represented by general formula (VII') or a pharmaceutically acceptable salt thereof, the method comprising: conducting an alkyl-removing or cycloalkyl-removing reaction of a compound represented by general formula (VII') in which R^{X1} is alkoxy or cycloalkoxy, or a pharmaceutically acceptable salt thereof under acidic conditions to give a compound represented by general formula (VII') in which R^{X1} is -OH, or a pharmaceutically acceptable salt thereof.

### Solution 10

The present disclosure provides a method for a compound represented by general formula (VII) or a pharmaceutically acceptable salt thereof, the method comprising: 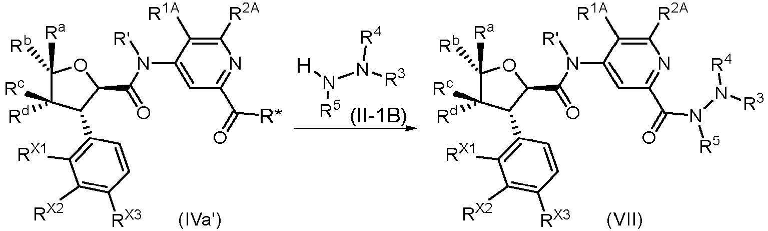
conducting an amidation reaction of a compound represented by general formula (IVa') or a salt thereof with a compound represented by general formula (II-1B) or a salt thereof (preferably hydrochloride) under alkaline conditions to give the compound represented by general formula (VII) or the pharmaceutically acceptable salt thereof,
wherein R* is selected from the group consisting of hydroxy, alkoxy, and halogen; when an amino group comprises a protecting group, the method further comprises a step of removing the protecting group; the amino protecting group is preferably Boc;
R^{1A}, R^{2A} , R', R^{a}, R^{b}, R^{c}, R^{d}, R^{X1}, R^{X2}, R^{X3}, R³, R⁴, and R⁵ are as defined in general formula (VII).

### Solution 10-1

The present disclosure provides a method for a compound represented by general formula (VII) or a pharmaceutically acceptable salt thereof, the method comprising: 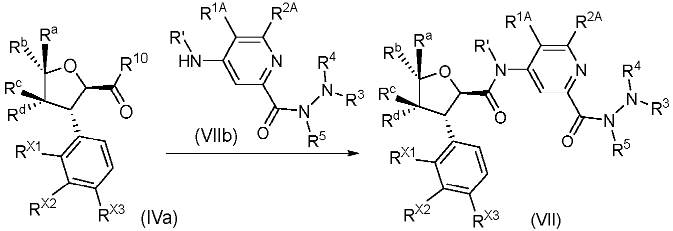 conducting a condensation reaction of a compound represented by general formula (IVa) or a salt thereof with a compound represented by general formula (VIIb) or a salt thereof (preferably hydrochloride) under alkaline conditions to give the compound represented by general formula (VII) or the pharmaceutically acceptable salt thereof, wherein:
R¹⁰ is halogen (preferably a chlorine atom) or OH; when R¹⁰ is OH, the reaction is conducted in the presence of a condensing agent or oxalyl chloride, preferably in the presence of a condensing agent;
R', R^{a}, R^{b}, R^{c}, R^{d}, R^{X1}, R^{X2}, R^{X3}, R^{1A}, R^{2A}, R³, R⁴, and R⁵ are as defined in general formula (VII).

### Solution 10-2

The present disclosure provides a method for a compound represented by general formula (VII) or a pharmaceutically acceptable salt thereof, the method comprising: conducting an alkyl-removing or cycloalkyl-removing reaction of a compound represented by general formula (VII) in which R^{X1} is alkoxy or cycloalkoxy, or a pharmaceutically acceptable salt thereof under acidic conditions to give a compound represented by general formula (VII) in which R^{X1} is -OH, or a pharmaceutically acceptable salt thereof.

### Solution 11-1

The present disclosure provides a method for a compound represented by general formula (VIII) or a pharmaceutically acceptable salt thereof, the method comprising: 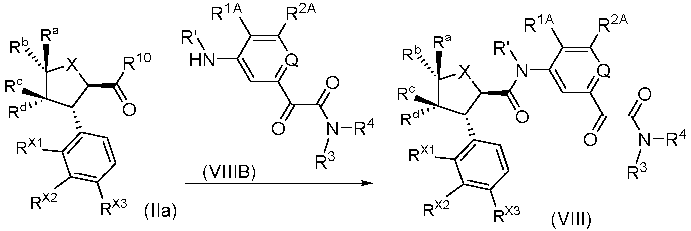 conducting a condensation reaction of a compound represented by general formula (IIa) or a salt thereof with a compound represented by general formula (VIIIB) or a salt thereof (preferably hydrochloride) under alkaline conditions to give the compound represented by general formula (VIII) or the pharmaceutically acceptable salt thereof, wherein:
R¹⁰ is halogen (preferably a chlorine atom) or OH; when R¹⁰ is OH, the reaction is conducted in the presence of a condensing agent or oxalyl chloride, preferably in the presence of a condensing agent;
R', X, R^{a}, R^{b}, R^{c}, R^{d}, R^{X1}, R^{X2}, R^{X3}, R^{1A}, R^{2A}, Q, R³, and R⁴ are as defined in general formula (VIII).

### Solution 11-2

The present disclosure provides a method for a compound represented by general formula (VIII) or a pharmaceutically acceptable salt thereof, the method comprising: 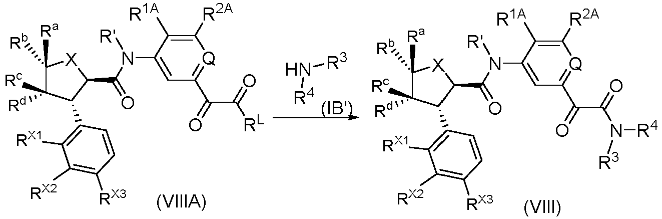 conducting a urethane exchange reaction of a compound represented by general formula (VIIIA) or a salt thereof with a compound represented by general formula (IB') or a salt thereof (preferably hydrochloride) to give the compound represented by general formula (VIII) or the pharmaceutically acceptable salt thereof, wherein:
R^{L} is alkoxy;
R', X, R^{a}, R^{b}, R^{c}, R^{d}, R^{X1}, R^{X2}, R^{X3}, R^{1A}, R^{2A}, Q, R³, and R⁴ are as defined in general formula (VIII).

### Solution 11-3

The present disclosure provides a method for a compound represented by general formula (VIII) or a pharmaceutically acceptable salt thereof, the method comprising: conducting an alkyl-removing or cycloalkyl-removing reaction of a compound represented by general formula (VIII) in which R^{X1} is alkoxy or cycloalkoxy, or a pharmaceutically acceptable salt thereof under acidic conditions to give a compound represented by general formula (VIII) in which R^{X1} is -OH, or a pharmaceutically acceptable salt thereof.

The above pinner reactions are based on a pinner amidine synthesis method. The specific mechanism is as follows: A starting-material cyano compound undergoes an alcoholysis reaction with an alcohol solvent in the presence of a catalyst to produce an imidate, and the imidate then undergoes an aminolysis or ammonolysis reaction with an amine or ammonia to produce an amidine.

The catalyst in the above pinner reactions may be an acid catalyst or an alkali catalyst. The acid catalyst is an organic acid or an inorganic acid, including, but not limited to, hydrochloric acid, hydrogen chloride gas, mercaptoacetic acid, N-acetylcysteine, and the like; mercaptoacetic acid is preferred. The alkali catalyst is an organic alkali or an inorganic alkali, including, but not limited to, sodium methoxide, sodium ethoxide, triethylamine, N,N-diisopropylethylamine, N,N-diisopropylethylenediamine, and the like; N,N-diisopropylethylamine or sodium methoxide is preferred, and sodium methoxide is more preferred.

The ammonia used in the above ammonolysis reaction is liquid ammonia, ammonia water, or an ammonium salt, preferably ammonium chloride. The amine used in the aminolysis reaction is an organic amine.

The above alcohol solvent includes, but is not limited to, methanol, ethanol, isopropanol, n-butanol, and the like.

The reagent that provides acidic conditions in the above synthesis solutions includes organic acids and inorganic acids; the organic acids include, but are not limited to, trifluoroacetic acid, formic acid, acetic acid, methanesulfonic acid, p-toluenesulfonic acid, Me₃SiCl, and TMSOTf, and the inorganic acids include, but are not limited to, hydrogen chloride, HCl in dioxane, hydrochloric acid in dioxane, hydrochloric acid, sulfuric acid, nitric acid, and phosphoric acid; HCl in dioxane or hydrochloric acid in dioxane is preferred. The reagent that provides acidic conditions in the alkyl-removing or cycloalkyl-removing reaction in the above synthesis solutions is preferably a Lewis acid, such as BBr₃ or ALCl₃; BBr₃ is preferred.

The reagent that provides alkaline conditions in the above synthesis solutions includes organic alkalis and inorganic alkalis; the organic alkalis include, but are not limited to, triethylamine, N,N-diisopropylethylamine, N,N-diisopropylethylenediamine, n-butyllithium, lithium diisopropylamide, potassium acetate, sodium tert-butoxide, potassium tert-butoxide, tetrabutylammonium fluoride, tetrabutylammonium fluoride in tetrahydrofuran, or 1,8-diazabicycloundec-7-ene, and the inorganic alkalis include, but are not limited to, sodium hydride, potassium phosphate, sodium carbonate, sodium acetate, potassium acetate, potassium carbonate, cesium carbonate, sodium hydroxide, lithium hydroxide, cesium fluoride, and potassium hydroxide; triethylamine or N,N-diisopropylethylamine is preferred. When R¹⁰ is halogen, the reagent that provides alkaline conditions is preferably triethylamine, and when R¹⁰ is OH, the reagent that provides alkaline conditions is preferably N,N-diisopropylethylamine. The condensation reactions are preferably conducted in the presence of a condensing agent as defined below. The alkali in the above synthesis solutions 1-1, 2-1, and 5-1 is preferably N,N-diisopropylethylamine.

In the above reactions, the condensing agent includes, but is not limited to, 1-(3-dimethylaminopropyl)-3-ethylcarbodiimide hydrochloride, *N,N'-*dicyclohexylcarbodiimide, *N,N'*-diisopropylcarbodiimide, O-benzotriazol-*N,N,N',N'-*tetramethyluronium tetrafluoroborate, 1-hydroxybenzotriazole, 1-hydroxy-7-azabenzotriazole, O-benzotriazol-*N,N,N',N'*-tetramethyluronium hexafluorophosphate, O-(7-azabenzotriazol-1-yl)-*N,N,N',N'*-tetramethyluronium hexafluorophosphate (HATU), 2-(7-oxybenzotriazole oxide)-*N,N,N',N'*-tetramethyluronium hexafluorophosphate, benzotriazol-1-yloxytris(dimethylamino)phosphonium hexafluorophosphate, or benzotriazol-1-yloxytripyrrolidinophosphonium hexafluorophosphate; HATU is preferred.

The condensation reactions in the above synthesis solutions, when R¹⁰ is halogen, are optionally conducted in the presence of a catalyst; the catalyst is preferably 4-dimethylaminopyridine.

The amidation reactions in the above synthesis solutions, when R* is hydroxy, are optionally conducted in the presence of a condensing agent; the condensing agent is as defined above.

The reactions of the above steps are preferably conducted in a solvent, including, but not limited to: pyridine, ethylene glycol dimethyl ether, acetic acid, methanol, ethanol, acetonitrile, n-butanol, toluene, tetrahydrofuran, dichloromethane, petroleum ether, ethyl acetate, n-hexane, dimethyl sulfoxide, 1,4-dioxane, water, N,N-dimethylformamide, N,N-dimethylacetamide, 1,2-dibromoethane, and mixtures thereof.

### BRIEF DESCRIPTION OF THE DRAWINGS

FIG. 1 shows a H-N HSQC spectrum of the compound of Example 16.
FIG. 2 shows a H-N HMBC spectrum of the compound of Example 16.
FIG. 3 shows the analgesic efficacy of the compound of Example 16 in a rat model of incision pain.
FIG. 4 shows the effect of the compound of Example 16 on body weight in a rat model of incision pain.
FIG. 5 shows the analgesic efficacy of the compound of Example 32 in a rat model of incision pain.
FIG. 6 shows the effect of the compound of Example 32 on body weight in a rat model of incision pain.

### DETAILED DESCRIPTION

The present disclosure is further described below with reference to examples, but these examples are not intended to limit the scope of the present disclosure.

### Examples

The structures of the compounds were determined by nuclear magnetic resonance (NMR) spectroscopy or/and mass spectrometry (MS). The NMR shifts (δ) are given in 10⁻⁶ (ppm). The NMR analyses were performed using a Bruker AVANCE-400 nuclear magnetic resonance instrument or Bruker AVANCE NEO 500M, with dimethyl sulfoxide-D6 (DMSO-*d₆*), chloroform-D (CDCl₃), and methanol-D4 (CD₃OD) as solvents and tetramethylsilane (TMS) as an internal standard.

The MS analyses were performed using an Agilent 1200/1290 DAD-6110/6120 Quadrupole MS liquid chromatography-mass spectrometry system (manufacturer: Agilent; MS model: 6110/6120 Quadrupole MS),
waters ACQuity UPLC-QD/SQD (manufacturer: waters; MS model: waters ACQuity Qda Detector/waters SQ Detector), and
THERMO Ultimate 3000-Q Exactive (manufacturer: THERMO; MS model: THERMO Q Exactive).

The high performance liquid chromatography (HPLC) analyses were performed using Agilent HPLC 1200DAD, Agilent HPLC 1200VWD, and Waters HPLC e2695-2489 high performance liquid chromatographs.

The chiral HPLC analyses were performed using an Agilent 1260 DAD high performance liquid chromatograph.

The preparative high performance liquid chromatography steps were performed using Waters 2545-2767, Waters 2767-SQ Detecor2, Shimadzu LC-20AP, and Gilson GX-281 preparative chromatographs.

The preparative chiral chromatography steps were performed using a Shimadzu LC-20AP preparative chromatograph.

The CombiFlash preparative flash chromatograph used was Combiflash Rf200 (TELEDYNE ISCO).

The thin-layer chromatography silica gel plates used were Yantai Huanghai HSGF254 or Qingdao GF254 silica gel plates. The silica gel plates used in the thin-layer chromatography (TLC) had a layer thickness of 0.15 mm-0.2 mm, and those used in the thin-layer chromatography separation and purification had a layer thickness of 0.4 mm-0.5 mm.

In the silica gel column chromatography, a 200-300 mesh silica gel (Huanghai, Yantai) was generally used as the carrier.

The kinase mean inhibition rates and IC₅₀ values were measured using a NovoStar microplate reader (BMG, Germany).

Known starting materials in the present disclosure may be synthesized by using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can all be performed under an argon atmosphere or a nitrogen atmosphere unless otherwise specified.

The argon atmosphere or nitrogen atmosphere means that the reaction flask was connected to a balloon containing about 1 L of argon or nitrogen gas.

The hydrogen atmosphere means that the reaction flask was connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 cycles of vacuumization and hydrogen filling.

The microwave reactions were performed using a CEM Discover-S 908860 microwave reactor.

In the examples, the solutions were aqueous solutions unless otherwise specified.

In the examples, the reaction temperature was room temperature, i.e., 20 °C-30 °C, unless otherwise specified.

The monitoring of the reaction progress in the examples was conducted by thin-layer chromatography (TLC). The developing solvent for reactions, the eluent system for column chromatography purification, and the developing solvent system for thin-layer chromatography include: A: a dichloromethane/methanol system, B: a n-hexane/ethyl acetate system, and C: a petroleum ether/ethyl acetate system. The volume ratio of the solvents was adjusted depending on the polarity of the compound, or by adding a small amount of basic or acidic reagents such as triethylamine and acetic acid.

### Example 1

### (2R,3S,4S,5R)-N-(2-Aminopyrimidin-5-yl)-3-(3,4-difluoro-2-methoxyphenyl)-4,5-dimethyl-5-(trifluoromethyl)tetrahydrofuran-2-carboxamide 1

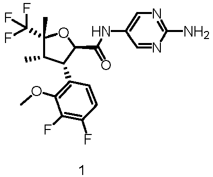

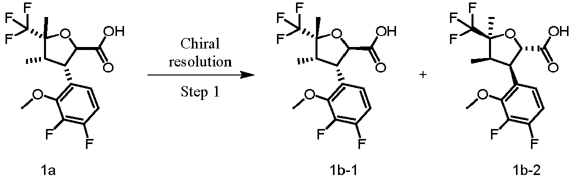

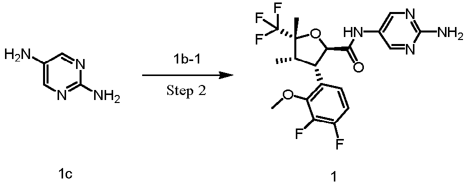

### Step 1

### (2R,3S,4S,5R)-3-(3,4-Difluoro-2-methoxyphenyl)-4,5-dimethyl-5-(trifluoromethyl)tetrahydrofuran-2-carboxylic acid 1b-1

### (2S,3R,4R,5S)-3-(3,4-Difluoro-2-methoxyphenyl)-4,5-dimethyl-5-(trifluoromethyl)tetrahydrofuran-2-carboxylic acid 1b-2

*rac*-(2*R*,3*S*,4*S*,5*R*)-3-(3,4-Difluoro-2-methoxyphenyl)-4,5-dimethyl-5-(trifluoromethyl)tetrahydrofuran-2-carboxylic acid **1a** (12 g, 33.87 mmol, prepared by the method disclosed in Example 3 on page 231 of the specification in the patent application "WO2021113627") was resolved on a chiral column (Waters SFC 150, chromatography column: DAICEL CHIRALPAK^{®}IC, 40 × 250 mm, 10 µm; mobile phase A: supercritical CO₂, mobile phase B: IPA, gradient ratio: A:B: 90:10, flow rate: 120 mL/min) to give title products **1b-1** (5.5 g, yield: 45.8%) and **1b-2** (5.08 g, yield: 42.3%).

MS m/z (ESI): 353.2 [M-1].

Single-configuration compound (shorter retention time) **1b-1** (5.5 g, yield: 45.8%). MS m/z (ESI): 353.2 [M-1].

Chiral HPLC analysis: retention time 2.414 min, purity: 99% (chromatography column: DAICEL CHIRALPAK^{®}IC, 100 × 3 mm, 3 µm; mobile phase A: supercritical CO₂, mobile phase B: IPA (0.1% DEA), gradient ratio: mobile phase A: 60%-95%, flow rate: 1.5 mL/min).

Single-configuration compound (longer retention time) **1b-2** (5.08 g, yield: 42.3%). MS m/z (ESI): 353.2 [M-1].

Chiral HPLC analysis: retention time 2.724 min, purity: 99% (chromatography column: DAICEL CHIRALPAK^{®}IC, 100 × 3 mm, 3 µm; mobile phase A: supercritical CO₂, mobile phase B: IPA (0.1% DEA), gradient ratio: mobile phase A: 60%-95%, flow rate: 1.5 mL/min).

### Step 2

### (2R,3S,4S,5R)-N-(2-Aminopyrimidin-5-yl)-3-(3,4-difluoro-2-methoxyphenyl)-4,5-dimethyl-5-(trifluoromethyl)tetrahydrofuran-2-carboxamide 1

Compound **1b-1** (22 mg, 62 µmol) and 2,5-diaminopyrimidine **1c** (10.3 mg, 93 µmol, Shanghai Bide) were dissolved in 1 mL of *N,N*-dimethylformamide, and *N,N-*diisopropylethylamine (24 mg, 186 µmol) and O-(7-azabenzotriazol-1-yl)-*N,N,N',N'-*tetramethyluronium hexafluorophosphate (HATU) (28.3 mg, 74 µmol) were added under an ice bath. With the temperature maintained, the mixture was left to react for 2 h. The reaction mixture was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound 1 (8 mg, yield: 28.8%).

MS m/z (ESI): 447.2 [M+1].

¹H NMR (500 MHz, CD₃OD): δ 8.39 (s, 2H), 7.13 (ddd, 1H), 6.98 (td, 1H), 5.05 (d, 1H), 4.26 (dd, 1H), 3.99 (d, 3H), 2.78 (p, 1H), 1.68-1.64 (m, 3H), 0.80 (dq, 3H).

### Example 2

### (2R,3S,4S,5R)-3-(3,4-Difluoro-2-methoxyphenyl)-N-(2-((R)-2,3-dihydroxypropoxy)pyridin-4-yl)-4,5-dimethyl-5-(trifluoromethyl)tetrahydrofuran-2-carboxamide 2

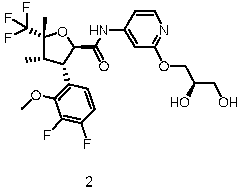

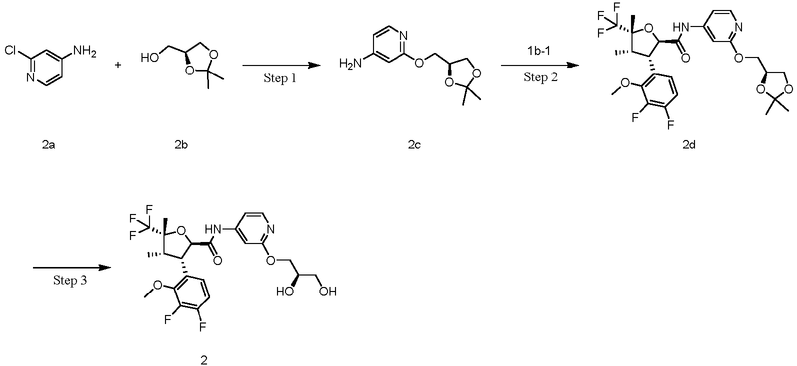

### Step 1

### (S)-(+)-2-((2,2-Dimethyl-1,3-dioxolan-4-yl)methoxy)pyridin-4-amine 2c

(*S*)-(+)-(2,2-Dimethyl-1,3-dioxolan-4-yl)methanol **2b** (616 mg, 4.66 mmol, Shanghai Accela) was dissolved in 10 mL of anhydrous *N,N*-dimethylformamide, and sodium hydride (199 mg, 5 mmol, 60% purity) was added under an ice bath. With the temperature maintained, the mixture was left to react for 1 h, and 2-chloro-4-aminopyridine **2a** (200 mg, 1.55 mmol, Shanghai Bide) was then added. The mixture was stirred at 160 °C for 10 h. The reaction mixture was cooled to room temperature, quenched with water, and extracted with dichloromethane (30 mL × 3). The organic phases were combined, dried over anhydrous sodium sulfate, filtered to remove the drying agent, and then concentrated under reduced pressure. The residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **2c** (140 mg, yield: 40%).

MS m/z (ESI): 225.2 [M+1].

### Step 2

### (2R,3S,4S,5R)-3-(3,4-Difluoro-2-methoxyphenyl)-N-(2-((S)-2,2-dimethyl-1,3-dioxolan-4-yl)methoxy)pyridin-4-yl)-4,5-dimethyl-5-(trifluoromethyl)tetrahydrofuran-2-carboxamide 2d

Compound **1b-1** (20 mg, 56.43 µmol) was dissolved in dichloromethane (0.8 mL), and oxalyl chloride (14.3 mg, 112 µmol) and one drop of *N,N*-dimethylformamide were added under an ice bath. The mixture was warmed to room temperature and left to react for 1 h. The reaction mixture was concentrated under reduced pressure, and the residue was dissolved in dichloromethane (1 mL). Compound **2c** (15.2 mg, 67.7 µmol), triethylamine (22.8 mg, 225.8 µmol), and 4-dimethylaminopyridine (1.4 mg, 11.2 µmol) were added. The mixture was stirred for 1 h. The reaction mixture was concentrated under reduced pressure to give crude title compound **2d** (31 mg). The product was directly used in the next step without purification.

MS m/z (ESI): 561.2 [M+1].

### Step 3

### (2R,3S,4S,5R)-3-(3,4-Difluoro-2-methoxyphenyl)-N-(2-((R)-2,3-dihydroxypropoxy)pyridin-4-yl)-4,5-dimethyl-5-(trifluoromethyl)tetrahydrofuran-2-carboxamide 2

Crude compound **2d** (30 mg, 53.5 µmol) was dissolved in dichloromethane (1 mL), and a 4 M solution of HCl in dioxane (0.5 mL) was added. The mixture was stirred for 1 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **2** (2 mg, yield: 7%). MS m/z (ESI): 521.2 [M+1].

¹H NMR (500 MHz, CD₃OD): δ 8.00 (dd, 1H), 7.41 (d, 1H), 7.30 (d, 1H), 7.17 (dd, 1H), 7.00 (td, 1H), 5.07 (s, 1H), 4.25 (dd, 1H), 4.05-3.84 (m, 5H), 3.64 (qd, 2H), 2.80 (t, 1H), 2.21 (t, 1H), 1.66 (s, 3H), 0.83 (dt, 3H).

### Example 3

### (2R,3S,4S,5R)-3-(3,4-Difluoro-2-methoxyphenyl)-N-(2-(2-hydroxypropan-2-yl)pyridin-4-yl)-4,5-dimethyl-5-(trifluoromethyl)tetrahydrofuran-2-carboxamide 3

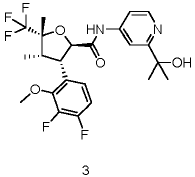

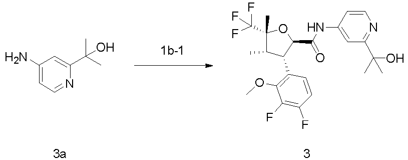

Compound **1b-1** (20 mg, 56 µmol) and 2-(4-aminopyridin-2-yl)propan-2-ol **3a** (12.9 mg, 84.6 µmol,Shanghai Accela) were dissolved in 1 mL of *N,N-*dimethylformamide, and *N,N*-diisopropylethylamine (22 mg, 169 µmol) and O-(7-azabenzotriazol-1-yl)-*N,N,N,N*-tetramethyluronium hexafluorophosphate (HATU) (26 mg, 68 µmol) were added under an ice bath. With the temperature maintained, the mixture was left to react for 2 h. The reaction mixture was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **3** (4 mg, yield: 14.5%).

MS m/z (ESI): 489.2 [M+1].

¹H NMR (500 MHz, CD₃OD): δ 8.34 (d, 1H), 7.90 (d, 1H), 7.58 (dd, 1H), 7.12 (ddd, 1H), 6.98 (td, 1H), 5.07 (d, 1H), 4.32 (dd, 1H), 4.00 (d, 3H), 2.79 (p, 1H), 1.66 (d, 3H), 1.51 (s, 6H), 0.81 (dt, 3H).

### Example 4-1 or Example 4-2

### (2R,3S,4S,5R)-3-(3,4-Difluoro-2-methoxyphenyl)-4,5-dimethyl-N-(2-(2-methyl-2H-tetrazol-5-yl)pyridin-4-yl)-5-(trifluoromethyl)tetrahydrofuran-2-carboxamide 4-1 or (2R,3S,4S,5R)-3-(3,4-Difluoro-2-methoxyphenyl)-4,5-dimethyl-N-(2-(1-methyl-1H-tetrazol-5-yl)pyridin-4-yl)-5-(trifluoromethyl)tetrahydrofuran-2-carboxamide 4-2

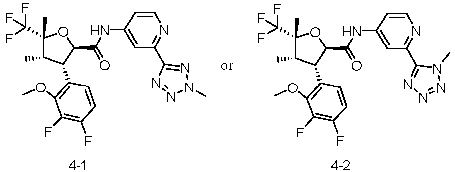

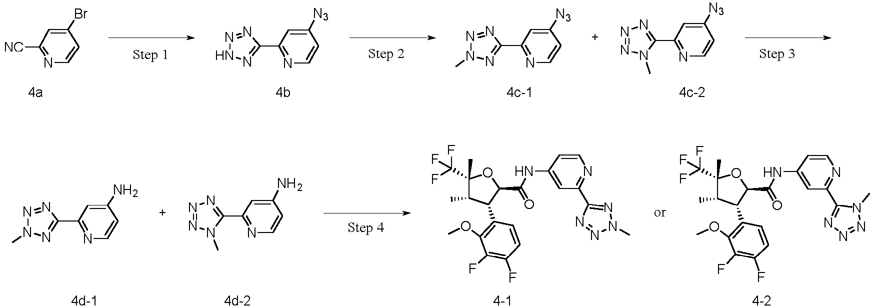

### Step 1

### 4-Azido-2-(2H-tetrazol-5-yl)pyridine 4b

4-Bromopyridine-2-carbonitrile **4a** (1 g, 5.46 mmol, Shanghai Accela) and sodium azide (535 mg, 8.22 mmol) were dissolved in *N,N-*dimethylformamide (25 mL), and ammonium chloride (1.0 g, 18.7 mmol) was added. The mixture was stirred at 120 °C for 4 h. The reaction mixture was cooled to room temperature, poured into water, and extracted with ethyl acetate (15 mL × 3). The organic phases were combined, dried over anhydrous sodium sulfate, filtered to remove the drying agent, and then concentrated under reduced pressure to give crude title compound **4b** (1 g). The product was directly used in the next step without purification.

MS m/z (ESI): 189.2 [M+1].

### Step 2

### A mixture of 4-azido-2-(2-methyl-2H-tetrazol-5-yl)pyridine 4c-1 and 4-azido-2-(1-methyl-1H-tetrazol-5-yl)pyridine 4c-2

Compound **4b** (1 g, 5.3149 mmol) was dissolved in *N*,*N*-dimethylformamide (20 mL), and potassium hydroxide (900 mg, 16 mmol) and iodomethane (1 g, 7 mmol) were added. The mixture was stirred for 16 h. The reaction mixture was filtered. Water was added to the filtrate, and extraction was performed with ethyl acetate (15 mL × 3). The organic phases were combined, dried over anhydrous sodium sulfate, filtered to remove the drying agent, and then concentrated under reduced pressure to give a mixture of crude title compounds **4c-1** and **4c-2** (1 g). The product was directly used in the next step without purification.

MS m/z (ESI): 203.2 [M+1].

### Step 3

### 2-(2-Methyl-2H-tetrazol-5-yl)pyridin-4-amine 4d-1

### 2-(1-Methyl-1H-tetrazol-5-yl)pyridin-4-amine 4d-2

The mixture of crude compounds **4c-1** and **4c-2** (400 mg, 1.97 mmol) was dissolved in methanol (20 mL), and 10% palladium on carbon catalyst (wet) (100 mg) was added. The mixture was stirred in a hydrogen atmosphere for 14 h. The reaction mixture was filtered, and the filtrate was concentrated under reduced pressure. The residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give the title compounds (40 mg and 30 mg, yields: 11.4% and 8.6%).

MS m/z (ESI): 177.2 [M+1].

Single-configuration compound (shorter retention time) (30 mg, yield: 8.6%).

HPLC analysis: retention time 0.54 min, purity: 99% (chromatography column: ACQUITY UPLC^{®}BEH, C18, 1.7 µm, 2.1 × 50 mm; mobile phase: water (10 mM ammonium bicarbonate), acetonitrile, gradient ratio: acetonitrile 10%-95%). Single-configuration compound (longer retention time) (40 mg, yield: 11.4%).

HPLC analysis: retention time 0.81 min, purity: 92% (chromatography column: ACQUITY UPLC^{®}BEH, C18, 1.7 µm, 2.1 × 50 mm; mobile phase: water (10 mM ammonium bicarbonate), acetonitrile, gradient ratio: acetonitrile 10%-95%).

### Step 4

### (2R,3S,4S,5R)-3-(3,4-Difluoro-2-methoxyphenyl)-4,5-dimethyl-N-(2-(2-methyl-2H-tetrazol-5-yl)pyridin-4-yl)-5-(trifluoromethyl)tetrahydrofuran-2-carboxamide 4-1 or (2R,3S,4S,5R)-3-(3,4-Difluoro-2-methoxyphenyl)-4,5-dimethyl-N-(2-(1-methyl-1H-tetrazol-5-yl)pyridin-4-yl)-5-(trifluoromethyl)tetrahydrofuran-2-carboxamide 4-2

Compound **1b-1** (20 mg, 56.43 µmol) was dissolved in dichloromethane (2 mL), and oxalyl chloride (40 mg, 315 µmol) and one drop of *N,N-*dimethylformamide were added under an ice bath. The mixture was warmed to room temperature and left to react for 1 h. The reaction mixture was concentrated under reduced pressure, and the residue was dissolved in dichloromethane (3 mL). Triethylamine (40 mg, 395 µmol) was added, and a solution of the compound corresponding to the longer retention time between compounds **4d-1** and **4d-2** (15.2 mg, 67.7 µmol) in dichloromethane (1 mL) was added dropwise at -10 °C. The mixture was stirred for 1 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **4-1 or 4-2**(4 mg, yield: 13.8%).

MS m/z (ESI): 513.2 [M+1].

¹H NMR (500 MHz, DMSO-*d₆*): δ 10.87 (s, 1H), 8.69-8.67 (m, 1H), 8.58-8.56 (m, 1H), 7.88-7.84 (m, 1H), 7.23-7.15 (m, 2H), 5.18-5.13 (m, 1H), 4.40 (s, 3H), 4.31-4.23 (m, 1H), 3.96 (s, 3H), 2.83-2.74 (m, 1H), 1.63 (s, 3H), 0.76-0.72 (m, 3H).

### Example 4-1 or Example 4-2 (2R,3S,4S,5R)-3-(3,4-Difluoro-2-methoxyphenyl)-4,5-dimethyl-N-(2-(2-methyl-2H-tetrazol-5-yl)pyridin-4-yl)-5-(trifluoromethyl)tetrahydrofuran-2-carboxamide 4-1 or (2R,3S,4S,5R)-3-(3,4-Difluoro-2-methoxyphenyl)-4,5-dimethyl-N-(2-(1-methyl-1H-tetrazol-5-yl)pyridin-4-yl)-5-(trifluoromethyl)tetrahydrofuran-2-carboxamide 4-2

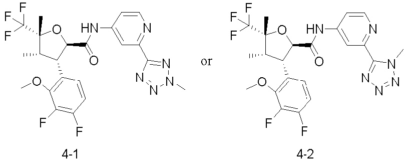

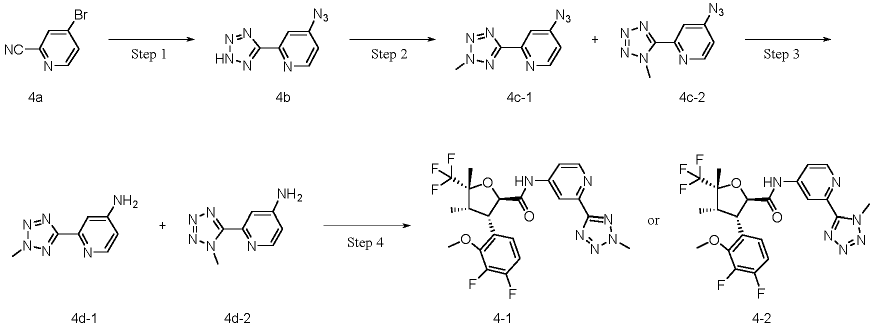

### Step 1

### 4-Azido-2-(2H-tetrazol-5-yl)pyridine 4b

4-Bromopyridine-2-carbonitrile **4a** (1 g, 5.46 mmol, Shanghai Accela) and sodium azide (535 mg, 8.22 mmol) were dissolved in *N,N*-dimethylformamide (25 mL), and ammonium chloride (1.0 g, 18.7 mmol) was added. The mixture was stirred at 120 °C for 4 h. The reaction mixture was cooled to room temperature, poured into water, and extracted with ethyl acetate (15 mL × 3). The organic phases were combined, dried over anhydrous sodium sulfate, filtered to remove the drying agent, and then concentrated under reduced pressure to give crude title compound **4b** (1 g). The product was directly used in the next step without purification.

MS m/z (ESI): 189.2 [M+1].

### Step 2

### A mixture of 4-azido-2-(2-methyl-2H-tetrazol-5-yl)pyridine 4c-1 and 4-azido-2-(1-methyl-1H-tetrazol-5-yl)pyridine 4c-2

Compound **4b** (1 g, 5.3149 mmol) was dissolved in *N,N-*dimethylformamide (20 mL), and potassium hydroxide (900 mg, 16 mmol) and iodomethane (1 g, 7 mmol) were added. The mixture was stirred for 16 h. The reaction mixture was filtered. Water was added to the filtrate, and extraction was performed with ethyl acetate (15 mL × 3). The organic phases were combined, dried over anhydrous sodium sulfate, filtered to remove the drying agent, and then concentrated under reduced pressure to give a mixture of crude title compounds **4c-1** and **4c-2** (1 g). The product was directly used in the next step without purification.

MS m/z (ESI): 203.2 [M+1].

### Step 3

### 2-(2-Methyl-2H-tetrazol-5-yl)pyridin-4-amine 4d-1

### 2-(1-Methyl-1H-tetrazol-5-yl)pyridin-4-amine 4d-2

The mixture of crude compounds **4c-1** and **4c-2** (400 mg, 1.97 mmol) was dissolved in methanol (20 mL), and 10% palladium on carbon catalyst (wet) (100 mg) was added. The mixture was stirred in a hydrogen atmosphere for 14 h. The reaction mixture was filtered, and the filtrate was concentrated under reduced pressure. The residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give the title compounds (40 mg and 30 mg, yields: 11.4% and 8.6%).

MS m/z (ESI): 177.2 [M+1].

Single-configuration compound (shorter retention time) (30 mg, yield: 8.6%).

HPLC analysis: retention time 0.54 min, purity: 99% (chromatography column: ACQUITY UPLC^{®}BEH, C18, 1.7 µm, 2.1 × 50 mm; mobile phase: water (10 mM ammonium bicarbonate), acetonitrile, gradient ratio: acetonitrile 10%-95%). Single-configuration compound (longer retention time) (40 mg, yield: 11.4%).

HPLC analysis: retention time 0.81 min, purity: 92% (chromatography column: ACQUITY UPLC^{®}BEH, C18, 1.7 µm, 2.1 × 50 mm; mobile phase: water (10 mM ammonium bicarbonate), acetonitrile, gradient ratio: acetonitrile 10%-95%).

### Step 4

### (2R,3S,4S,5R)-3-(3,4-Difluoro-2-methoxyphenyl)-4,5-dimethyl-N-(2-(2-methyl-2H-tetrazol-5-yl)pyridin-4-yl)-5-(trifluoromethyl)tetrahydrofuran-2-carboxamide 4-1 or (2R,3S,4S,5R)-3-(3,4-Difluoro-2-methoxyphenyl)-4,5-dimethyl-N-(2-(1-methyl-1H-tetrazol-5-yl)pyridin-4-yl)-5-(trifluoromethyl)tetrahydrofuran-2-carboxamide 4-2

Compound **1b-1** (20 mg, 56.43 µmol) was dissolved in dichloromethane (2 mL), and oxalyl chloride (40 mg, 315 µmol) and one drop of *N,N-*dimethylformamide were added under an ice bath. The mixture was warmed to room temperature and left to react for 1 h. The reaction mixture was concentrated under reduced pressure, and the residue was dissolved in dichloromethane (3 mL). Triethylamine (40 mg, 395 µmol) was added, and a solution of the compound corresponding to the shorter retention time between compounds **4d-1** and **4d-2** (15.2 mg, 67.7 µmol) in dichloromethane (1 mL) was added dropwise at -10 °C. The mixture was stirred for 1 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **4-1 or 4-2**(3 mg, yield: 10.3%).

MS m/z (ESI): 513.2 [M+1].

¹H NMR (500 MHz, DMSO-*d₆*): δ 10.80 (s, 1H), 8.61-8.57 (m, 1H), 8.52-8.51 (m, 1H), 7.75-7.71 (m, 1H), 7.22-7.16 (m, 2H), 5.16-5.13 (m, 1H), 4.43 (s, 3H), 4.29-4.25 (m, 1H), 3.95 (s, 3H), 2.77-2.74 (m, 1H), 1.61 (s, 3H), 0.74-0.72 (m, 3H).

### Example 5

### 4-((2R,3S,4S,5R)-3-(3,4-Difluoro-2-methoxyphenyl)-4,5-dimethyl-5-(trifluoromethyl)tetrahydrofuran-2-carboxamido)-N-methoxypicolinamide 5

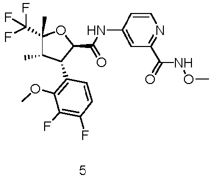

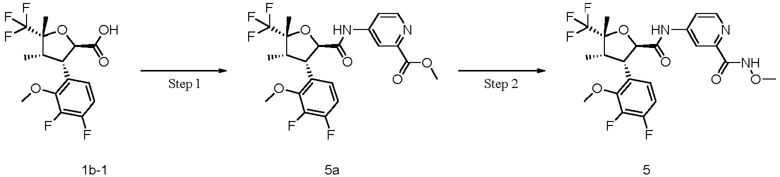

### Step 1

### Methyl 4-((2R,3S,4S,5R)-3-(3,4-difluoro-2-methoxyphenyl)-4,5-dimethyl-5-(trifluoromethyl)tetrahydrofuran-2-carboxamido)picolinate 5a

Compound **1b-1** (50 mg, 141.1 µmol) was dissolved in dichloromethane (5 mL), and oxalyl chloride (36 mg, 282 µmol) and one drop of *N,N-*dimethylformamide were added under an ice bath. The mixture was naturally warmed to room temperature and left to react for 1 h. The reaction mixture was concentrated under reduced pressure, and the residue was dissolved in dichloromethane (5 mL). Triethylamine (72 mg, 711 µmol) and 4-dimethylaminopyridine (3.5 mg, 28.2 µmol) were added, and methyl 4-aminopyridine-2-carboxylate (30 mg, 197.6 µmol) was added under an ice bath. The mixture was stirred for 14 h. The reaction mixture was concentrated under reduced pressure, and the residue was purified by silica gel column chromatography using eluent system B to give title compound **5a** (30 mg, yield: 43.5%).

MS m/z (ESI): 489.2 [M+1].

### Step 2

### 4-((2R,3S,4S,5R)-3-(3,4-Difluoro-2-methoxyphenyl)-4,5-dimethyl-5-(trifluoromethyl)tetrahydrofuran-2-carboxamido)-N-methoxypicolinamide 5

Compound **5a** (20 mg, 40.9 µmol) was dissolved in tetrahydrofuran (0.5 mL), methoxyamine hydrochloride (6.84 mg, 81.9 µmol,Shanghai Titan) was added, and a 1 M solution of lithium bis(trimethylsilyl)amide in tetrahydrofuran (0.2 mL) was added dropwise under an ice bath. With the temperature maintained, the mixture was left to react for 1 h. The reaction mixture was quenched with a saturated ammonium chloride solution and extracted with ethyl acetate (5 mL × 2). The organic phases were combined, dried over anhydrous sodium sulfate, filtered to remove the drying agent, and then concentrated under reduced pressure. The residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **5** (3 mg, yield: 14.5%).

MS m/z (ESI): 504.2 [M+1].

¹H NMR (500 MHz, CD₃OD): δ 8.48 (d, 1H), 8.25 (d, 1H), 7.90 (dd, 1H), 7.14 (dd, 1H), 7.00 (q, 1H), 5.10 (d, 1H), 4.35 (dd, 1H), 4.02 (d, 3H), 3.83 (s, 3H), 2.82 (p, 1H), 1.68 (s, 3H), 0.87-0.80 (m, 3H).

### Example 6

### (2R,3S,4S,5R)-N-(2-(1-Amino-2-hydroxyethyl)pyridin-4-yl)-3-(3,4-difluoro-2-methoxyphenyl)-4,5-dimethyl-5-(trifluoromethyl)tetrahydrofuran-2-carboxamide 6 (a mixture of diastereomers)

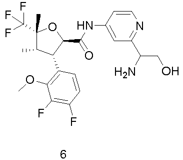

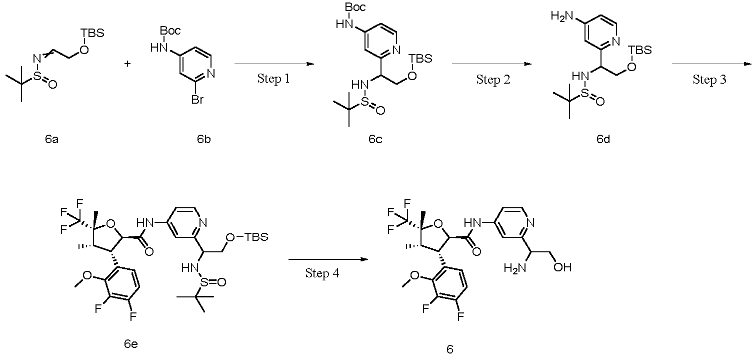

### Step 1

### tert-Butyl (2-(2-((tert-butyldimethylsilyl)oxy)-1-((tert-butylsulfinyl)amino)ethyl)pyridin-4-yl)carbamate 6c (a racemate)

0.8 mL of a 2.5 M solution of n-butyllithium in n-hexane was added to tetrahydrofuran (10 mL), and a solution of tert-butyl (2-bromopyridin-4-yl)carbamate **6b** (250 mg, 915 µmol,prepared by the method disclosed in "Dalton Transactions, 2016, vol. 45, # 32, p. 128-7-12813") in tetrahydrofuran (2 mL) was added dropwise at -78 °C. The mixture was left to react at that temperature for 1 h. A solution of N-(2-((tertbutyldimethylsilyl)oxy)ethylidene)-2-methylpropane-2-sulfinamide **6a** (250 mg, 56.43 µmol,prepared by the method for intermediate 25 disclosed on page 89 of the specification in the patent application "WO2008132502") in tetrahydrofuran (2 mL) was added. The mixture was stirred at that temperature for 2 h. The reaction mixture was quenched with a saturated sodium chloride solution at -78 °C and extracted with ethyl acetate (10 mL × 3). The organic phases were combined, dried over anhydrous sodium sulfate, filtered to remove the drying agent, and then concentrated under reduced pressure. The residue was purified by silica gel column chromatography using eluent system B to give title compound **6c** (170 mg, a racemate, yield: 40%).

MS m/z (ESI): 472.2 [M+1].

### Step 2

### N-(1-(4-Aminopyridin-2-yl)-2-((tert-butyldimethylsilyl)oxy)ethyl)-2-methylpropane-2-sulfinamide 6d (a racemate)

Compound **6c** (132 mg, 280 µmol) was dissolved in dichloromethane (3 mL), and 1 mL of trifluoroacetic acid was added. The mixture was stirred for 3 h. The reaction mixture was concentrated under reduced pressure, and the residue was dissolved in dichloromethane and washed with a saturated sodium bicarbonate solution. The organic phase was dried over anhydrous sodium sulfate, filtered to remove the drying agent, and then concentrated under reduced pressure to give crude title compound **6d** (104 mg, a racemate). The product was directly used in the next step without purification.

MS m/z (ESI): 372.2 [M+1].

### Step 3

### (2R,3S,4S,5R)-N-(2-(2-((tert-Butyldimethylsilyl)oxy)-1-((tert-butylsulfinyl)amino)ethyl)pyridin-4-yl)-3-(3,4-difluoro-2-methoxyphenyl)-4,5-dimethyl-5-(trifluoromethyl)tetrahydrofuran-2-carboxamide 6e (a mixture of diastereomers)

Compound **1b-1** (354 mg, 284 µmol) was dissolved in dichloromethane (3 mL), and oxalyl chloride (71 mg, 560 µmol) and one drop of *N,N-*dimethylformamide were added under an ice bath. The mixture was warmed to room temperature and left to react for 1 h. The reaction mixture was concentrated under reduced pressure, and the residue was dissolved in dichloromethane (3 mL). Compound **6d** (104 mg, 280 µmol) and *N,N-*diisopropylethylenediamine (110 mg, 851 µmol) were added under an ice bath. The mixture was stirred for 1 h. The reaction mixture was concentrated under reduced pressure, and the residue was purified by silica gel column chromatography using eluent system A to give title compound **6e** (150 mg, a mixture of diastereomers, yield: 75%).

MS m/z (ESI): 708.2 [M+1].

### Step 4

### (2R,3S,4S,5R)-N-(2-(1-Amino-2-hydroxyethyl)pyridin-4-yl)-3-(3,4-difluoro-2-methoxyphenyl)-4,5-dimethyl-5-(trifluoromethyl)tetrahydrofuran-2-carboxamide 6 (a mixture of diastereomers)

Compound **6e** (150 mg, 212 µmol) was dissolved in dichloromethane (2 mL), and a 4 M solution of hydrochloric acid in dioxane (5 mL) was added. The mixture was stirred for 1 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **6** (3 mg, a mixture of diastereomers, yield: 2.9%).

MS m/z (ESI): 490.2 [M+1].

¹H NMR (500 MHz, CD₃OD): δ 8.72-8.18 (m, 1H), 7.69 (s, 2H), 7.13 (ddd, 1H), 7.06-6.92 (m, 1H), 5.08 (d, 1H), 4.32 (dd, 1H), 4.00 (d, 3H), 3.86-3.54 (m, 2H), 2.80 (t, 1H), 1.66 (s, 3H), 1.30 (s, 1H), 0.88-0.71 (m, 3H).

### Example 6-1 or Example 6-2

### (2R,3S,4S,5R)-N-(2-((S)-1-Amino-2-hydroxyethyl)pyridin-4-yl)-3-(3,4-difluoro-2-methoxyphenyl)-4,5-dimethyl-5-(trifluoromethyl)tetrahydrofuran-2-carboxamide 6-1 or (2R,3S,4S,5R)-N-(2-((R)-1-Amino-2-hydroxyethyl)pyridin-4-yl)-3-(3,4-difluoro-2-methoxyphenyl)-4,5-dimethyl-5-(trifluoromethyl)tetrahydrofuran-2-carboxamide 6-2

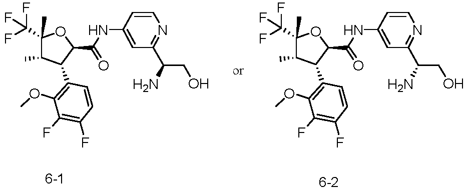

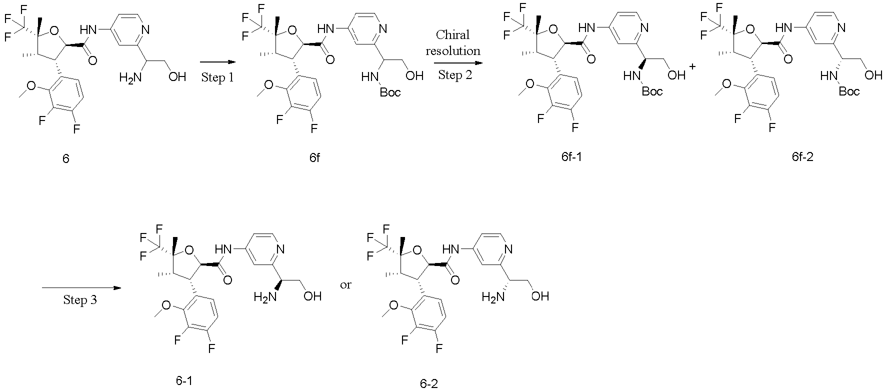

### Step 1

### tert-Butyl (1-(4-((2R,3S,4S,5R)-3-(3,4-difluoro-2-methoxyphenyl)-4,5-dimethyl-5-(trifluoromethyl)tetrahydrofuran-2-carboxamido)pyridin-2-yl)-2-hydroxyethyl)carbamate 6f (a mixture of diastereomers)

Compound **6** (190 mg, 388.2 µmol) was dissolved in dichloromethane (6 mL), and di-tert-butyl dicarbonate (85 mg, 388.2 µmol) was added. The mixture was stirred for 14 h. The reaction mixture was concentrated under reduced pressure to give crude title compound **6f** (230 mg, a mixture of diastereomers).

MS m/z (ESI): 590.2 [M+1].

### Step 2

### tert-Butyl ((S)-(1-(4-((2R,35,4S,5R)-3-(3,4-difluoro-2-methoxyphenyl)-4,5-dimethyl-5-(trifluoromethyl)tetrahydrofuran-2-carboxamido)pyridin-2-yl)-2-hydroxyethyl)carbamate 6f-1

### tert-Butyl ((R)-(1-(4-((2R,3S,4S,5R)-3-(3,4-difluoro-2-methoxyphenyl)-4,5-dimethyl-5-(trifluoromethyl)tetrahydrofuran-2-carboxamido)pyridin-2-yl)-2-hydroxyethyl)carbamate 6f-2

Compound **6f** (230 mg) was resolved on a chiral column (Gilson 281, chromatography column: CHIRALPAK IE, 20 × 250 mm, 10 µm; mobile phase A: n-hexane, mobile phase B: ethanol, gradient ratio: A:B: 90:10, flow rate: 20 mL/min)) to give the title compounds (65 mg and 114 mg, yields: 28.4% and 49.8%).

The chiral HPLC analysis method for the mixture of diastereomers **6f:** Agilent 1260 DAD, chromatography column: CHIRALPAK IE, 250 × 4.6 mm, 5 µm; mobile phase: n-hexane and ethanol (containing 0.1% diethylamine), gradient ratio: A:B: 90:10, flow rate: 1.0 mL/min.

Single-configuration compound (shorter retention time): 6.447 min, (65 mg, yield: 28.4%).

MS m/z (ESI): 590.2 [M+1].

Single-configuration compound (longer retention time): 8.484 min, (114 mg, yield: 49.8%).

MS m/z (ESI): 590.2 [M+1].

### Step 3

### (2R,3S,4S,5R)-N-(2-((S)-1-Amino-2-hydroxyethyl)pyridin-4-yl)-3-(3,4-difluoro-2-methoxyphenyl)-4,5-dimethyl-5-(trifluoromethyl)tetrahydrofuran-2-carboxamide 6-1 or

### (2R,3S,4S,SR)-N (2-((R)-1-Amino-2-hydroxyethyl)pyridin-4-yl)-3-(3,4-difluoro-2-methoxyphenyl)-4,5-dimethyl-5-(trifluoromethyl)tetrahydrofuran-2-carboxamide 6-2

The compound corresponding to the shorter retention time between compounds **6f-1** and **6f-2** (65 mg, 110 µmol) was dissolved in dichloromethane (4 mL), and a 4 M solution of hydrochloric acid in 1,4-dioxane (2 mL) was added. The mixture was left to react for 1 h and then concentrated under reduced pressure, and the residue was purified by high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **6-1** or **6-2** (37.6 mg, yield: 69.6%).

MS m/z (ESI): 490.2 [M+1].

¹H NMR (500 MHz, CD₃OD): δ 8.71-8.07 (m, 1H), 7.68 (s, 2H), 7.11 (ddd, 1H), 6.97 (td, 1H), 5.06 (d, 1H), 4.30 (dd, 1H), 3.98 (d, 3H), 3.89-3.52 (m, 2H), 2.78 (p,1H), 1.64 (s, 3H), 1.30 (d, 1H), 0.80 (dt, 3H).

### Example 6-1 or Example 6-2

### (2R,3S,4S,5R)-N-(2-((S)-1-Amino-2-hydroxyethyl)pyridin-4-yl)-3-(3,4-difluoro-2-methoxyphenyl)-4,5-dimethyl-5-(trifluoromethyl)tetrahydrofuran-2-carboxamide 6-1 or

### (2R,3S,4S,SR)-N (2-((R)-1-Amino-2-hydroxyethyl)pyridin-4-yl)-3-(3,4-difluoro-2-methoxyphenyl)-4,5-dimethyl-5-(trifluoromethyl)tetrahydrofuran-2-carboxamide 6-2

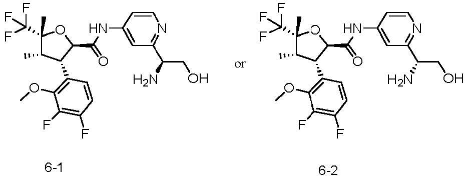

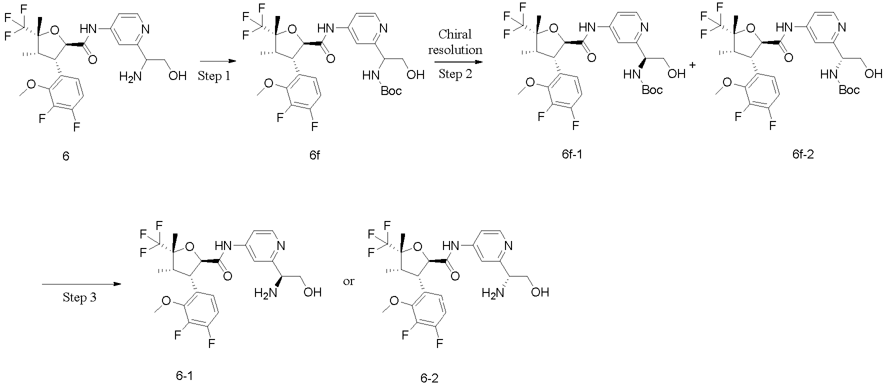

### Step 1

### tert-Butyl (1-(4-((2R,3S,4S,5R)-3-(3,4-difluoro-2-methoxyphenyl)-4,5-dimethyl-5-(trifluoromethyl)tetrahydrofuran-2-carboxamido)pyridin-2-yl)-2-hydroxyethyl)carbamate 6f (a mixture of diastereomers)

Compound **6** (190 mg, 388.2 µmol) was dissolved in dichloromethane (6 mL), and di-tert-butyl dicarbonate (85 mg, 388.2 µmol) was added. The mixture was stirred for 14 h. The reaction mixture was concentrated under reduced pressure to give crude title compound **6f** (230 mg, a mixture of diastereomers).

MS m/z (ESI): 590.2 [M+1].

### Step 2

### tert-Butyl ((S)-(1-(4-((2R,3S,4S,5R)-3-(3,4-difluoro-2-methoxyphenyl)-4,5-dimethyl-5-(trifluoromethyl)tetrahydrofuran-2-carboxamido)pyridin-2-yl)-2-hydroxyethyl)carbamate 6f-1

### tert-Butyl ((R)-(1-(4-((2R,3S,4S,5R)-3-(3,4-difluoro-2-methoxyphenyl)-4,5-dimethyl-5-(trifluoromethyl)tetrahydrofuran-2-carboxamido)pyridin-2-yl)-2-hydroxyethyl)carbamate 6f-2

Compound **6f** (230 mg) was resolved on a chiral column (Gilson 281, chromatography column: CHIRALPAK IE, 20 × 250 mm, 10 µm; mobile phase A: n-hexane, mobile phase B: ethanol, gradient ratio: A:B: 90:10, flow rate: 20 mL/min) to give the title compounds (65 mg and 114 mg, yields: 28.4% and 49.8%).

The chiral HPLC analysis method for the mixture of diastereomers **6f:** Agilent 1260 DAD, chromatography column: CHIRALPAK IE, 250 × 4.6 mm, 5 µm; mobile phase: n-hexane and ethanol (containing 0.1% diethylamine), gradient ratio: A:B: 90:10, flow rate: 1.0 mL/min.

Single-configuration compound (shorter retention time): 6.447 min, (65 mg, yield: 28.4%).

MS m/z (ESI): 590.2 [M+1].

Single-configuration compound (longer retention time): 8.484 min, (114 mg, yield: 49.8%).

MS m/z (ESI): 590.2 [M+1].

### Step 3

### (2R,3S,4S,5R)-N-(2-((S)-1-Amino-2-hydroxyethyl)pyridin-4-yl)-3-(3,4-difluoro-2-methoxyphenyl)-4,5-dimethyl-5-(trifluoromethyl)tetrahydrofuran-2-carboxamide 6-1 or

### (2R,3S,4S,5R)-N-(2-((R)-1-Amino-2-hydroxyethyl)pyridin-4-yl)-3-(3,4-difluoro-2-methoxyphenyl)-4,5-dimethyl-5-(trifluoromethyl)tetrahydrofuran-2-carboxamide 6-2

The compound corresponding to the longer retention time between compounds **6f-1** and **6f-2** (114 mg, 193 µmol) was dissolved in dichloromethane (4 mL), and a 4 M solution of hydrochloric acid in 1,4-dioxane (2 mL) was added. The mixture was left to react for 1 h and then concentrated under reduced pressure, and the residue was purified by high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **6-1** or **6-2** (33.3 mg, yield: 61.7%).

MS m/z (ESI): 490.2 [M+1].

¹H NMR (500 MHz, CD₃OD): δ 8.37 (s, 1H), 7.66 (s, 2H), 7.11 (ddd, 1H), 6.97 (td, 1H), 5.06 (d,1H), 4.30 (dd, 1H), 3.98 (d, 3H), 3.87-3.53 (m, 2H), 2.78 (p, 1H), 1.64 (s, 3H), 1.30 (d, 1H), 0.80 (dt, 3H).

### Example 7

### (2R,35,4S,5R)-N-(2-(2H-Tetrazol-5-yl)pyridin-4-yl)-3-(3,4-difluoro-2-methoxyphenyl)-4,5-dimethyl-5-(trifluoromethyl)tetrahydrofuran-2-carboxamide 7

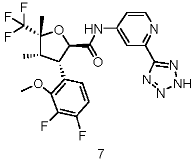

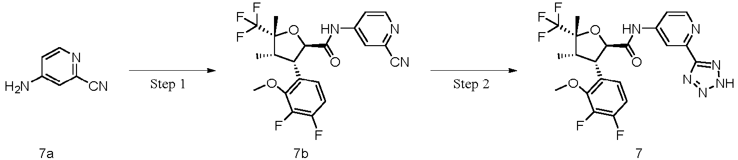

### Step 1

### (2R,3S,4S,5R)-N-(2-Cyanopyridin-4-yl)-3-(3,4-difluoro-2-methoxyphenyl)-4,5-dimethyl-5-(trifluoromethyl)tetrahydrofuran-2-carboxamide 7b

Compound **1b-1** (50 mg, 141 µmol) was dissolved in dichloromethane (10 mL), and oxalyl chloride (40 mg, 315 µmol) and one drop of *N,N-*dimethylformamide were added under an ice bath. The mixture was warmed to room temperature and left to react for 1 h. The reaction mixture was concentrated under reduced pressure, and the residue was dissolved in dichloromethane (3 mL). *N*,*N*-Diisopropylethylamine (60 mg, 464 µmol) was added, and a solution of 4-aminopyridine-2-carbonitrile **7a** (30 mg, 251 µmol, Shanghai Hanhong) in dichloromethane (1 mL) was added dropwise under an ice bath. The mixture was stirred for 2 h. The reaction mixture was concentrated under reduced pressure, and the residue was purified by silica gel column chromatography using eluent system B to give title compound **7b** (45 mg, yield: 70%).

MS m/z (ESI): 456.2 [M+1].

### Step 2

### (2R,3S,4S,5R)-N-(2-(2H-Tetrazol-5-yl)pyridin-4-yl)-3-(3,4-difluoro-2-methoxyphenyl)-4,5-dimethyl-5-(trifluoromethyl)tetrahydrofuran-2-carboxamide 7

Compound **7b** (20 mg, 44 µmol) and sodium azide (30 mg, 461 mmol) were dissolved in *N*,*N*-dimethylformamide (2 mL), and ammonium chloride (10 mg, 187 µmol) was added. The mixture was stirred at 110 °C for 4 h. The reaction mixture was cooled to room temperature, poured into water, and extracted with ethyl acetate (5 mL × 3). The organic phases were combined, dried over anhydrous sodium sulfate, filtered to remove the drying agent, and then concentrated under reduced pressure. The residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound 7 (12 mg, yield: 54%).

MS m/z (ESI): 499.2 [M+1].

¹H NMR (500 MHz, DMSO-*d₆*): δ 10.80 (s, 1H), 8.63-8.57 (m, 1H), 8.48-8.45 (m, 1H), 7.80 (s, 1H), 7.25-7.05 (m, 3H), 5.16-5.12 (m, 1H), 4.31-4.21 (m, 1H), 3.96 (s, 3H), 2.80-2.68 (m, 1H), 1.63 (s, 3H), 0.76-0.72 (m, 3H).

### Example 8

### (2R,3S,4S,5R)-N-(2-(1-Cyanocyclopropyl)pyridin-4-yl)-3-(3,4-difluoro-2-methoxyphenyl)-4,5-dimethyl-5-(trifluoromethyl)tetrahydrofuran-2-carboxamide 8

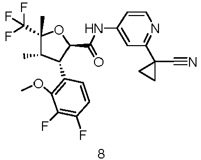

Title compound **8** (5 mg, yield: 4.6%) was obtained by using the synthesis scheme of Example 1 and replacing the starting material of step 2, compound **1c,** with 1-(4-aminopyridin-2-yl)cyclopropane-1-carbonitrile hydrochloride (prepared using the method disclosed in Example 98 on page 107 of the specification in the patent application "CN111196804").

MS m/z (ESI): 496.2 [M+1].

¹H NMR (500 MHz, CD₃OD): δ 8.33 (d, 1H), 7.99 (d, 1H), 7.58 (dd, 1H), 7.13 (ddd, 1H), 7.00 (td, 1H), 5.08 (d, 1H), 4.34 (dd, 1H), 4.02 (d, 3H), 2.81 (t, 1H), 1.74 (dt, 4H), 1.68 (s, 3H), 0.83 (dq, 3H).

### Example 9

### (2R,3S,4S,5R)-3-(3,4-Difluoro-2-methoxyphenyl)-4,5-dimethyl-5-(trifluoromethyl)-N-(2-ureidopyridin-4-yl)tetrahydrofuran-2-carboxamide 9

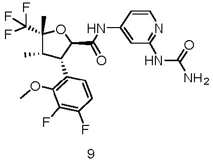

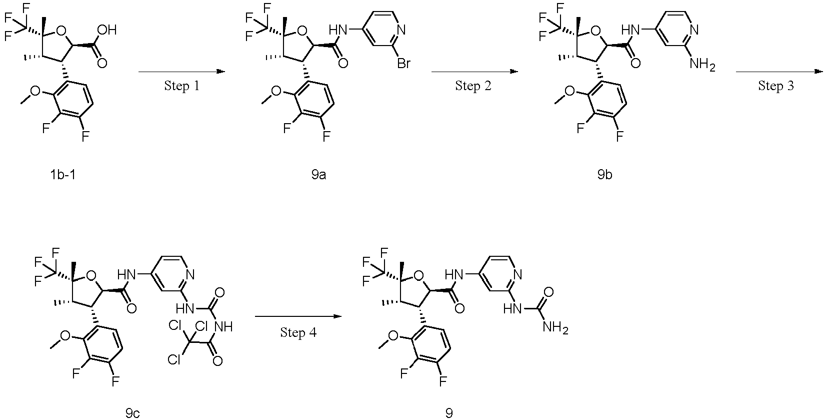

### Step 1

### (2R,3S,4S,5R)-N-(2-Bromopyiidin-4-yl)-3-(3,4-difluoro-2-methoxyphenyl)-4,5-dimethyl-5-(trifluoromethyl)tetrahydrofuran-2-carboxamide 9a

Compound **1b-1** (100 mg, 282.2 µmol) was dissolved in dichloromethane (5 mL), and oxalyl chloride (72 mg, 564 µmol) and one drop of *N,N-*dimethylformamide were added under an ice bath. The mixture was warmed to room temperature and left to react for 1 h. The reaction mixture was concentrated under reduced pressure, and the residue was dissolved in dichloromethane (5 mL). Triethylamine (143 mg, 1.4 mmol) and 4-dimethylaminopyridine (100 mg, 282.2 µmol) were added, and a solution of 2-bromo-4-aminopyridine (50 mg, 289 µmol) in dichloromethane (1 mL) was added dropwise under an ice bath. The mixture was stirred for 14 h. The reaction mixture was concentrated under reduced pressure, and the residue was purified by silica gel column chromatography using eluent system B to give title compound **9a** (90 mg, yield: 62.6%).

MS m/z (ESI): 509.2 [M+1].

### Step 2

### (2R,3S,4S,5R)-N-(2-Aminopyridin-4-yl)-3-(3,4-difluoro-2-methoxyphenyl)-4,5-dimethyl-5-(trifluoromethyl)tetrahydrofuran-2-carboxamide 9b

Compound **9a** (45 mg, 88.3 µmol) and urea (43.2 mg, 166.6 µmol) were dissolved in 1,4-dioxane (5 mL), and tris(dibenzylideneacetone)palladium (10 mg, 11 µmol,Shanghai Titan), 4,5-bis(diphenylphosphino)-9,9-dimethylxanthene (10 mg, 17 µmol,Shanghai Titan), and cesium carbonate (60 mg, 184 µmol) were added. The system was purged with nitrogen and stirred at 105 °C for 14 h. The reaction mixture was concentrated under reduced pressure, and the residue was purified by silica gel column chromatography using eluent system A to give title compound **9b** (39 mg, yield: 99%).

MS m/z (ESI): 446.2 [M+1].

### Step 3

### (2R,3S,4S,5R)-3-(3,4-Difluoro-2-methoxyphenyl)-4,5-dimethyl-N-(2-(3-(2,2,2-trichloroacetyl)ureido)pyridin-4-yl)-5-(trifluoromethyl)tetrahydrofuran-2-carboxamide 9c

Compound **9b** (39 mg, 87.5 µmol) was dissolved in tetrahydrofuran, and trichloroacetyl isocyanate (19.84 mg, 105.1 µmol,TCI) was added under an ice bath. With the temperature maintained, the mixture was left to react for 20 min. The reaction mixture was concentrated under reduced pressure to give crude title compound **9c** (50 mg). The product was directly used in the next step without purification.

MS m/z (ESI): 633.2 [M+1].

### Step 4

### (2R,3S,4S,5R)-3-(3,4-Difluoro-2-methoxyphenyl)-4,5-dimethyl-5-(trifluoromethyl)-N-(2-ureidopyridin-4-yl)tetrahydrofuran-2-carboxamide 9

Compound **9c** (50 mg, 78.9 µmol) was dissolved in a 7 M solution of ammonia in methanol (2 mL). The mixture was stirred for 10 min. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **9** (4 mg, yield: 10.3%).

MS m/z (ESI): 489.2 [M+1].

¹H NMR (500 MHz, CD₃OD): δ 8.10 (d, 1H), 7.54 (d, 1H), 7.17 (dd, 1H), 7.12 (ddd, 1H), 6.99 (td, 1H), 5.07 (dd, 1H), 4.37-4.29 (m, 1H), 4.01 (d, 3H), 2.80 (p, 1H), 1.67 (s, 3H), 0.83 (dq, 3H).

### Example 10

### (2R,3S,4S,5R)-3-(3,4-Difluoro-2-methoxyphenyl)-4,5-dimethyl-N-(2-(2-oxotetrahydropyrimidin-1(2H)-yl)pyridin-4-yl)-5-(trifluoromethyl)tetrahydrofuran-2-carboxamide 10

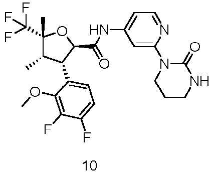

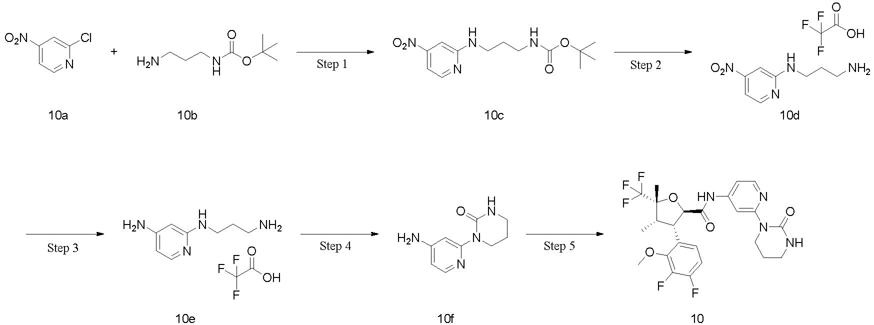

### Step 1

### tert-Butyl (3-((4-nitropyridin-2-yl)amino)propyl)carbamate 10c

2-Chloro-4-nitropyridine **10a** (1 g, 5.46 mmol, Shanghai Accela) and tert-butyl (3-aminopropyl)carbamate **10b** (1.73 g, 9.93 mmol, Shanghai Bide) were dissolved in *N,N-*dimethylformamide (25 mL), and N,N-diisopropylethylamine (2.58 g, 19.96 mmol) was added. The mixture was stirred at 110 °C for 4 h. The reaction mixture was cooled to room temperature, poured into water, and extracted with ethyl acetate (30 mL × 3). The organic phases were combined, dried over anhydrous sodium sulfate, filtered to remove the drying agent, and then concentrated under reduced pressure. The residue was purified by silica gel column chromatography using eluent system B to give title compound **10c** (300 mg, yield: 10.1%).

MS m/z (ESI): 297.2 [M+1].

### Step 2

*N*¹-(4-Nitropyridin-2-yl)propane-1,3-diamine 2,2,2-trifluoroacetate **10d** Compound **10c** (300 mg, 1.01 mol) was dissolved in dichloromethane (3 mL), and 3 mL of trifluoroacetic acid was added. The mixture was stirred for 3 h. The reaction mixture was concentrated under reduced pressure, and the residue was dissolved in dichloromethane and washed with a saturated sodium bicarbonate solution. The organic phase was dried over anhydrous sodium sulfate, filtered to remove the drying agent, and then concentrated under reduced pressure to give crude title compound **10d** (224 mg). The product was directly used in the next step without purification.

MS m/z (ESI): 197.2 [M+1].

### Step 3

### N²-(3-Aminopropyl)pyridine-2,4-diamine 2,2,2-trifluoroacetate 10e

Crude compound **10d** (224 mg, 722.04 µmol) was dissolved in methanol (5 mL), and 10% palladium on carbon catalyst (wet) (70 mg) was added. The mixture was stirred for 14 h in a hydrogen atmosphere. The reaction mixture was filtered, and the filtrate was concentrated under reduced pressure to give crude title compound **10e** (189 mg). The product was directly used in the next step without purification.

MS m/z (ESI): 167.2 [M+1].

### Step 4

### 1-(4-Aminopyridin-2-yl)tetrahydropyrimidin-2(1H)-one 10f

Crude compound **10e** (189 mg, 674 µmol) was dissolved in 5 mL of tetrahydrofuran, and *N,N-*diisopropylethylamine (441 mg, 3.4 mmol) and *N,N-*carbonyldiimidazole (CDI) (185 mg, 1.14 mmol) were added. The mixture was refluxed for 14 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **10f** (15 mg, yield: 6.8%).

MS m/z (ESI): 192.2 [M+1].

### Step 5

### (2R,3S,4S,5R)-3-(3,4-Difluoro-2-methoxyphenyl)-4,5-dimethyl-N-(2-(2-oxotetrahydropyrimidin-1(2H)-yl)pyridin-4-yl)-5-(trifluoromethyl)tetrahydrofuran-2-carboxamide 10

Compound **1b-1** (50 mg, 141.4 µmol) was dissolved in dichloromethane (3 mL), and oxalyl chloride (17 mg, 134 µmol) and one drop of *N,N-*dimethylformamide were added under an ice bath. The mixture was warmed to room temperature and left to react for 1 h. The reaction mixture was concentrated under reduced pressure, and the residue was dissolved in dichloromethane (3 mL). *N,N-*Diisopropylethylamine (43 mg, 332.7 µmol), 4-dimethylaminopyridine (8 mg, 65 µmol), and compound **10f** (15 mg, 78 µmol) were added. The mixture was stirred for 1 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **10** (3 mg, yield: 8.6%).

MS m/z (ESI): 529.2 [M+1].

¹H NMR (500 MHz, DMSO-*d₆*): δ 10.59 (s, 1H), 8.18 (d, 1H), 8.01 (d, 1H), 7.41 (dd, 1H), 7.20-7.15 (m, 1H), 7.11-7.08 (m, 1H), 6.87-6.85 (m, 1H), 5.08 (d, 1H), 4.23 (dd, 1H), 3.95 (s, 3H), 3.83-3.80 (m, 2H), 3.21-3.18 (m, 2H), 2.79-2.73 (m, 1H), 1.92-1.87 (m, 2H), 1.58 (s, 3H), 0.71 (d, 3H).

### Example 11

### (2R,3S,4S,5R)-N-(2-Acetamidopyridin-4-yl)-3-(3,4-difluoro-2-methoxyphenyl)-4,5-dimethyl-5-(trifluoromethyl)tetrahydrofuran-2-carboxamide 11

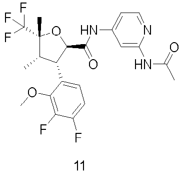

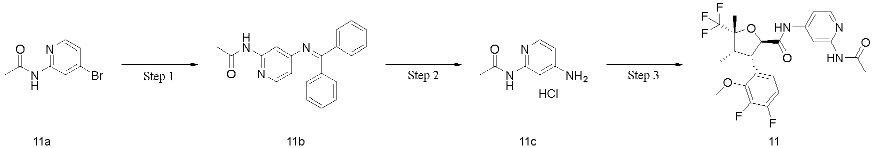

### Step 1

### N-(4-((Diphenylmethylene)amino)pyridin-2-yl)acetamide 11b

*N*-(4-Bromopyridin-2-yl)acetamide **11a** (500 mg, 2.32 mmol, Shanghai Accela) and benzophenonimine (510 mg, 2.81 mmol) were dissolved in 1,4-dioxane (20 mL), and tris(dibenzylideneacetone)palladium (220 mg, 240 mmol), 4,5-bis(diphenylphosphino)-9,9-dimethylxanthene (250 mg, 432 mmol), and cesium carbonate (2.1 g, 6.44 mmol) were added. The system was purged with nitrogen and stirred at 100 °C for 2 h. The reaction mixture was concentrated under reduced pressure, and the residue was purified by silica gel column chromatography using eluent system B to give title compound **11b** (430 mg, yield: 58.6%).

MS m/z (ESI): 316.2 [M+1].

### Step 2

### N-(4-Aminopyridin-2-yl)acetamide 11c

Compound **11b** (200 mg, 634.18 µmol) was dissolved in concentrated hydrochloric acid (5 mL). The mixture was stirred for 30 min. The reaction mixture was concentrated under reduced pressure. Ethyl acetate (5 mL) was added to the residue, and the mixture was stirred for 30 min and filtered. The filter cake was dried to give crude title compound **11c** (100 mg). The product was directly used in the next step without purification.

MS m/z (ESI): 152.2 [M+1].

### Step 3

### (2R,3S,4S,5R)-N-(2-Acetamidopyridin-4-yl)-3-(3,4-difluoro-2-methoxyphenyl)-4,5-dimethyl-5-(trifluoromethyl)tetrahydrofuran-2-carboxamide 11

Compound **1b-1** (50 mg, 141 µmol) and crude compound **11c** (50 mg, 266 µmol) were dissolved in 2 mL of *N,N*-dimethylformamide, and *N,N-*diisopropylethylamine (55 mg, 425.5 µmol) and *O*-(7-azabenzotriazol-1-yl)-*N,N,N',N'*-tetramethyluronium hexafluorophosphate (HATU) (80 mg, 210 µmol) were added under an ice bath. With the temperature maintained, the mixture was left to react for 2 h. The reaction mixture was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **11** (4 mg, yield: 5.8%).

MS m/z (ESI): 488.2 [M+1].

¹H NMR (500 MHz, DMSO-*d₆*):δ 10.70 (s, 1H), 10.42 (s, 1H), 8.25 (s, 1H), 8.16 (d, 1H), 7.51-7.46 (m, 1H), 7.19 (q, 1H), 7.14-7.08 (m, 1H), 5.09 (d, 1H), 4.25 (dd, 1H), 3.96 (d, 3H), 2.77 (p, 1H), 2.08 (s, 3H), 1.59 (s, 3H), 0.73 (d, 3H).

### Example 12

### (2R,3S,4S,5R)-3-(3,4-Difluoro-2-methoxyphenyl)-N-(2-(hydrazinecarbonyl)pyridin-4-yl)-4,5-dimethyl-5-(trifluoromethyl)tetrahydrofuran-2-carboxamide 12

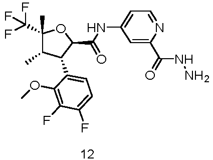

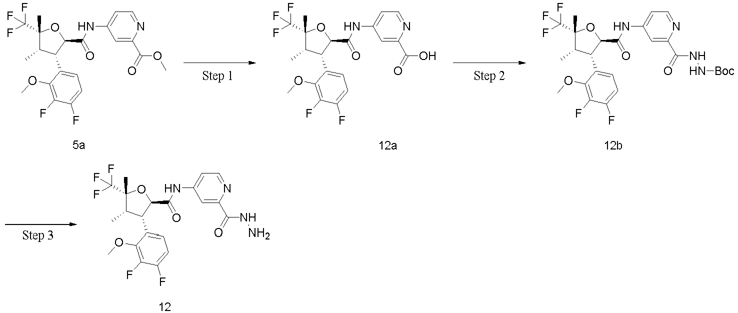

### Step 1

### 4-((2R,3S,4S,5R)-3-(3,4-Difluoro-2-methoxyphenyl)-4,5-dimethyl-5-(trifluoromethyl)tetrahydrofuran-2-carboxamido)picolinic acid 12a

Compound **5a** (700 mg, 1.43 mmol) was dissolved in methanol (5 mL) and water (5 mL), and lithium hydroxide monohydrate (400 mg, 1.43 mmol) was added. The mixture was stirred for 1 h. The reaction mixture was concentrated under reduced pressure and diluted with water, and the pH was adjusted to 3-4 with 1 M hydrochloric acid. The mixture was filtered, and the filter cake was washed with water and dried to give crude title compound **12a** (650 mg). The product was directly used in the next step without purification.

MS m/z (ESI): 473.1 [M-1].

### Step 2

### tert-Butyl 2-(4-((2R,3S,4S,5R)-3-(3,4-difluoro-2-methoxyphenyl)-4,5-dimethyl-5-(trifluoromethyl)tetrahydrofuran-2-carboxamido)picolinoyl)hydrazine-1-carboxylate 12b

Compound **12a** (50 mg, 105.4 µmol) and tert-butyl carbazate (16 mg, 93 µmol,Shanghai Bide) were dissolved in 1 mL of *N,N-*dimethylformamide, and *N,N-*diisopropylethylamine (41 mg, 317.2 µmol) and *O*-(7-azabenzotriazol-1-yl)-*N,N,N',N'-*tetramethyluronium hexafluorophosphate (HATU) (61 mg, 160.4 µmol) were added under an ice bath. With the temperature maintained, the mixture was left to react for 14 h. Water was added to the reaction mixture, and extraction was performed with ethyl acetate (10 mL × 3). The organic phases were combined, dried over anhydrous sodium sulfate, filtered to remove the drying agent, and then concentrated under reduced pressure. The residue was purified by silica gel column chromatography using eluent system A to give title compound **12b** (55 mg, yield: 88%).

MS m/z (ESI): 589.2 [M+1].

### Step 3

### (2R,3S,4S,5R)-3-(3,4-Difluoro-2-methoxyphenyl)-N-(2-(hydrazinecarbonyl)pyridin-4-yl)-4,5-dimethyl-5-(trifluoromethyl)tetrahydrofuran-2-carboxamide 12

Compound **12b** (50 mg, 105.4 µmol) was dissolved in dichloromethane (5 mL), and a 4 M solution of hydrogen chloride in 1,4-dioxane (3 mL) was added. The mixture was stirred for 1 h. The reaction mixture was concentrated under reduced pressure, and the residue was dissolved in methanol. Solid potassium carbonate was added for neutralization, and the mixture was filtered. The filtrate was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **12** (20 mg, yield: 48.2%).

MS m/z (ESI): 489.2 [M+1].

¹H NMR (500 MHz, DMSO-*d₆*): δ 10.76 (s, 1H), 9.82 (s, 1H), 8.47 (s, 1H), 8.24 (s, 1H), 7.82 (s, 1H), 7.17 (brs, 2H), 5.11 (d, 1H), 4.54 (s, 2H), 4.26 (t, 1H), 3.93 (s, 3H), 2.78(t, 1H), 1.62 (s, 3H), 0.74 (s, 3H).

### Example 13

### 4-((2R,3S,4S,5R)-3-(3,4-Difluoro-2-methoxyphenyl)-4,5-dimethyl-5-(trifluoromethyl)tetrahydrofuran-2-carboxamido)-N-(pyrrolidin-1-yl)picolinamide 13

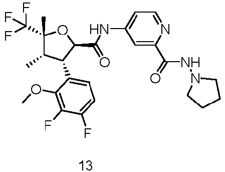

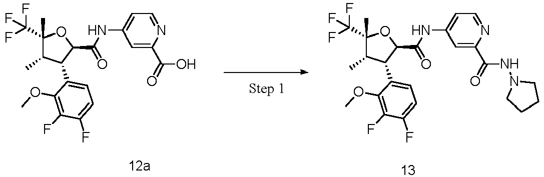

Compound **12a** (50 mg, 105.4 µmol) and 1-aminopyrrolidine hydrochloride (25.9 mg, 210.9 µmol,Shanghai Bide) were dissolved in 5 mL of *N,N*-dimethylformamide, and *N,N*-diisopropylethylamine (68.1 mg, 527 µmol) and *O*-(7-azabenzotriazol-1-yl)-*N,N,N'*,*N'*-tetramethyluronium hexafluorophosphate (HATU) (80.2 mg, 210.8 µmol) were added under an ice bath. With the temperature maintained, the mixture was left to react for 2 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **13** (12 mg, yield: 22%).

MS m/z (ESI): 543.2.2 [M+1].

¹H NMR (500 MHz, CDCl₃): δ 8.63 (d, 2H),8.40 (d, 1H), 8.19-8.17 (m, 1H), 7.86 (s, 1H), 7.24-7.07 (m, 1H), 7.05-6.90 (m, 1H), 5.00 (d, 1H), 4.08-4.04 (m, 1H), 4.00 (s, 3H), 3.01 (t, 4H), 2.76-2.73 (m, 1H), 1.96-1.94 (m, 4H), 1.69 (s, 3H), 0.79 (d, 3H).

### Example 14

### (2R,3S,4S,5R)-3-(3,4-Difluoro-2-methoxyphenyl)-4,5-dimethyl-N-(2-(pyrazolidine-1-carbonyl)pyridin-4-yl)-5-(trifluoromethyl)tetrahydrofuran-2-carboxamide 14

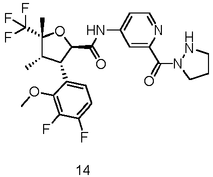

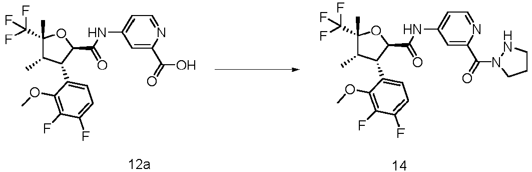

Compound **12a** (50 mg, 105.4 µmol) and pyrazolidine dihydrochloride (30.5 mg, 210.9 µmol,Shanghai Accela) were dissolved in 5 mL of *N,N-*dimethylformamide, and *N,N-*diisopropylethylamine (68.1 mg, 527 µmol) and *O*-(7-azabenzotriazol-1-yl)-*N,N,N',N'-*tetramethyluronium hexafluorophosphate (HATU) (80.2 mg, 210.8 µmol) were added under an ice bath. With the temperature maintained, the mixture was left to react for 2 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **14** (10 mg, yield: 18%).

MS m/z (ESI): 529.2 [M+1].

¹H NMR (500 MHz, CDCl₃): δ 8.56-8.47 (m, 2H),7.82-7.77 (m, 1H), 7.08-7.05 (m, 1H), 6.93-6.89 (m, 1H), 5.00 (d, 1H),4.08-4.04 (m, 1H), 4.00 (s, 3H), 3.78-3.75 (m, 1H), 3.21-3.09 (m, 2H), 2.76-2.73 (m, 1H), 2.23-2.01 (m, 3H), 1.67 (s, 3H), 0.89-0.87 (m,2H), 0.79 (d, 3H).

### Example 15

### (2R,3S,4S,5R)-3-(3,4-Difluoro-2-methoxyphenyl)-N-(2-(isoxazolidine-2-carbonyl)pyridin-4-yl)-4,5-dimethyl-5-(trifluoromethyl)tetrahydrofuran-2-carboxamide 15

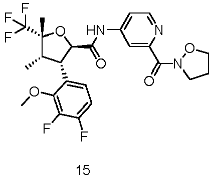

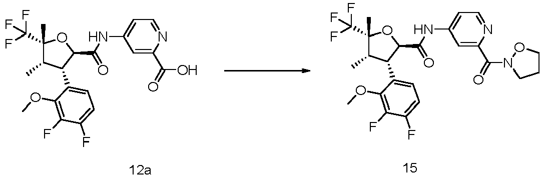

Compound **12a** (50 mg, 105.4 µmol) and isoxazolidine hydrochloride (23.1 mg, 210.7 µmol,Shanghai Accela) were dissolved in 5 mL of *N,N-*dimethylformamide, and *N,N-*diisopropylethylamine (54.5 mg, 421.6 µmol) and *O*-(7-azabenzotriazol-1-yl)-*N,N,N',N'-*tetramethyluronium hexafluorophosphate (HATU) (80.2 mg, 210.8 µmol) were added under an ice bath. With the temperature maintained, the mixture was left to react for 2 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **15** (10 mg, yield: 18%).

MS m/z (ESI): 530.2 [M+1].

¹H NMR (500 MHz, CDCl₃): δ 8.53 (s, 1H),7.97 (d, 2H), 7.26-7.09 (m, 1H), 6.92-6.87 (m, 1H), 5.05 (d, 1H), 4.41-4.04 (m, 6H), 4.00 (s, 3H), 2.77-2.74 (m, 1H), 2.44-2.39 (m, 2H), 1.68 (s, 3H), 0.80-0.77 (m, 3H).

### Example 16

### (2R,3S,4S,5R)-3-(3,4-Difluoro-2-methoxyphenyl)-N-(2-((Z)-(N'-methoxycarbamimidoyl)pyridin-4-yl)-4,5-dimethyl-5-(trifluoromethyl)tetrahydrofuran-2-carboxamide 16

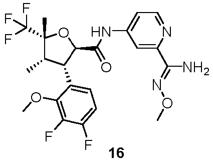

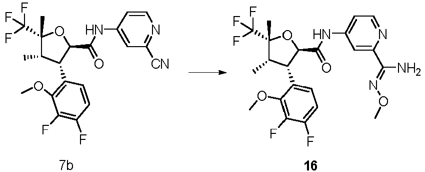

Compound **7b** (100 mg, 219.6 µmol) was dissolved in 10 mL of isopropanol, and *N,N-*diisopropylethylamine (85.1 mg, 658.8 µmol), mercaptoacetic acid (40.5 mg, 439 µmol, Shanghai Bide), and methoxyamine hydrochloride (55 mg, 658.8 µmol) were added. The mixture was left to react at 80 °C for 14 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **16** (10 mg, yield: 18%).

MS m/z (ESI): 503.2 [M+1].

¹H NMR (500 MHz, DMSO-*d₆*):δ 10.69 (s, 1H), 8.44 (d, 1H), 8.11 (d, 1H), 7.72 (dd, 1H), 7.18 (dt, 2H), 6.07 (s, 1H), 5.09 (d, 1H), 4.25 (dd, 1H), 3.96 (d, 3H), 3.79 (d, 3H), 2.78 (t, 1H), 2.01 (q, 1H), 1.61 (s, 3H), 0.73 (d, 3H).

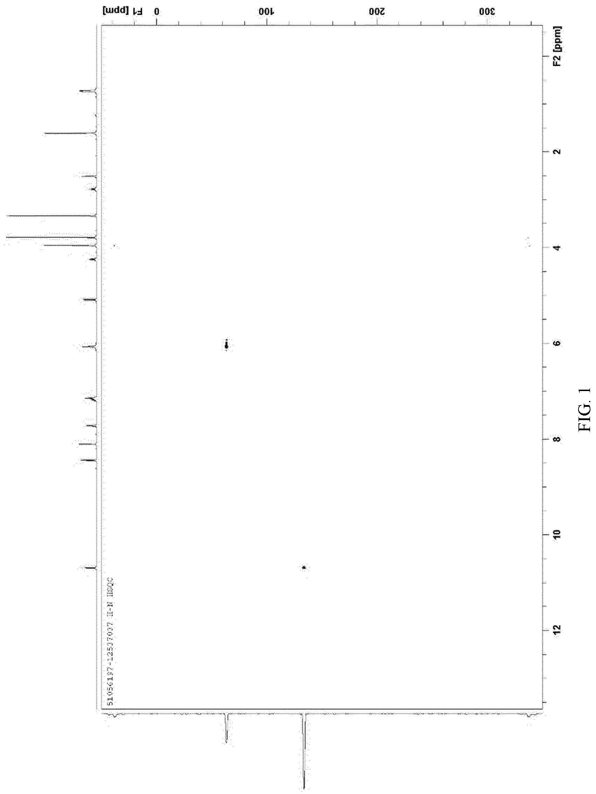

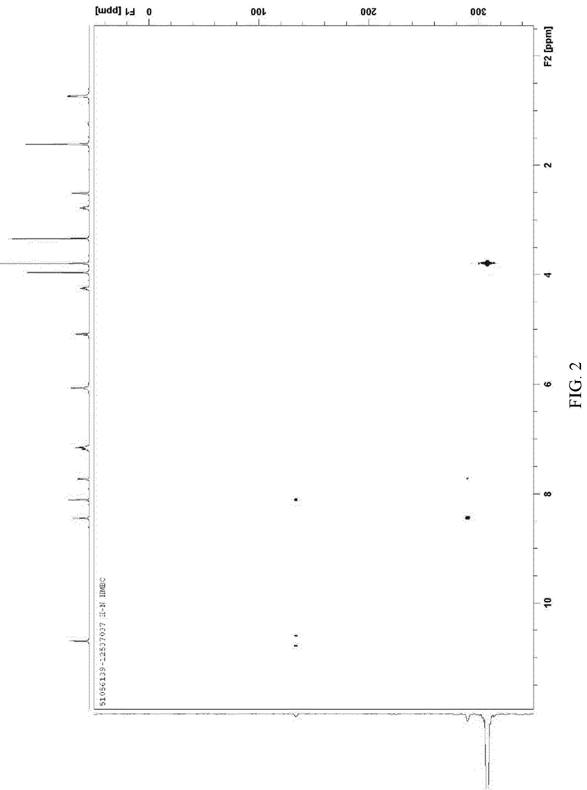

The H-N HSQC spectrum of the compound of Example 16 is shown in FIG. 1, and the H-N HMBC spectrum is shown in FIG. 2.

### Example 17

### 4-(2R,3S,4S,5R)-3-(3,4-Difluoro-2-methoxyphenyl)-4,5-dimethyl-5-(trifluoromethyl)tetrahydrofuran-2-carboxamido-N-((1-hydroxycyclopropyl)methyl)picolinamide 17

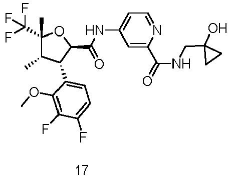

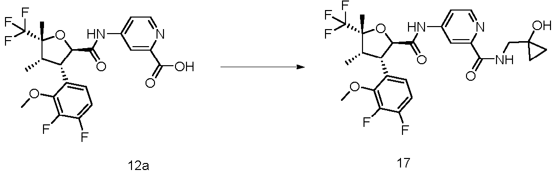

Compound **12a** (51.5 mg, 108.6 µmol) and 1-(aminomethyl)cyclopropanol (19 mg, 217.3 µmol,Shanghai Accela) were dissolved in 5 mL of *N,N-*dimethylformamide, and *N,N-*diisopropylethylamine (42 mg, 326 µmol) and *O*-(7-azabenzotriazol-1-yl)-*N,N,N',N'-*tetramethyluronium hexafluorophosphate (HATU) (80.6 mg, 217.2 µmol) were added under an ice bath. With the temperature maintained, the mixture was left to react for 2 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **17** (12 mg, yield: 20.3%).

MS m/z (ESI): 544.2 [M+1].

¹H NMR (500 MHz, CDCl₃):δ 8.79 (s, 2H), 8.47 (d, 1H), 8.27 (d, 1H), 8.03 (s, 1H), 7.08-7.05 (m, 1H), 6.94-6.89 (m, 1H), 5.04(d, 1H), 4.10-4.06 (m, 1H), 4.00 (s,3H), 3.62 (t, 2H), 2.76 (t, 2H), 1.70 (s,3H), 0.91-0.88 (m, 2H), 0.79 (d, 3H), 0.69-0.66 (m,2H).

### Example 18

### N-(Cyclopropylmethoxy)-4-((2R,3S,4S,5R)-3-(3,4-difluoro-2-methoxyphenyl)-4,5-dimethyl-5-(trifluoromethyl)tetrahydrofuran-2-carboxamido)picolinamide 18

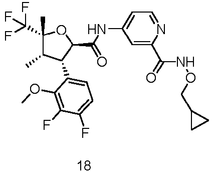

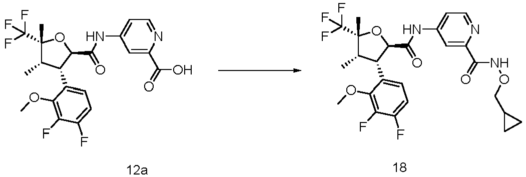

Compound **12a** (50 mg, 105.4 µmol) and *O*-cyclopropylmethylhydroxylamine hydrochloride (26 mg, 210.7 µmol,Shanghai Accela) were dissolved in 5 mL of *N,N-*dimethylformamide, and *N,N*-diisopropylethylamine (68.1 mg, 527 µmol) and *O*-(7-azabenzotriazol-1-yl)-*N,N,N',N'*-tetramethyluronium hexafluorophosphate (HATU) (80.2 mg, 210.8 µmol) were added under an ice bath. With the temperature maintained, the mixture was left to react for 2 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **18** (10 mg, yield: 26%).

MS m/z (ESI): 544.2 [M+1].

¹H NMR (500 MHz, CDCl₃): δ 10.23 (s, 1H), 8.61 (s, 1H), 8.40 (d, 1H), 8.15-8.13 (m, 1H), 7.86 (s, 1H), 7.09-7.05 (m, 1H), 6.93-6.88 (m, 1H), 5.01 (d, 1H), 4.08-4.05 (m, 1H), 4.00 (s, 3H), 3.87 (d, 2H), 2.75 (t, 1H), 1.69 (s, 3H), 0.89-0.86 (m, 1H), 0.80-0.78 (m, 3H), 0.65-0.61 (m, 2H), 0.36-0.33 (m, 2H).

### Example 19

### (2R,3S,4S,5R)-3-(3,4-Difluoro-2-methoxyphenyl)-4,5-dimethyl-N-(2-(2-methylhydrazine-1-carbonyl)pyridin-4-yl)-5-(trifluoromethyl)tetrahydrofuran-2-carboxamide 19

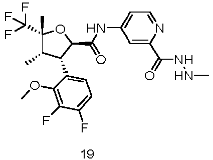

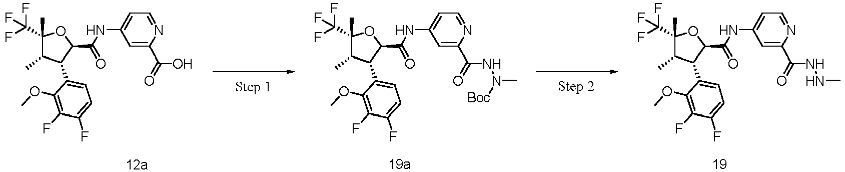

### Step 1

### tert-Butyl 2-(4-((2R,3S,4S,5R)-3-(3,4-difluoro-2-methoxyphenyl)-4,5-dimethyl-5-(trifluoromethyl)tetrahydrofuran-2-carboxamido)picolinoyl)-1-methylhydrazine-1-carboxylate 19a

Compound **12a** (40 mg, 84.3 µmol) and 1-tert-butoxycarbonyl-1-methylhydrazine (15 mg, 102.6 µmol,Shanghai Accela) were dissolved in 4 mL of *N,N-*dimethylformamide, and *N*,*N*-diisopropylethylamine (33 mg, 255.3 µmol) and *O*-(7-azabenzotriazol-1-yl)-*N*,*N*,*N'*,*N'*-tetramethyluronium hexafluorophosphate (HATU) (42 mg, 110.4 µmol) were added under an ice bath. With the temperature maintained, the mixture was left to react for 14 h. Water was added to the reaction mixture, and extraction was performed with dichloromethane (10 mL × 3). The organic phases were combined, dried over anhydrous sodium sulfate, filtered to remove the drying agent, and then concentrated under reduced pressure to give crude title compound **19a** (50 mg).

MS m/z (ESI): 603.2 [M+1].

### Step 2

### (2R,3S,4S,5R)-3-(3,4-Difluoro-2-methoxyphenyl)-4,5-dimethyl-N-(2-(2-methylhydrazine-1-carbonyl)pyridin-4-yl)-5-(trifluoromethyl)tetrahydrofuran-2-carboxamide 19

Compound **19a** (50 mg, 82.9 µmol) was dissolved in dichloromethane (5 mL), and trifluoroacetic acid (3 mL) was added. The mixture was stirred for 1 h. The reaction mixture was concentrated under reduced pressure, and the residue was dissolved in dichloromethane. The solution was neutralized with a saturated sodium bicarbonate solution and extracted with dichloromethane (5 mL × 3). The organic phases were combined, dried over anhydrous sodium sulfate, filtered to remove the drying agent, and then concentrated under reduced pressure. The residue was purified by silica gel column chromatography using eluent system A to give title compound **19** (30 mg, yield: 71.9%). MS m/z (ESI): 503.2 [M+1].

¹H NMR (500 MHz, DMSO-*d₆*): δ 10.74 (s,1H), 10.12 (s, 1H), 8.49 (d, 1H), 8.25 (s, 1H), 7.84 (d, 1H), 7.21-7.14 (m, 2H), 5.11 (d, 2H), 4.26 (t, 1H), 3.96 (s, 3H), 2.80-2.75 (m, 1H), 2.53 (s, 3H), 1.61 (s, 3H), 0.74 (d, 3H).

### Example 20

### (2R,3S,4S,5R)-N-(2-Carbamimidoylpyridin-4-yl)-3-(3,4-difluoro-2-methoxyphenyl)-4,5-dimethyl-5-(trifluoromethyl)tetrahydrofuran-2-carboxamide 20

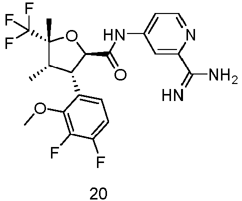

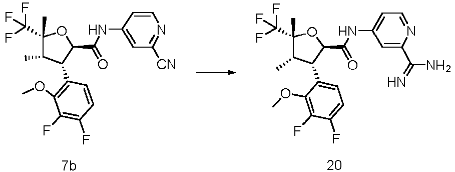

Compound **7b** (85 mg, 186.6 µmol) was dissolved in 2 mL of methanol, and sodium methoxide (10 mg, 185.1 µmol) and ammonium chloride (20 mg, 373.9 µmol) were added. The mixture was left to react at 70 °C for 6 h. The reaction mixture was cooled to room temperature and then filtered. The filtrate was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **20** (9 mg, yield: 10.2%).

MS m/z (ESI): 473.2 [M+1].

¹H NMR (500 MHz, DMSO-*d₆*): δ 8.48 (d, 1H), 8.36 (s, 1H), 7.79 (dd, 1H), 7.17 (dd, 2H), 5.12 (d, 1H), 4.26 (dd, 1H), 3.96 (d, 3H), 2.78 (p, 1H), 1.61 (s, 3H), 0.76-0.71 (m, 3H).

### Example 21

### (2R,3S,4S,5R)-3-(3,4-Difluoro-2-methoxyphenyl)-4,5-dimethyl-N-(2-((Z)-N'-methylcarbamimidoyl)pyridin-4-yl)-5-(trifluoromethyl)tetrahydrofuran-2-carboxamide 21

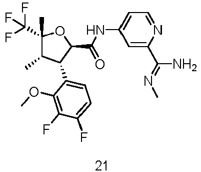

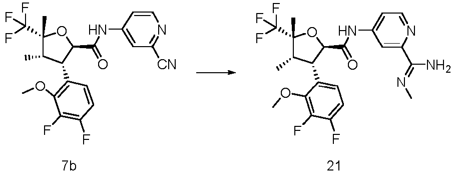

Compound **7b** (60 mg, 131.7 µmol) was dissolved in 5 mL of isopropanol, and *N,N-*diisopropylethylamine (51 mg, 395.2 µmol), mercaptoacetic acid (24.3 mg, 263.6 µmol, Shanghai Bide), and methylamine hydrochloride (26.7 mg,395.3 µmol) were added. The mixture was left to react at 80 °C for 14 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **21** (20 mg, yield: 31.2%).

MS m/z (ESI): 487.2 [M+1].

¹H NMR (500 MHz, CDCl₃): δ 8.45 (d, 1H), 8.40 (s, 1H), 8.28 (d, 1H), 7.13 (s, 1H), 6.92 (q, 1H), 5.05 (d, 1H), 4.14 (dd, 1H), 4.01 (d, 3H), 3.19 (s, 3H), 2.77 (p, 1H), 1.74 (s, 3H), 0.85-0.77 (m, 3H).

### Example 22

### (2R,3S,4S,5R)-3-(3,4-Difluoro-2-methoxyphenyl)-N-(2-(2-ethylhydrazine-1-carbonyl)pyridin-4-yl)-4,5-dimethyl-5-(trifluoromethyl)tetrahydrofuran-2-carboxamide 22

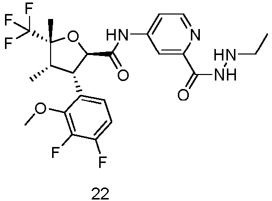

Title compound **22** was obtained by using the synthesis scheme of Example 13 and replacing the starting material compound 1-aminopyrrolidine hydrochloride with the compound ethylhydrazine hydrochloride (Shanghai Bide).

MS m/z (ESI): 517.2 [M+1].

¹H NMR (500 MHz, CD₃OD) δ 8.48 (d, 1H), 8.25 (s, 1H), 7.89 (d, 1H), 7.20-7.12 (m, 1H), 6.98-6.93(m, 1H), 5.08 (d, 1H), 4.33 (t, 1H), 4.00 (s, 3H), 2.94 (q, 2H), 2.80 (p, 1H), 1.66 (s, 3H), 1.14 (t, 3H), 0.82 (d, 3H).

### Example 23

### (2R,3S,4S,5R)-N-(2-(2-Cyclopropylhydrazine-1-carbonyl)pyridin-4-yl)-3-(3,4-difluoro-2-methoxyphenyl)-4,5-dimethyl-5-(trifluoromethyl)tetrahydrofuran-2-carboxamide 23

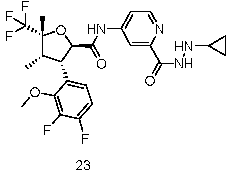

Title compound **23** (10 mg, yield: 15.7%) was obtained by using the synthesis scheme of Example 13 and replacing the starting material compound 1-aminopyrrolidine hydrochloride with the compound cyclopropylhydrazine hydrochloride.

MS m/z (ESI): 529.2 [M+1].

¹H NMR (500 MHz, CD₃OD): δ 8.50 (d, 1H), 8.26 (d, 1H), 7.90 (dd, 1H), 7.14 (ddd, 1H), 7.00 (td, 1H), 5.10 (d, 1H), 4.35 (dd, 1H), 4.02 (d, 3H), 2.82 (p, 1H), 2.69 (tt, 1H), 1.68 (d, 3H), 0.84 (dq, 3H), 0.64-0.57 (m, 2H), 0.57-0.49 (m, 2H).

### Example 24

### (2R,3S,4S,5R)-3-(3,4-Difluoro-2-methoxyphenyl)-N-(2-(2,2-dimethylhydrazine-1-carbonyl)pyridin-4-yl)-4,5-dimethyl-5-(trifluoromethyl)tetrahydrofuran-2-carboxamide 24

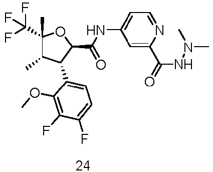

Title compound **24** (5 mg, yield: 23%) was obtained by using the synthesis scheme of Example 13 and replacing the starting material compound 1-aminopyrrolidine hydrochloride with the compound 1,1-dimethylhydrazine hydrochloride.

MS m/z (ESI): 517.2 [M+1].

¹H NMR (500 MHz, CD₃OD): δ 8.49 (d, 1H), 8.25 (d, 1H), 7.92 (dd, 1H), 7.14 (ddd, 1H), 7.05-6.96 (m, 1H), 5.10 (d, 1H), 4.34 (dd, 1H), 4.01 (d, 3H), 2.86-2.75 (m, 1H), 2.67 (s, 6H), 1.68 (s, 3H), 0.87-0.78 (m, 3H).

### Example 25

### (2R,3S,4S,5R)-N-(5-(2-Amino-2-oxoacetyl)-1H-pyrrol-3-yl)-3-(3,4-difluoro-2-methoxyphenyl)-4,5-dimethyl-5-(trifluoromethyl)tetrahydrofuran-2-carboxamide 25

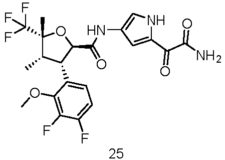

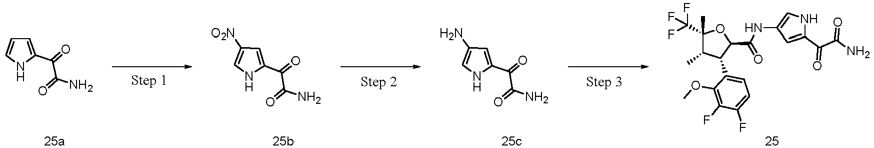

### Step 1

### 2-(4-Nitro-1H-pyrrol-2-yl)-2-oxoacetamide 25b

Concentrated nitric acid (4 mL) was added to acetic anhydride (10 mL) at -5 °C. The mixture was warmed to 0 °C, stirred for 30 min, and then cooled to -50 °C. A solution of 2-oxo-2-(1*H*-pyrrol-2-yl)acetamide **25a** (1.5 g, 10.86 mmol, prepared by the method disclosed in "Organic and Biomolecular Chemistry, 2009, vol. 7, # 10, p. 2187-2194") in chloroform (4 mL) was added. The mixture was slowly warmed to -30 °C and stirred for 1 h. The reaction mixture was poured into water (100 mL), the pH was adjusted to a neutral level with a saturated sodium carbonate solution, and extraction was performed with chloroform (30 mL × 3). The organic phases were combined, dried over anhydrous sodium sulfate, filtered to remove the drying agent, and then concentrated under reduced pressure. The residue was purified by silica gel column chromatography using eluent system A to give title compound **25b** (236 mg, yield: 11.8%).

MS m/z (ESI): 182.0 [M-1].

### Step 2

### 2-(4-Amino-1H-pyrrol-2-yl)-2-oxoacetamide 25c

Compound **25b** (80 mg, 436.8 µmol) was dissolved in methanol (10 mL), and 10% palladium on carbon catalyst (wet) (10 mg) was added. The mixture was stirred for 3 h in a hydrogen atmosphere. The reaction mixture was filtered, and the filtrate was concentrated under reduced pressure to give crude title compound **25c** (69 mg). The product was directly used in the next step without purification.

MS m/z (ESI): 154.2 [M+1].

### Step 3

### (2R,3S,4S,5R)-N-(5-(2-Amino-2-oxoacetyl)-1H-pyrrol-3-yl)-3-(3,4-difluoro-2-methoxyphenyl)-4,5-dimethyl-5-(trifluoromethyl)tetrahydrofuran-2-carboxamide 25

Compound **1b-1** (80 mg, 225.8 µmol) and crude compound **25c** (69 mg, 450.6 µmol) were dissolved in 2 mL of *N,N-*dimethylformamide, and *N,N*-diisopropylethylamine (29 mg, 225 µmol) and *O*-(7-azabenzotriazol-1-yl)-*N,N,N',N'*-tetramethyluronium hexafluorophosphate (HATU) (85 mg, 225.8 µmol) were added under an ice bath. The mixture was warmed to room temperature and stirred for 30 min. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **25** (2.5 mg, yield: 2.2%).

MS m/z (ESI): 490.0 [M+1].

¹H NMR (500 MHz, CD₃OD): δ 7.59 (d, 1H), 7.41 (s, 1H), 7.18-7.12 (m,1H), 7.02-6.96 (m, 1H), 5.02 (d, 1H), 4.27 (dd, 1H), 4.00 (s, 3H), 2.82-2.75 (m,1H), 1.67 (s, 3H), 1.40-1.32 (d, 3H), 0.86-0.80 (m, 1H).

### Example 26

### (2R,3S,4S,5R)-N-(2-(2-Amino-2-oxoacetyl)pyridin-4-yl)-3-(3,4-difluoro-2-methoxyphenyl)-4,5-dimethyl-5-(trifluoromethyl)tetrahydrofuran-2-carboxamide trifluoroacetate 26

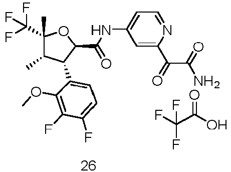

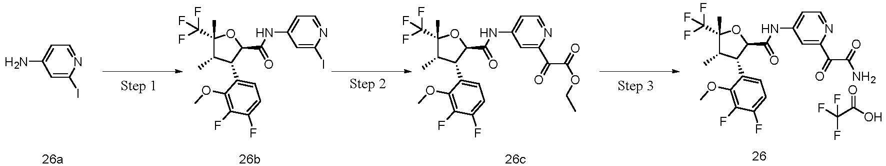

### Step 1

### (2R,3S,4S,5R)-3-(3,4-Difluoro-2-methoxyphenyl)-N-(2-iodopyridin-4-yl)-4,5-dimethyl-5-(trifluoromethyl)tetrahydrofuran-2-carboxamide 26b

Compound **1b-1** (500 mg, 1.41 mmol) was dissolved in dichloromethane (10 mL), and oxalyl chloride (628 mg, 4.94 mmol) and five drops of *N,N*-dimethylformamide were added under an ice bath. The mixture was warmed to room temperature and left to react for 1 h. The reaction mixture was concentrated under reduced pressure, and the residue was dissolved in dichloromethane (10 mL). *N,N*-Diisopropylethylamine (913 mg, 7.06 mmol) was added, and 2-iodopyridin-4-amine **26a** (342 mg, 1.55 mol, WuXi AppTec) and 4-dimethylaminopyridine (174 mg, 1.41 mmol) were added under an ice bath. The mixture was stirred for 2 h. The reaction mixture was concentrated under reduced pressure, and the residue was purified by silica gel column chromatography using eluent system B to give title compound **26b** (680 mg, yield: 86.6%).

MS m/z (ESI): 557.2 [M+1].

### Step 2

### Ethyl 2-(4-((2R,3S,4S,5R)-3-(3,4-difluoro-2-methoxyphenyl)-4,5-dimethyl-5-(trifluoromethyl)tetrahydrofuran-2-carboxamido)pyridin-2-yl)-2-oxoacetate 26c

Compound **26b** (680 mg, 1.22 mmol) was dissolved in tetrahydrofuran (15 mL), and a 1 M solution of isopropylmagnesium bromide in tetrahydrofuran (6.2 mL) was added under a nitrogen atmosphere and an ice bath. The mixture was warmed to room temperature and left to react for 1 h. Diethyl oxalate (1.79 g, 12.2 mmol) was added under an ice bath. The mixture was warmed to room temperature and left to react for another 2 h. After the reaction was complete, the reaction mixture was quenched with a saturated ammonium chloride solution and extracted with ethyl acetate (20 mL × 3). The organic phases were combined, washed with a saturated sodium chloride solution, dried over anhydrous sodium sulfate, filtered to remove the drying agent, and then concentrated under reduced pressure. The residue was purified by silica gel column chromatography using eluent system A to give title compound **26c** (340 mg, yield: 52.4%).

MS m/z (ESI): 531.2 [M+1].

### Step 3

### (2R,3S,4S,5R)-N-(2-(2-Amino-2-oxoacetyl)pyridin-4-yl)-3-(3,4-difluoro-2-methoxyphenyl)-4, 5-dimethyl-5-(trifluoromethyl)tetrahydrofuran-2-carboxamide trifluoroacetate 26

Compound **26c** (340 mg, 640.9 µmol) was dissolved in a 7 M solution of ammonia in methanol (10 mL). The mixture was stirred for 1 h. The reaction mixture was concentrated under reduced pressure, and the residue was purified by preparative high performance liquid chromatography (Waters-2545, chromatography column: Welch Xtimate Prep C18, 30 × 250 mm, 5 µm; mobile phase: aqueous phase (0.1% trifluoroacetic acid) and acetonitrile, gradient ratio: acetonitrile 30%-65%, flow rate: 30 mL/min) to give title compound **26** (108 mg, yield: 27.3%).

MS m/z (ESI): 502.2 [M+1].

¹H NMR (500 MHz, CD₃OD): δ 8.54 (d, 1H), 8.39 (d, 1H), 8.20 (dd, 1H), 7.12 (t, 1H), 6.98 (q, 1H), 5.16 (d, 1H), 4.35 (t, 1H), 4.00 (d, 3H), 2.84-2.78 (m, 1H), 1.66 (s, 3H), 0.83 (d, 3H).

### Example 27

### (2R,3S,4S,5R)-N-(3-Carbamimidoyl-4-fluorophenyl)-3-(3,4-difluoro-2-methoxyphenyl)-4,5-dimethyl-5-(trifluoromethyl)tetrahydrofuran-2-carboxamide 27

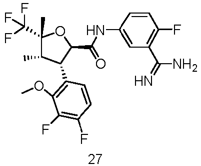

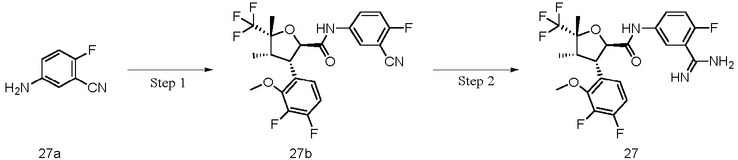

### Step 1

### (2R,3S,4S,5R)-N-(3-Cyano-4-fluorophenyl)-3-(3,4-difluoro-2-methoxyphenyl)-4,5-dimethyl-5-(trifluoromethyl)tetrahydrofuran-2-carboxamide 27b

Compound 1b-1 (260 mg, 734 µmol) was dissolved in dichloromethane (12 mL), and oxalyl chloride (279.7 mg, 2.2 mmol) and two drops of *N,N-*dimethylformamide were added under an ice bath. The mixture was warmed to room temperature and left to react for 1 h. The reaction mixture was concentrated under reduced pressure, and the residue was dissolved in dichloromethane (5 mL). N,N-Diisopropylethylamine (284 mg, 2.2 mmol) was added, and 5-amino-2-fluorobenzonitrile 27a (100 mg, 734.6 µmol, prepared by the method disclosed in "Bioorganic and Medicinal Chemistry Letters, 2006, vol. 16, # 19, p. 5176-5182") and a catalytic amount of 4-dimethylaminopyridine were added dropwise under an ice bath. The mixture was stirred for 2 h. The reaction mixture was concentrated under reduced pressure, and the residue was purified by silica gel column chromatography using eluent system B to give title compound **27b** (165 mg, yield: 47.5%).

MS m/z (ESI): 473.2 [M+1].

### Step 2

### (2R,3S,4S,5R)-N-(3-Carbamimidoyl-4-fluorophenyl)-3-(3,4-difluoro-2-methoxyphenyl)-4,5-dimethyl-5-(trifluoromethyl)tetrahydrofuran-2-carboxamide 27

Compound **27b** (83 mg, 175.7 µmol) was dissolved in 2 mL of methanol, and sodium methoxide (28.5 mg, 527 µmol) was added. The mixture was left to react at 35 °C for 1 h, and ammonium chloride (37.6 mg, 702.7 µmol) was then added. The mixture was left to react at 70 °C for 2 h. The reaction mixture was cooled to room temperature and then filtered. The filtrate was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **27** (8 mg, yield: 9%).

MS m/z (ESI): 490.2 [M+1].

¹H NMR (500 MHz, DMSO-*d₆*): δ 10.39 (s, 2H), 7.80 (dd, 1H), 7.69 (dt, 1H), 7.28-7.04 (m, 4H), 5.06 (d, 1H), 4.24 (dd, 1H), 3.95 (d, 3H), 2.76 (t, 1H), 2.06-1.94 (m, 1H), 1.60 (s, 3H), 0.78-0.62 (m, 3H).

### Example 28

### Hexyl ((4-((2R,3S,4S,5R)-3-(3,4-difluoro-2-methoxyphenyl)-4,5-dimethyl-5-(trifluoromethyl)tetrahydrofuran-2-carboxamido)pyridin-2-yl)(imino)methyl)carbamate 28

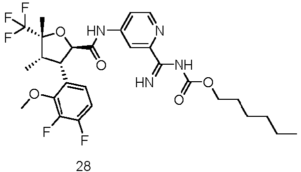

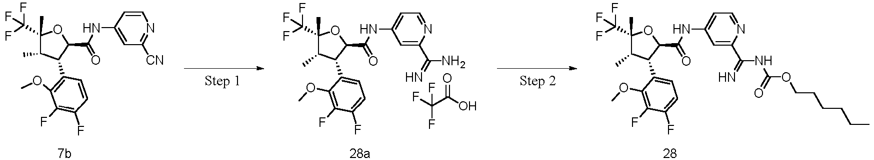

### Step 1

### (2R,3S,4S,5R)-N-(2-Carbamimidoylpyridin-4-yl)-3-(3,4-difluoro-2-methoxyphenyl)-4,5-dimethyl-5-(trifluoromethyl)tetrahydrofuran-2-carboxamide 2,2,2-trifluoroacetate 28a

Compound **7b** (2.8 g, 6.15 mmol) was dissolved in methanol (60 mL), and sodium methoxide (333 mg, 6.16 mmol) was added. The mixture was left to react at 35 °C for 5 h, and ammonium chloride (658 mg, 12.3 mmol) was then added. The mixture was left to react at 70 °C for 1.5 h. The reaction mixture was cooled to room temperature and then filtered. The filtrate was concentrated under reduced pressure, and the residue was purified by preparative high performance liquid chromatography (Waters-2545, chromatography column: Welch Xtimate Prep C18, 30 × 250 mm, 5 µm; mobile phase: aqueous phase (0.1% trifluoroacetic acid) and acetonitrile, gradient ratio: acetonitrile 35%-65%, flow rate: 30 mL/min) to give title compound **28a** (650 mg, yield: 18%).

MS m/z (ESI): 473.2 [M+1].

### Step 2

### Hexyl ((4-((2R,3S,4S,5R)-3-(3,4-difluoro-2-methoxyphenyl)-4,5-dimethyl-5-(trifluoromethyl)tetrahydrofuran-2-carboxamido)pyridin-2-yl)(imino)methyl)carbamate 28

Compound **28a** (100 mg, 170.5 µmol) was dissolved in tetrahydrofuran (3 mL), and *N,N-*diisopropylethylamine (66.1 mg, 511.5 µmol) and n-hexyl chloroformate (33.6 mg, 204.62 µmol, Shanghai Titan) were added. The mixture was stirred for 20 min and then concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **28** (46 mg, yield: 44.9%).

MS m/z (ESI): 601.1 [M+1].

¹H NMR (500 MHz, CD₃OD): δ 8.55 (d, 1H), 8.32 (s, 1H), 8.02 (d, 1H), 7.15 (t, 1H), 7.00 (q, 1H), 5.10 (d, 1H), 4.35 (t, 1H), 4.18 (t, 2H), 4.02 (s, 3H), 2.90-2.73 (m, 1H), 1.71 (d, 5H), 1.46-1.34 (m, 6H), 0.94 (q, 3H), 0.84 (d, 3H).

### Example 29

### Isopropyl ((4-((2R,3S,4S,5R)-3-(3,4-difluoro-2-methoxyphenyl)-4,5-dimethyl-5-(trifluoromethyl)tetrahydrofuran-2-carboxamido)pyridin-2-yl)(imino)methyl)carbamate 29

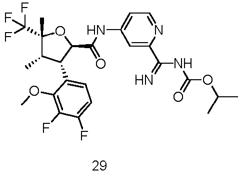

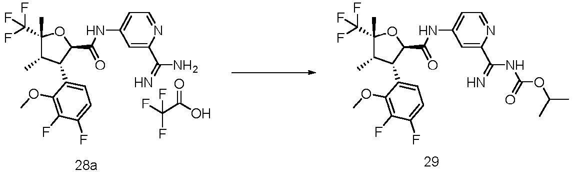

Compound **28a** (57 mg, 97.19 µmol) was dissolved in tetrahydrofuran (3 mL), and *N,N-*diisopropylethylamine (37.6 mg, 291.5 µmol) and isopropyl chloroformate (14.3 mg, 116.6 µmol, Shanghai Titan) were added. The mixture was stirred for 20 min and then concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **29** (28 mg, yield: 51.5%).

MS m/z (ESI): 559.2 [M+1].

¹H NMR (500 MHz, CD₃OD): δ 8.55 (d, 1H), 8.31 (d, 1H), 8.02 (dd, 1H), 7.15 (ddd, 1H), 7.00 (td, 1H), 5.10 (d, 1H), 5.01 (q, 1H), 4.35 (dd, 1H), 4.02 (d, 3H), 2.82 (p, 1H), 1.69 (s, 3H), 1.34 (d, 6H), 0.84 (dq, 3H).

### Example 30

### Hexyl ((5-((2R,3S,4S,5R)-3-(3,4-difluoro-2-methoxyphenyl)-4,5-dimethyl-5-(trifluoromethyl)tetrahydrofuran-2-carboxamido)-2-fluorophenyl)(imino)methyl)carbamate 30

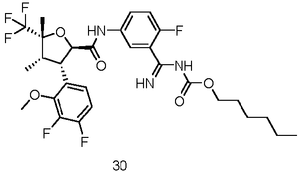

Title compound **30** (145 mg, yield: 76.6%) was obtained by using step 2 of the synthesis scheme of Example 28 and replacing the starting material compound **28a** with compound **27.**

MS m/z (ESI): 617.2 [M+1].

¹H NMR (500 MHz, CD₃OD): δ 7.85 (dd, 1H), 7.79 (dt, 1H), 7.20 (t, 1H), 7.15 (ddd, 1H), 6.99 (q, 1H), 5.06 (d, 1H), 4.31 (dd, 1H), 4.13 (t, 2H), 4.01 (d, 3H), 2.80 (p, 1H), 1.69 (d, 5H), 1.49-1.32 (m, 6H), 1.05-0.91 (m, 3H), 0.83 (dt, 3H).

### Example 31

### Isopropyl ((5-((2R,3S,4S,5R)-3-(3,4-difluoro-2-methoxyphenyl)-4,5-dimethyl-5-(trifluoromethyl)tetrahydrofuran-2-carboxamido)-2-fluorophenyl)(imino)methyl)carbamate 31

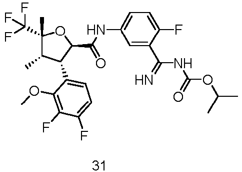

Title compound **31** was obtained by using the synthesis scheme of Example 29 and replacing the starting material compound **28a** with compound **27.**

MS m/z (ESI): 576.2 [M+1].

### Example 32

### (2R,3S,4S,5R)-3-(3,4-Difluoro-2-methoxyphenyl)-N-(2-((Z)-(N'-hydroxycarbamimidoyl)pyridin-4-yl)-4,5-dimethyl-5-(trifluoromethyl)tetrahydrofuran-2-carboxamide 32

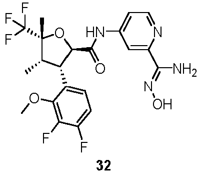

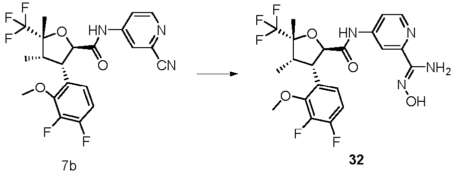

Compound **7b** (200 mg, 439.2 µmol) was dissolved in methanol (4 mL), and hydroxylamine hydrochloride (61 mg, 877.8 µmol) and N,N-diisopropylethylamine (60 mg, 464.2 µmol) were added. The mixture was stirred for 1.5 h. The reaction mixture was concentrated under reduced pressure, and the residue was dissolved in dichloromethane. The solution was washed with water and a saturated sodium chloride solution in sequence. The organic phase was concentrated under reduced pressure, and the residue was purified by silica gel column chromatography using eluent system A to give title compound **32** (160 mg, yield: 74.6%).

MS m/z (ESI): 489.0 [M+1].

¹H NMR (500 MHz, CDCl₃): δ 8.61 (s, 1H), 8.46 (d, 1H), 7.87 (dd, 1H), 7.83 (d, 1H), 7.09 (ddd, 1H), 6.92 (td, 1H), 5.75 (s, 2H), 5.02 (d, 1H), 4.10 (dd, 1H), 4.01 (d, 3H), 2.76 (p, 1H), 1.68 (s, 3H), 0.80 (dq, 3H).

### Example 33

### (2R,3S,4S,5R)-3-(3,4-Difluoro-2-hydroxyphenyl)-N-(2-((Z)-(N'-methoxycarbamimidoyl)pyridin-4-yl)-4,5-dimethyl-5-(trifluoromethyl)tetrahydrofuran-2-carboxamide 33

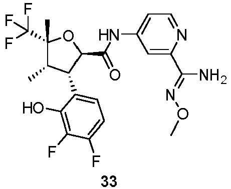

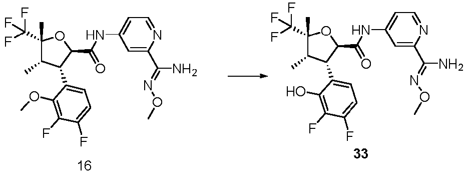

Compound **16** (1.5 g, 3 mmol) was dissolved in dichloromethane (15 mL), and boron tribromide (8.8 g, 6 mmol, 17% in dichloromethane) was added under an ice bath. The mixture was naturally warmed to room temperature and left to react for 16 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **33** (185 mg, yield: 12.7%).

MS m/z (ESI): 489.0 [M+1].

¹H NMR (500 MHz, DMSO-*d₆*): δ 10.70 (s, 1H), 10.47 (s, 1H), 8.44 (d, 1H), 8.11 (d, 1H), 7.72 (dd, 1H), 7.06-6.99 (m, 1H), 6.91-6.82 (m, 1H), 6.07 (s, 2H), 5.08 (d, 1H), 4.25 (dd, 1H), 3.79 (s, 3H), 2.84 (p, 1H), 1.60 (s, 3H), 0.75-0.68 (m, 3H).

### Example 34

### (2R,3S,4S,5R)-3-(2-Cyclobutoxy-3,4-difluorophenyl)-N-(2-((Z)-(N'-methoxycarbamoyl)pyridin-4-yl)-4,5-dimethyl-5-(trifluoromethyl)tetrahydrofuran-2-carboxamide 34

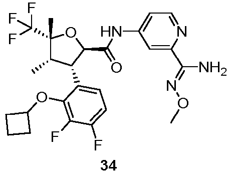

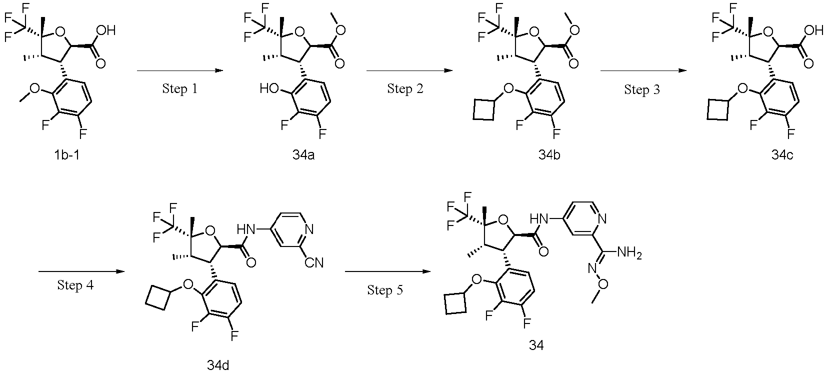

### Step 1

### Methyl (2R,3S,4S,5R)-3-(3,4-difluoro-2-hydroxyphenyl)-4,5-dimethyl-5-(trifluoromethyl)tetrahydrofuran-2-carboxylate 34a

Compound **1b-1** (1.0 g, 2.8 mmol) was dissolved in dichloromethane (20 mL), and a 1 M solution of boron tribromide in dichloromethane (8.5 mL) was added at 0 °C. The mixture was warmed to room temperature and stirred for 2 h. The reaction mixture was quenched with methanol (10 mL) and concentrated under reduced pressure to give crude title compound **34a** (1 g). The product was directly used in the next step without purification. MS m/z (ESI): 353.0 [M-1].

### Step 2

### Methyl (2R,3S,4S,5R)-3-(2-cyclobutoxy-3,4-difluorophenyl)-4,5-dimethyl-5-(trifluoromethyl)tetrahydrofuran-2-carboxylate 34b

Crude compound **34a** (600 mg, 1.7 mmol) was dissolved in *N,N*-dimethylacetamide (20 mL), and bromocyclobutane (2.28 g, 16.9 mmol, Shanghai Accela), cesium carbonate (2.76 g, 8.5 mmol), and potassium iodide (282 mg, 1.7 mmol) were added. The mixture was left to react at 80 °C for 7 h. The reaction mixture was cooled to room temperature and then filtered. The filtrate was concentrated under reduced pressure, and the residue was purified by silica gel column chromatography using eluent system C to give title compound **34b** (440 mg, yield: 63.6%).

### Step 3

### (2R,3S,4S,5R)-3-(2-Cyclobutoxy-3,4-difluorophenyl)-4,5-dimethyl-5-(trifluoromethyl)tetrahydrofuran-2-carboxylic acid 34c

Compound **34b** (440 mg, 1.1 mmol) was dissolved in tetrahydrofuran (5 mL), and a solution of lithium hydroxide monohydrate (175 mg, 4.2 mmol) in water (4.2 mL) was added under an ice bath. The mixture was warmed to room temperature and left to react for 2 h. The reaction mixture was concentrated under reduced pressure and diluted with water, the pH was adjusted to 3 with 1 M hydrochloric acid, and extraction was performed with ethyl acetate (10 mL × 3). The organic phases were combined, dried over anhydrous sodium sulfate, and filtered to remove the drying agent, and the filtrate was then concentrated under reduced pressure to give crude title compound 34c (420 mg). The product was directly used in the next step without purification.

MS m/z (ESI): 393.3 [M-1].

### Step 4

### (2R,3S,4S,5R)-N-(2-Cyanopyridin-4-yl)-3-(2-cyclobutoxy-3,4-difluorophenyl)-4,5-dimethyl-5-(trifluoromethyl)tetrahydrofuran-2-carboxamide 34d

Crude compound **34c** (420 mg, 1.1 mmol) was dissolved in dichloromethane (5 mL), and oxalyl chloride (406 mg, 3.2 mmol) and one drop of N,N-dimethylformamide were added under an ice bath. With the temperature maintained, the mixture was left to react for 1 h. The reaction mixture was concentrated under reduced pressure, and the residue was dissolved in dichloromethane (5 mL). *N,N*-Diisopropylethylamine (413 mg, 3.2 mmol) and compound **7a** (254 mg, 2.1 mmol) were added. The mixture was stirred for 16 h. The reaction mixture was concentrated under reduced pressure, and the residue was purified by silica gel column chromatography using eluent system B to give title compound **34d** (480 mg, yield: 91%).

MS m/z (ESI): 496.1 [M+1].

### Step 5

### (2R,3S,4S,5R)-3-(2-Cyclobutoxy-3,4-difluorophenyl)-N-(2-((Z)-(N'-methoxycarbamoyl)pyridin-4-yl)-4,5-dimethyl-5-(trifluoromethyl)tetrahydrofuran-2-carboxamide 34

Compound **34d** (100 mg, 221.8 µmol) was dissolved in 3 mL of isopropanol, and mercaptoacetic acid (38 mg, 412.5 µmol), methoxyamine hydrochloride (51 mg, 610.6 µmol), and *N,N*-diisopropylethylamine (79 mg, 611.2 µmol) were added. The mixture was left to react at 80 °C for 14 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound **34** (70 mg, yield: 63.9%).

MS m/z (ESI): 543.2 [M+1].

¹H NMR (500 MHz, CD₃OD): δ 8.42 (d, 1H), 8.09 (d, 1H), 7.71 (dd, 1H), 7.12-7.08 (m, 1H), 6.98-6.92 (m, 1H), 5.05 (d, 1H), 4.71-4.65 (m, 1H), 4.36 (dd, 1H), 3.86 (s, 3H), 2.82-2.76 (m, 1H), 2.44-2.36 (m, 1H), 2.35-2.28 (m, 1H), 2.27-2.20 (m, 1H), 2.19-2.12 (m, 1H), 1.80-1.71 (m, 1H), 1.67 (s, 3H), 1.59-1.49 (m, 1H), 0.81 (d, 3H).

### Example 35

### 4-((2R,3S,4S,5R)-3-(2-Cyclobutoxy-3,4-difluorophenyl)-4,5-dimethyl-5-(trifluoromethyl)tetrahydrofuran-2-carboxamido)-N-methoxypicolinamide 35

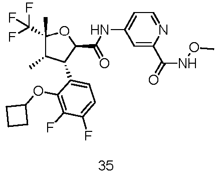

Title compound 35 (30 mg, yield: 41.6%) was obtained by using the synthesis scheme of Example 5 and replacing the starting material of step 1, compound **1b-1,** with compound **34c.**

MS m/z (ESI): 544.2 [M+1].

¹H NMR (500 MHz, CD₃OD): δ 8.46 (d, 1H), 8.23 (d, 1H), 7.88 (dd, 1H), 7.12-7.08 (m, 1H), 6.98-6.93 (m, 1H), 5.06 (d, 1H), 4.72-4.65 (m, 1H), 4.38 (dd, 1H), 3.81 (s, 3H), 2.83-2.77 (m, 1H), 2.43-2.37 (m, 1H), 2.34-2.29 (m, 1H), 2.28-2.21 (m, 1H), 2.20-2.12 (m, 1H), 1.80-1.71 (m, 1H), 1.67 (s, 3H), 1.59-1.50 (m, 1H), 0.81 (d, 3H).

### Example 36

### (2R,3S,4S,5R)-3-(2-Cyclobutoxy-3,4-difluorophenyl)-N-(2-((Z)-(N'-hydroxycarbamimidoyl)pyridin-4-yl)-4,5-dimethyl-5-(trifluoromethyl)tetrahydrofuran-2-carboxamide 36

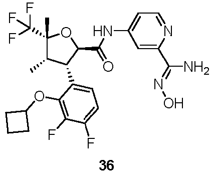

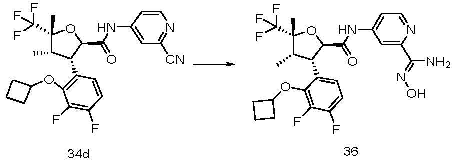

Compound **34d** (100 mg, 210.8 µmol) was dissolved in methanol (3 mL), and hydroxylamine hydrochloride (29 mg, 417.3 µmol) and N,N-diisopropylethylamine (27 mg, 208.9 µmol) were added. The mixture was stirred for 1.5 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-40%, flow rate: 30 mL/min) to give title compound 36 (80 mg, yield: 75%).

MS m/z (ESI): 527.3 [M-1].

¹H NMR (500 MHz, CD₃OD): δ 8.42 (d, 1H), 8.02 (d, 1H), 7.73 (dd, 1H), 7.12-7.08 (m, 1H), 6.97-6.92 (m, 1H), 5.04 (d, 1H), 4.71-4.65 (m, 1H), 4.36 (dd, 1H), 2.83-2.76 (m, 1H), 2.43-2.36 (m, 1H), 2.35-2.28 (m, 1H), 2.27-2.20 (m, 1H), 2.19-2.12 (m, 1H), 1.80-1.71 (m, 1H), 1.67 (s, 3H), 1.59-1.49 (m, 1H), 0.81 (d, 3H).

### Example 37

### (2R,3S,4S,5R)-3-(3,4-Difluoro-2-methoxyphenyl)-N-(2-((Z)-(N'-methoxy-d₃)carbamimidoyl)pyridin-4-yl)-4,5-dimethyl-5-(trifluoromethyl)tetrahydrofuran-2-carboxamide 37

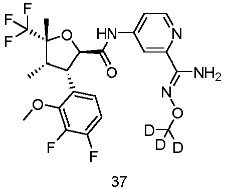

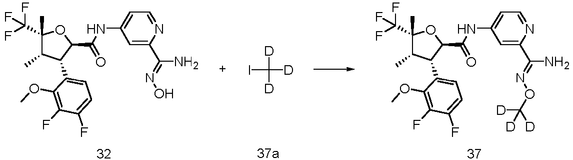

Compound 32 (102 mg, 208.8 µmol) was dissolved in N,N-dimethylformamide (1 mL), and a solution of lithium hydroxide monohydrate (8.8 mg, 208.8 µmol) in water (0.1 mL) was added. After 1 h of stirring, a solution of deuterated iodomethane 37a (36.3 mg, 250.6 µmol, Energy) in N,N-dimethylformamide (1 mL) was added. After 0.5 h of stirring, the reaction mixture was filtered, and the filtrate was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 35%-45%, flow rate: 30 mL/min) to give title compound 37 (15 mg, yield: 14.2%).

MS m/z (ESI): 506.3 [M+1].

¹H NMR (500 MHz, DMSO-*d₆*): δ 10.68 (s, 1H), 8.44 (d, 1H), 8.10 (d, 1H), 7.71 (dd, 1H), 7.24-7.08 (m, 2H), 6.06 (s, 2H), 5.08 (d, 1H), 4.24 (dd, 1H), 3.95 (d, 3H), 2.77 (t, 1H), 1.60 (s, 3H), 0.80-0.64 (m, 3H).

### Example 38

### (2R,3S,4S,5R)-N-(2-(1-Aminocyclopropyl)pyridin-4-yl)-3-(3,4-difluoro-2-methoxyphenyl)-4,5-dimethyl-5-(trifluoromethyl)tetrahydrofuran-2-carboxamide 38

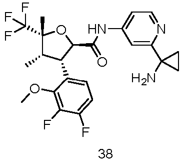

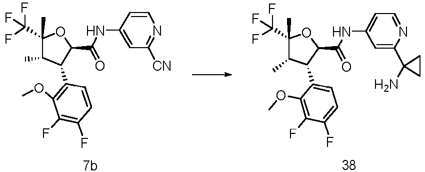

Compound **7b** (80 mg, 175.7 µmol) was dissolved in tetrahydrofuran (10 mL), and tetraisopropyl titanate (100 mg, 351.8 µmol, Shanghai Accela) was added. After 10 min of stirring, a 1 M solution of ethylmagnesium bromide in tetrahydrofuran (0.75 mL) was added dropwise. The mixture was stirred for 0.5 h, quenched with a saturated ammonium chloride solution, and extracted with ethyl acetate (5 mL × 3). The organic phases were combined, dried over anhydrous sodium sulfate, and filtered. The filtrate was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-42%, flow rate: 30 mL/min) to give title compound **38** (10 mg, yield: 11.7%).

MS m/z (ESI): 484.4 [M-1].

¹H NMR (500 MHz, CDCl₃): δ 8.52-8.36 (m, 2H), 7.65 (s, 1H), 7.25-7.24 (m, 1H), 7.10-7.06 (m, 1H), 6.93-6.88 (m, 1H), 5.01-4.99 (m, 1H), 4.14-4.06 (m, 1H), 4.01 (s, 3H), 2.76-2.71 (m, 1H), 2.00 (br, 2H), 1.68 (s, 3H), 1.35-1.24 (m, 2H), 1.15-1.10 (m, 2H), 0.85-0.72 (m, 3H).

### Example 39

### (2R,3S,4S,5R)-3-(3,4-Difluoro-2-methoxyphenyl)-N-(2-(2-hydroxy-2-(1-hydroxycyclopropyl)ethoxy)pyridin-4-yl)-4,5-dimethyl-5-(trifluoromethyl)tetrahydrofuran-2-carboxamide 39 (a mixture of diastereomers)

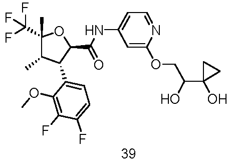

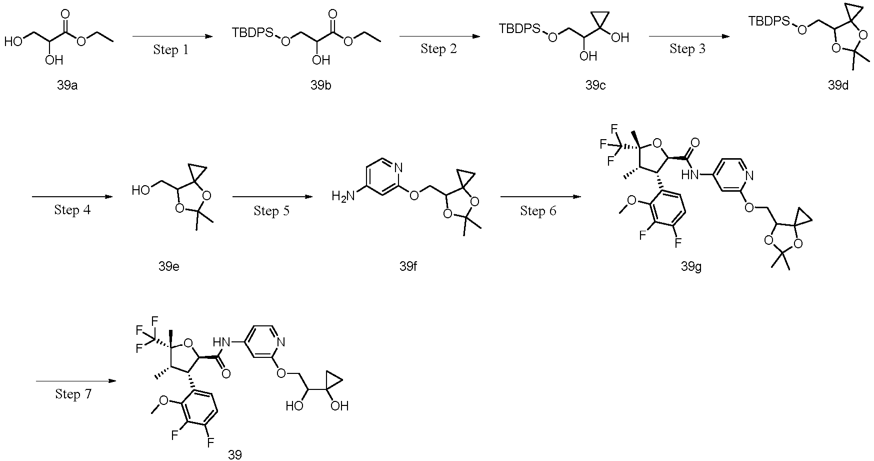

### Step 1

### Ethyl (±)-3-((tert-butyldiphenylsilyl)oxy)-2-hydroxypropionate 39b

Ethyl (±)-2,3-dihydroxypropionate **39a** (1 g, 7.5 mmol, prepared by the method disclosed in "Synthesis, 2016, vol. 48, # 21, art. no. SS-2016-H0270-PSP, p. 3696-3700") was dissolved in dichloromethane (20 mL), and imidazole (761.3 mg, 11.2 mmol) and tert-butyl(chloro)diphenylsilane (2.5 g, 9 mmol) were added. The mixture was stirred for 2 h. The reaction mixture was cooled to room temperature and then concentrated under reduced pressure, and the residue was purified by silica gel column chromatography using eluent system B to give title compound **39b** (2.2 g, yield: 79.2%).

### Step 2

### (±)-1-(2-((tert-Butyldiphenylsilyl)oxy)-1-hydroxyethyl)cyclopropan-1-ol 39c

Compound **39b** (2.2 g, 5.9 mmol) was dissolved in tetrahydrofuran (50 mL), tetraisopropyl titanate (1.68 g, 5.9 mmol) was added, and a 2 M solution of ethylmagnesium bromide in tetrahydrofuran (8.9 mL) was added dropwise under an ice bath. The mixture was naturally warmed to room temperature and stirred for 3 h. The reaction mixture was concentrated under reduced pressure, and the residue was dissolved in dichloromethane (50 mL). The solution was poured into a saturated aqueous ammonium chloride solution (50 mL), and the mixture was stirred for 30 min and then filtered. The filter cake was washed with dichloromethane, and the filtrate was collected. The organic phase was separated, washed with a saturated sodium chloride solution, dried over anhydrous sodium sulfate, and filtered. The filtrate was concentrated under reduced pressure, and the residue was purified by silica gel column chromatography using eluent system B to give title compound **39c** (550 mg, yield: 26.1%).

MS m/z (ESI): 355.2 [M-1].

### Step 3

### (±)-tert-Butyl((5,5-dimethyl-4,6-dioxaspiro[2.4]hept-7-yl)methoxy)diphenylsilane 39d

Compound **39c** (550 mg, 1.5 mmol), 2,2-dimethoxypropane (482 mg, 4.6 mmol, Shanghai Taitan), and p-toluenesulfonic acid (26.6 mg, 154.2 µmol) were dissolved in acetone (10 mL). The mixture was stirred for 16 h. The reaction mixture was concentrated under reduced pressure, and the residue was purified by silica gel column chromatography using eluent system B to give title compound **39d** (500 mg, yield: 81.7%).

### Step 4

### (±)-(5,5-Dimethyl-4,6-dioxaspiro[2.4]hept-7-yl)methanol 39e

Compound **39d** (500 mg, 1.3 mmol) was dissolved in tetrahydrofuran (10 mL), and tetrabutylammonium fluoride (284 mg, 1.3 mmol) was added. The mixture was stirred for 2 h. The reaction mixture was concentrated under reduced pressure to give crude title compound **39e** (200 mg). The product was directly used in the next step without purification.

### Step 5

### (±)-2-((5,5-Dimethyl-4,6-dioxaspiro[2.4]hept-7-yl)methoxy)pyridin-4-amine 39f

Crude compound **39e** (115 mg, 727 µmol) was dissolved in *N*-methylpyrrolidone (5 mL), and sodium hydride (40 mg, 1 mmol, purity 60%) was added under an ice bath. The mixture was warmed to room temperature and stirred for 1 h, and a solution of 2-chloro-4-aminopyridine (35 mg, 272 µmol, Shanghai Accela) in N-methylpyrrolidone (5 mL) was then added dropwise. The mixture was heated to 160 °C and left to react for 3 h. The reaction mixture was cooled to room temperature, diluted with water, and extracted with ethyl acetate (15 mL × 3). The organic phases were combined, dried over anhydrous sodium sulfate, and filtered. The filtrate was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-42%, flow rate: 30 mL/min) to give title compound **39f** (8 mg, yield: 11.7%).

MS m/z (ESI): 251.3 [M+1].

### Step 6

### (2R,3S,4S,5R)-3-(3,4-Difluoro-2-methoxyphenyl)-N-(2-((5,5-dimethyl-4,6-dioxaspiro[2.4]hept-7-yl)methoxy)pyridin-4-yl)-4,5-dimethyl-5-(trifluoromethyl)tetrahydrofuran-2-carboxamide 39g (a mixture of diastereomers)

Compound **1b-1** (20 mg, 56.5 µmol) was dissolved in dichloromethane (1 mL), and oxalyl chloride (14.3 mg, 113 µmol) and one drop of N,N-dimethylformamide were added under an ice bath. The mixture was warmed to room temperature and left to react for 1 h. The reaction mixture was concentrated under reduced pressure, and the residue was dissolved in dichloromethane (1 mL). **39f** (14 mg, 56 µmol), 4-dimethylaminopyridine (1.4 mg, 11 µmol), and N,N-diisopropylethylamine (22 mg, 169.4 µmol) were added under an ice bath. The mixture was stirred for 2 h. The reaction mixture was concentrated under reduced pressure to give crude title compound **39g** (30 mg). The product was directly used in the next step without purification.

MS m/z (ESI): 587.5 [M+1].

### Step 7

### (2R,3S,4S,5R)-3-(3,4-Difluoro-2-methoxyphenyl)-N-(2-(2-hydroxy-2-(1-hydroxycyclopropyl)ethoxy)pyridin-4-yl)-4,5-dimethyl-5-(trifluoromethyl)tetrahydrofuran-2-carboxamide 39 (a mixture of diastereomers)

Crude compound **39g** (30 mg, 51.1 µmol) was dissolved in dichloromethane (1 mL), and a 4 M solution of hydrogen chloride in 1,4-dioxane (0.5 mL) was added under an ice bath. The mixture was stirred for 0.5 h. The reaction mixture was made neutral with a saturated sodium carbonate solution and concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-40%, flow rate: 30 mL/min) to give title compound **39** (a mixture of diastereomers, 7 mg, yield: 25%).

MS m/z (ESI): 545.4 [M-1].

¹H NMR (500 MHz, CD₃OD): δ 7.99 (d, 1H), 7.25 (d, 1H), 7.16 (dd, 1H), 7.12 (ddd, 1H), 7.00 (td, 1H), 5.06 (d, 1H), 4.56 (dd, 1H), 4.34 (ddd, 2H), 4.01 (d, 3H), 3.61 (dd, 1H), 2.86-2.74 (m, 1H), 1.67 (s, 3H), 0.87-0.78 (m, 3H), 0.68 (dt, 4H).

### Example 40

### (2R,3S,4S,5R)-N-(2-(1-Carboxamidocyclopropyl)pyridin-4-yl)-3-(3,4-difluoro-2-methoxyphenyl)-4,5-dimethyl-5-(trifluoromethyl)tetrahydrofuran-2-carboxamide 40

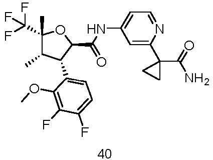

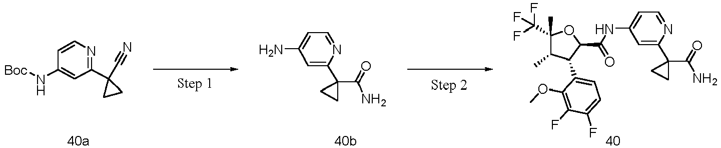

### Step 1

### 1-(4-Aminopyridin-2-yl)cyclopropane-1-carboxamide 40b

tert-Butyl (2-(1-cyanocyclopropyl)pyridin-4-yl)carbamate **40a** (50 mg, 192.8 µmol, prepared by the method disclosed in Example 98 on page 107 of the specification in the patent application "CN111196804A") was dissolved in concentrated sulfuric acid (0.25 mL). The mixture was stirred for 16 h. The reaction mixture was made neutral with a 4 N sodium hydroxide solution, diluted with N,N-dimethylformamide (0.5 mL), and filtered, and the filtrate was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-40%, flow rate: 30 mL/min) to give title compound **40b** (20 mg, yield: 58.5%).

MS m/z (ESI): 178.0 [M+1].

### Step 2

### (2R,3S,4S,5R)-N-(2-(1-Carboxamidocyclopropyl)pyridin-4-yl)-3-(3,4-difluoro-2-methoxyphenyl)-4,5-dimethyl-5-(trifluoromethyl)tetrahydrofuran-2-carboxamide 40

Compound **1b-1** (50 mg, 141.4 µmol) was dissolved in dichloromethane (1 mL), and oxalyl chloride (53.7 mg, 423.4 µmol) and one drop of N,Ndimethylformamide were added under an ice bath. The mixture was warmed to room temperature and left to react for 1 h. The reaction mixture was concentrated under reduced pressure, and the residue was dissolved in dichloromethane (3 mL). N,N-Diisopropylethylamine (91.2 mg, 705.6 µmol), 4-dimethylaminopyridine (1.7 mg, 14 µmol), and compound **40b** (30 mg, 169.3 µmol) were added under an ice bath. The mixture was stirred for 1 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **40** (4 mg, yield: 5.5%).

MS m/z (ESI): 512.4 [M-1].

¹H NMR (500 MHz, CD₃OD): δ 8.39 (d, 1H), 7.74 (d, 1H), 7.65 (dd, 1H), 7.13 (dd, 1H), 7.00 (q, 1H), 5.08 (d, 1H), 4.33 (dd, 1H), 4.02 (d, 3H), 2.81 (p, 1H), 1.67 (s, 3H), 1.56 (q, 2H), 1.24 (q, 2H), 0.86-0.80 (m, 3H).

### Example 41

### (2R,3S,4S,5R)-N-(2-(Amino(methyl)phosphoryl)pyridin-4-yl)-3-(3,4-difluoro-2-methoxyphenyl)-4,5-dimethyl-5-(trifluoromethyl)tetrahydrofuran-2-carboxamide 41 (a mixture of diastereomers)

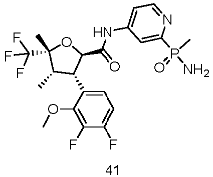

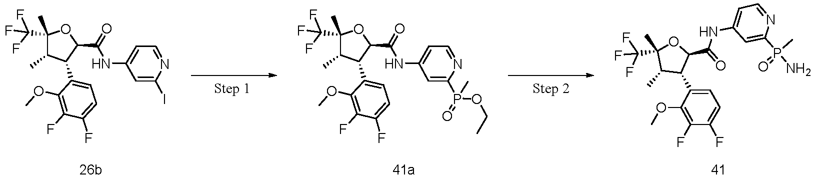

### Step 1

### Ethyl (4-((2R,3S,4S,5R)-3-(3,4-difluoro-2-methoxyphenyl)-4,5-dimethyl-5-(trifluoromethyl)tetrahydrofuran-2-carboxamido)pyridin-2-yl)(methyl)phosphinate 41a (a mixture of diastereomers)

Diethyl methylphosphonite (31.8 mg, 233.7 µmol, Shanghai Macklin) was weighed out, and water (4.2 mg, 233.7 µmol) was added at 0 °C. The mixture was stirred for 16 h, and compound 26b (100 mg, 179,8 µmol), N,N-diisopropylethylamine (69.7 mg, 539.3 µmol), [1,1'-bis(diphenylphosphino)ferrocene]palladium(II) dichloride (13.2 mg, 8 µmol), and toluene (1 mL) were then added. The system was purged with nitrogen and left to react at 105 °C for 4 h. The reaction mixture was concentrated under reduced pressure, and the residue was purified by silica gel column chromatography using eluent system A to give title compound **41a** (80 mg, yield: 83%).

MS m/z (ESI): 537.4 [M+1].

### Step 2

### (2R,3S,4S,5R)-N-(2-(Amino(methyl)phosphoryl)pyridin-4-yl)-3-(3,4-difluoro-2-methoxyphenyl)-4,5-dimethyl-5-(trifluoromethyl)tetrahydrofuran-2-carboxamide 41 (a mixture of diastereomers)

Compound **41a** (60 mg, 111.9 µmol) was dissolved in tetrahydrofuran (1 mL), and a 1 M solution of lithium bis(trimethylsilyl)amide in tetrahydrofuran (1 mL) was added under an ice bath. With the temperature maintained, the mixture was left to react for 0.5 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **41** (a mixture of diastereomers, 2 mg, yield: 3.5%).

MS m/z (ESI): 508.4 [M+1].

¹H NMR (500 MHz, CD₃OD): δ 8.61 (d, 1H), 8.22 (dd, 1H), 7.87 (dt, 1H), 7.14 (t, 1H), 7.06-6.96 (m, 1H), 5.10 (d, 1H), 4.34 (dd, 1H), 4.01 (d, 3H), 2.88-2.75 (m, 1H), 1.72 (d, 3H), 1.68 (s, 3H), 0.84 (dt, 3H).

### Example 42

### (2R,3S,4S,5R))-N-(2-(1-Amino-2-(cyclopropylmethoxy)ethyl)pyridin-4-yl)-3-(3,4-difluoro-2-methoxyphenyl)-4,5-dimethyl-5-(trifluoromethyl)tetrahydrofuran-2-carboxamide 42 (a mixture of diastereomers)

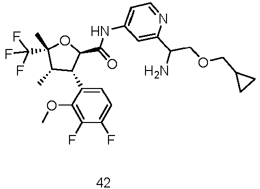

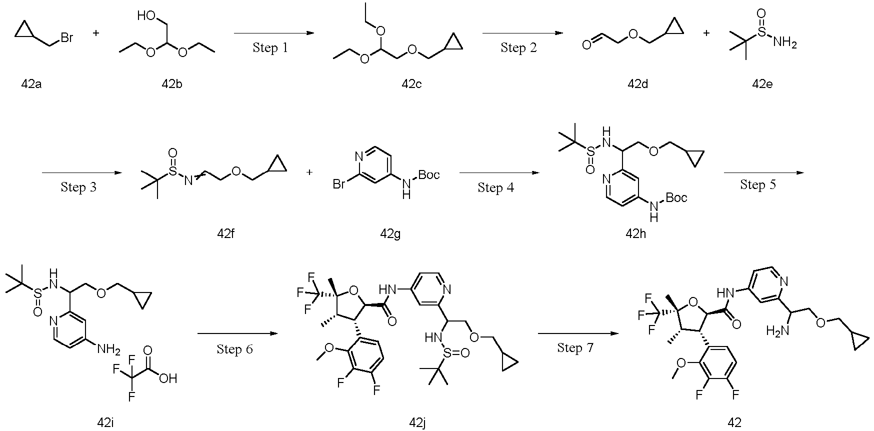

### Step 1

### ((2,2-Diethoxyethoxy)methyl)cyclopropane 42c

Sodium hydride (2.38 g, 59.5 mmol, purity 60%) was mixed with tetrahydrofuran (40 mL), and a solution of 2,2-diethoxyethanol **42b** (5 g, 37.2 mmol, Shanghai Titan) in tetrahydrofuran (40 mL) was added dropwise under an ice bath. After the dropwise addition, the mixture was warmed to room temperature and left to react for 1 h. A solution of bromomethylcyclopropane **42a** (7.54 g, 55.9 mmol, Nanjing PharmaBlock) in tetrahydrofuran (40 mL) and sodium iodide (56 mg, 372.6 µmol) were added. The mixture was left to react at 80 °C for 4 days. The reaction mixture was cooled to room temperature and then filtered. The filtrate was concentrated under reduced pressure and then dissolved in dichloromethane (100 mL). The solution was washed with a saturated sodium chloride solution, dried over anhydrous sodium sulfate, and filtered. The filtrate was concentrated under reduced pressure to give crude title compound **42c** (5.1 g). The product was directly used in the next step without purification.

### Step 2

### 2-(Cyclopropylmethoxy)acetaldehyde 42d

Crude compound **42c** (1 g, 5.3 mmol) was dissolved in tetrahydrofuran (16 mL), and a 2 M sulfuric acid solution (8 mL) was added. The mixture was stirred for 1 h, then heated to 60 °C, and left to react for 1 h. The reaction mixture was cooled to room temperature, then made neutral with sodium bicarbonate, and extracted with ethyl acetate (20 mL × 3). The organic phases were combined, dried over anhydrous sodium sulfate, and filtered. The filtrate was concentrated under reduced pressure, and the residue was purified by silica gel column chromatography using eluent system B to give title compound **42d** (570 mg, yield: 94%).

### Step 3

### N-(2-(Cyclopropylmethoxy)ethylidene)-2-methylpropane-2-sulfinamide 42f

Compound **42d** (0.57 g, 5 mmol) and 2-methylpropane-2-sulfinamide **42e** (605.2 mg, 5 mmol, Shanghai Bide) were dissolved in dichloromethane (10 mL), and anhydrous cupric sulfate (2 g, 12,5 mmol) was added. The system was purged with nitrogen and stirred for 16 h. The reaction mixture was filtered. The filtrate was concentrated under reduced pressure, and the residue was purified by silica gel column chromatography using eluent system B to give title compound **42f** (750 mg, yield: 69%).

MS m/z (ESI): 218.4 [M+1].

### Step 4

### tert-Butyl (±)-(2-(1-((tert-butylsulfinyl)amino)-2-(cyclopropylmethoxy)ethyl)pyridin-4-yl)carbamate 42h

tert-Butyl (2-bromopyridin-4-yl)carbamate **42g** (439.9 mg, 1.6 mmol, Shanghai Leyan) was dissolved in tetrahydrofuran (10 mL), and a 2.5 M solution of n-butyllithium in n-hexane (1.4 mL) was slowly added dropwise at -78 °C. The mixture was left to react at that temperature for 1 h, and a solution of compound **42f** (0.35 g, 1.6 mmol) in tetrahydrofuran (2 mL) was then added. The mixture was left to react at -78 °C for another 3 h. A saturated ammonium chloride solution was added to the reaction mixture, and extraction was performed with ethyl acetate (15 mL × 3). The organic phases were combined, dried over anhydrous sodium sulfate, and filtered. The filtrate was concentrated under reduced pressure, and the residue was purified by silica gel column chromatography using eluent system B to give title compound **42h** (450 mg, yield: 67.9%).

MS m/z (ESI): 412.4 [M+1].

### Step 5

### (±)-N-(1-(4-Aminopyridin-2-yl)-2-(cyclopropylmethoxy)ethyl)-2-methylpropane-2-sulfinamide 2,2,2-trifluoroacetate 42i

Compound **42h** (75 mg, 182.2 µmol) was dissolved in dichloromethane (1.3 mL), and trifluoroacetic acid (0.5 mL) was added under an ice bath. The mixture was warmed to room temperature and left to react for 1.5 h. The reaction mixture was concentrated under reduced pressure to give crude title compound **42i** (77 mg). The product was directly used in the next step without purification.

MS m/z (ESI): 312.4 [M+1].

### Step 6

### (2R,3S,4S,5R)-N-(2-(1-((tert-Butylsulfinyl)amino)-2-(cyclopropylmethoxy)ethyl)pyridin-4-yl)-3-(3,4-difluoro-2-methoxyphenyl)-4,5-dimethyl-5-(trifluoromethyl)tetrahydrofuran-2-carboxamide 42j (a mixture of diastereomers)

Compound **1b-1** (57.9 mg, 163.4 µmol) was dissolved in dichloromethane (2 mL), and oxalyl chloride (51.9 mg, 408.6 µmol) and one drop of *N,N-*dimethylformamide were added under an ice bath. The mixture was warmed to room temperature and left to react for 1 h. The reaction mixture was concentrated under reduced pressure, and the residue was dissolved in dichloromethane (2 mL). The solution was set aside for later use. Crude compound **42i** (77 mg, 181 µmol) was dissolved in dichloromethane (4 mL), and N,N-diisopropylethylamine (211.2 mg, 1.6 mmol) and 4-dimethylaminopyridine (4 mg, 32.6 µmol) were added under an ice bath. The solution set aside earlier was added dropwise, and the mixture was warmed to room temperature and stirred for 1 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **42j** (10 mg, yield: 9.4%).

MS m/z (ESI): 648.4 [M+1].

### Step 7

### (2R,35,4S,5R)-N-(2-(1-Amino-2-(cyclopropylmethoxy)ethyl)pyridin-4-yl)-3-(3,4-difluoro-2-methoxyphenyl)-4,5-dimethyl-5-(trifluoromethyl)tetrahydrofuran-2-carboxamide 42 (a mixture of diastereomers)

Compound **42j** (10 mg, 15.4 µmol) was dissolved in dichloromethane (1.5 mL), and a 4 M solution of hydrogen chloride in 1,4-dioxane (0.2 mL) was added. The mixture was stirred for 1 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give title compound **42** (a mixture of diastereomers, 2 mg, yield: 23.8%).

MS m/z (ESI): 544.2 [M+1].

¹H NMR (500 MHz, CD₃OD): δ 8.40 (d, 1H), 7.74 (d, 1H), 7.61 (dd, 1H), 7.14 (t, 1H), 7.00 (q, 1H), 5.09 (d, 1H), 4.33 (dd, 1H), 4.14 (dd, 1H), 4.01 (d, 3H), 3.73 (dd, 1H), 3.60 (dd, 1H), 3.31 (d, 2H), 2.21 (t, 1H), 1.68 (s, 3H), 1.01 (d, 1H), 0.88-0.80 (m, 3H), 0.52-0.43 (m, 2H), 0.17 (q, 2H).

### Example 43

### 4-((2R,3S,4S,5R)-3-(3,4-Difluoro-2-methoxyphenyl)-4,5-dimethyl-5-(trifluoromethyl)tetrahydrofuran-2-carboxamido)-2-((Z)-N'-hydroxycarbamimidoyl)pyridine 1-oxide 43

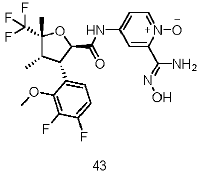

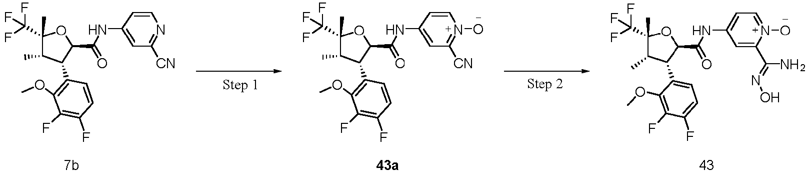

### Step 1

### 2-Cyano-4-((2R,3S,4S,5R)-)-3-(3,4-difluoro-2-methoxyphenyl)-4,5-dimethyl-5-(trifluoromethyl)tetrahydrofuran-2-carboxamido)pyridine 1-oxide 43a

Compound **7b** (300 mg, 658.8 µmol) was dissolved in chloroform (6 mL), and 3-chloroperoxybenzoic acid (268 mg, 1.3 mmol, purity 85%) was added. The mixture was stirred at 65 °C for 16 h. The reaction mixture was cooled to room temperature and purified by silica gel column chromatography using eluent system A to give title compound **43a** (105 mg, yield: 33.8%).

MS m/z (ESI): 472.2 [M+1].

### Step 2

### 4-((2R,3S,4S,5R)-3-(3,4-Difluoro-2-methoxyphenyl)-4,5-dimethyl-5-(trifluoromethyl)tetrahydrofuran-2-carboxamido)-2-((Z)-N-hydroxycarbamimidoyl)pyridine 1-oxide 43

Compound **43a** (100 mg, 212.1 µmol) was dissolved in methanol (2 mL), and hydroxylamine hydrochloride (30 mg, 431.7 µmol) and *N,N*-diisopropylethylamine (30 mg, 232.1 µmol) were sequentially added. The mixture was stirred at 45 °C for 1.5 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-40%, flow rate: 30 mL/min) to give title compound **43** (45 mg, yield: 42%).

MS m/z (ESI): 505.1 [M+1].

¹H NMR (500 MHz, DMSO-*d₆*): δ 10.71 (s, 1H), 10.16 (s, 1H), 8.21 (d, 1H), 8.19 (d, 1H), 7.69 (dd, 1H), 7.22-7.10 (m, 2H), 6.78 (s, 2H), 5.07 (d, 1H), 4.24 (dd, 1H), 3.95 (d, 3H), 2.76 (p, 1H), 1.60 (s, 3H), 0.73 (dd, 3H).

### Example 44

### 4-((2R,3S,4S,5R)-3-(3,4-Difluoro-2-methoxyphenyl)-4,5-dimethyl-5-(trifluoromethyl)tetrahydrofuran-2-carboxamido)-2-((Z)-N-methoxycarbamimidoyl)pyridine 1-oxide 44

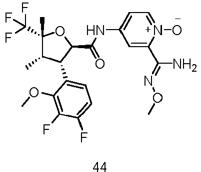

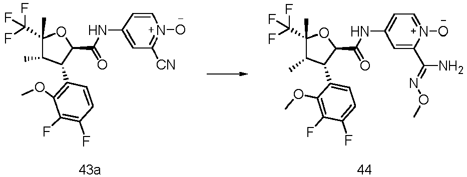

Compound **43a** (412 mg, 874 µmol) was dissolved in isopropanol (8 mL), and methoxyamine hydrochloride (219 mg, 2.6 mmol), mercaptoacetic acid (161 mg, 1.7 mmol), and N,N-diisopropylethylamine (339 mg, 2.6 mmol) were added. The mixture was stirred at 85 °C for 2 h. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-40%, flow rate: 30 mL/min) to give title compound **44** (207 mg, yield: 45.6%).

MS m/z (ESI): 519.2 [M+1].

¹H NMR (500 MHz, DMSO-*d₆*): δ 10.79 (s, 1H), 8.21 (d, 1H), 8.12 (d, 1H), 7.77 (dd, 1H), 7.22-7.11 (m, 2H), 7.10-7.00 (m, 2H), 5.08 (d, 1H), 4.24 (dd, 1H), 3.96 (t, 3H), 3.78 (d, 3H), 2.77 (p, 1H), 1.60 (s, 3H), 0.72 (d, 3H).

### Example 45

### (2R,3S,4S,5R)-N-(2-((Z)-(N'-Cyclopropoxycarbamimidoyl)pyridin-4-yl)-3-(3,4-difluoro-2-methoxyphenyl)-4,5-dimethyl-5-(trifluoromethyl)tetrahydrofuran-2-carboxamide 45

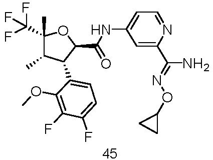

Title compound **45** (3 mg, yield: 5%) was obtained by using the synthesis scheme of Example 16 and replacing the starting material compound methoxyamine hydrochloride with O-cyclopropylhydroxylamine hydrochloride (prepared by the method disclosed in "Organic and biomolecular chemistry, 2020, vol. 18, # 17, p. 3281-3287").

MS m/z (ESI): 529.1 [M+1].

¹H NMR (500 MHz, CDCl₃): δ 8.55 (s, 1H), 8.44 (d, 1H), 8.02 (dd, 1H), 7.73 (d, 1H), 7.10 (t, 1H), 6.93 (q, 1H), 5.57 (s, 2H), 5.02 (d, 1H), 4.15-3.96 (m, 5H), 2.77 (p, 1H), 1.72 (s, 3H), 0.90 (t, 1H), 0.84-0.79 (m, 4H), 0.71 (q, 2H).

### Example 46

### 2-Carbamimidoyl-4-((2R,3S,4S,5R)-3-(3,4-difluoro-2-methoxyphenyl)-4,5-dimethyl-5-(trifluoromethyl)tetrahydrofuran-2-carboxamido)pyridine 1-oxide 2,2,2-trifluoroacetate 46

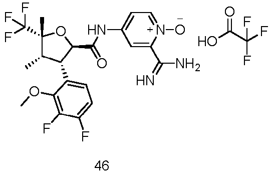

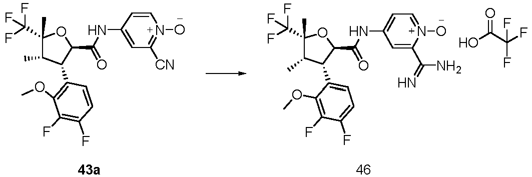

Compound **43a** (100 mg, 212.1 µmol) was dissolved in tetrahydrofuran (1 mL), and 1 M lithium bis(trimethylsilyl)amide (636.4 µL) was added. The mixture was stirred for 30 min. The reaction mixture was concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (1%_{∘} trifluoroacetic acid) and acetonitrile, gradient ratio: acetonitrile 30%-42%, flow rate: 30 mL/min) to give title compound **46** (2.8 mg, yield: 1.4%).

MS m/z (ESI): 489.4 [M+1].

¹H NMR (500 MHz, CD₃OD): δ 8.41 (dd, 2H), 7.92 (dd, 1H), 7.14 (t, 1H), 7.01 (t, 1H), 5.36 (s, 1H), 4.33 (dd, 1H), 4.02 (d, 3H), 2.82 (t, 1H), 1.68 (s, 3H), 0.84 (d, 3H).

### Example 47

### (2R,3S,4S,5R)-3-(3,4-Difluoro-2-methoxyphenyl)-N-(2-(1-hydroxycyclopropyl)pyridin-4-yl)-4,5-dimethyl-5-(trifluoromethyl)tetrahydrofuran-2-carboxamide 47

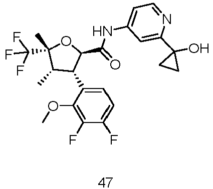

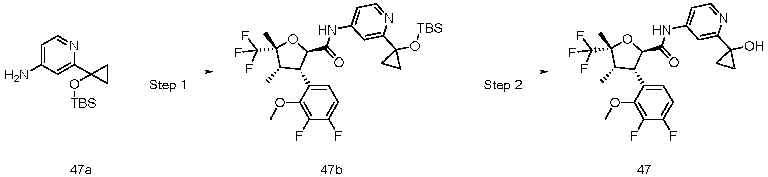

### Step 1

### (2R,3S,4S,5R)-N-(2-(1-((tert-Butyldimethylsilyl)oxy)cyclopropyl)pyridin-4-yl)-3-(3,4-difluoro-2-methoxyphenyl)-4,5-dimethyl-5-(trifluoromethyl)tetrahydrofuran-2-carboxamide 47b

Compound **1b-1 (80** mg, 225.8 µmol) was dissolved in dichloromethane (3 mL), and oxalyl chloride (60 mg, 472.7 µmol) and one drop of *N,N-*dimethylformamide were added under an ice bath. The mixture was warmed to room temperature and left to react for 1 h. The reaction mixture was concentrated under reduced pressure, and the residue was dissolved in dichloromethane (3 mL). *N,N*-Diisopropylethylamine (60 mg, 464.2 µmol), 4-dimethylaminopyridine (12 mg, 97.4 µmol), and 2-(1-((tertbutyldimethylsilyl)oxy)cyclopropyl)pyridin-4-amine **47a** (65 mg, 245.8 µmol, prepared by the method disclosed in Example 169 on page 196 of the specification in the patent application "WO2022053022") were added. The mixture was stirred for 1 h. The reaction mixture was concentrated under reduced pressure, and the residue was purified by silica gel column chromatography using eluent system B to give title compound **47b** (25 mg, yield: 18.4%).

MS m/z (ESI): 601.3 [M+1].

### Step 2

### (2R,3S,4S,5R)-3-(3,4-Difluoro-2-methoxyphenyl)-N-(2-(1-hydroxycyclopropyl)pyridin-4-yl)-4,5-dimethyl-5-(trifluoromethyl)tetrahydrofuran-2-carboxamide 47

Compound **47b** (30 mg, 49.9 µmol) was dissolved in tetrahydrofuran (2 mL), and a 1 M solution of tetrabutylammonium fluoride in tetrahydrofuran (2 mL) was added. The mixture was stirred for 30 min. The reaction mixture was diluted with ethyl acetate and washed with water. The organic phase was then separated and concentrated under reduced pressure, and the residue was purified by preparative high performance liquid chromatography (Waters-2545, chromatography column: YMC Triart-Exrs C18, 30 × 150 mm, 5 µm; mobile phase: aqueous phase (10 mmol/L ammonium bicarbonate) and acetonitrile, gradient ratio: acetonitrile 30%-40%, flow rate: 30 mL/min) to give title compound **47** (5 mg, yield: 20.5%).

MS m/z (ESI): 487.1 [M+1].

¹H NMR (500 MHz, CD₃OD): δ 8.29-8.28 (m, 1H), 7.76-7.75 (m, 1H), 7.48-7.46 (m, 1H), 7.14-7.12 (m, 1H), 7.11-6.97 (m, 1H), 5.08-5.06 (m, 1H), 4.33-4.29 (m, 1H), 4.00 (s, 3H), 2.81-2.78 (m, 1H), 1.66 (s, 3H), 1.23-1.22 (m, 2H), 1.10-1.08 (m, 2H), 0.83-0.80 (m, 3H).

### Biological Evaluations

### Test Example 1: Determination of Inhibitory Activity of Compounds of Present Disclosure Against Nav1.8

The objective of the experiment was to investigate the effects of the compounds on the Nav1.8 ion channel in *ex vivo* experiments. The Nav1.8 ion channel is stably expressed on HEK293 cells. After the Nav1.8 current stabilized, the effects of the compounds on the Nav1.8 ion channel were obtained by comparing the magnitudes of the Nav1.8 currents before and after the compounds were applied.

### 1. Materials and instruments

1) A patch clamp amplifier: patch clamp PC-505B (WARNER instruments)/MultiClamp 700A (Axon instrument)
2) A digital-to-analog converter: Digidata 1440A (Axon CNS)/Digidata 1550A (Axon instruments)
3) A micromanipulator: MP-225 (SUTTER instrument)
4) An inverted microscope: TL4 (Olympus)
5) Glass micropipette puller: PC-10 (NARISHIGE)
6) Microelectrode glass capillaries: B12024F (Wuhan Vital Sense Scientific Instruments Co., Ltd.)
7) Dimethyl sulfoxide (DMSO) D2650 (Sigma-Aldrich)
8) TTX AF3014 (Affix Scientific)

### 2. Procedures

### 2.1. Compound solution preparation

The compounds for preparing the intracellular and extracellular fluids were purchased from Sigma (St. Louis, MO) except for the NaOH and KOH for the acid-base titrations. The extracellular fluid (mM) was: NaCl, 137; KCl, 4; CaCl₂, 1.8; MgCl₂, 1; HEPES, 10; glucose, 10; pH 7.4 (NaOH titration). The intracellular fluid (mM) was: aspartic acid, 140; MgCl₂, 2; EGTA, 11; HEPES, 10; pH 7.2 (CsOH titration). All test and control compound solutions contained 1 µM TTX.

The test compounds were stored at a concentration of 9 mM in dimethyl sulfoxide (DMSO). On the testing day, the compounds were further dissolved in the extracellular fluid to prepare the required concentrations.

### 2.2. Manual patch clamp testing process

1) After the compounds were prepared into solutions with specified concentrations, the compound solutions were sequentially added to channels in ascending order according to their concentrations, and each channel was labeled.
2) A cell was transferred to the perfusion chamber. Positive pressure was applied inside the electrode, and the electrode tip was brought into contact with the cell. The three-way valve of the suction device was switched to the state where all three ways were open, and negative pressure was then applied to the electrode, allowing a high-resistance seal to form between the electrode and the cell. The application of negative pressure was continued to rupture the cell membrane, leading to the formation of a current pathway.
3) After the cell membrane rupture current stabilized, perfusion was performed with different concentrations in sequence. If the current remained stable for at least one minute, perfusion was performed with the next concentration. The perfusion time for each concentration did not exceed five minutes.
4) The perfusion chamber was cleaned. The perfusion chamber was rinsed with the compound solutions in descending order according to their concentrations, with each concentration applied for 20 s. Finally, the perfusion chamber was rinsed with the extracellular fluid for 1 min.

### 2.3. Test voltage equation (resting) and results

The cell was clamped at -80 mV and then depolarized to 10 mV using a square wave lasting for 10 ms to obtain a Nav1.8 current. This procedure was repeated once every 5 seconds. The maximum current induced by the square wave was detected. After the current stabilized, perfusion was performed with the test compounds. After the reaction stabilized, the strength of the blocking was calculated.

### 3. Data analysis

The data were stored on a computer system and analyzed. Data acquisition and analysis were performed using pCLAMP 10 (Molecular Devices, Union City, CA), and the results were reviewed by a manager. "Current stabilized" means that the changes in current over time were within a limited range. The magnitude of the current after stabilization was used to calculate the effect of the compound at this solubility.

The inhibitory activity of the compounds of the present disclosure against Nav1.8 was determined through the above assay. The IC₅₀ values obtained are shown in Table 1.

**Table 1. IC₅₀ for the inhibition of Nav1.8 channel activity by the compounds of the present disclosure**

| Example No. | IC₅₀ (nM) |
|---|---|
| 2 | 2.89 |
| 3 | 2.65 |
| 4-1 or 4-2 (synthesized from the compound with the longer retention time between 4d-1 and 4d-2) | 5.3 |
| 5 | 0.71 |
| 6-1 or 6-2 (synthesized from the compound with the shorter retention time between 6f-1 and 6f-2) | 2.23 |
| 6-1 or 6-2 (synthesized from the compound with the longer retention time between 6f-1 and 6f-2) | 0.85 |
| 6 | 1.87 |
| 9 | 3.24 |
| 11 | 1.54 |
| 14 | 2.12 |
| 15 | 1.78 |
| 16 | 0.33 |
| 18 | 1.89 |
| 19 | 1.01 |
| 20 | 0.24 |
| 21 | 1.35 |
| 22 | 0.68 |
| 23 | 2.85 |
| 24 | 2.12 |
| 26 | 1.09 |
| 27 | <0.16 |
| 32 | 0.93 |
| 37 | 1.86 |
| 38 | 2.2 |
| 39 | 2.68 |
| 43 | 0.30 |
| 44 | 0.53 |

| | |
|---|---|
| Conclusion: The compounds of the present disclosure have significant inhibitory effects on Nav1.8 channel activity. | |

### Test Example 2: Pharmacokinetic Evaluation

### I. SD rat testing

### 1. Abstract

SD rats were used as test animals. After intragastric (i.g.) administration of the example compounds to SD rats, the plasma concentrations of the compounds at different time points were determined by the LC/MS/MS method. The pharmacokinetic behaviors of the compounds of the present disclosure in SD rats were studied, and their pharmacokinetic profiles were evaluated.

### 2. Protocol

### 2.1. Test compounds

Compounds 16, 32, and 38.

### 2.2. Test animals

Compounds 16 and 32: Four male SD rats, provided by Vital River Laboratory Animal Technology Co., Ltd. The animals were fasted overnight and then intragastrically dosed. Compound 38: Two male SD rats, provided by Zhejiang Vital River Laboratory Animal Technology Co., Ltd. The animals were fasted overnight and then intragastrically dosed.

### 2.3. Compound solution preparation

Compounds 16 and 32: Certain amounts of the test compounds were weighed out, and 5% DMSO + 5% tween 80 + 90% normal saline was added to prepare 0.2 mg/mL colorless clear solutions.

Compound 38: A certain amount of the test compound was weighed out, and 25% PEG400 + 75% (10% TPGS + 1% HPMC K100LV) was added to prepare a 5 mg/mL colorless clear solution.

### 2.4. Administration

Compounds 16 and 32: The administration dose was 2 mg/kg, and the administration volume was 10.0 mL/kg.

Compound 38: The administration dose was 50 mg/kg, and the administration volume was 10.0 mL/kg.

### 3. Procedures

0.2-mL blood samples were collected from the orbit before administration and 0.25, 0.5, 1.0, 2.0, 4.0, 6.0, 8.0, 11.0, and 24.0 h after administration, placed into EDTA-K2 anticoagulation test tubes, and centrifuged at 10,000 rpm for 1 min (4 °C), and the plasma was separated within 1 h and stored with dry ice before analysis. The procedures from the blood collection to the centrifugation were performed under ice-bath conditions. Access to food was given 2 h after administration.

Compounds 16 and 32: Determination of the plasma concentrations of the test compounds in SD rats after the compounds were administered at different concentrations: A 25-µL sample of the SD rat plasma collected at each time point after administration was taken, and 200 µL of acetonitrile containing an internal standard (verapamil, 100 ng/mL) was added. The mixture was vortexed and centrifuged at 3700 rpm for 10 min. 0.1 µL of the supernatant was taken for LC/MS/MS analysis.

Compound 38: Determination of the plasma concentration of the test compound in SD rats after the compound was administered at different concentrations: A 25-µL sample of the SD rat plasma collected at each time point after administration was taken, and 200 µL of acetonitrile containing an internal standard (dexamethasone, 100 ng/mL) was added. The mixture was vortexed and centrifuged at 4000 rpm for 15 min. 1 µL of the supernatant was taken for LC/MS/MS analysis.

### 4. Pharmacokinetic parameters

**Table 2. The pharmacokinetic parameters of the compounds of the present disclosure**

| Compound No. | Administration dose (mg/kg) | Plasma concentration Cmax (ng/mL) | Area under curve AUC₀₋ₜ (h*ng/mL) | Half-life T1/2 (h) | Clearance rate CL/F (mL/min/kg) | Bioavailability (%) |
|---|---|---|---|---|---|---|
| 16 | 2.0 | 120 | 329 | 9.1 | 101 | 51.3 |
| 32 | 2.0 | 85.7 | 321 | 2.6 | 96.9 | 24.7 |
| 38 | 50 | 1445 | 18428 | 5.84 | 42 | - |

| | | | | | | |
|---|---|---|---|---|---|---|
| Conclusion: The compounds of the present disclosure demonstrated high plasma concentrations and high exposure in SD rats, indicating that the compounds of the present disclosure have significant pharmacokinetic advantages. | | | | | | |

### II. C57 mouse testing

### 1. Abstract

C57 mice were used as test animals. After intragastric (i.g.)/intravenous (i.v.) administration of the compounds of the present disclosure to C57 mice, the plasma concentrations of the compounds at different time points were determined by the LC/MS/MS method. The pharmacokinetic behaviors of the compounds of the present disclosure in C57 mice were studied, and their pharmacokinetic profiles were evaluated.

### 2. Protocol

### 2.1. Test compounds

Compound 16 and compound 32.

### 2.2. Test animals

Thirty-six C57 mice, with an equal number of males and females, provided by Vital River Laboratory Animal Technology Co., Ltd., with the production licenses SCXK (Zhejiang) 2019-0001 and SCXK (Beijing) 2019-0006. The mice were evenly divided into 4 groups of 9, with 3 mice per time point per group, and dosed by intragastric and intravenous administration.

### 2.3. Compound solution preparation

Certain amounts of the test compounds were weighed out, and 5% DMSO + 5% tween 80 + 90% normal saline was added to prepare 0.1 mg/mL colorless clear solutions (for the intragastric administration groups) and 0.1 mg/mL colorless clear solutions (for the intravenous administration groups).

### 2.4. Administration

The intragastric administration groups: The administration dose was 2.0 mg/kg, and the administration volume was 20 mL/kg.

The intravenous administration groups: The administration dose was 1.0 mg/kg, and the administration volume was 10 mL/kg.

### 3. Procedures

The intragastric administration groups: 0.1-mL blood samples were collected from the orbit before administration and 0.25, 0.5, 1.0, 2.0, 4.0, 6.0, 8.0, 11.0, and 24.0 h after administration, placed into EDTA-K2 anticoagulation test tubes, and centrifuged at 10,000 rpm for 1 min (4 °C), and the plasma was separated within 1 h and stored at -80 °C before analysis. The procedures from the blood collection to the centrifugation were performed under ice-bath conditions.

The intravenous administration groups: Blood samples were collected before administration and 5 min and 0.25, 0.5, 1.0, 2.0, 4.0, 8.0, 11.0, and 24 h after administration and were treated in the same way as those collected from the intragastric administration groups.

Determination of the plasma concentrations of the test compounds in C57 mice after the compounds were administered at different concentrations: Compound 16: A 20-µL sample of the C57 mouse plasma collected at each time point after administration was taken, and 200 µL of acetonitrile containing 100 ng/mL camptothecin (internal standard) was added to each sample to precipitate protein. The mixture was vortexed for 5 min and centrifuged at 3700 rpm for 10 min. 50 µL of the supernatant was taken, and 100 µL of water was added. The mixture was vortexed for 5 min, and 1 µL of the mixture was taken for LC/MS/MS analysis. Compound 32: A 20-µL sample of the C57 mouse plasma collected at each time point after administration was taken, and 200 µL of acetonitrile containing verapamil (internal standard, 20 ng/mL) was added to each sample to precipitate protein. The mixture was vortexed for 5 min and centrifuged at 3700 rpm for 10 min. 90 µL of the supernatant was taken, and 90 µL of water was added. The mixture was vortexed for 5 min, and 0.1 µL of the mixture was taken for LC/MS/MS analysis.

### 4. Pharmacokinetic parameters

**Table 3. The pharmacokinetic parameters of the compounds of the present disclosure**

| Compound No. | Route of administration/ administration dose (mg/kg) | Plasma concentration Cmax (ng/mL) | Area under curve AUC₀₋ₜ (h*ng/mL) | Half-life T1/2 (h) | Clearance rate CL/F (mL/min/kg) | Apparent distribution volume Vss (mL/kg) | Bioavailability F (%) |
|---|---|---|---|---|---|---|---|
| 16 | i.g. /2.0 | 266 | 1252 | 7.5 | 24.4 | - | 87.2 |
| | i.v./1.0 | 377 | 718 | 8.1 | 21.1 | 9972 | |
| 32 | i.g. /2.0 | 260 | 1564 | 3.2 | 21.2 | - | 82.6 |
| | i.v./1.0 | 616 | 947 | 3.3 | 16.1 | 3925 | |

| | | | | | | | |
|---|---|---|---|---|---|---|---|
| Conclusion: The compounds of the present disclosure demonstrated high plasma concentrations, high exposure, low clearance rates, and relatively high bioavailability in C57 mice, indicating that the compounds of the present disclosure have pharmacokinetic advantages. III. CD-1 mouse testing | | | | | | | |

### 1. Abstract

CD-1 mice were used as test animals. After intragastric (i.g.) administration of the compound of the present disclosure to CD-1 mice, the plasma concentrations of the compound at different time points were determined by the LC/MS/MS method. The pharmacokinetic behavior of the compound of the present disclosure in CD-1 mice was studied, and its pharmacokinetic profile was evaluated.

### 2. Protocol

### 2.1. Test compounds

### Compound 38.

### 2.2. Test animals

Nine male CD-1 mice (3 mice per time point), provided by Vital River Laboratory Animal Technology Co., Ltd. (license: SCXK (Zhejiang) 2019-0001). The mice were intragastrically dosed.

### 2.3. Compound solution preparation

A certain amount of the test compound was weighed out, and 25% PEG400 + 75% (10% TPGS + 1% HPMC K100LV) was added to prepare a 10 mg/mL yellowish suspension.

### 2.4. Administration

The administration dose was 100 mg/kg, and the administration volume was 10 mL/kg.

### 3. Procedures

The intragastric administration groups: 0.1-mL blood samples were collected from the orbit before administration and 0.25, 0.5, 1.0, 2.0, 4.0, 6.0, 8.0, 11.0, and 24.0 h after administration, placed into EDTA-K2 anticoagulation test tubes, and centrifuged at 10,000 rpm for 1 min (4 °C), and the plasma was separated within 1 h and stored at -80 °C before analysis. The procedures from the blood collection to the centrifugation were performed under ice-bath conditions.

Determination of the plasma concentration of the test compound in CD-1 mice after the compound was administered at different concentrations: A 20-µL sample of the CD-1 mouse plasma collected at each time point after administration was taken, and 200 µL of acetonitrile containing an internal standard (dexamethasone, 100 ng/mL) was added to each sample to precipitate protein. The mixture was vortexed for 5 min and centrifuged at 4000 rpm for 15 min. 40 µL of the supernatant was taken, and 120 µL of water was added. The mixture was vortexed for 5 min, and 0.5 µL of the mixture was taken for LC/MS/MS analysis.

### 4. Pharmacokinetic parameters

**Table 4. The pharmacokinetic parameters of the compound of the present disclosure**

| Compound No. | Plasma concentration Cmax (ng/mL) | Area under curve AUC₀₋ₜ (h*ng/mL) | Half-life T1/2 (h) | Clearance rate CL/F (mL/min/kg) |
|---|---|---|---|---|
| 38 | 6280 | 76714 | 5.8 | 20.3 |

| | | | | |
|---|---|---|---|---|
| Conclusion: The compound of the present disclosure demonstrated a high plasma concentration and high exposure in CD-1 mice, indicating that the compound of the present disclosure has significant pharmacokinetic advantages. | | | | |

### IV. Dog testing

### 1. Abstract

Dogs were used as test animals. After intragastric (i.g.) administration of the example compound to dogs, the plasma concentrations of the compound at different time points were determined by the LC/MS/MS method. The pharmacokinetic behavior of the compound of the present disclosure in dogs was studied, and its pharmacokinetic profile was evaluated.

### 2. Protocol

### 2.1. Test compounds

The compound of Example 43.

### 2.2. Test animals

Four dogs, with an equal number of males and females, provided by Weifang Shengnuo Laboratory Animal Breeding Co., Ltd (license: SCXK (Beijing) 2020-0009) and Beijing Institute of Xieerxin Biology Resource Co., Ltd (license: SCXK (Shandong) 20230001). The animals were fasted overnight and then intragastrically dosed.

### 2.3. Compound solution preparation

A certain amount of the example compound was weighed out, and 5% DMSO + 20% PG + 20% PEG400 + 55% normal saline was added to prepare a 0.4 mg/mL colorless clear solution.

### 2.4. Administration

The administration dose was 2 mg/kg, and the administration volume was 10.0 mL/kg.

### 3. Procedures

0.5-mL blood samples were collected from a forelimb vein before administration and 0.25, 0.5, 1.0, 2.0, 4.0, 6.0, 8.0, 11.0, 24.0, 48.0, and 72 h after administration, placed into EDTA-K2 anticoagulation test tubes, and centrifuged at 10,000 rpm for 1 min (4 °C), and the plasma was separated within 1 h and stored with dry ice before analysis. The procedures from the blood collection to the centrifugation were performed under ice-bath conditions. Access to food was given 2 h after administration.

Determination of the plasma concentration of the test compound in dogs after the compound was administered at different concentrations: A 50-µL sample of the dog plasma collected at each time point after administration was taken, and 250 µL of acetonitrile containing an internal standard (labetalol, 1 µg/mL) was added. The mixture was vortexed and centrifuged at 3700 rpm for 10 min. 0.5 µL of the supernatant was taken for LC/MS/MS analysis.

### 4. Pharmacokinetic parameters

**Table 5. The pharmacokinetic parameters of the compound of the present disclosure in dogs**

| Compound No. | Plasma concentration Cmax (ng/mL) | Area under curve AUC₀₋ₜ (h*ng/mL) | Half-life T1/2 (h) | Clearance rate CL/F (mL/min/kg) |
|---|---|---|---|---|
| 43 | 5675 | 250247 | 34.5 | 0.128 |

| | | | | |
|---|---|---|---|---|
| Conclusion: The compound of the present disclosure demonstrated a high plasma concentration, high exposure, a long half-life, and a low clearance rate in dogs, indicating that the compound of the present disclosure has significant pharmacokinetic advantages. | | | | |

### Test Example 3: Solubility of Compound of Present Disclosure

### 1. Materials

Reagents: dimethyl sulfoxide (chromatographically pure, Sigma-Aldrich, Cat. No. 472301-4X4L), ethanol (chromatographically pure, CNW, Cat. No. 4.016362.4000), acetonitrile (chromatographically pure, FISHER, Cat. No. A998-4), NaH₂PO₄·2H₂O (analytically pure, Sinopharm Chemical Reagent Co., Ltd., Cat. No. 20040717), ammonium acetate (chromatographically pure, ALADDIN, Cat. No. 17836-250G), FaSSIF/FeSSIF/FaSSGF powder (Biorelevant, Cat. No. FFF02), FaSSIF buffer concentrate (Biorelevant, Cat. No. FASBUF), FeSSIF buffer concentrate (Biorelevant, Cat. No. FESBUF), and ultrapure water (made in-house using an ELGA CHORUS laboratory water purification system).

Instrument: Agilent 1200 DAD liquid chromatograph (Agilent, U.S.)

### 2. Material solution preparation

### 2.1. FassIF solution preparation

FaSSIF solution: 2.0825 g of FaSSIF medium solution and 48.055 g of ultrapure water were weighed into a 50-mL beaker, and 0.112 g of FaSSIF/FeSSIF/FaSSGF powder was then added to the beaker. The mixture was stirred to dissolve the powder, and the solution was then equilibrated by standing for 2 h before use (the solution should be used within 48 h).

### 2.2. FessIF solution preparation

FeSSIF solution: 4.0705 g of FeSSIF medium solution and 45.97 g of ultrapure water were weighed into a 50-mL beaker, and 0.56 g of FaSSIF/FeSSIF/FaSSGF powder was then added to the beaker. The mixture was stirred to dissolve the powder, forming the desired solution (the solution should be used within 48 h).

### 3. Procedures

Dissolution tests in the FassIF and FessIF solutions.

3.1. A proper amount of the test compound was precisely weighed out and dissolved in DMSO to prepare a 10 mM stock solution. 10 µL of the stock solution (concentration 10 mM, dissolved in DMSO) was precisely measured out and well mixed with 990 µL of an organic solvent mixture (usually DMSO:acetonitrile:ethanol = 1:1:1) in a 2-mL sample flask to form a 100 µM clear sample solution. The solution was filtered using a 0.22 µm organic-phase microporous filter membrane, and the subsequent filtrate was taken as a reference solution.

3.2. 1 mg of the test sample was dissolved in 900 µL of the FassIF solution (or the FessIF solution). The mixture was vigorously mixed (ultrasonic dispersion was used for solid chunks that are difficult to disperse) to form a solution, and the solution was prepared in duplicate. The solutions were shaken in a 37 °C thermostatic shaker for 24 h and then centrifuged at 12,000 rpm for 30 min. The supernatants were used as sample solutions and transferred for liquid chromatography analysis.

### 4. Results

Solubility (µM) = sample peak area/reference peak area × reference solution concentration (µM) × sample solution dilution factor.

The average of two measurements was taken as the final solubility in the FassIF and FessIF solutions.

**Table 6. The solubility of the compound of the present disclosure**

| Compound No. | FassIF solution (µM) | FessIF solution (µM) |
|---|---|---|
| 20 | 307.78 | 2406.73 |

| | | |
|---|---|---|
| Conclusion: The compound of the present disclosure has excellent solubility in both the FassIF solution and the FessIF solution. | | |

### Test Example 4: Pharmacodynamic Study

### 1. Objective

To evaluate the analgesic efficacy of the compounds of the present disclosure in inhibiting pain in rats of a rat incision pain model.

### 2. Test compounds

The compounds of Example 16 and Example 32.

A 25% PEG400 + 75% (10% TPGS + 1% HPMC K100LV) solution was used.

### 3. Method and materials

3.1. Test animals and housing conditions

Test animals: SD rats, purchased from Beijing Vital River Laboratory Animal Technology Co., Ltd. (license number: SCXK (Zhejiang) 2019-0001), weighing about 180 g on purchase.

Housing conditions: 5 animals/cage, a 12/12-hour light/dark cycle, a constant temperature of 23±1 °C with humidity at 50 to 60%, *ad libitum* access to food and water.

### 3.2. Grouping of animals

After acclimatization, the SD rats were grouped as follows:

| Group | Number of animals | Administration dose and route |
|---|---|---|
| Vehicle control | 9 | 25% PEG400 + 75% (10% TPGS + 1% HPMC K100LV) solution (i.g./one dose) |
| Example 16 | 9 | 200 mpk (i.g./one dose) |
| Example 16 | 9 | 100 mpk(i.g./one dose) |
| Example 16 | 9 | 50 mpk(i.g./one dose) |
| Vehicle control | 9 | 25% PEG400 + 75% (10% TPGS + 1% HPMC K100LV) solution (i.g./one dose) |
| Example 32 | 9 | 200 mpk (i.g./one dose) |
| Example 32 | 9 | 100 mpk(i.g./one dose) |
| Example 32 | 9 | 30 mpk(i.g./one dose) |

| | | |
|---|---|---|
| Note: One dose means that only one dose was administered; i.g. stands for intragastric administration. | | |

### 3.3. Method:

Nine SD rats, weighing 170-190 g, were selected for this study. The mechanical pain threshold was measured using an electronic aesthesiometer. Then an incision pain surgery was performed. During the surgery, after the rats were anesthetized with Zoletil (Zoletil-50, 250 mg, diluted with normal saline to 50 mL after being dissolved, injected at a dose of 2 mL per 200 g of body weight), a 1 cm long incision was made in the middle of the plantar surface of the left hind paw using a No. 10 scalpel blade, cutting through the skin and fascia, and the incision was closed using 3-0 sterile silk sutures. The injured area was disinfected with penicillin, and the animals were returned to their original place to recover overnight. After the overnight recovery, oral gavage administration was performed. Five hours after administration (about 24 h after surgery), the mechanical pain threshold was measured using an electronic aesthesiometer.

### 3.4. Statistical analysis

Data were recorded using Excel statistical software: The averages were calculated using avg; the SD values were calculated using STDEV; the SEM values were calculated using STDEV/SQRT (the number of animals per group). Plotting was performed using GraphPad Prism software. Statistical analysis of the data was performed using one-way ANOVA and t-test.

Percent increase in threshold (%) = [(Gₜ - G₀)/G₀] × 100(%), where Gₜ is the plantar pain threshold of the administration group, and G₀ is the plantar pain threshold of the vehicle group.

### 4. Results

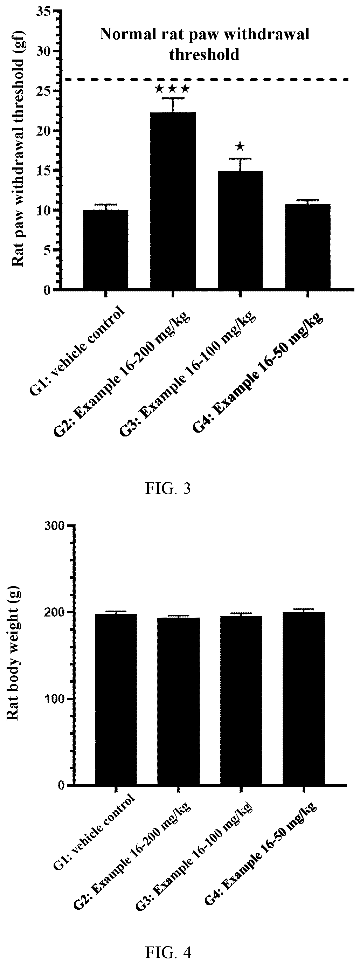

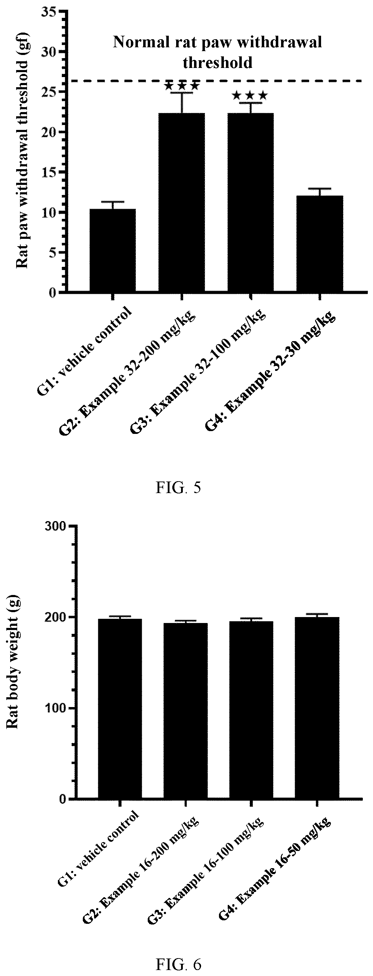

The analgesic efficacy of the compound of Example 16 in the rat incision pain model is shown in FIG. 3 and Table 7, and its effect on body weight is shown in FIG. 4. The analgesic efficacy of the compound of Example 32 in the rat incision pain model is shown in FIG. 5 and Table 7, and its effect on body weight is shown in FIG. 6.

**Table 7. The analgesic efficacy of the compounds of the present disclosure in the rat incision pain model**

| Group | Route of administration | Dose (mpk) | Pain threshold (gf) | Percent increase in threshold (%) | p (vs. vehicle control) | Number of animals/ group |
|---|---|---|---|---|---|---|
| Vehicle control | i.g./one dose | / | 10.0±0.7 | / | / | 9 |
| Example 16 | i.g./one dose | 200 | 22.3±1.8 | 122 | <0.001 | 9 |
| Example 16 | i.g./one dose | 100 | 14.9±1.5 | 49 | <0.05 | 9 |
| Example 16 | i.g./one dose | 50 | 10.8±0.5 | 7 | | 9 |
| Vehicle control | i.g./one dose | / | 10.4±0.9 | / | / | 9 |
| Example 32 | i.g./one dose | 200 | 22.4±2.5 | 114 | <0.001 | 9 |
| Example 32 | i.g./one dose | 100 | 22.3±1.3 | 114 | <0.001 | 9 |
| Example 32 | i.g./one dose | 30 | 12.0±0.9 | 15 | - | 9 |

| | | | | | | |
|---|---|---|---|---|---|---|
| Note: One dose means that only one dose was administered; i.g. stands for intragastric administration. | | | | | | |

### 5. Conclusion

The pain threshold of normal rats (weighing 170-190 g) was 26.2±1.6 gf, and the pain threshold of the vehicle control group was 10.0±0.7 gf. The pain thresholds for the compound of Example 16 at 200, 100, and 50 mg/kg were 22.3, 14.9, and 10.8 gf, respectively, which were significantly 122% (p < 0.001), 49% (p < 0.05), and 7% higher than those of the vehicle control group, respectively. The pain threshold for 200 mg/kg was significantly higher than that for 100 mg/kg (p < 0.01), and the pain threshold for 100 mg/kg was significantly higher than that for 50 mg/kg (p < 0.05). The analgesic effect was significantly dose-dependent, and the administration did not influence the body weight of rats.

The pain threshold of normal rats (weighing 170-190 g) was 26.3±0.8 gf, and the pain threshold of the vehicle control group was 10.4±0.9 gf. The pain thresholds for the compound of Example 32 at 200, 100, and 30 mg/kg were 22.4, 22.3, and 12.0 gf, respectively, which were significantly 114% (p < 0.001), 114% (p < 0.001), and 15% higher than those of the vehicle control group, respectively. The pain threshold for 200 mg/kg was comparable to that for 100 mg/kg, indicating that the analgesic efficacy had plateaued. The pain threshold for 100 mg/kg was significantly higher than that for 30 mg/kg (p < 0.01). The analgesic effect was significantly dose-dependent, and the administration did not influence the body weight of rats.

## Claims

1. A compound represented by general formula (I) or a pharmaceutically acceptable salt thereof: 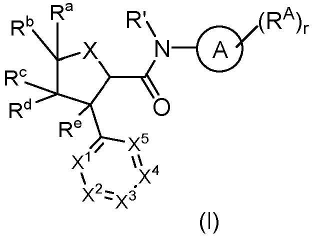 wherein:
ring A is phenyl or 6-membered heteroaryl;
each R^{A} is identical or different and is independently selected from the group consisting of a deuterium atom, halogen, hydroxy, cyano, alkyl, alkoxy, haloalkyl, haloalkoxy, hydroxyalkyl, hydroxyalkoxy, alkoxyalkyl, alkenyl, alkynyl, -NR³R⁴, -alkylene-NR³R⁴, -O-alkylene-NR³R⁴, -C(=NR⁵)R⁶, -S(O)ᵥNR³R⁴, -NR⁵S(O)ᵥR⁶, -S(O)ᵥR⁶, - S(=NR⁵)(O)R⁶, -NR⁵C(O)R⁶, -NR⁵C(O)NR³R⁴, -C(O)NR⁵-OR⁶, -P(O)R⁷R⁸, -C(O)NR⁵-NR³R⁴, -C(=NR⁵)NR⁵-OR⁶, -C(O)NR⁵-alkylene-Cy, -C(=NR⁵)NR³R⁴, -C(O)-C(O)-NR³R⁴, -S(=NR⁵)NR³R⁴, -S(=NR⁵)R⁶, -S(=NR⁵)(O)NR³R⁴, -Si(O)NR³R⁴, -Si(R⁶)₃, - OR⁶, cycloalkyl, heterocyclyl, aryl, heteroaryl, cycloalkylalkyl, heterocyclylalkyl, -C(O)-cycloalkyl, -C(O)-heterocyclyl, -alkylene-O-alkylene-cycloalkyl, -alkylene-O-cycloalkyl, -O-alkylene-heteroaryl, and -O-alkylene-heterocyclyl, wherein the alkyl, alkoxy, alkoxyalkyl, alkenyl, alkynyl, alkylene, cycloalkyl, heterocyclyl, aryl, heteroaryl, cycloalkylalkyl, and heterocyclylalkyl are each independently and optionally substituted with one or more R⁰¹;
Cy is cycloalkyl or heterocyclyl, and the cycloalkyl and heterocyclyl are each independently and optionally substituted with one or more substituents selected from the group consisting of a deuterium atom, halogen, hydroxy, cyano, oxo, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, and heteroaryl;
each R³, R⁴, and R⁵ is identical or different and is independently selected from the group consisting of a hydrogen atom, alkyl, deuterated alkyl, alkoxy, deuterated alkoxy, alkenyl, alkynyl, NR²⁰R²¹, C(O)NR²⁰R²¹, NR²²C(O)R²³, C(O)R²³, C(O)OR²³, OC(O)R²³, S(O)ᵥR²³, S(O)ᵥOR²³, OS(O)ᵥR²³, S(O)ᵥNR²⁰R²¹, OR²³, cycloalkyl, heterocyclyl, aryl, and heteroaryl, wherein the alkyl, alkoxy, alkenyl, alkynyl, cycloalkyl, heterocyclyl, aryl, and heteroaryl are each independently and optionally substituted with one or more R⁰¹;
alternatively, R³ and R⁴, together with the nitrogen atom to which they are attached, form heterocyclyl; the heterocyclyl is optionally substituted with one or more R⁰¹;
each R⁶, R⁷, and R⁸ is identical or different and is independently selected from the group consisting of a hydrogen atom, alkyl, alkoxy, haloalkyl, deuterated alkyl, haloalkoxy, deuterated alkoxy, hydroxyalkyl, alkenyl, alkynyl, NR²⁰R²¹, C(O)NR²⁰R²¹, NR²²C(O)R²³, C(O)R²³, C(O)OR²³, OC(O)R²³, S(O)ᵥR²³, S(O)ᵥOR²³, OS(O)ᵥR²³, S(O),NR²⁰R²¹, OR²³, cycloalkyl, heterocyclyl, cycloalkylalkyl, heterocyclylalkyl, aryl, and heteroaryl; the alkyl, alkoxy, alkenyl, alkynyl, cycloalkyl, heterocyclyl, cycloalkylalkyl, heterocyclylalkyl, aryl, and heteroaryl are each independently and optionally substituted with one or more R⁰¹;
each R⁰¹ is identical or different and is independently selected from the group consisting of a deuterium atom, halogen, hydroxy, cyano, oxo, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, heteroaryl, =CR^{7a}R^{8a}, -NR³R⁴, -alkylene-NR³R⁴, -O-alkylene-NR³R⁴, -C(=NR⁵)R⁶, -S(O)ᵥNR³R⁴, -NR⁵S(O)ᵥR⁶, -S(O)ᵥR⁶, -S(=NR⁵)(O)R⁶, -NR^{S}C(O)R⁶, - NR⁵C(O)NR³R⁴, -C(O)NR⁵-OR⁶, -P(O)R⁷R⁸, -C(O)NR⁵-NR³R⁴, -C(=NR⁵)NR⁵-OR⁶, - C(O)NR⁵-alkylene-Cy, -C(=NR⁵)NR³R⁴, -C(O)-C(O)-NR³R⁴, -S(=NR⁵)NR³R⁴, - S(=NR⁵)R⁶, -S(=NR⁵)(O)NR³R⁴, -Si(O)NR³R⁴, -OR⁶, -Si(R^{6a})₃, -O-alkylene-heteroaryl, and -O-alkylene-heterocyclyl; the alkyl, alkenyl, alkynyl, alkoxy, alkylene, cycloalkyl, heterocyclyl, aryl, and heteroaryl are each independently and optionally substituted with one or more substituents selected from the group consisting of a deuterium atom, halogen, hydroxy, cyano, oxo, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, and heteroaryl;
each R^{6a}, R^{7a}, and R^{8a} is identical or different and is independently selected from the group consisting of a hydrogen atom, a deuterium atom, halogen, hydroxy, cyano, amino, -NH alkyl, -N(alkyl)₂, acetyl, alkyl, alkenyl, alkynyl, alkoxy, haloalkyl, haloalkoxy, hydroxyalkyl, cycloalkyl, heterocyclyl, aryl, and heteroaryl;
R' is selected from the group consisting of a hydrogen atom, alkyl, haloalkyl, deuterated alkyl, alkoxy, deuterated alkoxy, hydroxyalkyl, cycloalkyl, and heterocyclyl;
X is O or S;
R^{a} and R^{b} are identical or different and are each independently selected from the group consisting of a hydrogen atom, a deuterium atom, halogen, hydroxy, cyano, amino, alkyl, deuterated alkyl, alkenyl, alkynyl, alkoxy, deuterated alkoxy, haloalkyl, haloalkoxy, hydroxyalkyl, cycloalkyl, heterocyclyl, cycloalkyloxy, and heterocyclyloxy; the alkyl, alkoxy, alkenyl, alkynyl, cycloalkyl, heterocyclyl, cycloalkyloxy, and heterocyclyloxy are optionally substituted with one or more R⁰²;
with the proviso that R^{a} and R^{b} are not simultaneously hydrogen;
R^{c} and R^{d} are identical or different and are each independently selected from the group consisting of a hydrogen atom, a deuterium atom, halogen, hydroxy, cyano, amino, alkyl, alkenyl, alkynyl, alkoxy, haloalkyl, deuterated alkyl, haloalkoxy, deuterated alkoxy, hydroxyalkyl, cycloalkyl, heterocyclyl, cycloalkyloxy, and heterocyclyloxy; the alkyl, alkoxy, alkenyl, alkynyl, cycloalkyl, heterocyclyl, cycloalkyloxy, and heterocyclyloxy are optionally substituted with one or more R⁰²;
alternatively, R^{a} and R^{b}, together with the carbon atom to which they are attached, form cycloalkyl or heterocyclyl; alternatively, R^{c} and R^{d}, together with the carbon atom to which they are attached, form cycloalkyl or heterocyclyl; wherein the cycloalkyl or heterocyclyl is independently and optionally substituted with one or more R⁰²;
R^{e} is selected from the group consisting of a hydrogen atom, a deuterium atom, halogen, hydroxy, cyano, amino, alkyl, alkoxy, haloalkyl, haloalkoxy, deuterated alkyl, deuterated alkoxy, and hydroxyalkyl;
each R⁰² is identical or different and is independently selected from the group consisting of a deuterium atom, halogen, hydroxy, cyano, oxo, amino, an amide group, alkyl, alkenyl, alkynyl, alkoxy, haloalkyl, haloalkoxy, hydroxyalkyl, cycloalkyl, heterocyclyl, aryl, and heteroaryl;
each R²⁰, R²¹, and R²² is identical or different and is independently selected from the group consisting of a hydrogen atom, alkyl, deuterated alkyl, alkoxy, deuterated alkoxy, alkenyl, alkynyl, cycloalkyl, heterocyclyl, aryl, and heteroaryl; the alkyl, alkoxy, alkenyl, alkynyl, cycloalkyl, heterocyclyl, aryl, and heteroaryl are each independently and optionally substituted with one or more groups selected from the group consisting of a deuterium atom, halogen, hydroxy, cyano, amino, alkyl, alkoxy, haloalkyl, haloalkoxy, and hydroxyalkyl;
each R²³ is identical or different and is independently selected from the group consisting of a hydrogen atom, alkyl, deuterated alkyl, alkoxy, deuterated alkoxy, alkenyl, alkynyl, cycloalkyl, heterocyclyl, aryl, and heteroaryl; the alkyl, alkoxy, alkenyl, alkynyl, cycloalkyl, heterocyclyl, aryl, and heteroaryl are each independently and optionally substituted with one or more groups selected from the group consisting of a deuterium atom, halogen, hydroxy, cyano, amino, alkyl, alkoxy, haloalkyl, haloalkoxy, and hydroxyalkyl;
X¹ is CR^{X1} or N;
X² is CR^{X2} or N;
X³ is CR^{X3} or N;
X⁴ is CR^{X4} or N;
X⁵ is CR^{X5} or N;
X¹, X², X³, X⁴, and X⁵ are not simultaneously N;
R^{X1}, R^{X2}, R^{X3}, R^{X4}, and R^{X5} are identical or different and are each independently selected from the group consisting of a hydrogen atom, a deuterium atom, halogen, hydroxy, cyano, amino, an amide group, nitro, alkyl, alkenyl, alkynyl, alkoxy, haloalkyl, deuterated alkyl, haloalkoxy, deuterated alkoxy, hydroxyalkyl, cycloalkyl, heterocyclyl, -O-(CH₂)ₙ-cycloalkyl, -O-(CH₂)ₛ-heterocyclyl, aryl, and heteroaryl, wherein the alkyl, alkoxy, alkenyl, alkynyl, cycloalkyl, heterocyclyl, aryl, and heteroaryl are each independently and optionally substituted with one or more R⁰³;
each R⁰³ is identical or different and is independently selected from the group consisting of a deuterium atom, halogen, hydroxy, cyano, oxo, amino, an amide group, alkyl, alkenyl, alkynyl, alkoxy, haloalkyl, haloalkoxy, hydroxyalkyl, cycloalkyl, heterocyclyl, aryl, and heteroaryl;
each v is identical or different and is independently selected from the group consisting of 0, 1, and 2;
n is selected from the group consisting of 0, 1, 2, 3, 4, and 5;
s is selected from the group consisting of 0, 1, 2, 3, 4, and 5; and
r is selected from the group consisting of 0, 1, 2, 3, 4, and 5;
with the proviso that 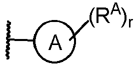 is not

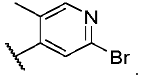

2. The compound or the pharmaceutically acceptable salt thereof according to claim 1, being a compound represented by general formula (II) or a pharmaceutically acceptable salt thereof: 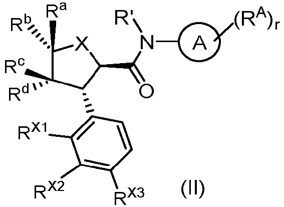 wherein:
R^{a} and R^{b} are different and are each independently selected from the group consisting of a hydrogen atom, halogen, hydroxy, cyano, amino, C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, 3- to 10-membered cycloalkyl, 3- to 10-membered heterocyclyl, 3- to 10-membered cycloalkyloxy, and 3- to 10-membered heterocyclyloxy; the 3- to 10-membered cycloalkyl, 3- to 10-membered heterocyclyl, 3- to 10-membered cycloalkyloxy, and 3- to 10-membered heterocyclyloxy are optionally substituted with one or more R⁰²;
R^{c} and R^{d} are different and are each independently selected from the group consisting of a hydrogen atom, halogen, hydroxy, cyano, amino, C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, 3- to 10-membered cycloalkyl, 3- to 10-membered heterocyclyl, 3- to 10-membered cycloalkyloxy, and 3- to 10-membered heterocyclyloxy; the 3- to 10-membered cycloalkyl, 3- to 10-membered heterocyclyl, 3- to 10-membered cycloalkyloxy, and 3- to 10-membered heterocyclyloxy are optionally substituted with one or more R⁰²;
ring A, R^{A}, R', X, R^{X1}, R^{X2}, R^{X3}, R⁰², and r are as defined in claim 1.

3. The compound or the pharmaceutically acceptable salt thereof according to claim 1 or 2, wherein ring A is selected from the group consisting of 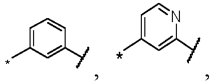 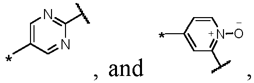 wherein the * end is attached to -NR', and the end is attached to R^{A}.

4. The compound or the pharmaceutically acceptable salt thereof according to any one of claims 1 to 3, wherein R^{A} is selected from the group consisting of F, Cl, amino, cyano, 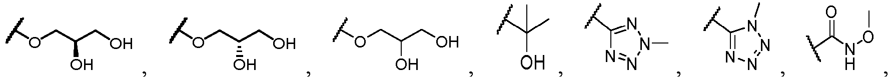 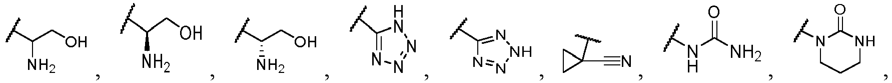 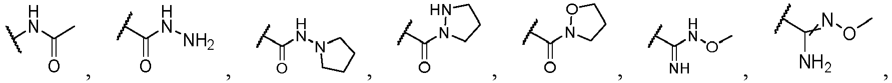 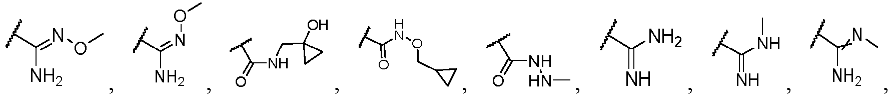 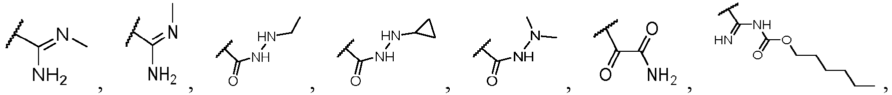 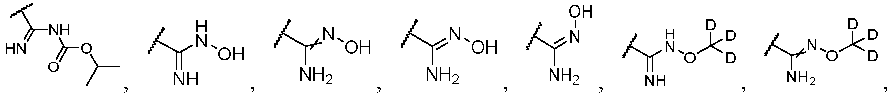 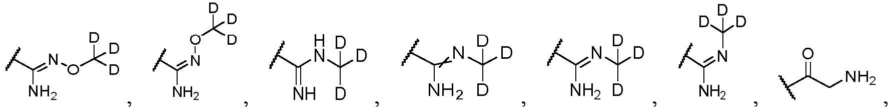 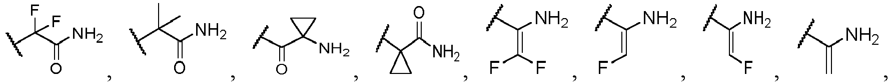 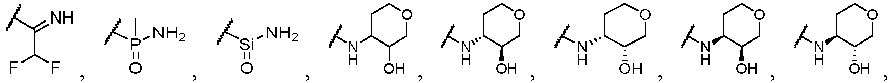 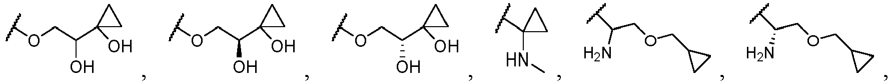 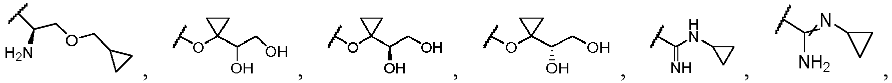 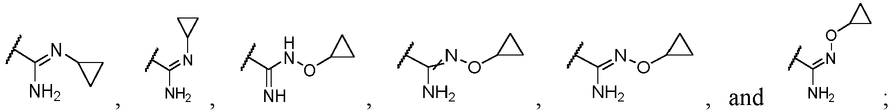 preferably, R^{A} is selected from the group consisting of 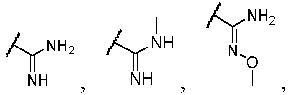 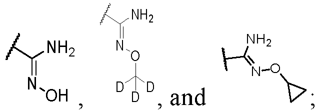 more preferably, R^{A} is

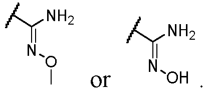

5. The compound or the pharmaceutically acceptable salt thereof according to claim 1 or 2, being a compound represented by general formula (IV) or a pharmaceutically acceptable salt thereof: 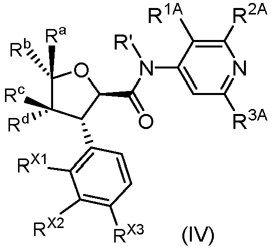
wherein R^{1A} and R^{2A} are identical or different and are each independently a hydrogen atom or R^{A};
R^{3A} is R^{A};
R^{a}, R^{b}, R^{c}, R^{d}, R^{X1}, R^{X2}, R^{X3}, R', and R^{A} are as defined in claim 2.

6. The compound or the pharmaceutically acceptable salt thereof according to claim 1 or 2, being a compound represented by general formula (VI') or a pharmaceutically acceptable salt thereof: 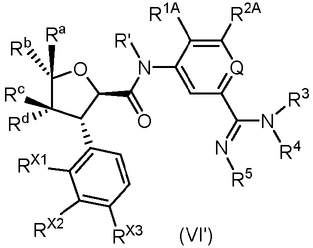
wherein Q is selected from the group consisting of CR^{5A}, N, and N⁺-O⁻;
R^{5A} is R^{1A};
R^{1A}, R^{2A}, R', R^{a}, R^{b}, R^{c}, R^{d}, R^{X1}, R^{X2}, R^{X3}, R³, R⁴, and R⁵ are as defined in claim 5.

7. The compound or the pharmaceutically acceptable salt thereof according to any one of claims 1 to 3, 5, and 6, wherein R³ is selected from the group consisting of a hydrogen atom, C₁₋₆ alkyl, OR²³, and C(O)OR²³, and R²³ is as defined in general formula (I); and/or R⁴ is a hydrogen atom; preferably, R³ is selected from the group consisting of a hydrogen atom, hydroxy, C₁₋₆ alkyl, C₁₋₆ alkoxy, deuterated C₁₋₆ alkoxy, -O-3- to 6-membered cycloalkyl, and C(O)OR²³, and R²³ is C₁₋₈ alkyl; and/or R⁴ is a hydrogen atom; more preferably, R³ is selected from the group consisting of a hydrogen atom, methyl, ethyl, hydroxy, methoxy, cyclopropyl, 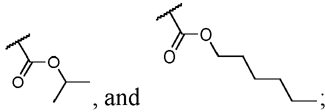 and/or R⁴ is a hydrogen atom; most preferably, R³ and R⁴ are both hydrogen atoms.

8. The compound or the pharmaceutically acceptable salt thereof according to any one of claims 1 to 3, 5, 6, and 7, wherein R⁵ is selected from the group consisting of a hydrogen atom, C₁₋₆ alkyl, OR²³, 3- to 6-membered cycloalkyl, and C(O)OR²³, and R²³ is as defined in claim 1; preferably, R⁵ is selected from the group consisting of a hydrogen atom, hydroxy, C₁₋₆ alkyl, C₁₋₆ alkoxy, deuterated C₁₋₆ alkoxy, -O-3- to 6-membered cycloalkyl, and C(O)OR²³, and R²³ is C₁₋₈ alkyl; more preferably, R⁵ is hydroxy or C₁₋₆ alkoxy; most preferably, R⁵ is hydroxy or methoxy.

9. The compound or the pharmaceutically acceptable salt thereof according to any one of claims 1 to 3 and 5 to 8, wherein R⁵ is selected from the group consisting of a hydrogen atom, methyl, hydroxy, methoxy, deuterated methoxy, -O-cyclopropyl, 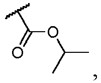 and 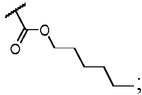 preferably, R⁵ is selected from the group consisting of a hydrogen atom, methyl, hydroxy, methoxy, deuterated methoxy, and -O-cyclopropyl.

10. The compound or the pharmaceutically acceptable salt thereof according to any one of claims 1 to 9, wherein R^{a} is C₁₋₆ alkyl; preferably, R^{a} is CH₃.

11. The compound or the pharmaceutically acceptable salt thereof according to any one of claims 1 to 10, wherein R^{b} is C₁₋₆ haloalkyl; preferably, R^{b} is CF₃.

12. The compound or the pharmaceutically acceptable salt thereof according to any one of claims 1 to 11, wherein R^{c} is a hydrogen atom, and R^{d} is C₁₋₆ alkyl; preferably, R^{c} is a hydrogen atom, and R^{d} is CH₃.

13. The compound or the pharmaceutically acceptable salt thereof according to any one of claims 1 to 12, wherein R' is a hydrogen atom.

14. The compound or the pharmaceutically acceptable salt thereof according to any one of claims 1 to 13, wherein R^{X1} is selected from the group consisting of hydroxy, C₁₋₆ alkoxy, and 3- to 6-membered cycloalkyloxy; preferably, R^{X1} is selected from the group consisting of hydroxy, methoxy, and 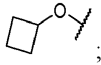 more preferably, R^{X1} is methoxy.

15. The compound or the pharmaceutically acceptable salt thereof according to any one of claims 1 to 14, wherein R^{X2} is halogen; preferably, R^{X2} is a fluorine atom.

16. The compound or the pharmaceutically acceptable salt thereof according to any one of claims 1 to 15, wherein R^{X3} is halogen; preferably, R^{X3} is a fluorine atom.

17. A compound or a pharmaceutically acceptable salt thereof, being selected from the group consisting of the following compounds:

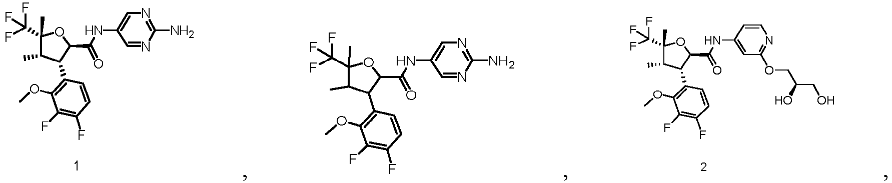

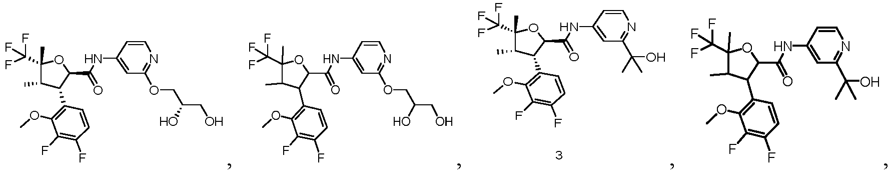

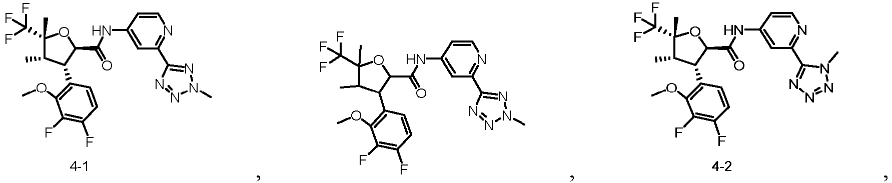

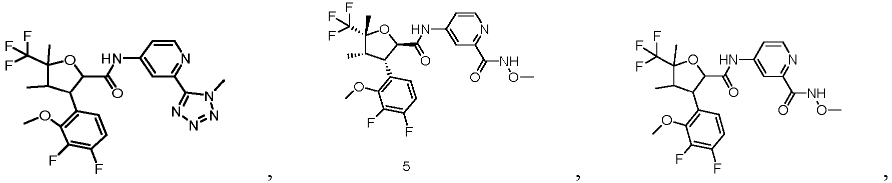

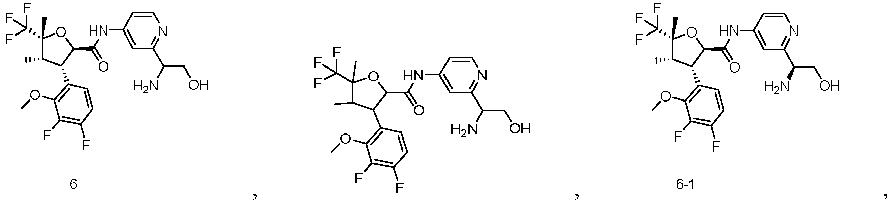

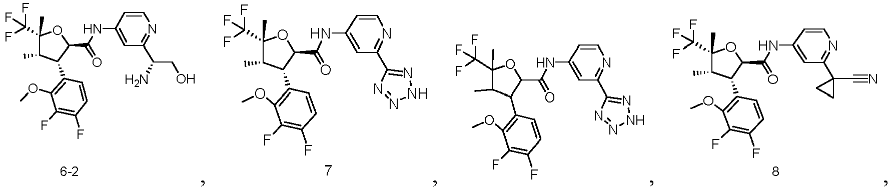

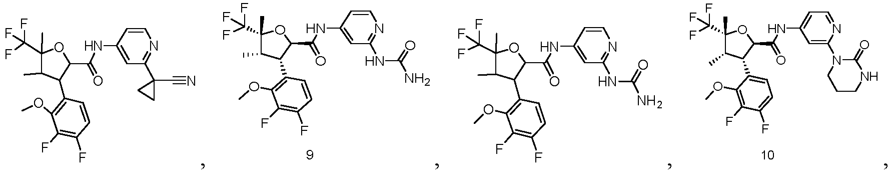

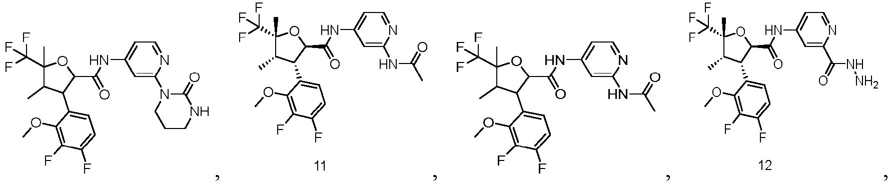

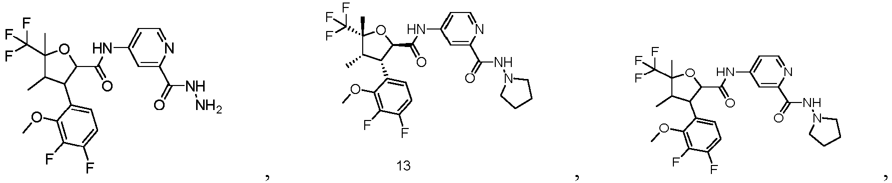

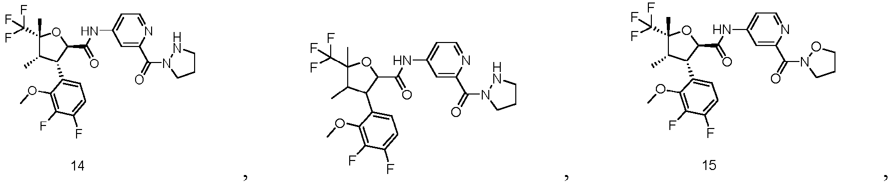

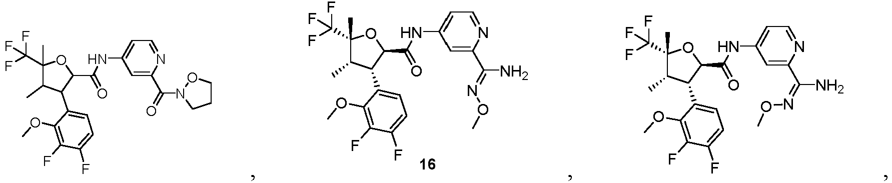

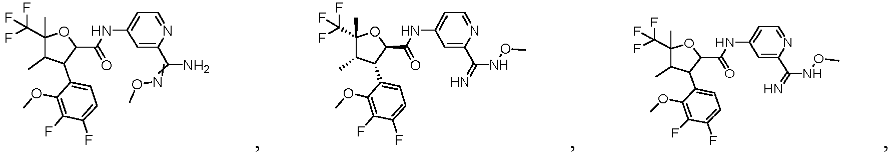

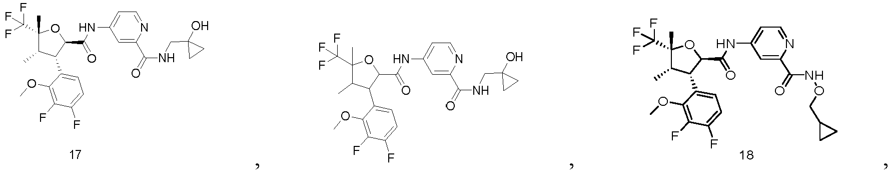

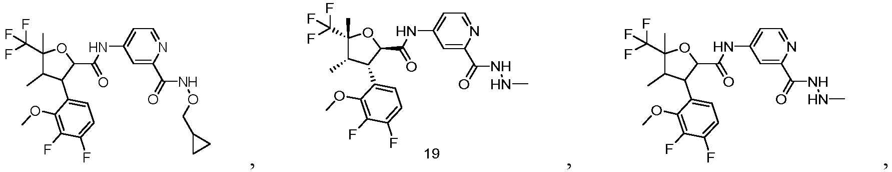

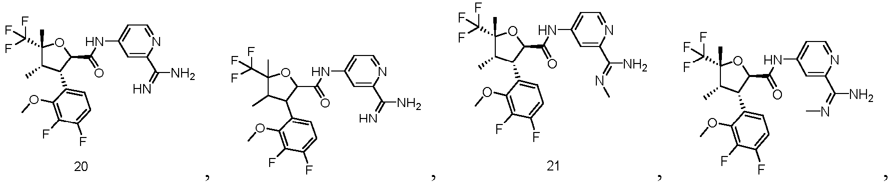

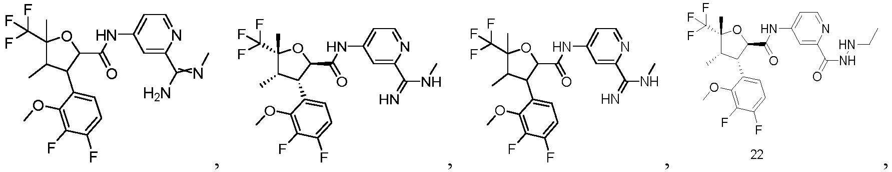

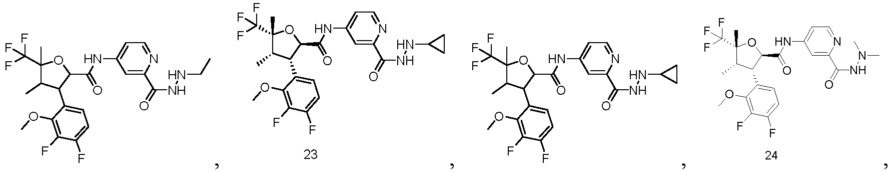

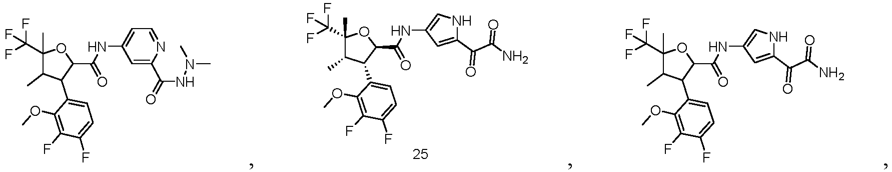

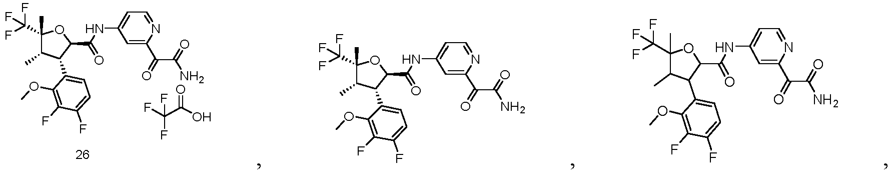

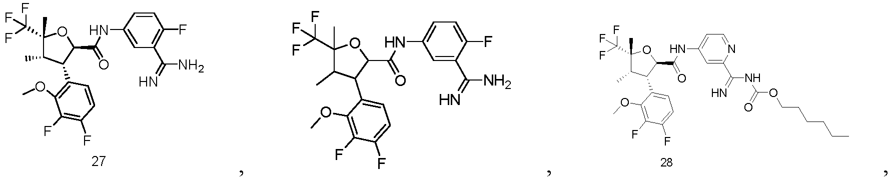

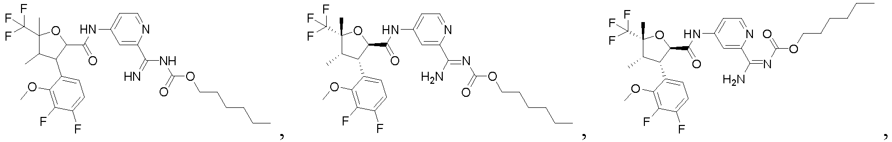

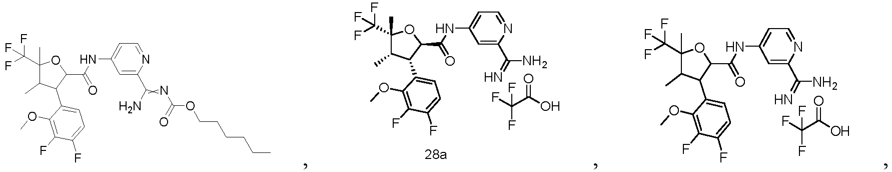

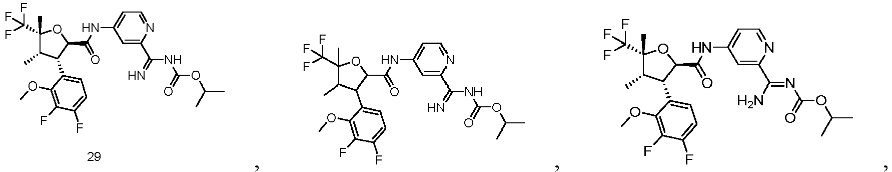

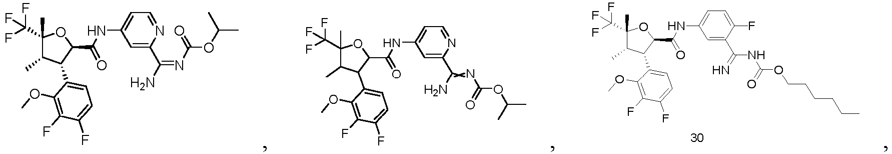

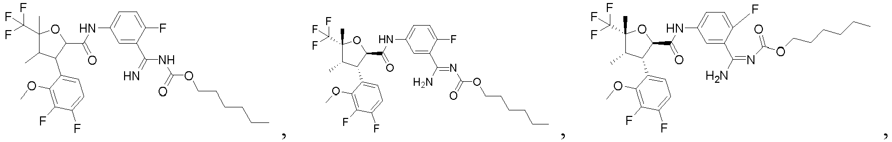

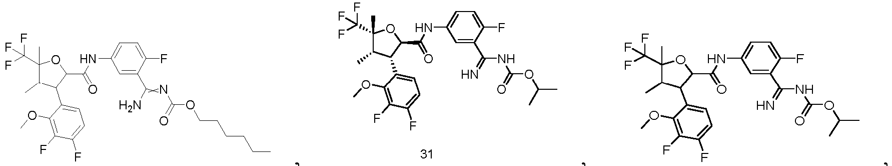

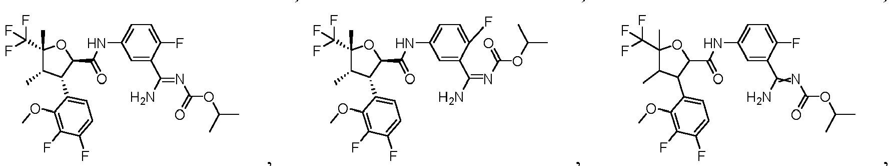

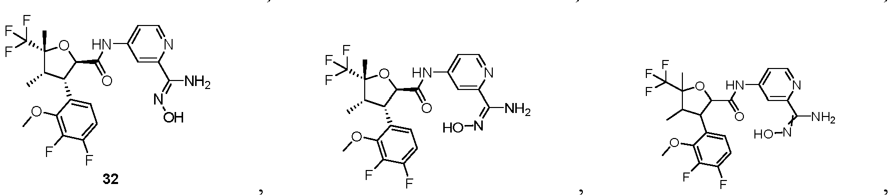

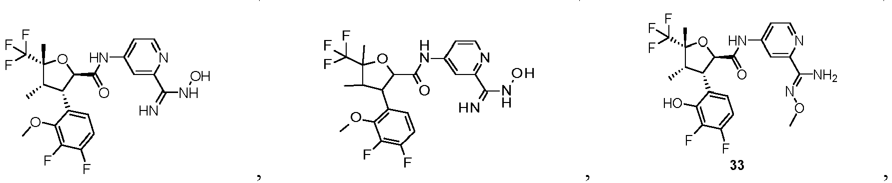

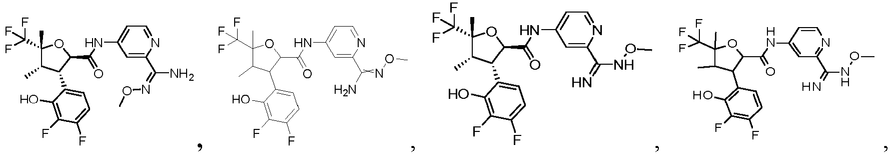

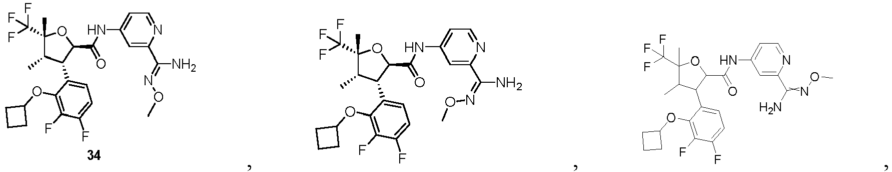

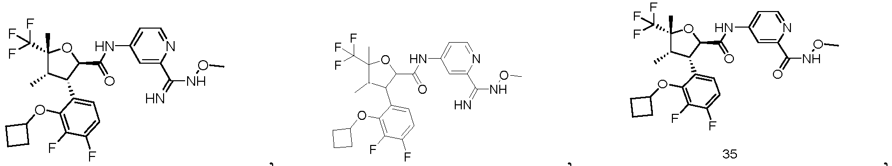

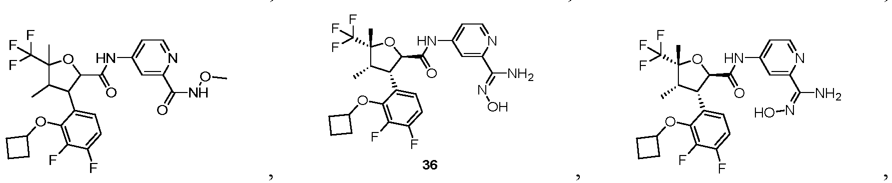

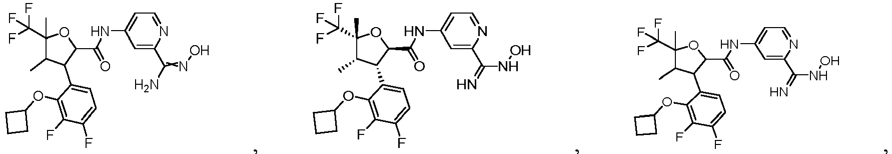

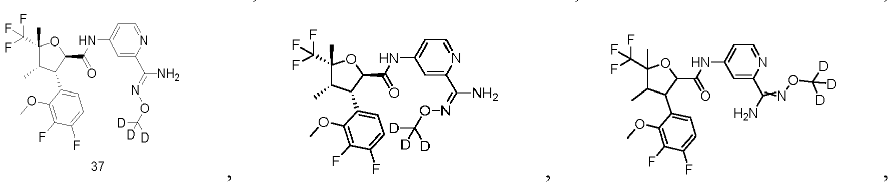

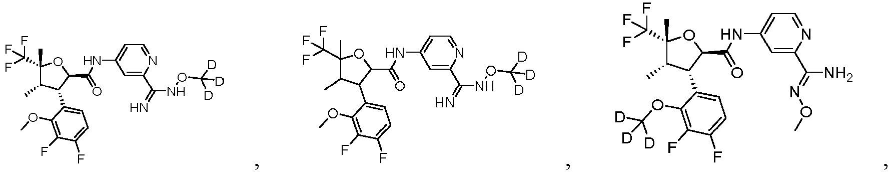

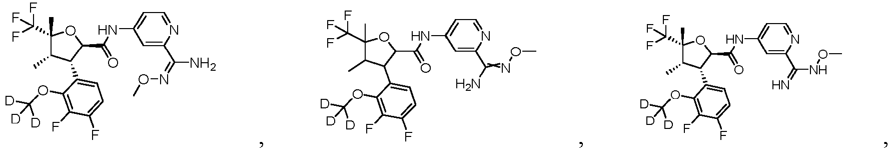

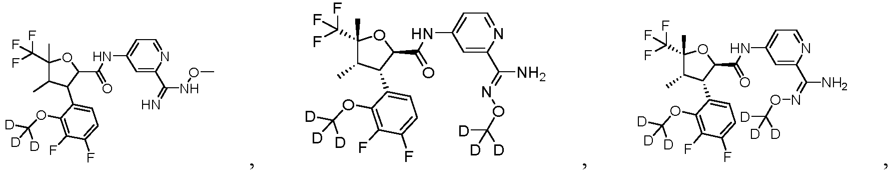

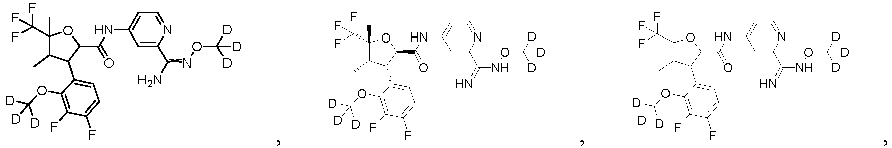

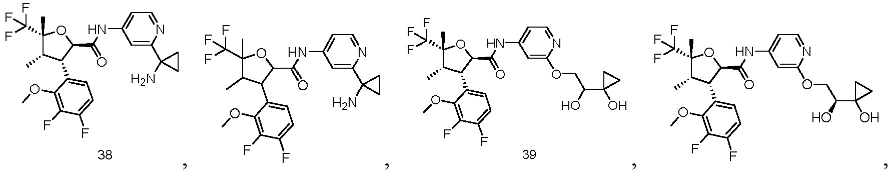

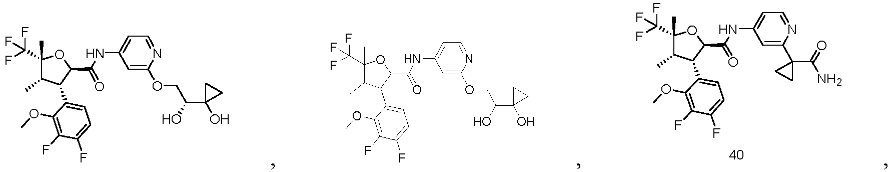

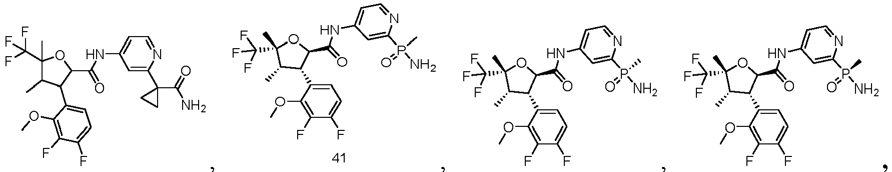

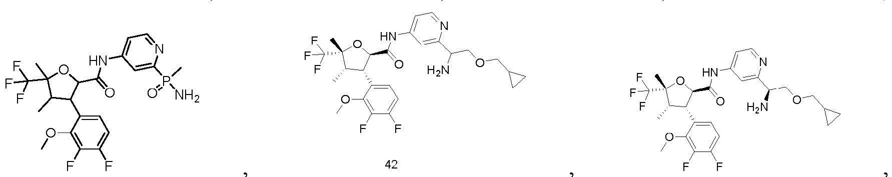

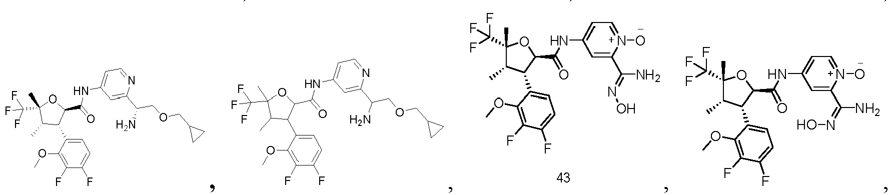

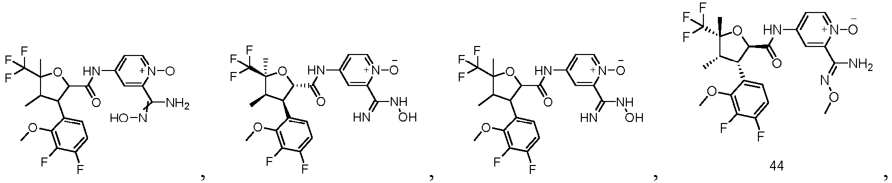

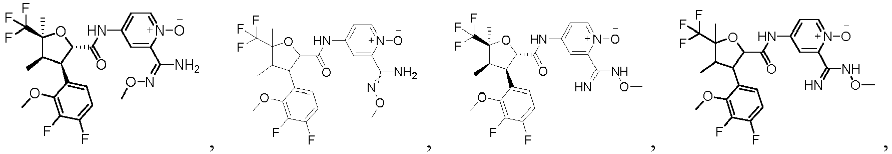

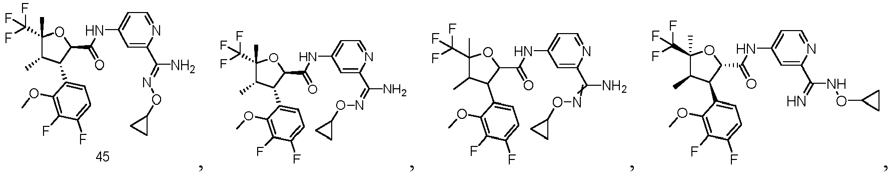

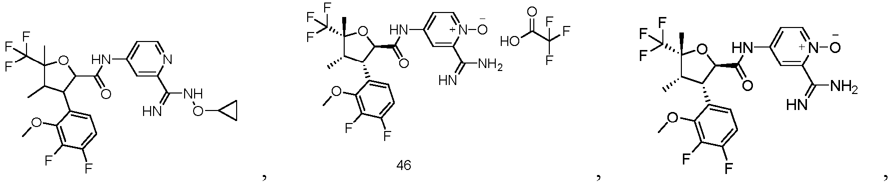

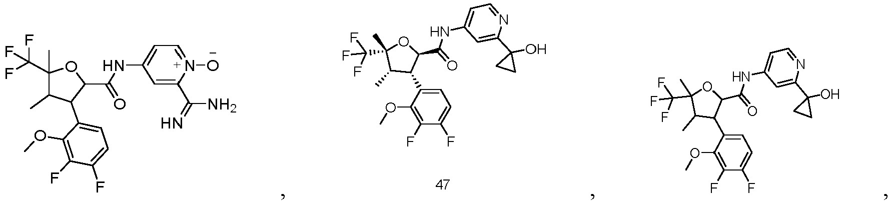

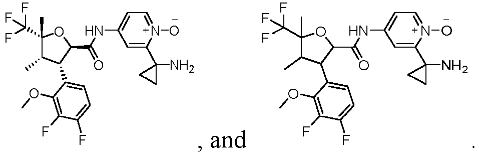

18. The compound represented by general formula (I) or the pharmaceutically acceptable salt thereof according to claim 1, being a compound represented by general formula (VI'A) or a salt thereof: 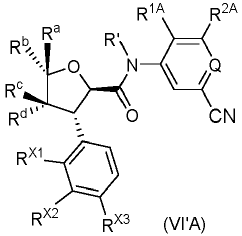 wherein Q, R^{1A}, R^{2A}, R', R^{a}, R^{b}, R^{c}, R^{d}, R^{X1}, R^{X2}, and R^{X3} are as defined in claim 6.

19. A compound or a salt thereof, being selected from the group consisting of the following compounds: 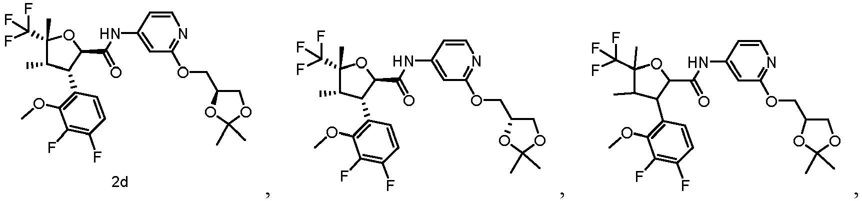 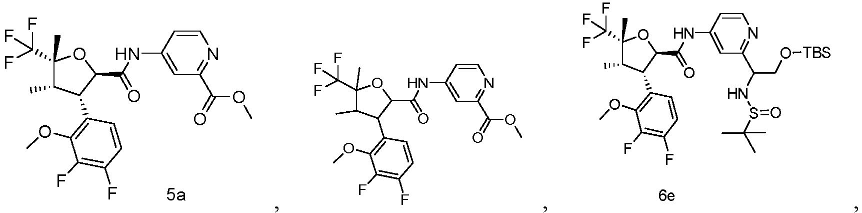 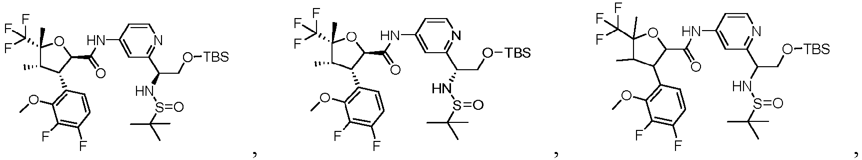 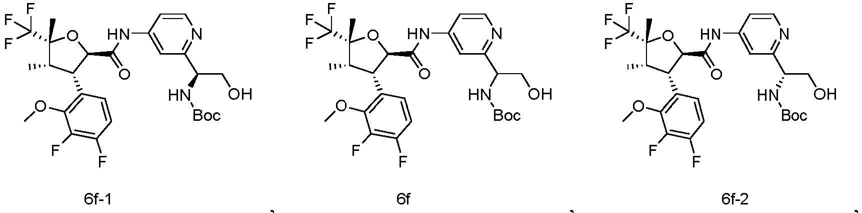 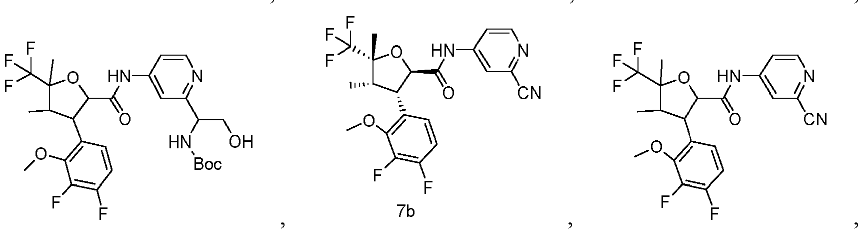 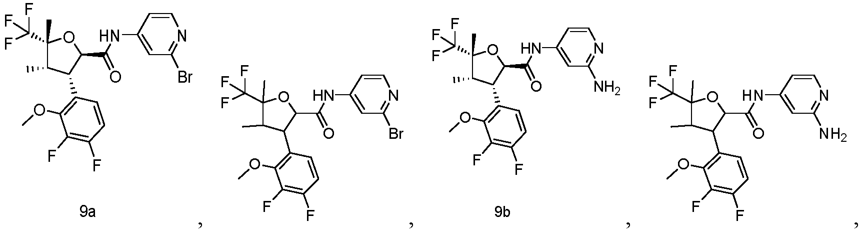 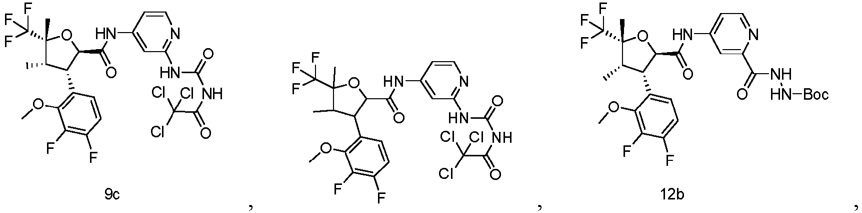 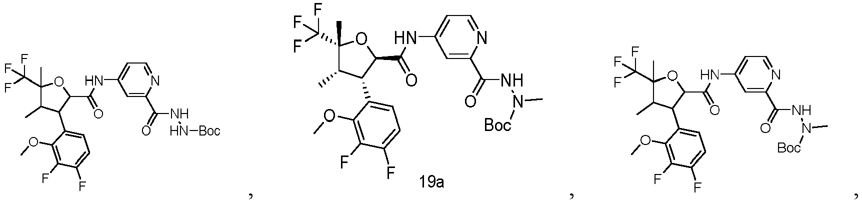 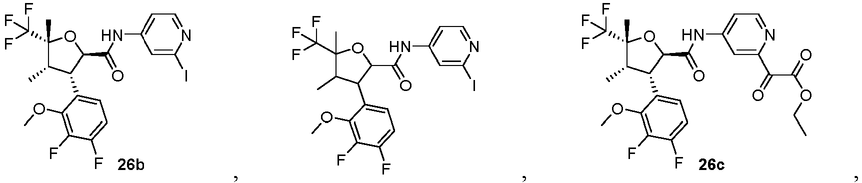 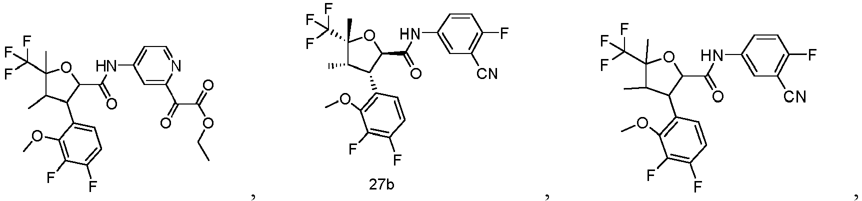 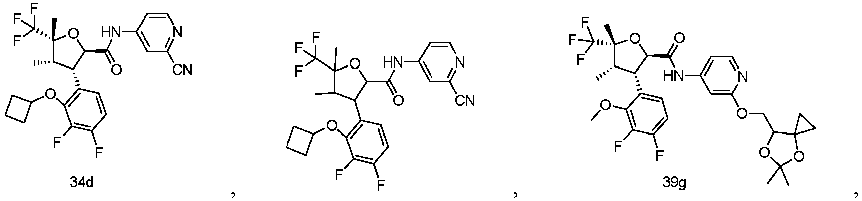 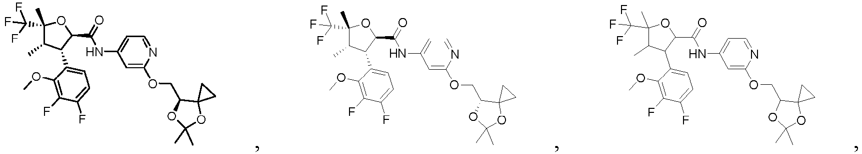 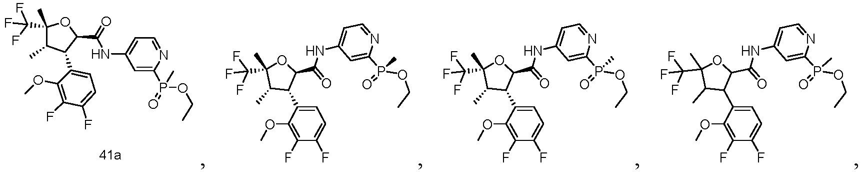 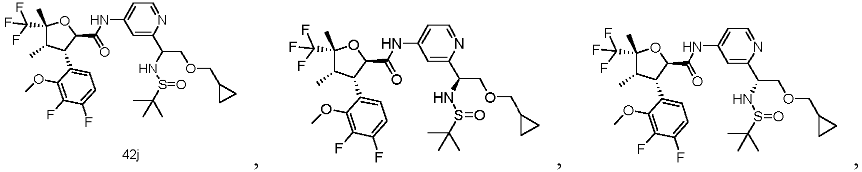 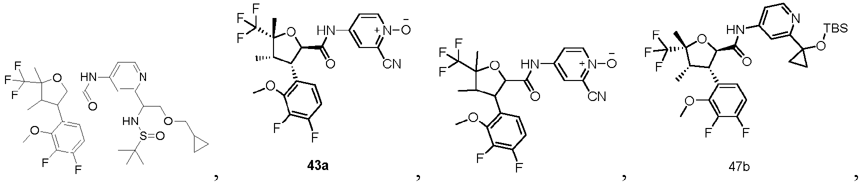 and

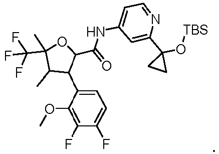

20. A method for preparing a compound represented by general formula (I) or a pharmaceutically acceptable salt thereof, comprising: 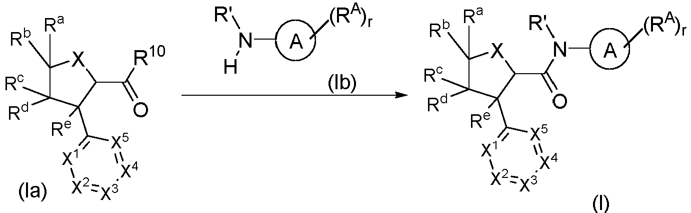 conducting a condensation reaction of a compound represented by general formula (Ia) or a salt thereof with a compound represented by general formula (Ib) or a salt thereof to give the compound represented by general formula (I) or the pharmaceutically acceptable salt thereof, wherein:
R¹⁰ is halogen (preferably a chlorine atom) or OH;
ring A, R^{A}, R', R^{a}, R^{b}, R^{c}, R^{d}, R^{e}, X, X¹, X², X³, X⁴, X⁵, and r are as defined in claim 1.

21. A method for preparing a compound represented by general formula (VI') or a pharmaceutically acceptable salt thereof, comprising: 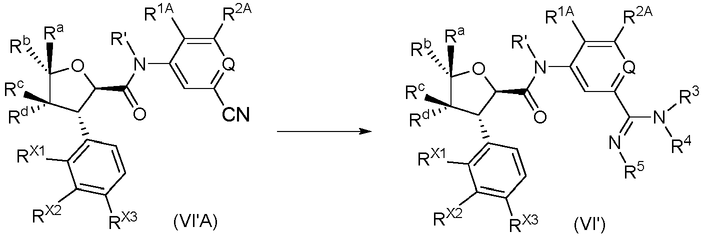 reacting a compound represented by general formula (VI'A) or a salt thereof with NH₂-R⁵ or a salt thereof (preferably hydrochloride) to give the compound represented by general formula (VI') or the pharmaceutically acceptable salt thereof, wherein:
R³ and R⁴ are both hydrogen atoms;
R', R^{a}, R^{b}, R^{c}, R^{d}, R^{X1}, R^{X2}, R^{X3}, R^{1A}, R^{2A}, Q, and R⁵ are as defined in claim 6.

22. A pharmaceutical composition, comprising the compound or the pharmaceutically acceptable salt thereof according to any one of claims 1 to 17, and one or more pharmaceutically acceptable carriers, diluents, or excipients.

23. Use of the compound or the pharmaceutically acceptable salt thereof according to any one of claims 1 to 17 or the pharmaceutical composition according to claim 22 in the preparation of a medicament for inhibiting a voltage-gated sodium channel, wherein preferably, the voltage-gated sodium channel is Nav1.8.

24. Use of the compound or the pharmaceutically acceptable salt thereof according to any one of claims 1 to 17 or the pharmaceutical composition according to claim 22 in the preparation of a medicament for treating and/or alleviating pain and pain-related diseases, multiple sclerosis, Charcot-Marie-Tooth syndrome, incontinence, pathological cough, or cardiac arrhythmia, wherein preferably, the pain is selected from the group consisting of chronic pain, acute pain, inflammatory pain, cancer pain, postsurgical pain, neuropathic pain, musculoskeletal pain, primary pain, gut pain, and idiopathic pain; the postsurgical pain is preferably selected from the group consisting of bunionectomy pain, herniorrhaphy pain, and abdominoplasty pain.
